# EUROPÄISCHE PATENTSCHRIFT

(11) **EP 0 666 852 B1**
(45) Veröffentlichungstag und Bekanntmachung des Hinweises auf die Patenterteilung: **22.01.1997**
(21) Anmeldenummer: 93924072.7
(22) Anmeldetag: 28.10.1993
(51) Int. Cl.: C07D 239/42, A01N 47/36, C07D 239/46, C07D 251/16, C07D 239/48, C07D 239/52, C07D 251/22, C07D 251/52

(54) **NEUE PHENYLSULFONYLHARNSTOFFE, IHRE DARSTELLUNG UND IHRE VERWENDUNG ALS HERBIZIDE UND PFLANZENWACHSTUMSREGULATOREN**
NEW PHENYLSULPHONYL UREAS, THEIR PREPARATION AND THEIR USE AS HERBICIDES AND PLANT-GROWTH REGULATORS
NOUVELLES PHENYLSULFONYLUREES, LEUR PREPARATION ET LEUR UTILISATION COMME HERBICIDES ET COMME REGULATEURS DE CROISSANCE VEGETALE

(30) Priorität: 31.10.1992 DE 4236902
(43) Veröffentlichungstag der Anmeldung: 16.08.1995
(73) Patentinhaber: Hoechst Schering AgrEvo GmbH, 13509 Berlin (DE)
(72) Erfinder: SCHNABEL, Gerhard, D-63868 Gro wallstadt (DE); WILLMS, Lothar, D-56204 Hillscheid (DE); BAUER, Klaus, D-63456 Hanau (DE); BIERINGER, Hermann, D-65817 Eppstein am Taunus (DE)
(86) Internationale Anmeldenummer: EP9302998
(87) Internationale Veröffentlichungsnummer: WO9410154

(56) Entgegenhaltungen:
- EP-A- 0 001 515
- EP-A- 0 116 518
- EP-A- 0 235 449
- US-A- 4 892 946

## Beschreibung

Die Erfindung betrifft das technische Gebiet der Herbizide und Pflanzenwachstumsregulatoren, insbesondere der Herbizide zur selektiven Bekämpfung von Unkräutern und Ungräsern in Nutzpflanzenkulturen.

Es ist bekannt, daß heterocyclisch substituierte Phenylsulfonylharnstoffe, die am Phenylring eine Amino- bzw. eine funktionalisierte Aminogruppe tragen, herbizide und pflanzenwachstumsregulierende Eigenschaften besitzen. (EP-A-1515, US-A-4,892,946; US-A-4,981,509; EP-A-116518, US-A-4,664,695 und US-A-4,632,695).

Überraschenderweise wurde nun gefunden, daß bestimmte heterocyclisch substituierte Phenylsulfonylharnstoffe als Herbizide oder Pflanzenwachstumsregulatoren besonders gut geeignet sind.

Gegenstand der vorliegenden Erfindung sind Verbindungen der Formel (I) oder deren Salze, 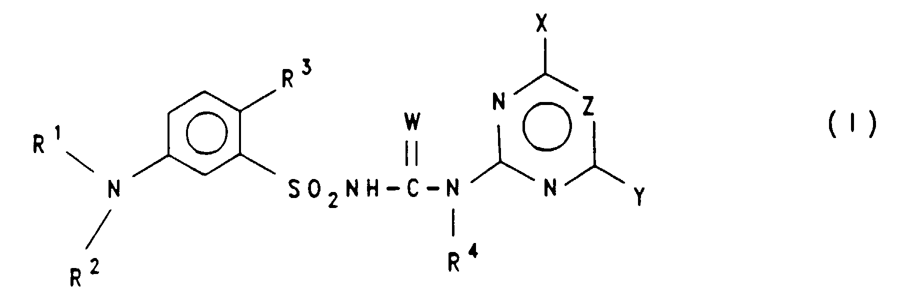 worin
- R¹: C₁-C₆-Alkyl, C₂-C₆-Alkenyl, C₂-C₆-Alkinyl, wobei die 3 letztgenannten Reste unabhängig voneinander unsubstituiert oder durch einen oder mehrere Reste aus der Gruppe Halogen, C₁-C₄-Alkoxy, CONH₂, C₁-C₄-Alkylthio, CN, CHO, (C₁-C₆-Alkyl)-carbonyl, (C₃-C₆-Cycloalkyl)-carbonyl, C₁-C₄-Alkylsulfonyl, Carboxy, (C₁-C₄-Alkoxy)-carbonyl, (C₂-C₄-Alkenyloxy)-carbonyl, (C₂-C₄-Alkinyloxy)-carbonyl, NO₂, NH₂, Mono- und Di-(C₁-C₆)-Alkylamino substituiert sind,
- R²: CO-R⁵, COO-R⁶, CO-SR⁷, CO-NR⁸R⁹, CS-NR¹⁰R¹¹, CS-OR¹², CS-SR¹³, SO₂R¹⁴, SO₂NR¹⁵R¹⁶,
- R³: COR¹⁷, CO-OR¹⁸, CO-NR¹⁹R²⁰, CO-SR²¹, CO-O-N=CR²²R²³, CSR²⁴, CSSR²⁵, CS-OR²⁶, CS-NR²⁷R²⁸, C(=NR²⁹)R³⁰,
- R⁴: H, C₁-C₆-Alkyl, C₂-C₆-Alkenyl, C₂-C₆-Alkinyl, wobei die 3 letztgenannten Reste unabhängig voneinander unsubstituiert oder durch einen oder mehrere Reste aus der Gruppe Halogen substituiert sind,
- R⁵: H, C₁-C₆-Alkyl, C₂-C₆-Alkenyl, C₂-C₆-Alkinyl, wobei die drei letztgenannten Reste unabhängig voneinander unsubstituiert oder durch einen oder mehrere Reste aus der Gruppe Halogen, C₁-C₄-Alkoxy, C₁-C₄-Alkylthio und NR³¹R³² substituiert sind, oder unsubstituiertes oder substituiertes C₃-C₈-Cycloalkyl, unsubstituiertes oder substituiertes Phenyl, unsubstituiertes oder substituiertes Heteroaryl oder Phenyl-C₁-C₄-alkyl, das am Phenylring unsubstituiert oder substituiert ist,
- R⁶: C₁-C₆-Alkyl, C₂-C₆-Alkenyl, C₂-C₆-Alkinyl, wobei die drei letztgenannten Reste unabhängig voneinander unsubstituiert oder durch einen oder mehrere Reste aus der Gruppe Halogen, C₁-C₄-Alkoxy, C₁-C₄-Alkylthio und NR³¹R³² substituiert sind, oder C₃-C₆-Cycloalkyl, das unsubstituiert oder durch einen oder mehrere Reste aus der Gruppe Halogen, C₁-C₄-Alkyl und C₁-C₄-Alkoxy substituiert ist, oder C₃-C₆-Cycloalkyl-C₁-C₃-alkyl,
- R⁷: analog R⁶,
- R⁸: H, C₁-C₆-Alkyl, C₂-C₆-Alkenyl, C₂-C₆-Alkinyl, wobei die 3 letztgenannten Reste unabhängig voneinander unsubstituiert oder durch einen oder mehrere Reste aus der Gruppe Halogen substituiert sind, oder (C₁-C₆-Alkoxy)-carbonyl oder C₁-C₄-Alkoxy,
- R⁹: H, C₁-C₆-Alkyl, C₂-C₆-Alkenyl, C₂-C₆-Alkinyl, wobei die 3 letztgenannten Reste unabhängig voneinander unsubstituiert oder durch einen oder mehrere Reste aus der Gruppe Halogen, C₁-C₄-Alkoxy, C₁-C₄-Alkylthio und NR³¹R³² substituiert sind, oder CO-R³³, CO-OR³⁴, CO-NR³⁵R³⁶, CSNR³⁵R³⁶, CS-R³³ oder CS-OR³⁴ oder
- R⁸, R⁹: zusammengenommen einen bivalenten Rest der Formel -(CH₂)₄-, -(CH₂)₅-, -CH₂CH₂-O-CH₂CH₂-, -CH₂CH₂NHCH₂CH₂-, wobei die letztgenannten 4 Reste unsubstituiert oder durch C₁-C₄-Alkyl substituiert sind,
- R¹⁰: analog R⁸,
- R¹¹: analog R⁹,
- R¹²: analog R⁶,
- R¹³: analog R⁶,
- R¹⁴: C₁-C₆-Alkyl, C₂-C₆-Alkenyl, C₂-C₆-Alkinyl, wobei die drei letztgenannten Reste unabhängig voneinander unsubstituiert oder durch einen oder mehrere Reste aus der Gruppe Halogen, C₁-C₄-Alkoxy, C₁-C₄-Alkylthio und NR³¹R³² substituiert sind,
- R¹⁵: analog R⁸,
- R¹⁶: H, C₁-C₆-Alkyl, C₂-C₆-Alkenyl, C₂-C₆-Alkinyl, wobei die 3 letztgenannten Reste unabhängig voneinander unsubstituiert oder durch einen oder mehrere Reste aus der Gruppe Halogen substituiert sind,
- R¹⁷: analog R⁵,
- R¹⁸: C₁-C₆-Alkyl, C₂-C₆-Alkenyl, C₂-C₆-Alkinyl, wobei die drei letztgenannten Reste unabhängig voneinander unsubstituiert oder durch einen oder mehrere Reste aus der Gruppe Halogen, C₁-C₄-Alkoxy, C₁-C₄-Alkylthio und NR³¹R³² substituiert sind, oder C₃-C₆-Cycloalkyl, das unsubstituiert oder durch einen oder mehrere Reste aus der Gruppe Halogen, C₁-C₄-Alkyl und C₁-C₄-Alkoxy substituiert ist, oder C₃-C₆-Cycloalkyl-C₁-C₃-alkyl oder H,
- R¹⁹: analog R⁸,
- R²⁰: analog R⁹,
- R²¹: analog R¹⁸,
- R²²: H, C₁-C₄-Alkyl, C₃-C₅-Alkenyl oder C₃-C₅-Alkinyl,
- R²³: H, C₁-C₄-Alkyl, C₃-C₅-Alkenyl oder C₃-C₅-Alkinyl,
- R²⁴: analog R¹⁷,
- R²⁵: analog R¹⁸,
- R²⁶: analog R¹⁸,
- R²⁷: analog R⁸,
- R²⁸: analog R⁹,
- R²⁹: H, OH, NH₂, NHR³⁷, N(R³⁷)₂, C₁-C₄-Alkyl, C₁-C₄-Alkoxy, C₂-C₄-Alkenyl oder C₂-C₄-Alkinyl, wobei die letztgenannten 4 Reste unabhängig voneinander unsubstituiert oder durch einen oder mehrere Reste aus der Gruppe Halogen, C₁-C₃-Alkoxy und C₁-C₃-Alkylthio substituiert sind,
- R³⁰: H, C₁-C₆-Alkyl, C₁-C₆-Alkoxy, C₂-C₆-Alkenyl, C₂-C₆-Alkinyl, wobei die letztgenannten vier Reste unabhängig voneinander unsubstituiert oder durch einen oder mehrere Reste aus der Gruppe Halogen, C₁-C₃-Alkoxy und C₁-C₃-Alkylthio substituiert sind,
- die R³¹: unabhängig voneinander H, C₁-C₄-Alkyl, (C₁-C₄-Alkyl)-carbonyl, (C₁-C₄-Alkoxy)-carbonyl oder CHO,
- die R³²: unabhängig voneinander H oder C₁-C₄-Alkyl,
- R³³: analog R⁵,
- R³⁴: analog R⁶,
- R³⁵, R³⁶: unabhängig voneinander H, C₁-C₆-Alkyl, C₂-C₆-Alkenyl, C₂-C₆-Alkinyl,
wobei die 3 letztgenannten Reste unabhängig voneinander unsubstituiert oder durch einen oder mehrere Reste aus der Gruppe Halogen substituiert sind,
- R³⁷: C₁-C₄-Alkyl, C₂-C₄-Alkenyl oder C₂-C₄-Alkinyl, wobei die drei genannten Reste unabhängig voneinander unsubstituiert oder durch einen oder mehrere Reste aus der Gruppe Halogen, C₁-C₄-Alkoxy und C₁-C₄-Alkylthio substituiert sind,
- W: O oder S,
- X, Y: unabhängig voneinander Wasserstoff, Halogen, C₁-C₆-Alkyl, C₁-C₆-Alkoxy oder C₁-C₆-Alkylthio, wobei die letztgenannten drei Reste unsubstituiert oder durch einen oder mehrere Reste aus der Gruppe Halogen, C₁-C₄-Alkoxy und C₁-C₄-Alkylthio substituiert sind, oder Mono- oder Di-(C₁-C₄-alkyl)amino, C₃-C₆-Cycloalkyl, C₃-C₅-Alkenyl, C₃-C₅-Alkenyloxy oder C₃-C₅-Alkinyloxy und
- Z: CH oder N
bedeuten.

In der Formel (I) und im folgenden können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die Kohlenstoffgerüste mit 1 bis 4 C-Atomen, bzw. bei ungesättigten Gruppen mit 2 bis 4 C-Atomen bevorzugt. Alkylreste, auch in den zusammengesetzten Bedeutungen wie Alkoxy, Haloalkyl usw., bedeuten z. B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste, Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Halogen bedeutet Fluor, Chlor, Brom oder lod. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl. Entsprechendes gilt für Haloalkenyl und andere durch Halogen substituierte Reste.

Aryl bedeutet ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl; Aryloxy bedeutet vorzugsweise die den genannten Arylresten entsprechenden Oxy-Reste, insbesondere Phenoxy.
Heteroaryl bedeutet ein mono-, bi- oder polycyclisches aromatisches System, in dem mindestens 1 Ring ein oder mehrere Heteroatome enthält, beispielsweise Pyridyl, Pyrimidinyl, Pyridazinyl, Pyrazinyl, Thienyl, Thiazolyl, Oxazolyl, Furyl, Pyrrolyl, Pyrazolyl und Imidazolyl, aber auch bicyclische oder polycyclische aromatische oder araliphatische Verbindungen, z. B. Chinolinyl, Benzoxazolyl etc.

Substituiertes Aryl, Heteroaryl, Phenyl, Benzyl, bzw. substituierte bicyclische Reste mit aromatischen Anteilen bedeuten beispielsweise einen vom unsubstituierten Grundkörper abgeleiteten substituierten Rest, wobei die Substituenten beispielsweise ein oder mehrere, vorzugsweise 1, 2 oder 3 Reste aus der Gruppe Halogen, Alkyl, Haloalkyl, Alkoxy, Haloalkoxy, Alkylthio, Hydroxy, Amino, Nitro, Cyano, Alkoxycarbonyl, Alkylcarbonyl, Formyl, Carbamoyl, Mono- und Dialkylaminocarbonyl, Mono- und Dialkylamino, Alkylsulfinyl und Alkylsulfonyl bedeuten und bei Resten mit C-Atomen solche mit 1 bis 4 C-Atomen, insbesondere 1 oder 2 C-Atomen, bevorzugt sind. Bevorzugt sind dabei in der Regel Substituenten aus der Gruppe Halogen, z. B. Fluor und Chlor, C₁-C₄-Alkyl, vorzugsweise Methyl oder Ethyl, C₁-C₄-Haloalkyl, vorzugsweise Trifluormethyl, C₁-C₄-Alkoxy, vorzugsweise Methoxy oder Ethoxy, C₁-C₄-Haloalkoxy, Nitro und Cyano. Besonders bevorzugt sind dabei die Substituenten Methyl, Methoxy und Chlor.

Gegebenenfalls substituiertes Phenyl ist beispielsweise Phenyl, das unsubstituiert oder ein- oder mehrfach, vorzugsweise bis zu dreifach durch gleiche oder verschiedene Reste aus der Gruppe Halogen, C₁-C₄-Alkyl, C₁-C₄-Alkoxy, C₁-C₄-Halogenalkyl, C₁-C₄-Halogenalkoxy und Nitro substituiert ist, z.B. o-, m- und p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Methoxyphenyl.

Ein heteroaromatischer Rest ist vorzugsweise 5- oder 6-gliedrig und enthält 1, 2 oder 3 Heteroatome, vorzugsweise aus der Gruppe N, O und S. Der Rest kann benzokondensiert sein. Es kommen Reste wie Oxiranyl, Pyrrolidinyl, Piperidyl, Dioxolanyl, Pyrazolyl, Morpholyl, Furyl, Tetrahydrofuryl, Indolyl, Chinolinyl, Pyrimidyl, Azepinyl, Imidazolyl, Triazolyl, Thienyl und Oxazolyl in Frage.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in den allgemeinen Formeln (I) nicht gesondert angegeben sind. Die durch ihre spezifische Raumform definierten möglichen Stereoisomeren, wie Enantiomere, Diastereomere, Z- und E-lsomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Die Verbindungen der Formel (I) können Salze bilden, bei denen der Wasserstoff der -SO₂-NH-Gruppe oder auch andere acide Wasserstoffatome (z.B. aus COOH u.a.) durch ein für die Landwirtschaft geeignetes Kation ersetzt wird. Diese Salze sind beispielsweise Metall-, insbesondere Alkali- oder Erdalkalisalze, oder auch Ammoniumsalze oder Salze mit organischen Aminen. Ebenso kann Salzbildung durch Anlagerung einer Säure an basische Gruppen, wie z.B. Amino und Alkylamino, erfolgen. Geeignete Säuren hierfür sind starke anorganische und organische Säuren, beispielsweise HCI, HBr, H₂SO₄ oder HNO₃.

Vor allem aus Gründen der höheren herbiziden Wirkung, besseren Selektivität, und/oder besseren Herstellbarkeit sind Verbindungen der Formel (I) oder deren Salze von besonderem Interesse, worin
- R¹: C₁-C₄-Alkyl, C₂-C₄-Alkenyl oder C₂-C₄-Alkinyl, vorzugsweise C₁-C₄-Alkyl, Allyl oder Propargyl oder
- R²: CO-R⁵, COOR⁶, CO-NR⁸R⁹, CS-NR¹⁰R¹¹, SO₂R¹⁴ oder SO₂NR¹⁵R¹⁶ oder
- R³: COR¹⁷, COOR¹⁸, CONR¹⁹R²⁰ oder CO-ON = CR²²R²³, vorzugsweise COOR¹⁸, oder
- R⁴: H oder C₁-C₄-Alkyl, vorzugsweise H oder Methyl, oder
- R⁵: H, C₁-C₆-Alkyl, das unsubstituiert oder durch einen oder mehrere Reste aus der Gruppe Halogen oder durch C₁-C₄-Alkoxy, C₁-C₄-Alkylthio oder NR³¹R³² substituiert ist, oder C₃-C₆-Cycloalkyl, unsubstituiertes oder substituiertes Phenyl, unsubstituiertes oder substituiertes Benzyl oder unsubstituiertes oder substituiertes Heteroaryl, vorzugsweise H, C₁-C₆-Alkyl, C₁-C₄-Haloalkyl, Cyclopropyl, Cyclopentyl, Cyclohexyl, Phenyl oder Heteroaryl, wobei die letztgenannten zwei Reste unsubstituiert oder durch einen oder mehrere Reste aus der Gruppe C₁-C₄-Alkyl, C₁-C₄-Alkoxy und Halogen substituiert sind, oder
- R⁶: C₁-C₆-Alkyl, C₂-C₆-Alkenyl, C₂-C₆-Alkinyl, C₁-C₆-Haloalkyl oder C₃-C₆-Cycloalkyl, vorzugsweise C₁-C₄-Alkyl, C₁-C₄-Haloalkyl, Allyl, Propargyl oder C₃-C₆-Cycloalkyl, oder
- R⁷: C₁-C₄-Alkyl, oder
- R⁸: Wasserstoff, C₁-C₆-Alkyl, C₁-C₄-Haloalkyl, C₁-C₄-Alkoxy oder (C₁-C₄-Alkoxy)-carbonyl, oder
- R⁹: Wasserstoff, C₁-C₆-Alkyl, das unsubstituiert oder durch einen oder mehrere Reste aus der Gruppe Halogen, C₁-C₄-Alkoxy und NR³¹R³² substituiert ist, oder CO-R³³, CO-OR³⁴ oder CO-NR³⁵R³⁶ oder
- R⁸ und R⁹: zusammengenommen einen bivalenten Rest der Formel -(CH₂)₄-, -(CH₂)₅- oder -CH₂CH₂-O-CH₂CH₂- oder
- R¹⁴: C₁-C₆-Alkyl, C₁-C₆-Haloalkyl, vorzugsweise C₁-C₄-Alkyl oder C₁-C₄-Haloalkyl oder
- R¹⁵, R¹⁶: unabhängig voneinander Wasserstoff oder C₁-C₄-Alkyl oder
- R¹⁷: Wasserstoff oder C₁-C₄-Alkyl, C₁-C₄-Haloalkyl, C₃-C₆-Cycloalkyl, Phenyl oder Heteroaryl, wobei die letztgenannten zwei Reste unsubstituiert oder substituiert sind, oder
- R¹⁸: Wasserstoff, C₁-C₄-Alkyl, C₂-C₆-Alkenyl oder C₂-C₆-Alkinyl, wobei die letztgenannten 3 Reste unsubstituiert oder durch einen oder mehrere Reste aus der Gruppe Halogen, C₁-C₄-Alkoxy, C₁-C₄-Alkylthio und NR³¹R³² substituiert sind, oder C₃-C₆-Cycloalkyl oder C₃-C₆-Cycloalkyl-C₁-C₃-alkyl oder
- R²²: Wasserstoff oder C₁-C₂-Alkyl oder
- R²³: Wasserstoff oder C₁-C₂-Alkyl oder
- R²⁹: Wasserstoff, Hydroxy, Amino, NHCH₃, N(CH₃)₂, C₁-C₄-Alkyl oder C₁-C₄-Alkoxy, oder
- R³⁰: Wasserstoff oder C₁-C₄-Alkyl oder
- die R³¹: unabhängig voneinander H oder C₁-C₄-Alkyl oder
- die R³²: unabhängig voneinander H oder C₁-C₄-Alkyl oder
- R³³: Wasserstoff, C₁-C₄-Alkyl, C₁-C₄-Haloalkyl, C₃-C₆-Cycloalkyl oder Phenyl, das unsubstituiert oder durch ein oder mehrere Reste aus der Gruppe Halogen, C₁-C₄-Alkyl und C₁-C₄-Alkoxy substituiert ist, oder
- R³⁴: C₁-C₄-Alkyl, Allyl, Propargyl oder Cycloalkyl oder
- R³⁵: Wasserstoff oder C₁-C₄-Alkyl oder
- R³⁶: Wasserstoff oder C₁-C₄-Alkyl oder
- X: C₁-C₄-Alkyl, C₁-C₄-Alkoxy, C₁-C₄-Haloalkyl, C₁-C₄-Alkylthio, Halogen oder Mono- oder Di-(C₁-C₂-alkyl)-amino, vorzugsweise Methyl, Ethyl, Methoxy, Ethoxy, Methylthio, Ethylthio, Chlor, NHCH₃ oder N(CH₃)₂, oder
- Y: C₁-C₄-Alkyl, C₁-C₄-Alkoxy, C₁-C₄-Haloalkyl oder C₁-C₄-Alkylthio, vorzugsweise Methyl, Ethyl, Methoxy oder Ethoxy,
bedeutet, oder vorzugsweise solche Verbindungen der Formel (I) oder deren Salze, worin zwei oder mehrere der vorstehend genannten besonderen oder bevorzugten Bedeutungen für die Reste R¹ bis R³⁶ in Formel (I) kombiniert vorhanden sind.

Bevorzugte Verbindungen der Formel (I) oder deren Salze sind solche, worin
- R¹: Methyl, Ethyl, n-Propyl, i-Propyl oder Allyl,
- R²: CO-R⁵, COOR⁶, CO-NR⁸R⁹, CS-NR¹⁰R¹¹, SO₂R¹⁴ oder SO₂NR¹⁵R¹⁶,
- R⁵: H, C₁-C₄-Alkyl, C₁-C₂-Haloalkyl, Cyclopropyl, Phenyl, Benzyl oder Heteroaryl mit 5 oder 6 Ringatomen, wobei die letztgenannten 3 Reste unsubstituiert oder durch einen oder mehrere Halogenatome substituiert sind,
- R⁶: C₁-C₄-Alkyl, Allyl, Propargyl oder Cyclopropyl,
- R⁸: H, C₁-C₄-Alkyl, C₁-C₄-Haloalkyl oder (C₁-C₄-Alkoxy)-carbonyl,
- R⁹: H oder C₁-C₄-Alkyl,
- R¹⁰: H oder C₁-C₄-Alkyl,
- R¹¹: H oder C₁-C₄-Alkyl,
- R¹⁴: C₁-C₄-Alkyl,
- R¹⁵: H oder C₁-C₄-Alkyl und
- R¹⁶: H oder C₁-C₄-Alkyl
bedeuten.

Besonders bevorzugt steht R⁵ für H, CH₃, C₂H₅, n- oder i-C₃H₇, n-, i-, t- oder 2-Butyl, n-Pentyl, CF₃, CH₂Cl, CHCl₂, CCl₃, CH₂Br, CH₂CCl₃, Cyclopropyl, Phenyl, Thienyl, Furyl oder Pyridyl, wobei die letztgenannten 4 Reste durch 1 bis 3 Halogenatome substituiert sein können.

Weiterer Gegenstand der vorliegenden Erfindung sind Verfahren zur Herstellung der erfindungsgemäßen Verbindungen der Formel (I) oder deren Salze, dadurch gekennzeichnet, daß man
a) eine Verbindung der Formel (II) 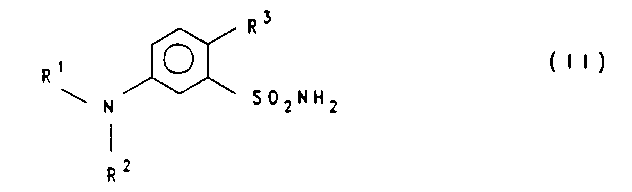 mit einem heterocyclischen Carbamat der Formel (III) 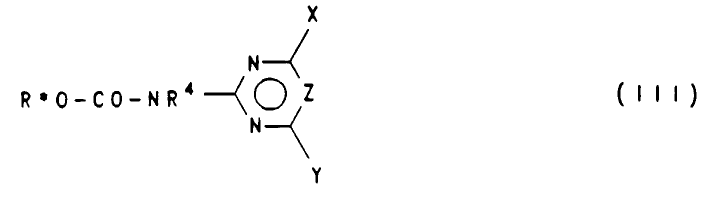 worin R* gegebenenfalls substituiertes Phenyl oder C₁-C₄-Alkyl bedeutet, umsetzt oder
b) ein Sulfonylisocyanat der Formel (IV) 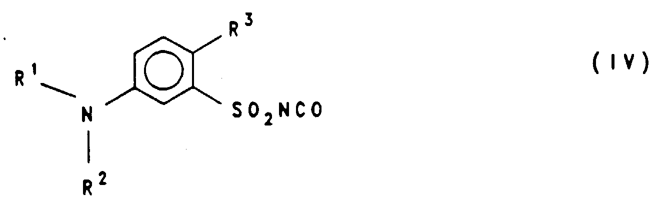 mit einem Aminoheterocyclus der Formel (V) 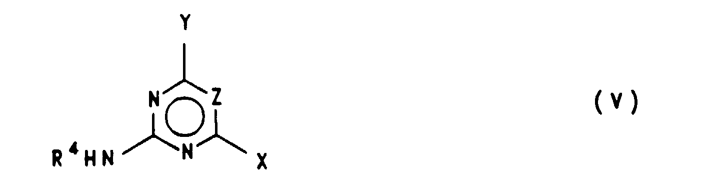 umsetzt oder
c) eine Verbindung der Formel (II) mit einem (Thio)Isocyanat der Formel (X) 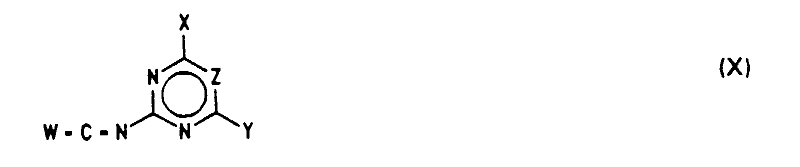 in Gegenwart einer Base umsetzt,
wobei in den obigen Formeln (II) bis (V) und (X) die Reste R¹ bis R⁴, W, X, Y und Z wie in Formel (I) definiert sind und bei den Varianten a) und b) zunächst Verbindungen der Formel (I) erhalten werden, in denen W = O ist.

Die Umsetzung der Verbindungen der Formeln (II) und (III) erfolgt vorzugsweise basenkatalysiert in einem inerten organischen Lösungsmittel, wie z. B. Dichlormethan, Acetonitril, Dioxan oder THF bei Temperaturen zwischen 0° C, vorzugsweise 20° C und dem Siedepunkt des Lösungsmittels. Als Base werden dabei beispielsweise organische Aminbasen, wie 1,8-Diazabicyclo-[5.4.0]undec7-en (DBU), insbesondere bei R* = (subst.) Phenyl (vgl. EP-A-44 807), oder Trimethylaluminium oder Triethylaluminium, letztere insbesondere bei R* = Alkyl (vgl. EP-A-166 516), verwendet.

Die Sulfonamide (II) und die Sulfonylisocyanate (IV) sind neue Verbindungen. Sie und ihre Herstellung sind ebenfalls Gegenstand der Erfindung.

Man erhält die Verbindungen der Formel (II) z. B. ausgehend von Verbindungen der Formel (Vl). 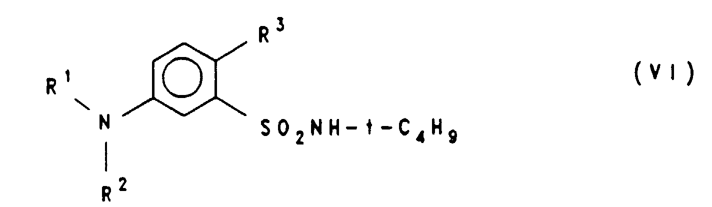 worin R¹, R² und R³ wie in Formel (I) definiert sind, durch Umsetzung mit einer starken Säure (vgl. hierzu WO-89/10921).

Als starke Säuren kommen z.B. Mineralsäuren, wie H₂SO₄ oder HCl, oder starke organische Säuren, wie Trifluoressigsäure in Frage. Die Abspaltung der t.-Butyl-Schutzgruppe erfolgt beispielsweise bei Temperaturen von -20°C und der jeweiligen Rückflußtemperatur des Reaktionsgemisches, vorzugsweise bei 0°C bis 40°C. Die Umsetzung kann in Substanz oder auch in einem inerten Solvens, wie z.B. Dichlormethan oder Trichlormethan, durchgeführt werden.

Die Verbindungen der Formel (VI), werden z. B. aus den Verbindungen der Formel (VI'), welche der Formel (Vl) entspricht, worin aber R² = H ist, durch Umsetzung mit geeigneten Elektrophilen, wie z. B. Säurechloriden, Säureanhydriden, Isocyanaten, Thioisocyanaten, Sulfochloriden oder Amidosulfochloriden, erhalten (vgl. hierzu: A.L.J. Beckniter in J. Zabicky, "The Chemistry of Amides", S. 73-185, Interscience, New York, 1970; E.J. Corey et al., Tetrahedron Lett. 1978, 1051;
H.J. Saunders, R.J. Slocombe, Chem. Rev. 43, 203 (1948);
S. Ozaki, Chem. Rev. 72, 457, 469 (1972);
G. Zölß, Arzneim.-Forsch. 33, 2 (1983);
Houben-Weyl-Hagemann, "Methoden der organischen Chemie", 4. Aufl. Bd. E4, S. 485 ff., Thieme Verlag Stuttgart, 1983;
J. Golinsky, M. Mahosza, Synthesis 1978, 823;
Houben-Weyl-Müller, "Methoden der organischen Chemie", 4. Aufl. Bd. IX, S. 338-400 und 605-622, Thieme Velag Stuttgart, 1955;
Houben-Weyl-Klarmann, "Methoden der organischen Chemie", 4. Aufl. Bd. E 11/2, S. 1020-22, Thieme Verlag Stuttgart, 1985;
S. Krishnamurthey, Tetrahedron Lett. 23, 3315 (1982).).

Die hierzu benötigten N-monosubstituierten Aminobenzol-Derivate, z. B. N-Alkylaminobenzol-Derivate, der Formel (VI') (siehe Formel (VI), R² = H), werden durch Monoalkylierung der Aniline der Formel (VI") (= Formel (VI), R¹ = H und R² = H) nach Standardmethoden gewonnen. Beispielsweise führt die N-Acylierung der Aniline der Formel (Vl") mit Carbonsäurechloriden oder Carbonsäureanhydriden zu den enstprechenden N-Acyl-Anilinen. Die nachfolgende Reduktion der Acylaminogruppe zu der analogen N-monosubstituierten Aminofunktion, z. B. Alkylaminofunktion (s. o.), mit geeigneten Reduktionsmitteln, wie z. B. Borandimethylsulfid-Komplex, führt in sehr guten Ausbeuten zu den Anilin-Derivaten der Formel (VI') (= Formel (VI), R² = H); siehe S. Krishnamurthey, Tetrahedron Lett. 23, 3315 (1982).

Die genannten Anilin-Derivate der Formel (VI') (= Formel (VI), R¹ und R² = H), werden nach literaturbekannten Verfahren durch Reduktion der Nitrogruppe der Verbindungen (VII), z. B. durch Hydrierung mit Wasserstoff in Gegenwart eines geeigneten Katalysators, wie Pd-C oder Raney-Nickel, oder durch Reduktion mit Eisen in essigsaurem Medium erhalten.
(vgl. hierzu: H. Berrie, G.T. Neuhold, F.S. Spring, J. Chem. Soc. 1952, 2042; M. Freifelder, "Catalytic Hydrogenation in Organic Synthesis: Procedures and Commentary", J. Wiley and Sons, New York (1978), Kap. 5).

Die aromatischen Sulfonamide der Formel (VII) können aus den Sulfonsäuren der Formel (VIII) gewonnen werden.

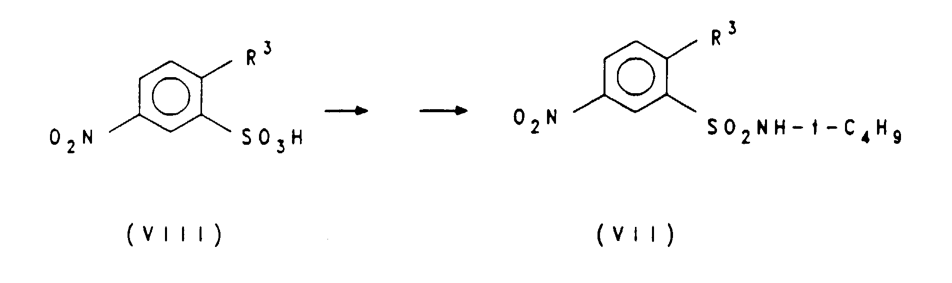

Zunächst erfolgt die Überführung der Sulfonsäuregruppe der Verbindungen (VIII) in die Sulfochloride, z. B. nach Standardmethoden wie Umsetzung von Phosphoroxychlorid oder Thionylchlorid mit Kalium-Salzen der entsprechenden Sulfonsäuren in inerten Solventien wie Acetonitril und/oder Sulfolan oder in Substanz durch Erwärmen zum Rückfluß (vgl. Houben-Weyl-Klamann, "Methoden der organischen Chemie", 4. Aufl. Bd. E XI/2, S. 1067-1073, Thieme Verlag Stuttgart, 1985).

Die Sulfonamidbildung aus den Sulfochloriden mit tert.-Butylamin in Ethanol liefert die Verbindungen (VII) in guten Ausbeuten (vgl. analoge Umsetzungen in WO 89/10921).

Die Sulfonsäuren der Formel (VIII) sind aus der kommerziell erhältlichen 2-Methyl-5-nitrobenzolsulfonsäure darstellbar.

Durch Oxidation der Methylgruppe von 2-Methyl-5-nitrobenzolsulfonsäure durch Standardmethoden, wie z. B. die Umsetzung mit Kaliumpermanganat zur Carbonsäurefunktion sowie gegebenenfalls nachfolgende Derivatisierung, wie z. B. Veresterung oder Amidbildung, wird der Substituent R³ eingeführt.
(vgl. hierzu: Houben-Weyl-Falbe: "Methoden der organischen Chemie", 4. Aufl. Bd. E V/1, Thieme Verlag Stuttgart, 1985, S. 199-202).

Durch eine Reihe von Standardreaktionen läßt sich eine breite Variation des Rests R³ erzielen:
- Carbonsäureamide sind durch Umsetzung von Säureanhydriden bzw. Säurechloriden mit Aminen erhältlich
   (vgl. hierzu: A.L.J. Beckwith in J. Zabicky, "The Chemistry of Amides", S. 73-185, Interscience, New York 1970).
- Thiocarbonsäure-S-ester sind aus Carbonsäuren und Thiolen unter Carbonylaktivierung, wie z.B. mit Diphenylphosphinsäurechlorid, darstellbar (vgl. hierzu: D. Scholz, D. Eigner, Monatsh. Chemie 110, 759 (1979), S. Yamada et al., J. Org. Chem. 39, 3302 (1974).);
- Thiocarbonsäure-O-ester können durch Reaktion von Carbonsäureestern mit geeigneten Reagenzien, wie z. B. P₄S₁₀, hergestellt werden (vgl. hierzu: S.O. Lauresson et. al., Bull. Soc. Chim. Belg. 87, 293 (1978); T. Nishiwak: et. al., Chem. Lett. 1980, 401.);
- Thiocarbonsäureamide werden aus Carbonsäureamiden durch "Schwefelung" z.B. mit Phosphorpentasulfid, erhalten (Houben-Weyl-Falbe, "Methoden der organischen Chemie", 4. Aufl. Bd. E 5/2, S. 1242-1247 (und dort zitierte Literatur), Thieme-Verlag Stuttgart, 1985);
- Aldehyde können aus Carbonsäuren bzw. Estern mit Hilfe einer Reihe von Reduktionsmitteln synthetisiert werden
   (Houben-Weyl-Falbe, "Methoden der organischen Chemie", 4. Aufl. Bd. E 3, S. 418 ff., Thieme Verlag Stuttgart, 1983).
- funktionalisierte Aldehyd-Derivate, wie z. B. Oxime, Oximether, Hydrazone, u.s.w., lassen sich durch Kondensation aus den Aldehyden mit den entsprechenden Aminen gewinnen (vgl. hierzu: Houben-Weyl-Müller, "Methoden der organischen Chemie", 4. Aufl. Bd. 7/1, S. 453-474, Thieme Verlag Stuttgart, 1954);
- N-Alkoxy-Carbonsäure-imid-ester lassen sich aus Hydroxamsäure-Bromid-Derivaten und Alkoholaten darstellen (E.C. Tayler, F. Kienzle, J. Org. Chem. 36, 233 (1971);
- Carbonsäure-imid-ester können aus Carbonsäure-chlorid-imiden und entsprechenden Alkoholaten gewonnen werden
   (vgl. hierzu: H. Böhme, O. Müller, Chem. Ber. 98, 1450 (1965); G. Bock, Chem. Ber. 100, 2870 (1967);
- O-Acyl-Oxime sind z. B. durch Umsetzung von Carbonsäureanhydriden und Ketoximen darstellbar
   (vgl. hierzu: S. Bittner, Y. Knobler, M. Frankel, Tetrahedron Lett. 1965, 95);
- Hydroxamsäureamide können durch Umsetzung von Hydroxamsäurechlorid-Derivaten mit Aminen erhalten werden (vgl. hierzu: Houben-Weyl-Müller, "Methoden der organischen Chemie", 4. Aufl. Bd. 10/4, S. 209 ff., Thieme-Verlag Stuttgart, 1968).

Alternativ können die Sulfonamide der Formel II, worin R¹ und R² ungleich H sind, durch Aminolyse der entsprechenden Sulfochloride der Formel (IXb) erhalten werden, 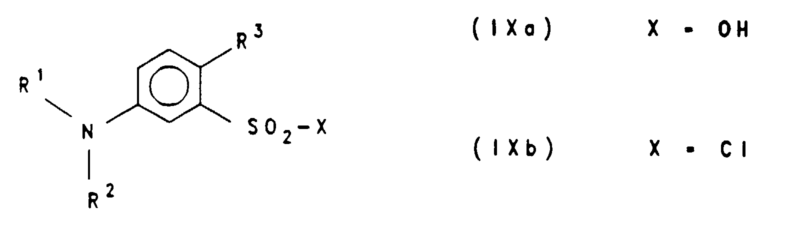 die wiederum aus den Sulfonsäuren (IXa) durch die genannten Standard methoden (s. genannte Umsetzungen von (VIII) nach (VII)) leicht erhältlich sind.

Die Verbindungen (IXa) können wiederum aus der Nitrobenzolsulfonsäure (VIII) durch die Folge von Umsetzungen
(1) Reduktion analog der Reduktion von (VII),
(2) N-Alkylierung und
(3) N-Acylierung,
letztere wie bei der Herstellung von (Vl) beschrieben, erhalten werden.

Die für die Umsetzung der Verbindungen (II) nach Variante a) benötigten Carbamate der Formel (III) sind literaturbekannt oder lassen sich analog bekannten Verfahren herstellen (vgl. EP-A-70804 oder US-A-4,480,101).

Aus den genannten Verbindungen der Formel (III) und (VIII) sowie deren Vorstufen lassen sich auch die für die Verfahrensvariante b) einsetzbaren Verbindungen der Formeln (IV) und (V) nach oder analog allgemein bekannten Methoden herstellen.

Die Phenylsulfonylisocyanate der Formel (IV) lassen sich z. B. analog den Verfahren aus EP-A-184385 aus Verbindungen der Formel (II), z. B. mit Phosgen, herstellen.

Die Umsetzung der Verbindungen (IV) mit den Aminoheterocyclen der Formel (V) führt man vorzugsweise in inerten, aprotischen Lösungsmitteln, wie z. B. Dioxan, Acetonitril oder Tetrahydrofuran bei Temperaturen zwischen 0° C und der Siedetemperatur des Lösungsmittels durch.

Die (Thio-)lsocyanate der Formel (X) sind nach literaturbekannten Verfahren darstellbar (vgl. EP-A-232067, EP-A-166516).

Die Umsetzung von Verbindungen der Formel (X) mit Sulfonamiden der Formel (II) erfolgt bei Temperaturen zwischen -10°C und +10°C, bevorzugt zwischen +20 und +80°C, in einem inerten aprotischen Lösungsmittel, wie z.B. Aceton oder Acetonitril in Gegenwart einer geeigneten Base, wie z.B. Triethylamin oder Kaliumcarbonat.

Die Salze der Verbindungen der Formel (I) werden vorzugsweise in inerten Lösungsmitteln wie z. B. Wasser, Methanol, Aceton, Dichlormethan, Tetrahydrofuran, Toluol oder Heptan, bei Temperaturen von 0 bis 100°C hergestellt. Geeignete Basen zur Herstellung der erfindungsgemäßen Salze sind beispielsweise Alkalicarbonate, wie Kaliumcarbonat, Alkali- und Erdalkalihydroxide, Ammoniak oder eine geeignete Aminbase, wie Triethylamin oder Ethanolamin. Als Säuren zur Salzbildung eignen sich z.B. HCI, HBr, H₂SO₄ oder HNO₃.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die erfindungsgemäßen Verbindungen der Formel (I) oder deren Salze weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im einzelnen seien beispielsweise einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung, z.B. durch Auslösen von Desikkation und Wuchstauchung, eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) oder deren Salze enthalten.

Die Verbindungen der Formel (I) oder deren Salze können auf verschiedene Art formuliert werden, je nachdem welche biologischen und/oder chemischphysikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluent and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calcium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z. B. Sorbitanfettsäureester oder Polyoxethylensorbitanester wie z. B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z. B. oben bei den anderen Formulierungstypen bereits aufgeführt sind, hergestellt werden.

Emulsionen, z. B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z. B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Die agrochemischen Zubereitungen enthalten in der Regel 0,1 bis 99 Gew.-%, insbesondere 0,1 bis 95 Gew.-%, Wirkstoff der Formel (I) oder deren Salze.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30, vorzugsweise meistens 5 bis 20 Gew.-% an Wirkstoff. versprühbare Lösungen etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Wirkstoffe in Mischungsformulierungen oder im Tank-Mix sind beispielsweise bekannte Wirkstoffe einsetzbar, wie sie in z.B. aus Weed Research 26, 441-445 (1986), oder "The Pesticide Manual", 9th edition, The British Crop Protection Council, 1990/91, Bracknell, England, und dort zitierter Literatur beschrieben sind. Als literaturbekannte Herbizide, die mit den Verbindungen der Formel (I) kombiniert werden können, sind z. B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet): acetochlor; acifluorfen; aclonifen; AKH 7088, d. h. [[[1-[5-[2-Chloro-4-(trifluoromethyl)-phenoxy]-2-nitrophenyl]-2-methoxyethylidene]-amino]-oxy]essigsäure und -essigsäuremethylester; alachlor; alloxydim; ametryn; amidosulfuron; amitrol; AMS, d. h. Ammoniumsulfamat; anilofos; asulam; atrazin; aziprotryn; barban; BAS 516 H, d. h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; carbetamide; CDAA, d. h. 2-Chlor-N,N-di-2-propenylacetamid; CDEC, d. h. Diethyldithiocarbaminsäure-2-chlorallylester; CGA 184927, d. h. 2-[4-[(5-Chlor-3-fluor-2-pyridinyl)-oxy]phenoxy]-propansäure und 2-propynylester; chlomethoxyfen; chloramben; chlorazifop-butyl, pirifenop-butyl; chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clomazone; clomeprop; cloproxydim; clopyralid; cyanazine; cycloate; cycloxydim; cycluron; cyperquat; cyprazine; cyprazole; 2,4-DB; dalapon; desmediphan; desmetryn; di-allate; dicamba; dichlobenil; dichlorprop; diclofop-methyl; diethatyl; difenoxuron; difenzoquat; diflufenican; dimefuron; dimethachlor; dimethametryn; dimethazone, clomazon; dimethipin; dimetrasulfuron, cinosulfuron; dinitramine; dinoseb; dinoterb; diphenamid; dipropetryn; diquat; dithiopyr; diuron; DNOC; eglinazine-ethyl; EL 177, d. h. 5-Cyano-1-(1,1-dimethylethyl)-N-methyl-3H-pyrazole-4-carboxamid; endothal; EPTC; esprocarb; ethalfluralin; ethametsulfuron-methyl; ethidimuron; ethiozin; ethofumesate; F5231, d. h. N-[2-Chlor-4-fluor-5-[4-(3-fluorpropyl)-4,5-dihydro-5-oxo-1H-tetrazol-1-yl]-phenyl]-ethansulfonamid; F6285, d. h. 1-[5-(N-Methylsulfonyl)-amino-2,4-dichlorophenyl]-3-methyl-4-difluoromethyl-1,2,4-triazol-5-on; fenoprop; fenoxan, s. clomazon; fenoxaprop-ethyl; fenuron; flamprop-methyl; flazasulfuron; fluazifop und dessen Esterderivate; fluchloralin; flumetsulam; N-[2,6-Difluorphenyl]-5-methyl-(1,2,4)-triazolo[1,5a]pyrimidin-2-sulfonamid; flumeturon; flumipropyn; fluorodifen; fluoroglycofen-ethyl; fluridone; flurochloridone; fluroxypyr; flurtamone; fomesafen; fosamine; furyloxyfen; glufosinate; glyphosate; halosaten; haloxyfop und dessen Esterderivate; hexazinone; Hw 52, d. h. N-(2,3-Dichlorphenyl)-4-(ethoxymethoxy)-benzamid; imazamethabenz-methyl; imazapyr; imazaquin; imazethamethapyr; imazethapyr; imazosulfuron; ioxynil; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; methabenzthiazuron; metham; methazole; methoxyphenone; methyldymron; metobromuron; metolachlor; metoxuron; metribuzin; metsulfuron-methyl; MH; molinate; monalide; monocarbamide dihydrogensulfate; monolinuron; monuron; MT 128, d. h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d. h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiazon; oxyfluorfen; paraquat; pebulate; pendimethalin; perfluidone; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Esterderivate; propazine; propham; propyzamide; prosulfalin; prosulfocarb; prynachlor; pyrazolinate; pyrazon; pyrazosulfuron-ethyl; pyrazoxyfen; pyridate; quinclorac; quinmerac; quinofop und dessen Esterderivate, quizalofop und dessen Esterderivate; quizalofop-ethyl; quizalofop-p-tefuryl; renriduron; dymron; S 275, d. h. 2-[4-Chlor-2-fluor-5-(2-propynyloxy)-phenyl]-4,5,6,7-tetrahydro-2H-indazol; S 482, d. h. 2-[7-Fluor-3,4-dihydro-3-oxo-4-(2-propynyl)-2H-1,4-benzoxazin-6-yl]-4,5,6,7-tetrahydro-1H-isoindol-1,3(2H)-dion; secbumeton; sethoxydim; siduron; simazine; simetryn; SN 106279, d. h. 2-[[7-[2-Chlor-4-(trifluor-methyl)-phenoxy]-2-naphthalenyl]-oxyl-propansäure und -methylester; sulfometuron-methyl; sulfazuron; flazasulfuron; TCA; tebutam; tebuthiuron; terbacil; terbucarb; terbuchlor; terbumeton; terbuthylazine; terbutryn; TFH 450, d. h. N,N-Diethyl-3-[(2-ethyl-6-methylphenyl)-sulfonyl]-1H-1,2,4-triazol-1-carboxamid; thiazafluron; thifensulfuron-methyl; thiobencarb; tiocarbazil; tralkoxydim; tri-allate; triasulfuron; triazofenamide; tribenuron-methyl; triclopyr; tridiphane; trietazine; trifluralin; trimeturon; vernolate; WL 110547, d. h. 5-Phenoxy-1-[3-(trifluormethyl)-phenyl]-1H-tetrazol.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser, und anschließend auf die Pflanzen, Pflanzenteile oder den landwirtschaftlich oder industriell genützten Boden, auf dem die Pflanzen stehen oder in dem sie heranwachsen oder als Saat vorliegen, appliziert. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

### A) Chemische Beispiele

### a) 2-Carboxy-nitrobenzolsulfonsäure

Zu einer Lösung aus 106,0 g (0,40 Mol) 2-Methyl-5-nitro-benzolsulfonsäure und 80,0 g (0,58 Mol) Kaliumcarbonat in 1300 ml Wasser werden bei 80° C portionsweise 400,0 g (2,53 Mol) Kaliumpermanganat innerhalb von 2 h zugesetzt. Die Reaktionstemperatur wird dabei zwischen 80 und 95° C gehalten.
Nach weiteren 4 h Nachrühren wird von dem entstandenen Feststoff abfiltriert. Das Filtrat wird mit konzentrierter Salzsäure angesäuert (pH = 1). Die ausgefallene, farblose 2-Carboxy-5-nitro-benzolsulfonsäure wird über einen Büchnertrichter abgesaugt und bei ca. 50° C/100 Torr getrocknet (82,0 g; 83,7 % d. Th.;
Schmp.: > 300° C.

### b) 2-Methoxycarbonyl-5-nitrobenzolsulfonsäure

Eine Suspension aus 190,0 g (0,77 mol) 2-Carboxy-5-nitrobenzolsulfonsäure, 10 ml DMF und 250 ml (3,43 mol) Thionylchlorid wird 3 h zum Sieden erhitzt. Nach dem Abtrennen von unlöslichen Bestandteilen mittels Filtration wird das Filtrat eingeengt. Zu dem so erhaltenen Rückstand werden 200 ml (4,94 mol) Methanol zugesetzt. Nach beendeter Zugabe wird das Reaktionsgemisch an 0°C abgekühlt. Der ausgefallene Feststoff wird abfiltriert und getrocknet. Man erhält so 70,9 g (35,3 % d.Th.) der farblosen, kristallinen 2-Methoxycarbonyl-5-nitrobenzolsulfonsäure (Schmp.: 92-94°C). Durch Abdestillieren flüchtiger Komponenten der Mutterlauge erhält man eine zweite Fraktion (62,5 g, 31,1 % d.Th.).

### c) 2-Methoxycarbonyl-5-nitro-benzolsulfonchlorid

Eine Lösung aus 70,9 g (0,27 mol) 2-Methoxycarbonyl-5-nitrobenzolsulfonsäure in 300 ml Methanol wird unter kräftigem Rühren vorsichtig mit einer Lösung aus
17,3 g (0,27 mol) Kaliumhydroxid (88 %ig) und 100 ml Methanol versetzt. Nach dem Abkühlen auf 0°C wird das ausgefallene Salz abfiltriert, getrocknet und anschließend in 150 ml Sulfolan, 150 ml Acetonitril und 10 ml Dimethylformamid suspendiert. Nach dem Zutropfen von 100 ml (1,07 mol) Phosphoroxychlorid wird das Gemisch für 2,5 h zum Sieden erhitzt. Anschließend wird das Reaktionsgemisch auf Eiswasser gegossen. Das ausgefallene 2-Methoxycarbonyl-5-nitrobenzolsulfochlorid (60,1 g, 70 % d.Th.) wird über einen Büchnertrichter abgesaugt und unter reduziertem Druck von Lösungsmittelspuren befreit.
Schmp.: 86-88°C.

### d) N-tert.-Butyl-2-methoxycarbonyl-5-nitrobenzolsulfonamid

Zu einer Lösung aus 34,4 g (0,12 mol) 2-Methoxycarbonyl-5-nitrobenzolsulfochlorid in 200 ml Essigsäureethylester und 250 ml Ethanol werden bei 0°C 50 ml
(0,48 mol) tert.-Butylamin langsam zugetropft. Anschließend wird die Reaktionslösung noch 10 min bei Raumtemperatur gerührt. Nach der Zugabe von 500 ml Wasser kristallisiert ein farbloser Feststoff aus. Nach dem Abfiltrieren und Trocknen werden 28,1 g (89 % d.Th.) an N-tert.-Butyl-2-methoxycarbonyl-5-nitrobenzolsulfochlorid erhalten. Schmp.: 121-124°C.

### e) N-tert.-Butyl-5-amino-2-methoxycarbonyl-benzolsulfonamid

25,08 g (0,079 mol) N-tert.-Butyl-2-methoxycarbonyl-5-nitrobenzolsulfonamid werden in 1000 ml MeOH gelöst. Nach der Zugabe von 0,50 g Pd-C (10 %ig) wird das Gemisch unter einer Wasserstoffatmosphäre (1 atm.) solange geschüttelt, bis die Wasserstoffaufnahme beendet ist. Nach dem Abtrennen des Katalysators wird das Lösungsmittel abdestilliert. Der Rückstand wird mit wenig Essigester ausgerührt. Das erhaltene N-tert.-Butyl-5-amino-2-methoxycarbonyl-benzolsulfonamid (18,3 g; 80,9 % d.Th.) schmilzt bei 193°C.

### f) N-tert:Butyl-5-formylamino-2-methoxycarbonyl-benzolsulfonamid

Bei 0°C werden 13 ml Essigsäureanhydrid vorsichtig mit 6,5 ml Ameisensäure versetzt. Anschließend wird das Gemisch für 2 h auf 50-60°C erwärmt. Nun wird eine Lösung aus 16,0 g (0,056 mol) N-tert.-Butyl-5-amino-2-methoxy-carbonyl-benzolsulfonamid und 50 ml DMF bei 0°C zugetropft. Nach dem Entfernen des Kühlbades wird noch 4 h bei Raumtemperatur gerührt. Danach wird das Reaktionsgemisch in 800 ml Essigsäureethylester aufgenommen und mit dreimal 150 ml Wasser gewaschen. Nach dem Trocknen der organischen Phase über Magnesiumsulfat und dem Abdestillieren des Solvens wird der Rückstand aus Essigsäureethylestern/n-Heptan umkristallisiert. Das erhaltene N-tert.-Butyl-5-formylamino-2-methoxycarbonylbenzolsulfonamid (14,23 g, 82 % d.Th.) schmilzt bei 113-114°C.

### g) N-tert.-Butyl-2-methoxycarbonyl-5-methylamino-benzolsulfonamid

9,92 g (0,032 mol) N-tert.-Butyl-5-formylamino-2-methoxycarbonylbenzolsulfonamid werden in 50 ml CHCl₃ gelöst und anschließend bei 0°C mit 5 ml (0,053 mol) Boran-Dimethylsulfid-Komplex versetzt. Nach 1 h bei 0°C und 3 h bei Raumtemperatur wird das Reaktionsgemisch auf 0°C abgekühlt. Nach der Zugabe von 30 ml Methanol wird das Gemisch mit Wasser gewaschen. Nach dem Trocknen der organischen Phase über Magnesiumsulfat und dem Abdestillieren des Solvens werden 9,23 g (98 % d.Th.) an N-tert.-Butyl-2-methoxycarbonyl-5-methylaminobenzolsulfonamid erhalten. Schmp.: 120-124°C.

### h) N-tert.-Butyl-5-[N-(ethoxycarbonylamino-thiocarbonyl)-methylamino]-2-methoxycarbonyl-benzolsulfonamid

1,20 g (4,0 mmol) N-tert.-Butyl-2-methoxycarbonyl-5-methylamino-benzolsulfonamid werden in 5 ml wasserfreiem DMF gelöst und mit 0,60 g (4,3 mmol) 95 %igem Ethoxycarbonylsenföl versetzt. Nach 3 h bei Raumtemperatur wird das Gemisch in Essigsäureethylester aufgenommen und mit 1 N Salzsäure und Wasser gewaschen. Nach dem Trocknen der organischen Phase über Magnesiumsulfat wird das Solvens abdestilliert. Der Rückstand wird in wenig Essigsäureethylester aufgenommen und mit Heptan bei -25°C gefällt. Nach dem Absaugen und Trocknen werden 1,29 g (75 % d.Th.) des farblosen N-tert.-Butyl-5-[N-(ethoxycarbonylamino-thiocarbonyl)-methylamino]-2-methoxycarbonyl-benzolsulfonamid erhalten. Schmp.: 110°C.

### i) 5-[N-(Ethoxycarbonylamino-thiocarbonyl)-methylamino]-2-methoxy-carbonyl-benzolsulfonamid

1,05 g (2,4 mmol) N-tert.-Butyl-5-[N-(ethoxycarbonylaminothiocarbonyl)-N-methyl-amino]-2-methoxycarbonyl-benzolsulfonamid werden 18 h lang in 15 ml Trifluoressigsäure bei Raumtemperatur gerührt. Anschließend werden flüchtige Komponenten bei 12 Torr/50°C abdestilliert. Der Rückstand wird in Toluol suspendiert. Nach dem erneuten Einengen der Suspension bei 12 Torr/50°C wird ein hochviskoser Rückstand (0,85 g, 93 % d.Th.) erhalten, der ohne weitere Reinigung direkt zu dem entsprechenden Sulfonylharnstoff umgesetzt wird (vgl. Beispiel j).

### j) N-[(4,6-Dimethoxypyrimidin-2-yl)-aminocarbonyl]-5-[N-(ethoxycarbonylaminothiocarbonyl)-methylamino]-2-methoxycarbonyl-benzolsulfonamid (siehe Tabellenbeispiel 2192)

Eine Suspension aus 0,69 g (1,84 mmol) 5-[N-(Ethoxycarbonylaminothiocarbonyl)-methylamino]-2-methoxycarbonyl-benzolsulfonamid und 0,55 g (1,99 mmol) 4,6-Dimethoxy-2-phenoxycarbonylamino-pyrimidin in 5 ml Acetonitril wird mit 1,2 ml DBU versetzt. Nach 17 h Rühren bei Raumtemperatur wird das Solvens abdestilliert. Der Rückstand wird in Wasser aufgenommen und mit Diethylether gewaschen. Nach dem Ansäuern der wäßrigen Phase mit konzentrierter Salzsäure (pH = 1) fällt ein farbloser Feststoff aus. Dieser wird abfiltriert und mit wenig Methanol ausgerührt. Man erhält so 0,61 g (60 % d.Th.) an N-[(4,6-Dimethoxy-pyrimidin-2-yl)-aminocarbonyl]-5-[N-(ethoxycarbonylamino-thiocarbonyl)-methylaminol-2-methoxycarbonyl-benzolsulfonamid. Schmp.: 144-145°C.

### k) 5-(N-Acetyl-methylamino)-N-[4,6-dimethoxy-pyrimidin-2-yl)-aminocarbonyl]-2-methoxycarbonylbenzolsulfonamid (siehe Tabelle 1, Beispiel 89)

Zu einem Gemisch aus 0,75 g (2,6 mMol) 5-(N-Acetyl-methylamino)-2-methoxycarbonylbenzolsulfonamid und 0,73 g (2,6 mMol) 4,6-Dimethoxy-2-(phenoxycarbonylamino)-pyrimidin in 10 ml CH₃CN werden 0,45 ml DBU zugesetzt. Nach der Aufarbeitung analog Beispiel j) erhält man 0,59 g des gewünschten Sulfonylharnstoffs mit Schmp. 197°C.

### I) 2-Methoxycarbonyl-5-(N-methoxycarbonyl-N-methyl-amino)-benzolsulfonylisocyanat

5,0 g 2-Methoxycarbonyl-5-(N-methoxycarbonyl-N-methyl-amino)-benzolsulfonamid werden in 17 ml 1,2-Dichlorethan suspendiert. Nach der Zugabe von 4 ml Thionylchlorid wird das Reaktionsgemisch 5 h lang zum Sieden erhitzt. Nach dem Abkühlen auf Raumtemperatur setzt man 0,3 ml Pyridin hinzu, leitet Phosgen in das Reaktionsgemisch ein und erhitzt die Lösung 4 h zum Rückfluß. Nach dem Einengen des Reaktionsgemisches erhält man 6,3 g eines Öls, das direkt in die nachfolgenden Umsetzung eingesetzt wird (Beispiele m, n, o).

### m) 2-Methoxycarbonyl-5-(N-methoxycarbonyl-N-methyl-amino)-N-[(4,6-dimethylpyrimidin-2-yl)-aminocarbonyl]-benzolsulfonamid (siehe Tabelle 1, Beispiel 1.183)

Zu einer Lösung aus 0,8 g 2-Amino-4,6-dimethylpyrimidin in 20 ml Dichlorethan wird eine Lösung aus 2,1 g 2-Methoxycarbonyl-5-(N-methoxycarbonylmethylamino)-benzolsulfonylisocyanat (Beispiel I)) und 20 ml Dichlorethan zugesetzt. Nach 8 h Rühren bei 40°C wird das Reaktionsgemisch eingeengt. Der Rückstand wird sukzessive mit 1N Salzsäure und Methanol intensiv ausgerührt. Man erhält so 0,47 g des gewünschten Sulfonylharnstoffes als festen Schaum;
¹H-NMR (DMSO, 80 MHz): δ (ppm) = 2,5 (s, 6H, 2 CH₃), 3,3 (s, 3H, N-CH₃), 3,7 (s, 3H, O-CH₃), 3,9 (s, 3H, OCH₃), 7,0 (s, 1H, H_{Pyrimidin}) 7,8 (s, 2H, 3-H, und 4-H), 8,1 (s, 1H, 6-H), 10,6 (s, 1H, NH), 13,3 (s, 1H, SO₂-NH).

### n) N-[(4-Chlor-6-methoxypyrimidin-2-yl)-aminocarbonyl]-2-methoxycarbonyl-5-(N-methoxycarbonyl-N-methyl-aminol-benzolsulfonamid (siehe Tabelle 1, Beispiel 1.185)

Analog Beispiel m) werden 0,9 g 2-Amino-4-chlor-6-methoxypyrimidin mit 2,1 g 2-Methoxycarbonyl-5-(N-methoxycarbonyl-N-methyl-amino)-benzolsulfonylisocyanat (Beispiel I)) zur Reaktion gebracht. Ausbeute: 0,45 g des gewünschten Sulfonylharnstoffs als festen Schaum;
¹H-NMR (DMSO, 80 MHz): δ (ppm) = 3,3 (s, 3H, N-CH₃), 3,7 (s, 3H, O-CH₃), 3,8 (s, 3H, O-CH₃), 4,0 (s, 3H, O-CH₃), 6,8 (s, 1H, H_{Pyrimin}), 7,7 (s, 2H, 3-H, und 4-H), 10,9 (s, 1H, NH), 12,0 (s, 1H, SO₂-NH).

### o) N-[N-(4-Methoxy-6-methyltriazin-2-yl)-aminocarbonyl]-2-methoxycarbonyl-5-(N-methoxycarbonyl-N-methyl-amino)-benzolsulfonamid (siehe Tabelle 1, Beispiel 1.180)

Analog Beispiel m) werden 0,85 g 2-Amino-4-methoxy-6-methyltriazin mit 2,1 g 2-Methoxycarbonyl-5-(N-methoxycarbonyl-N-methyl-amino)-benzolsulfonylisocyanat (Beispiel I)) zur Reaktion gebracht. Ausbeute: 0,47 g des gewünschten Sulfonylharnstoffs als festen Schaum;
¹H-NMR (DMSO, 80 MHz): δ (ppm) = 2,4 (s, 3H, CH₃), 3,3 (s, 3H, N-CH₃), 3,7 (s, 3H, OCH₃), 3,8 (s, 3H, OCH₃), 4,0 (s, 3H, O-CH₃), 7,8 (s, 2H, 3-H, 4-H), 8,1 (s, 1H, 6-H), 11,0 (s, 1H, NH), 12,3 (s, 14, SO₂NH).

Die übrigen in der Tabelle 1 beschriebenen Verbindungen erhält man auf analoge Weise zu den Beispielen a) bis o).

Folgende Abkürzungen werden in der Tabelle 1 verwendet:

| | |
|---|---|
| No. | = Beispielnummer |
| Fp. | = Festpunkt (Schmelzpunkt) in °C |
| Me | = Methyl |
| Et | = Ethyl |
| Pr | = n-Propyl |
| n-Pr | = n-Propyl |
| i-Pr | = i-Propyl |
| c-Pr | = Cyclopropyl |
| Bu | = Butyl |
| Ph | = Phenyl |

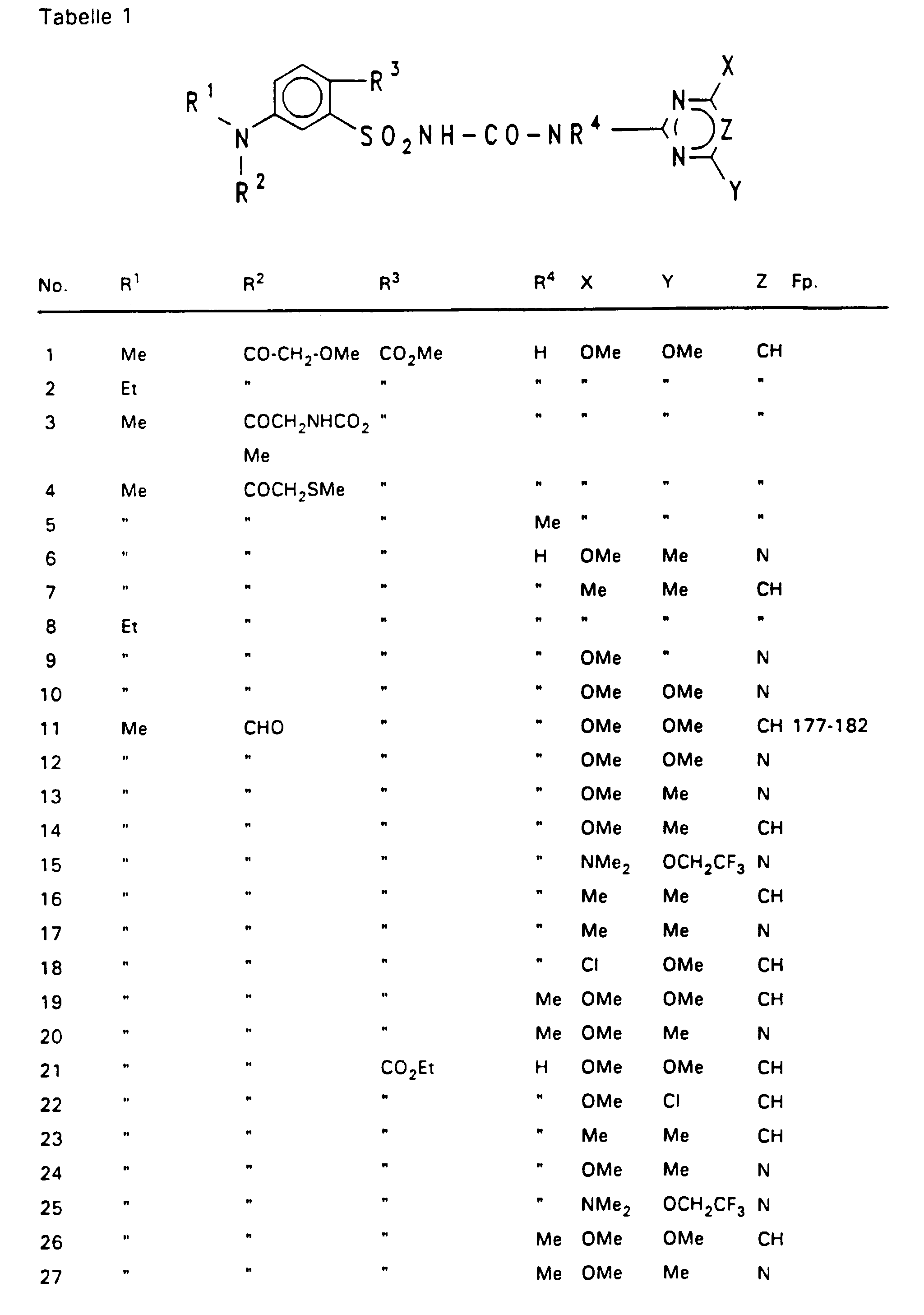

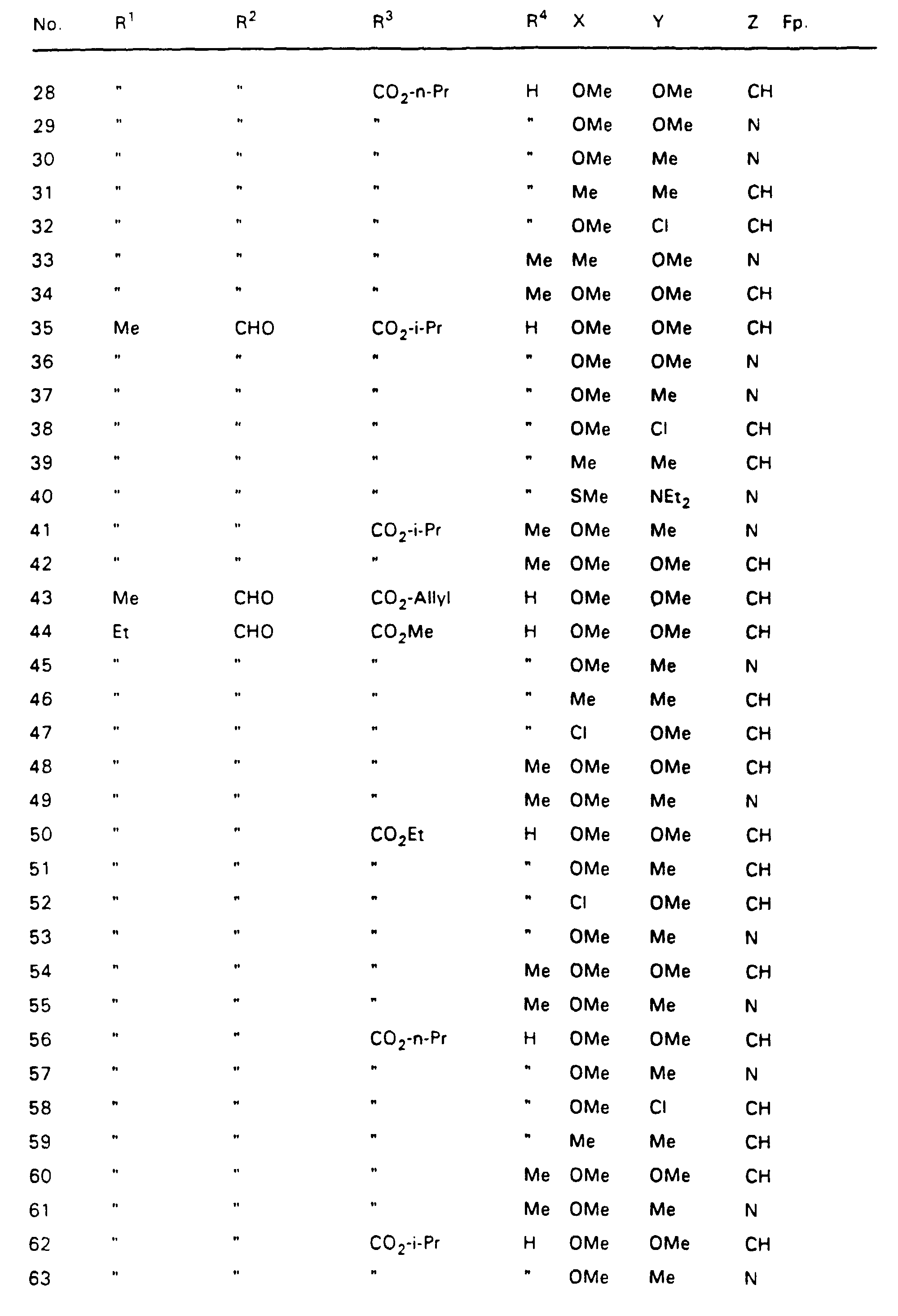

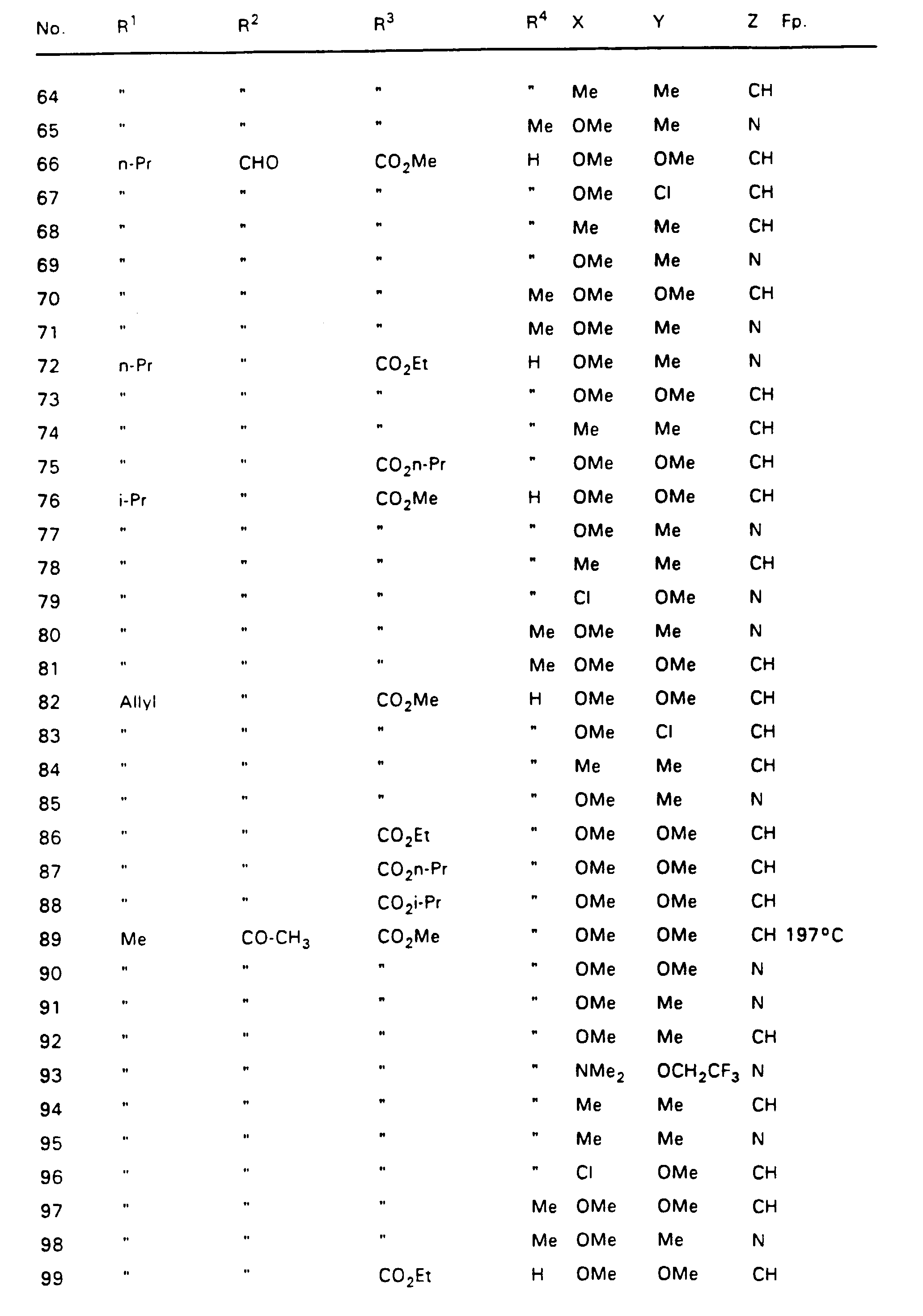

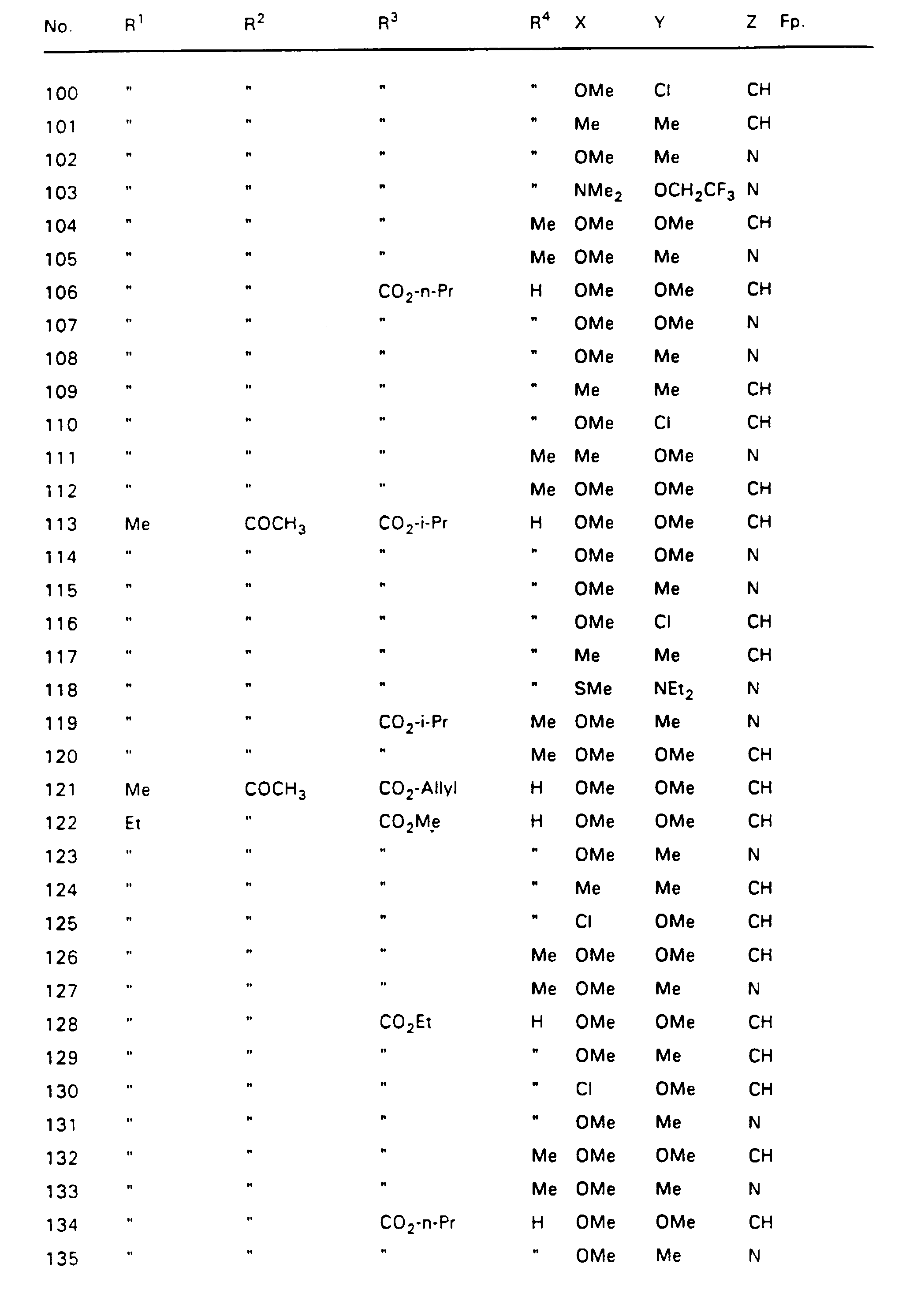

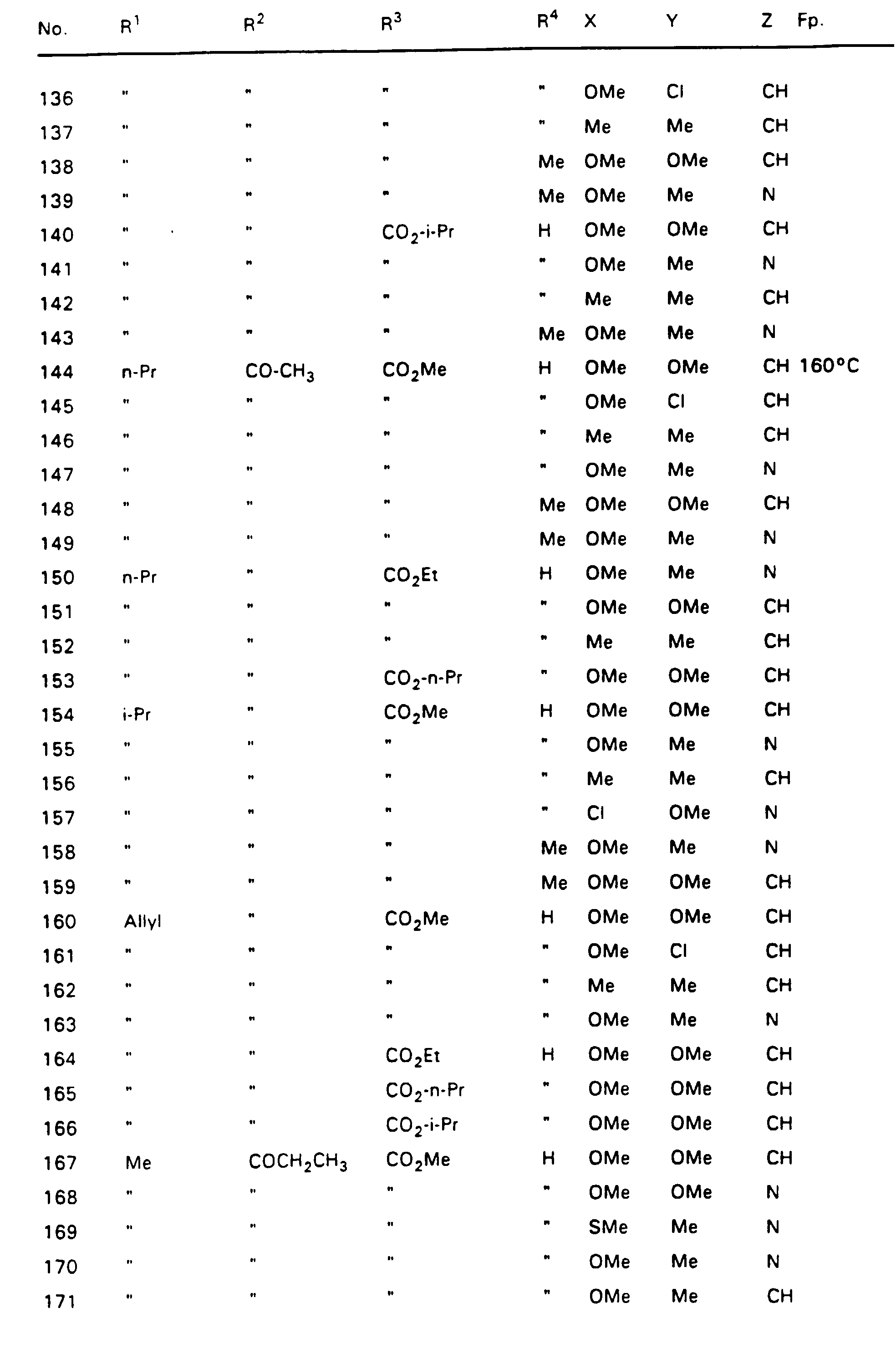

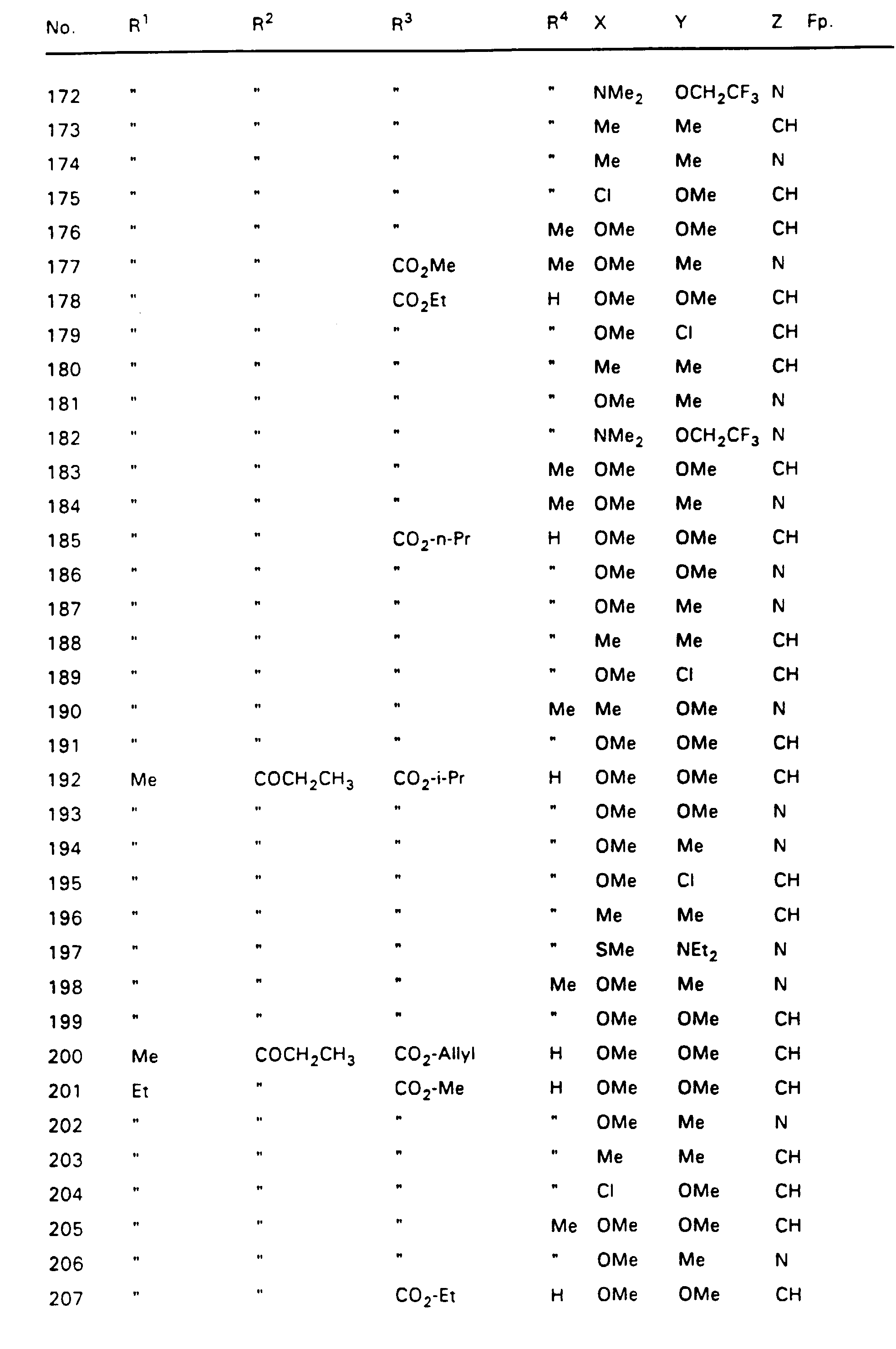

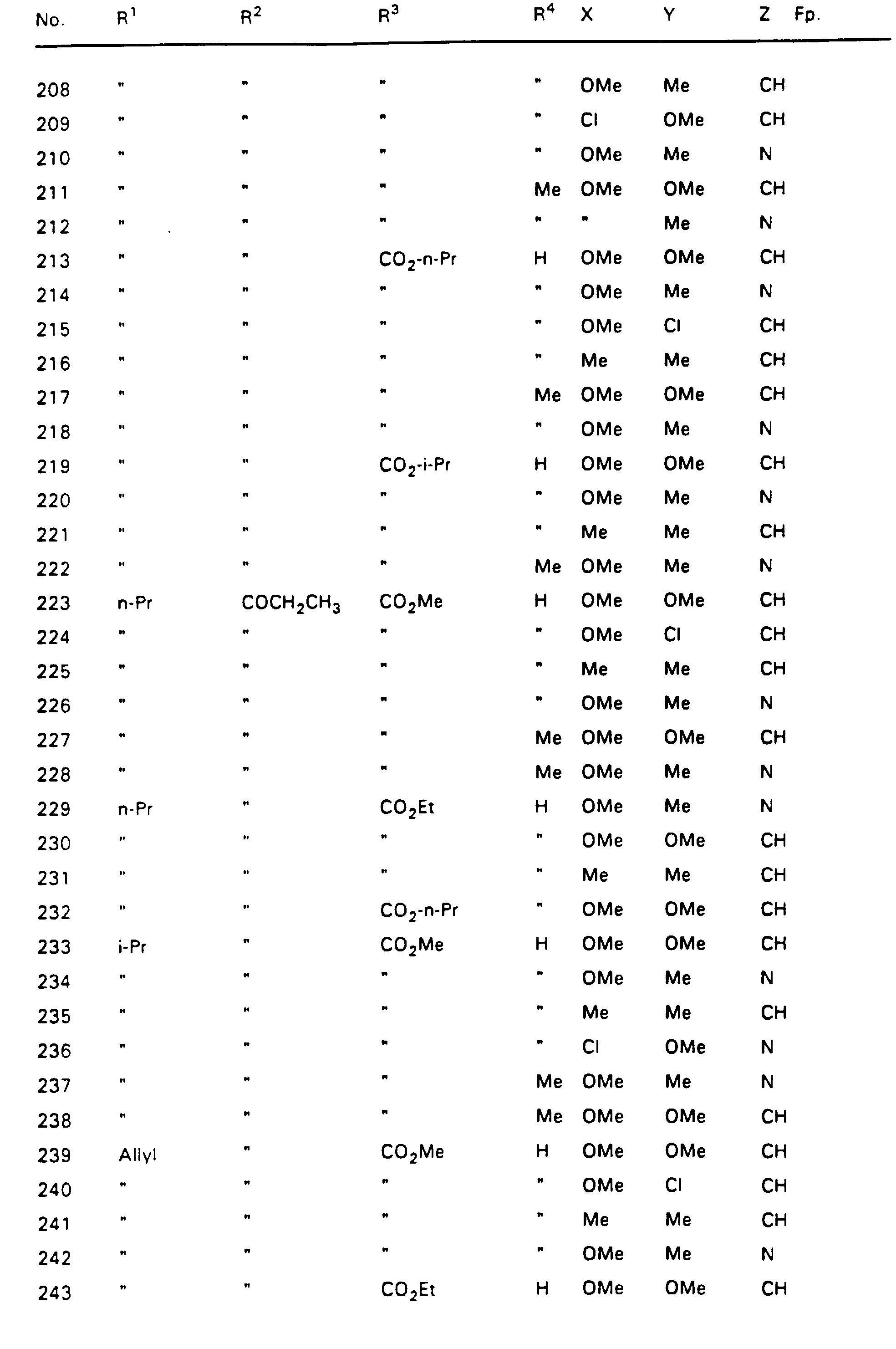

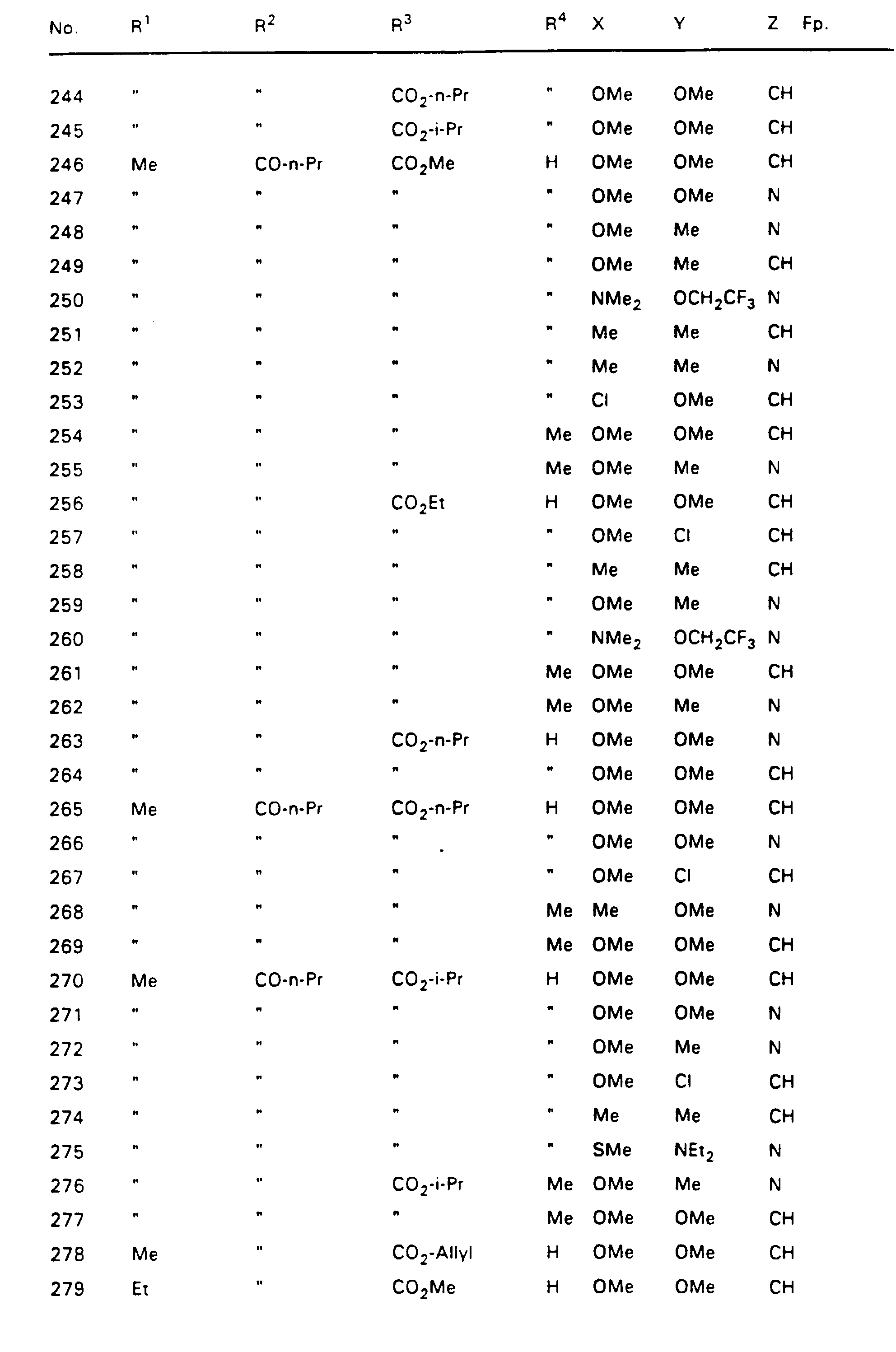

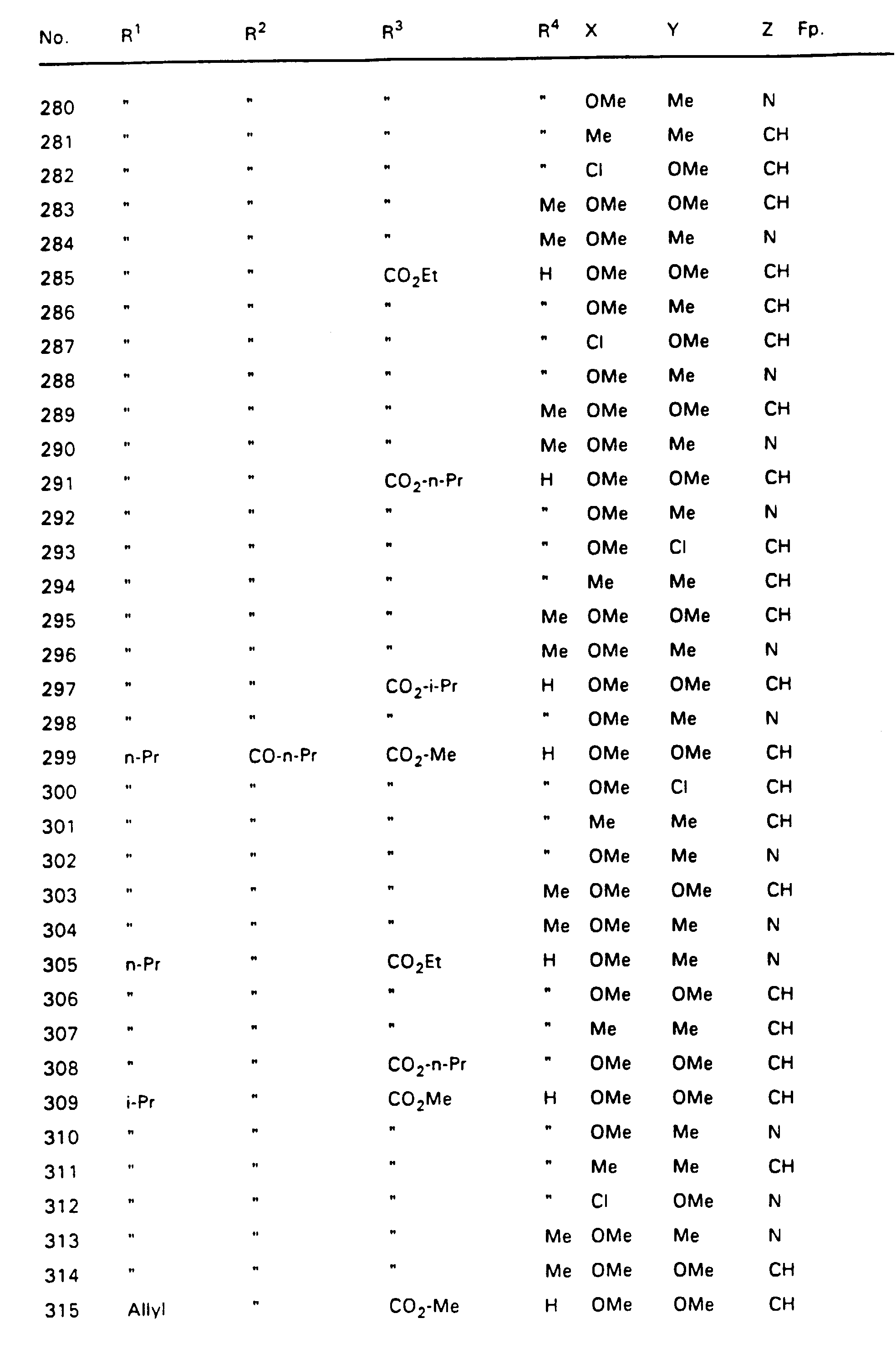

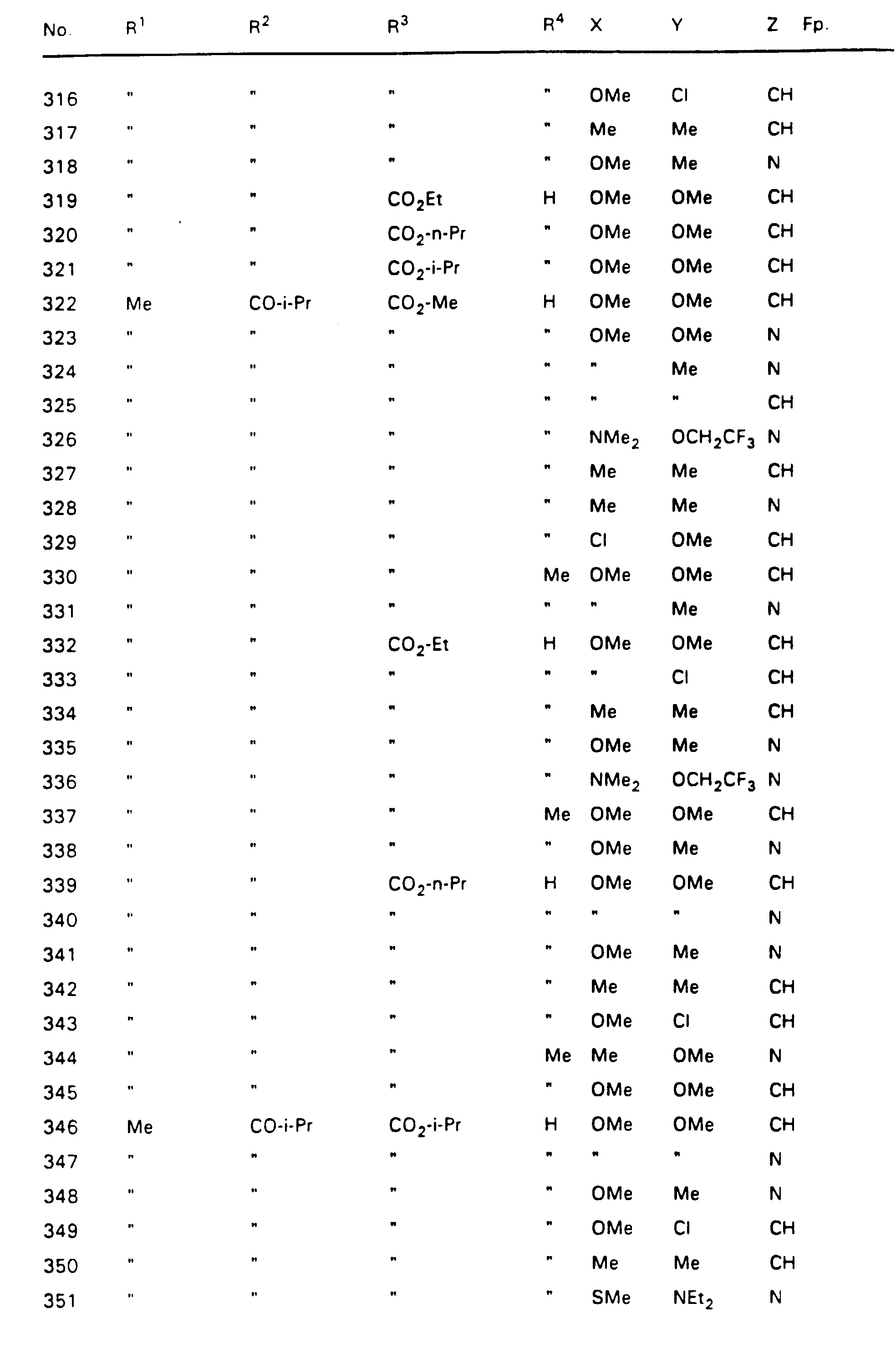

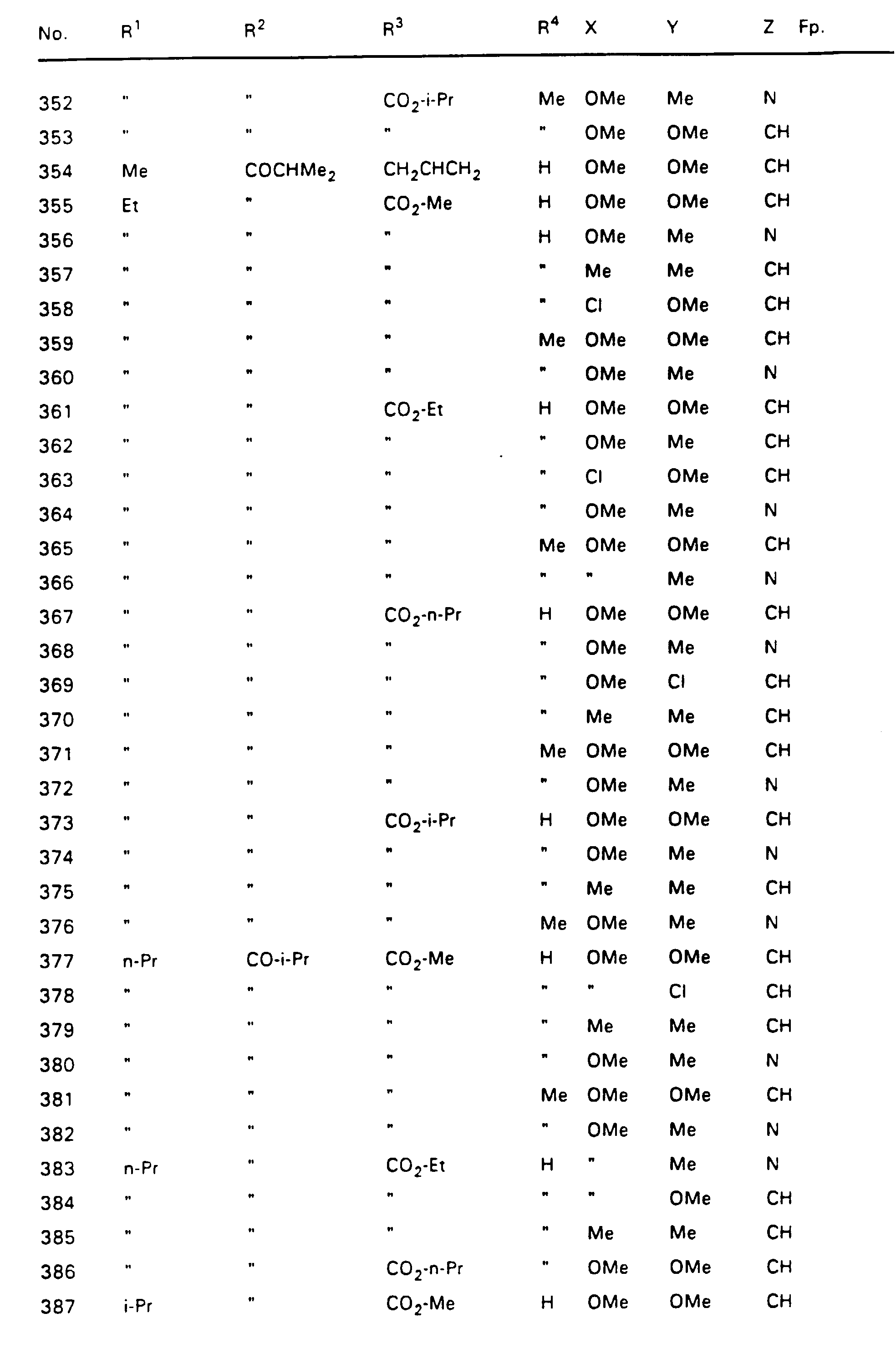

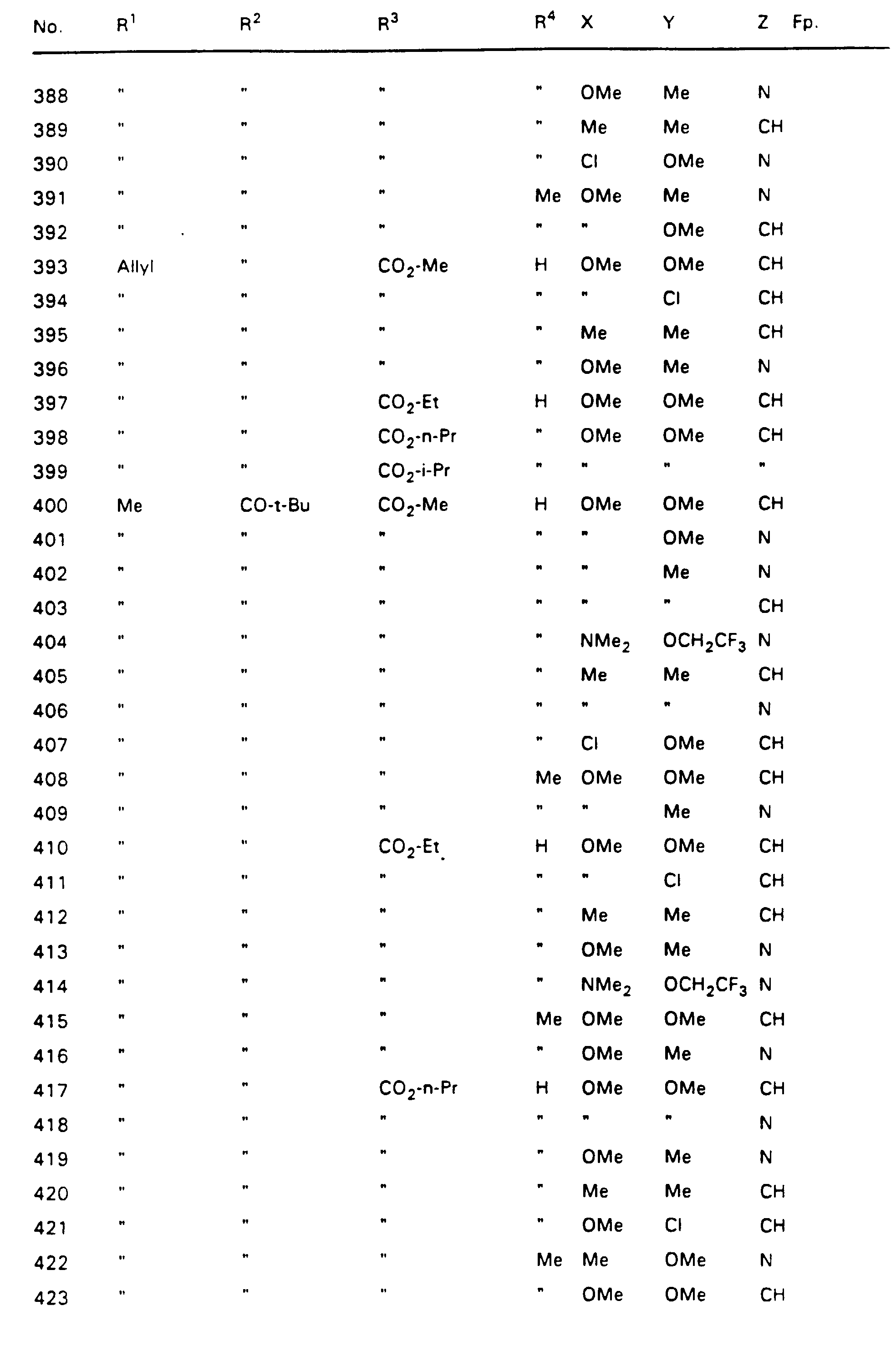

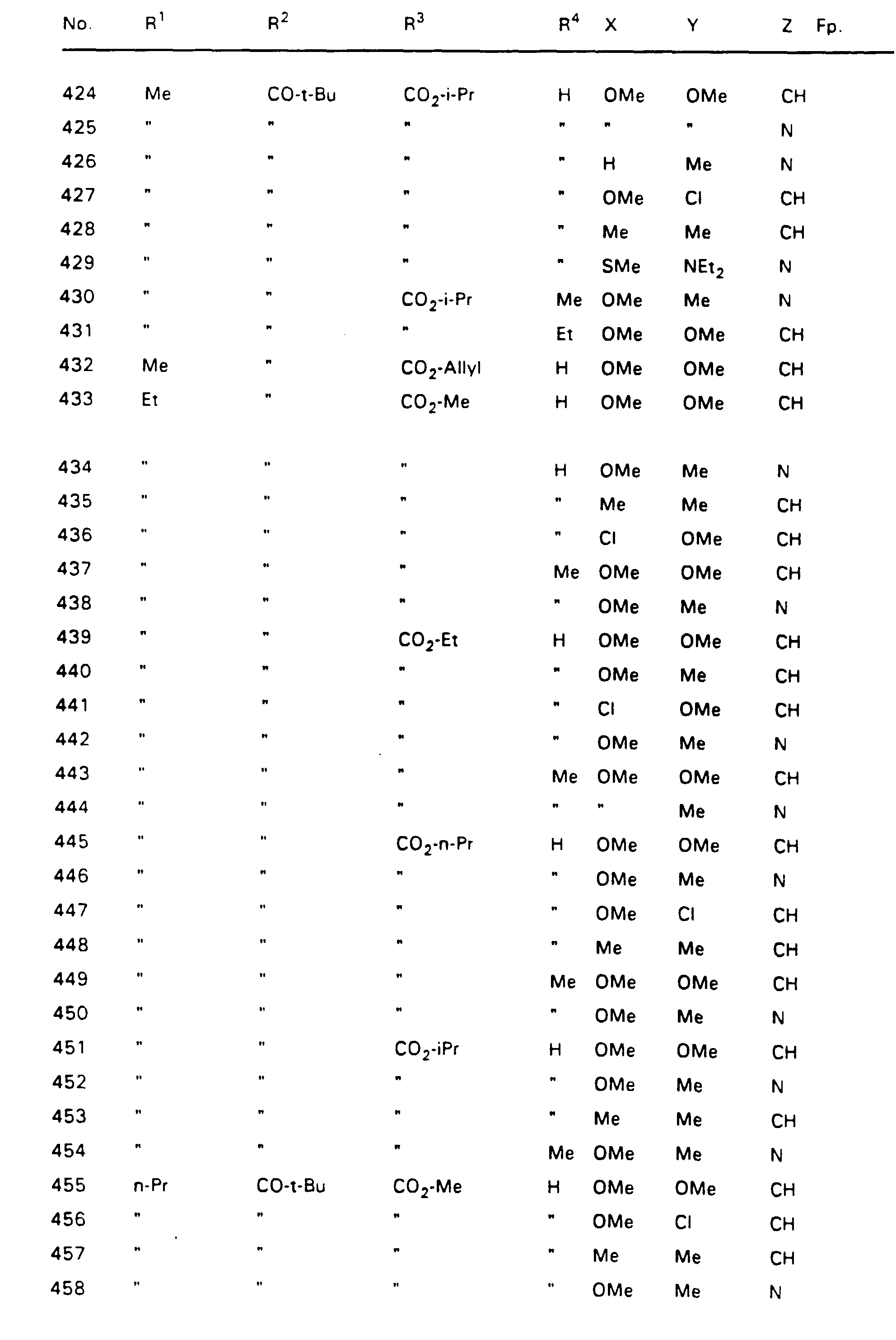

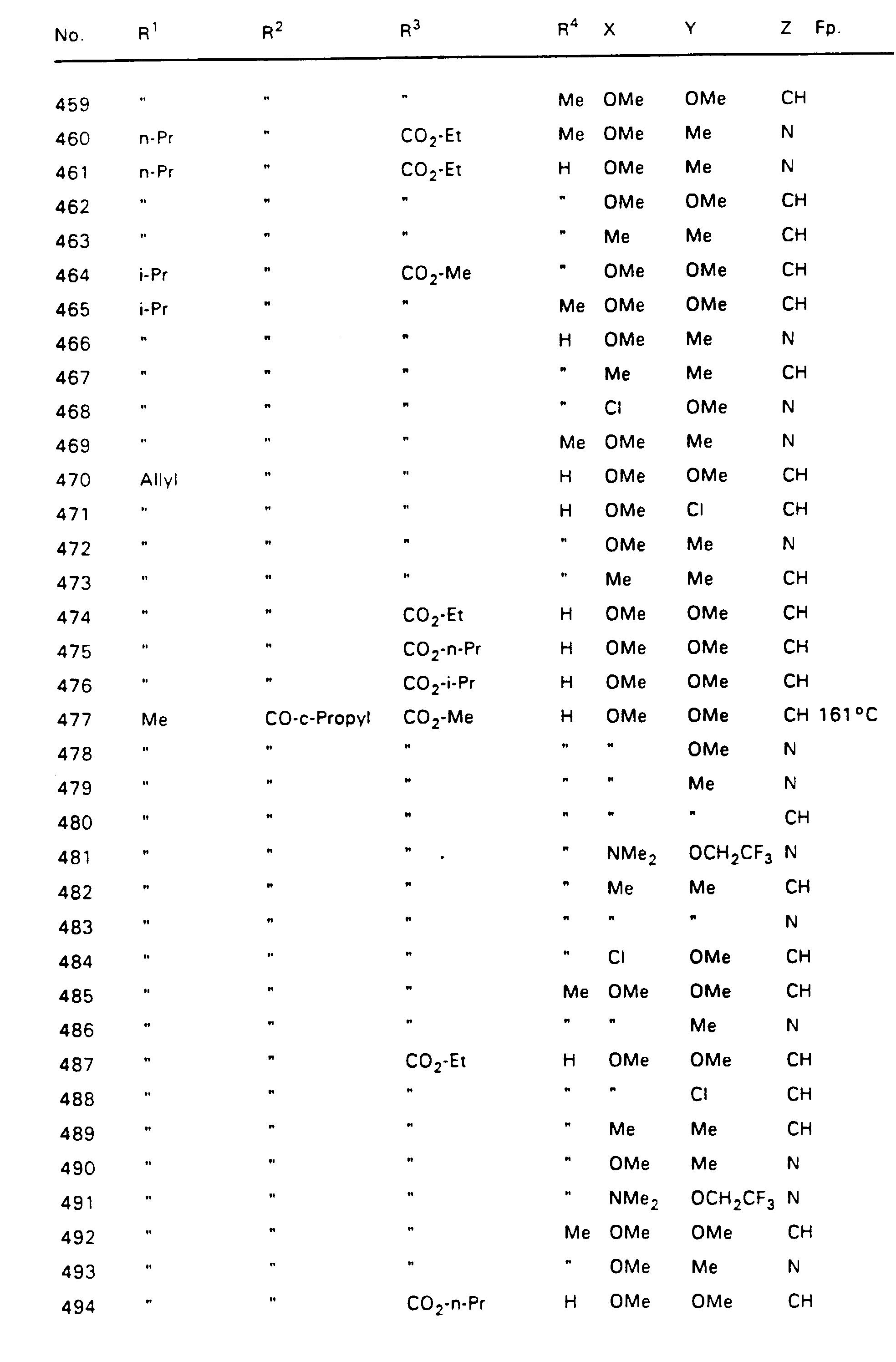

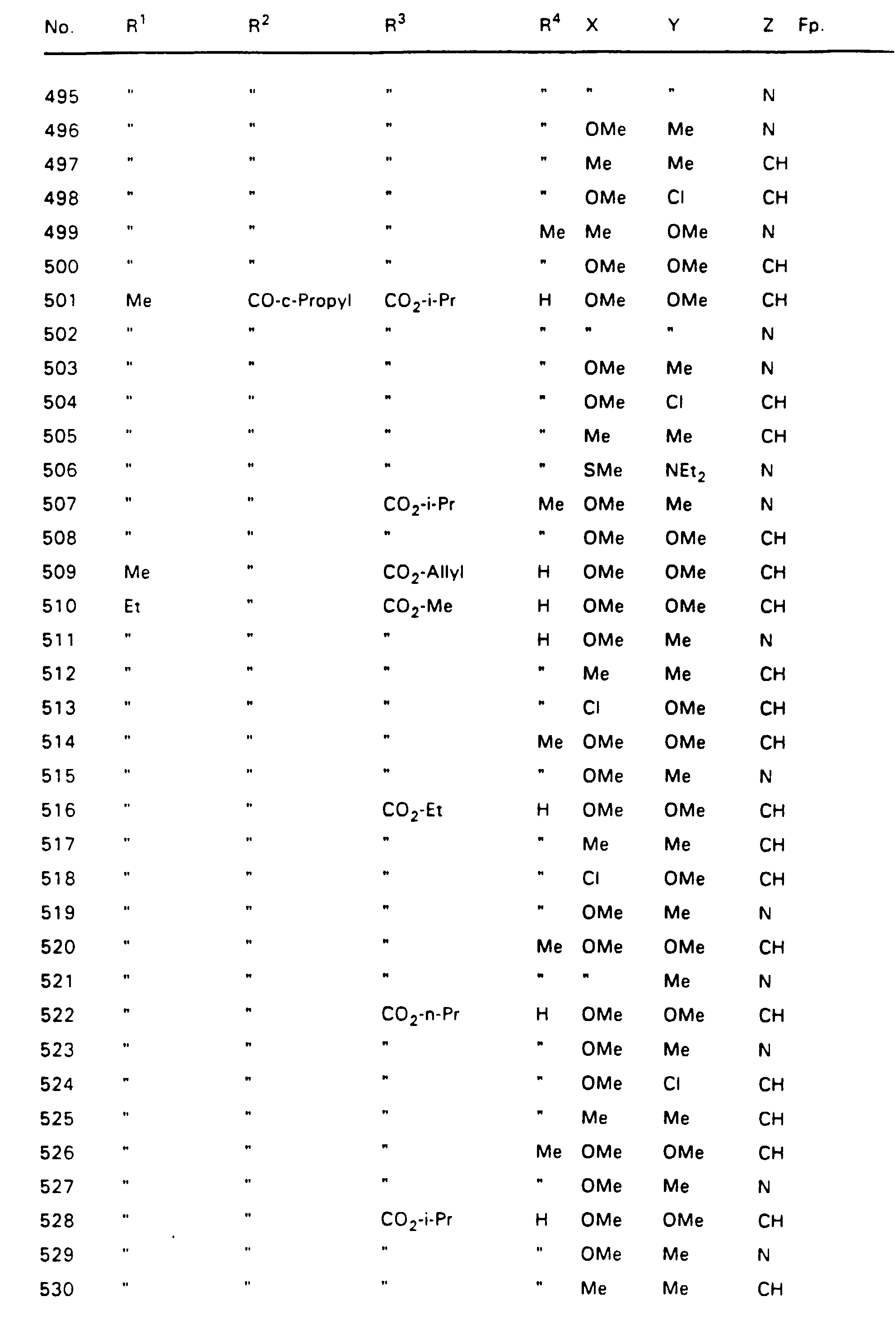

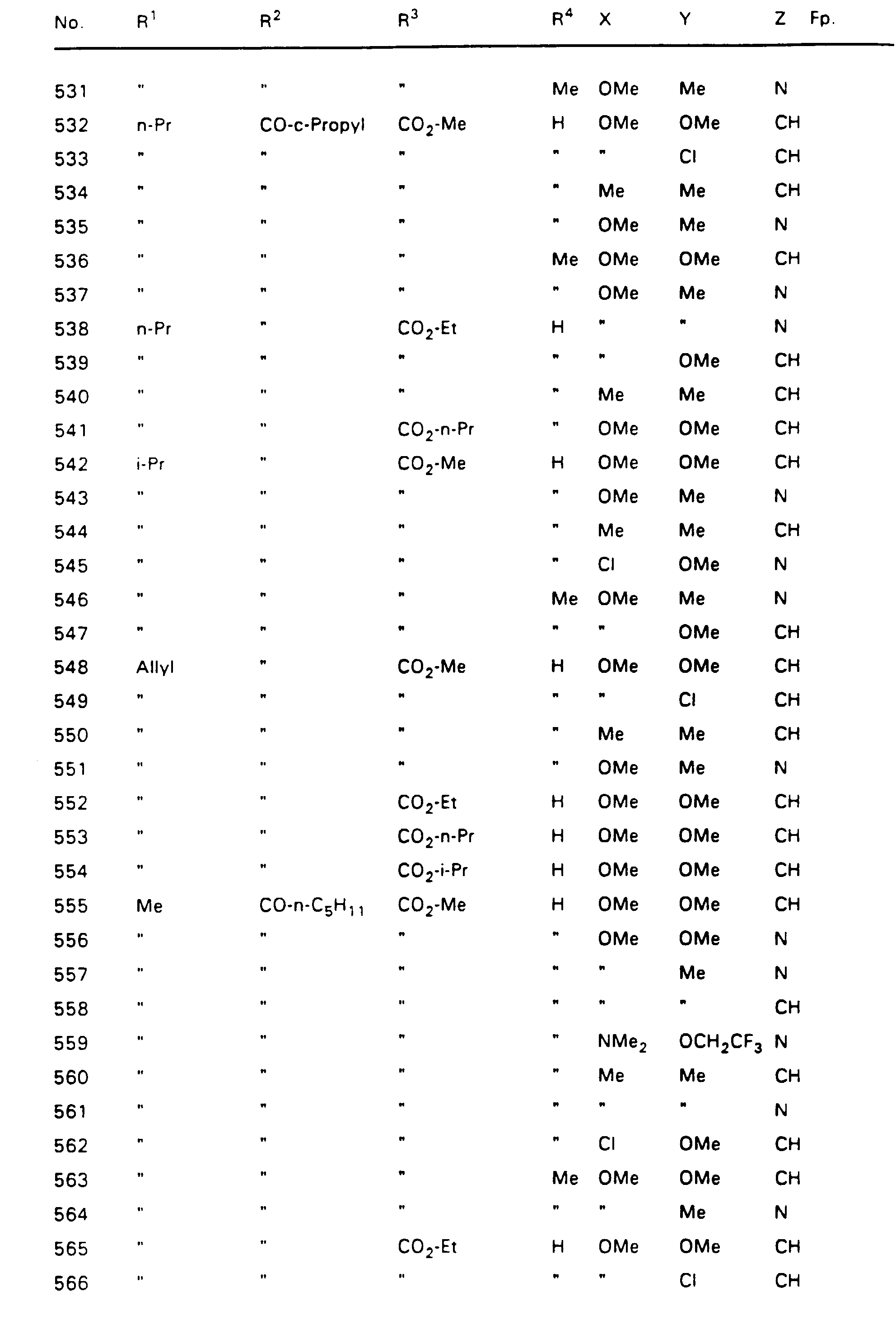

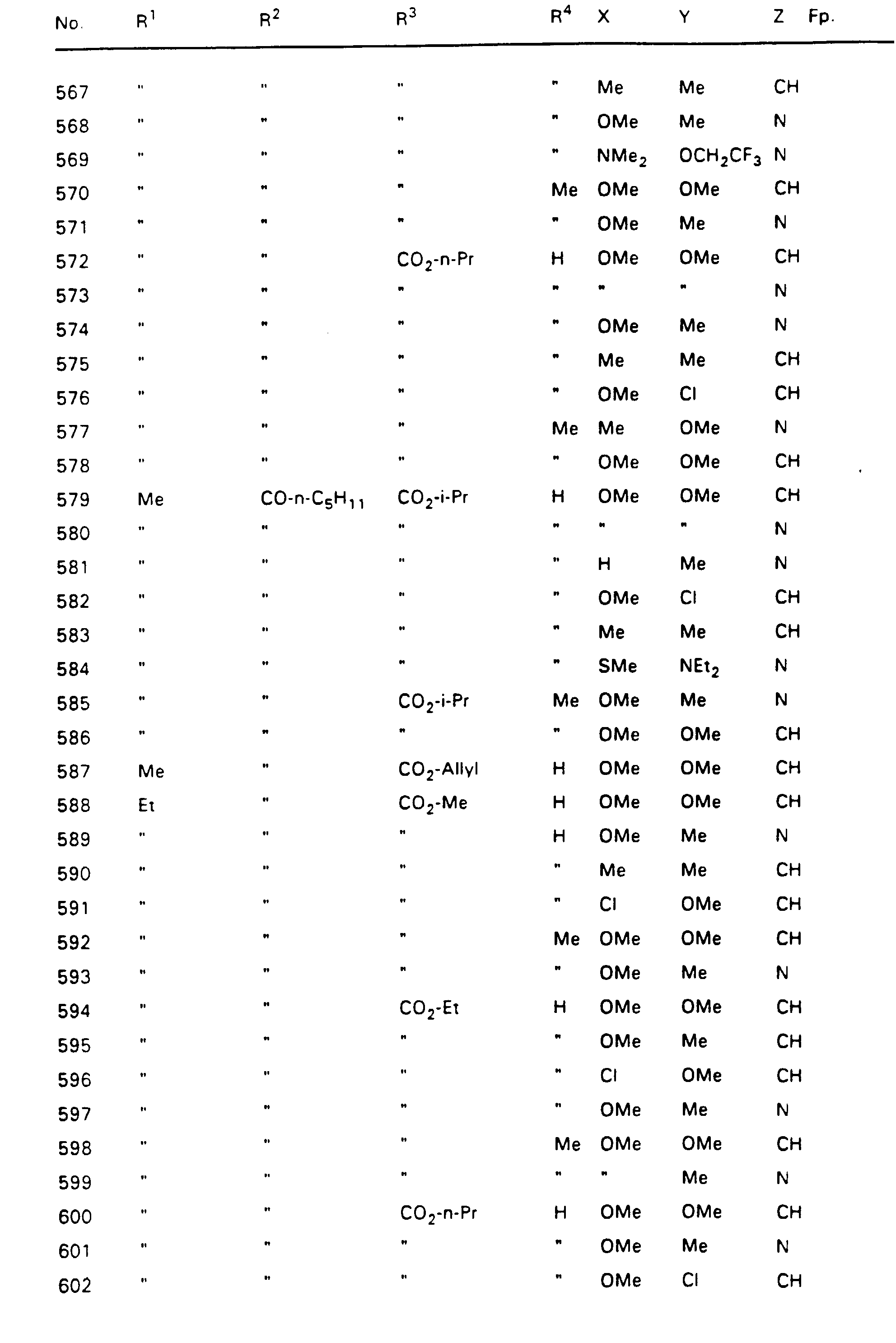

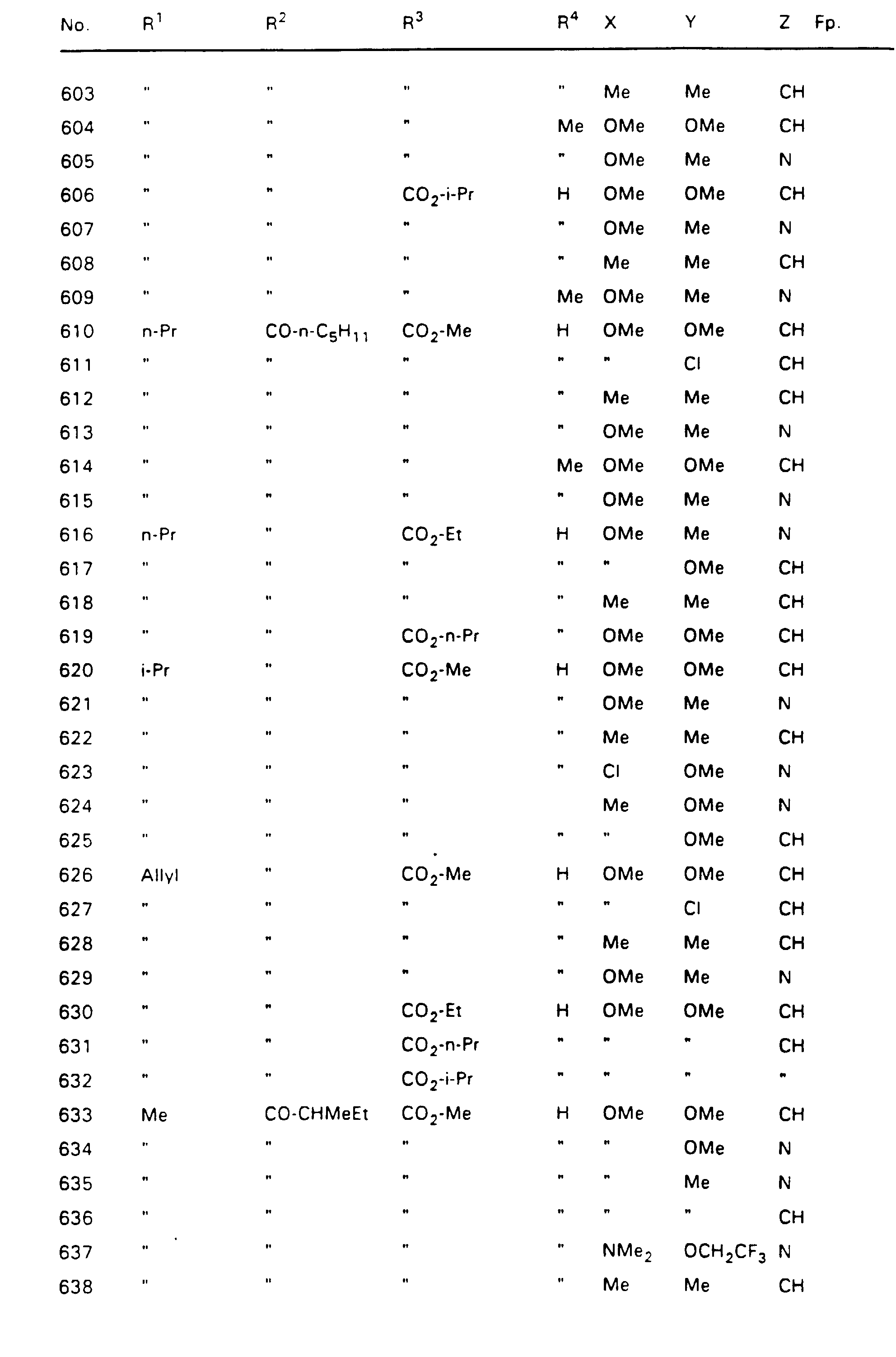

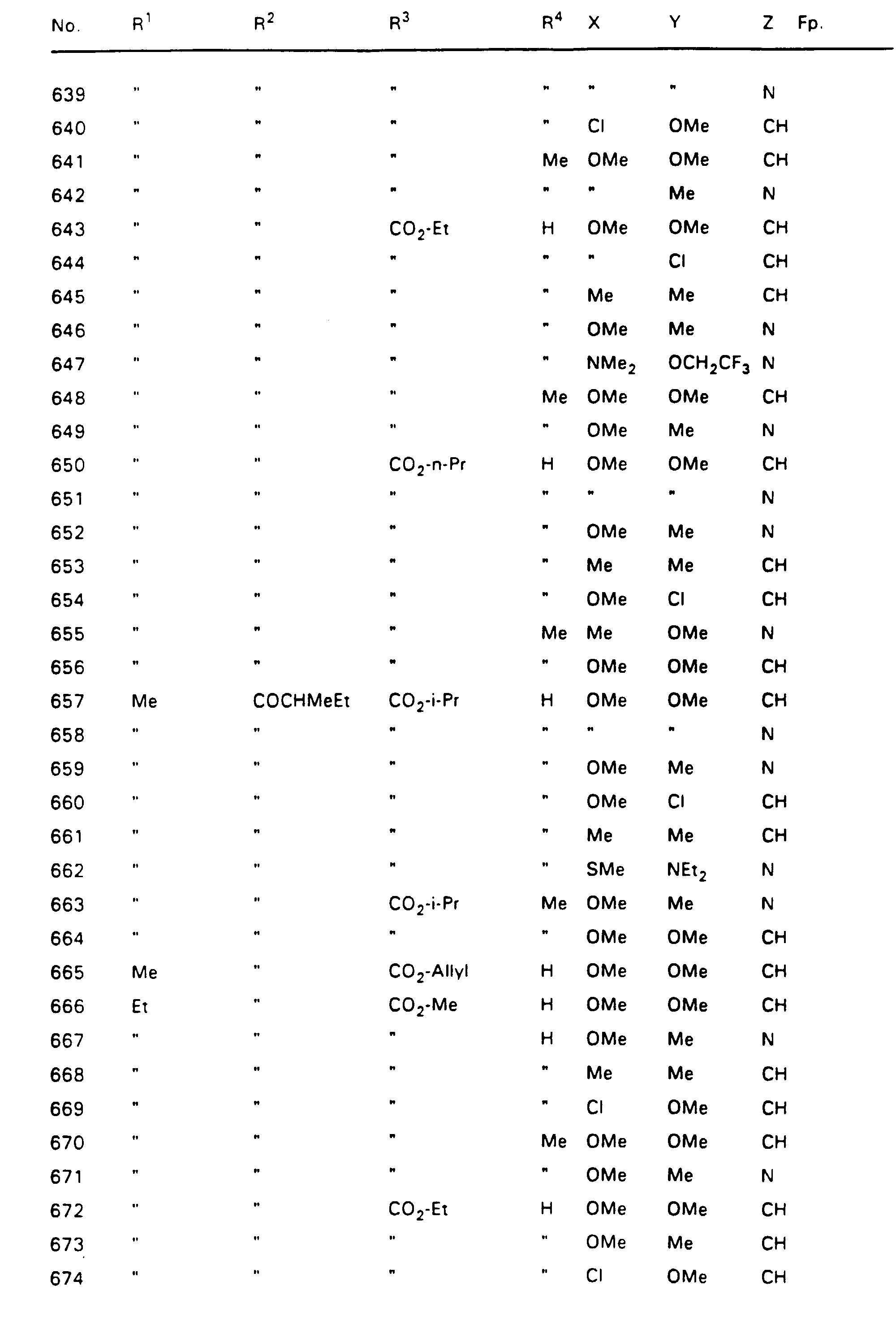

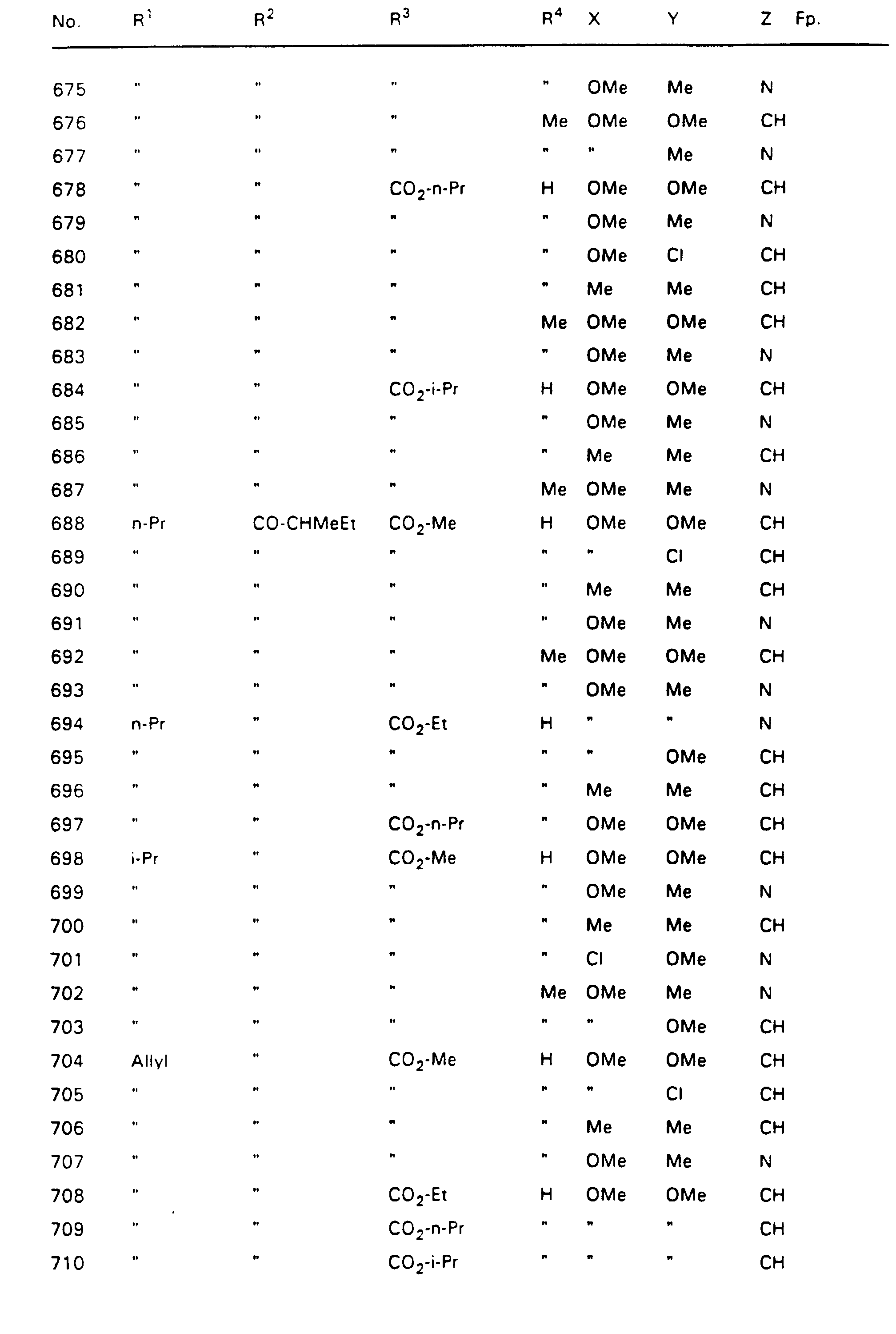

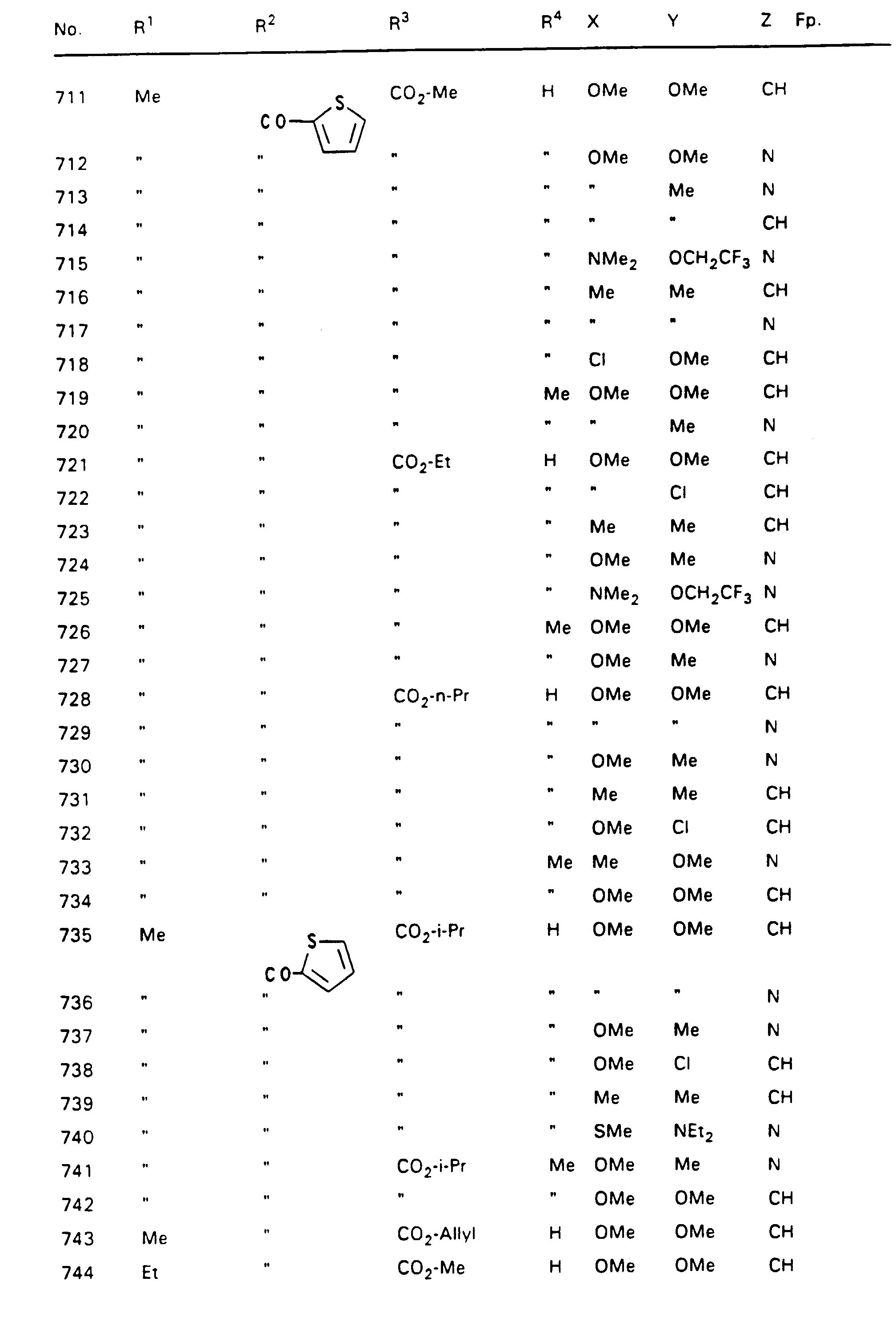

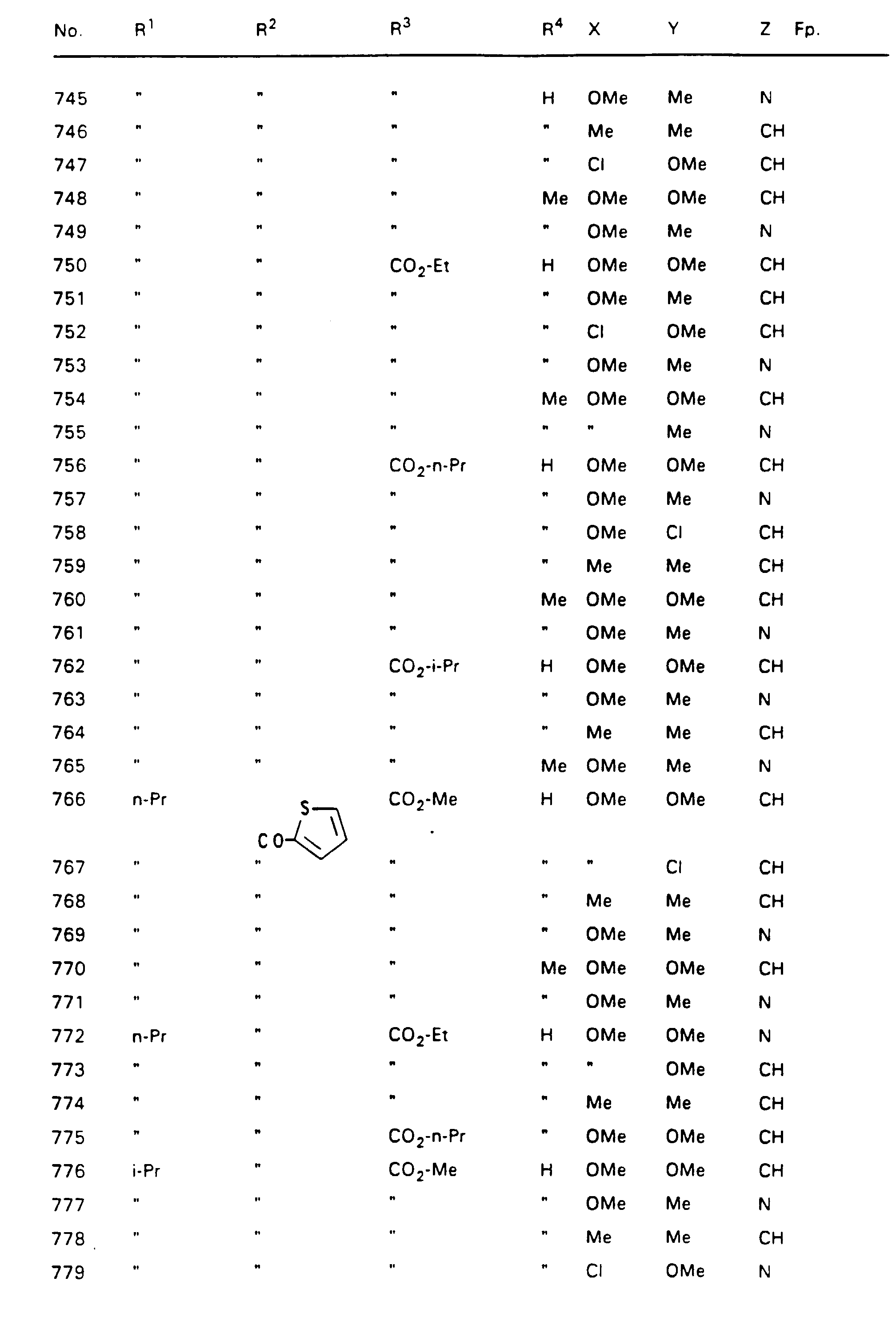

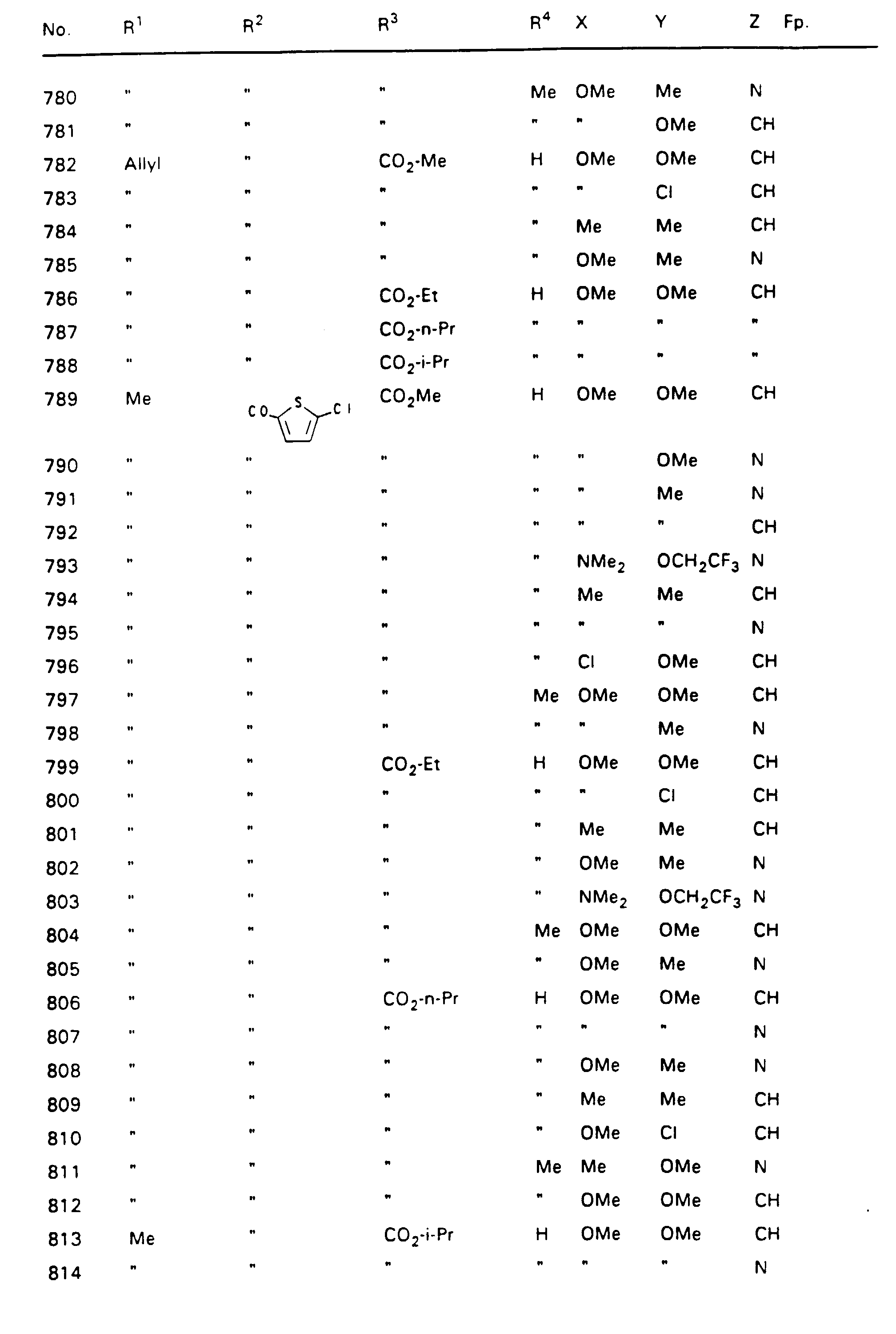

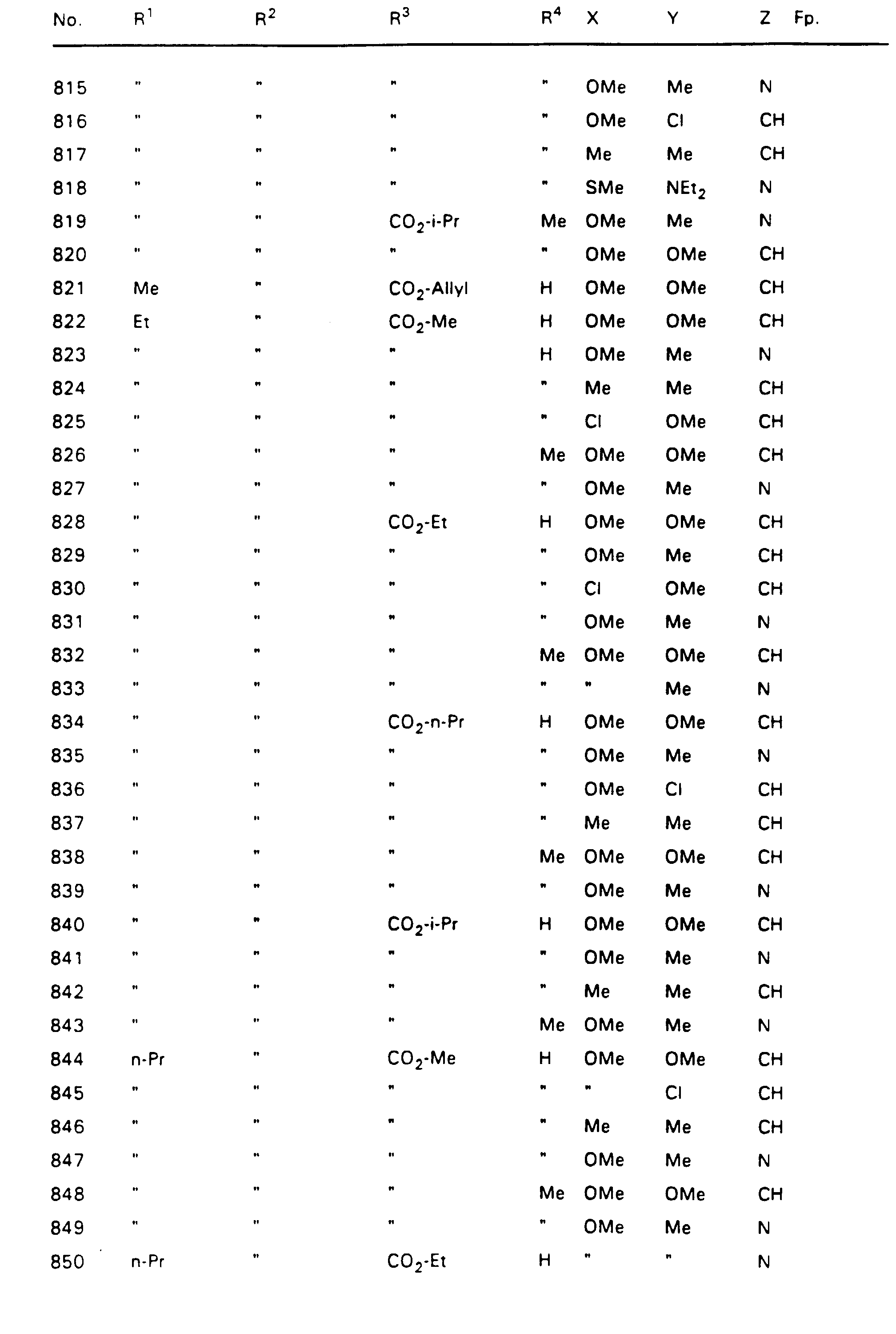

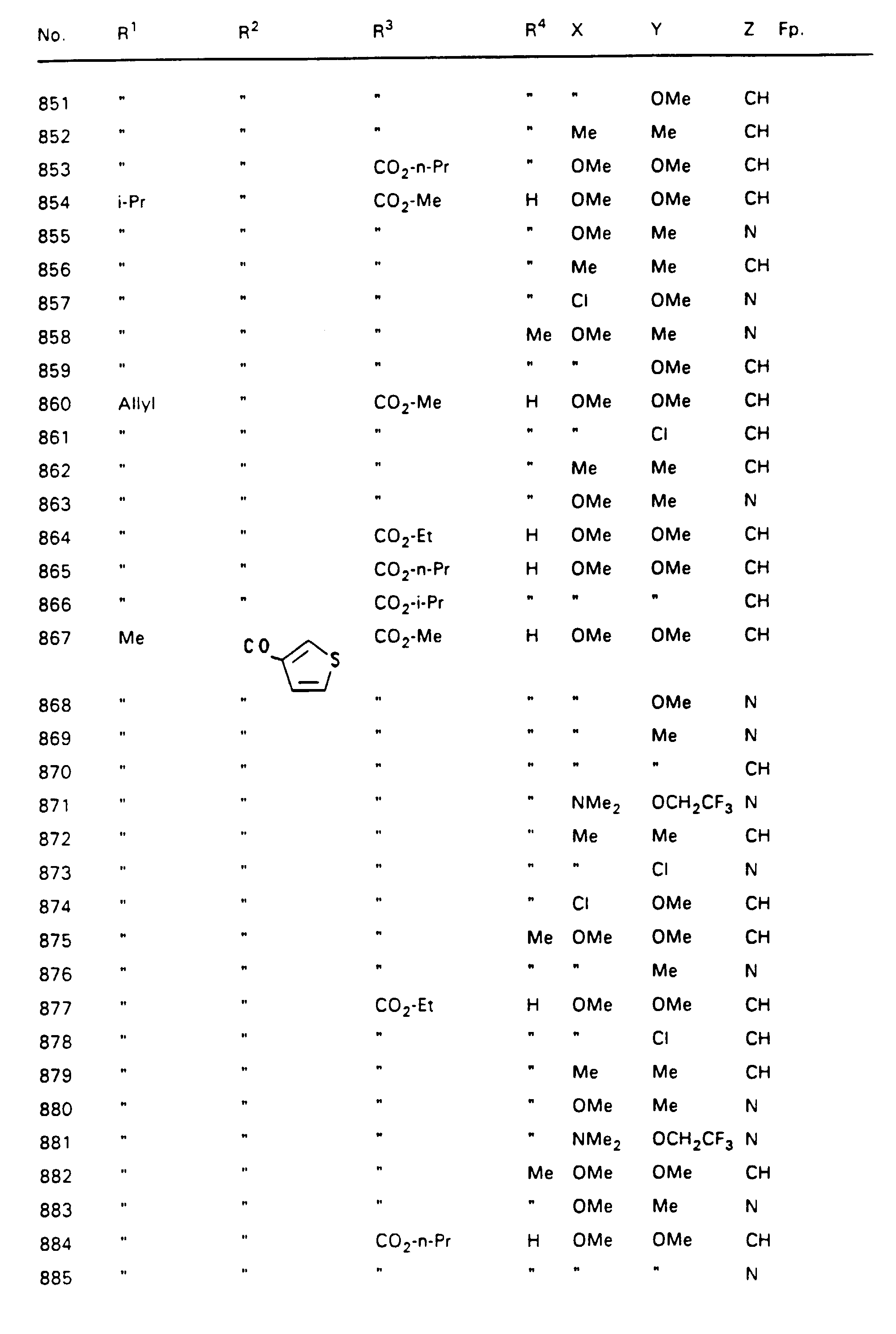

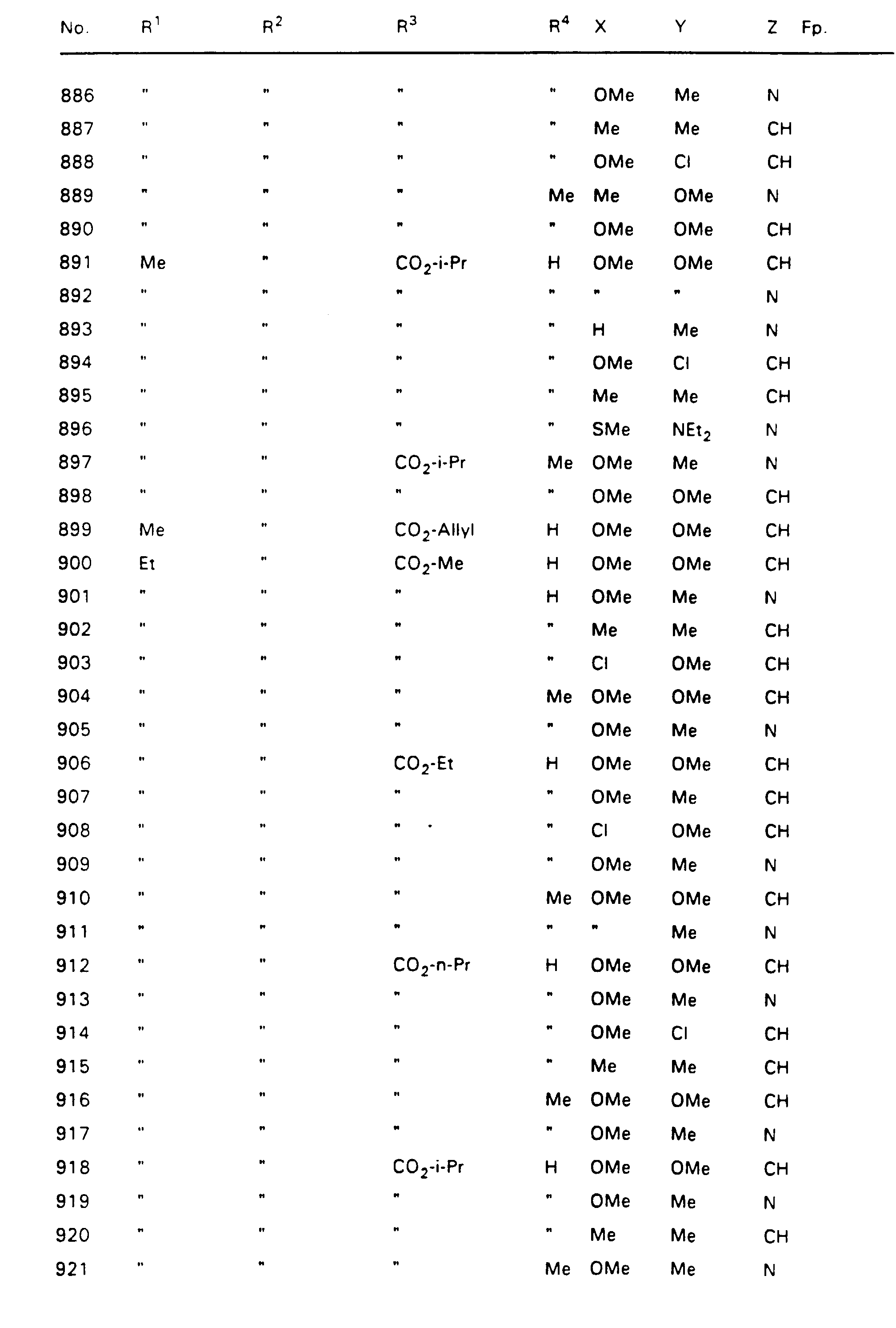

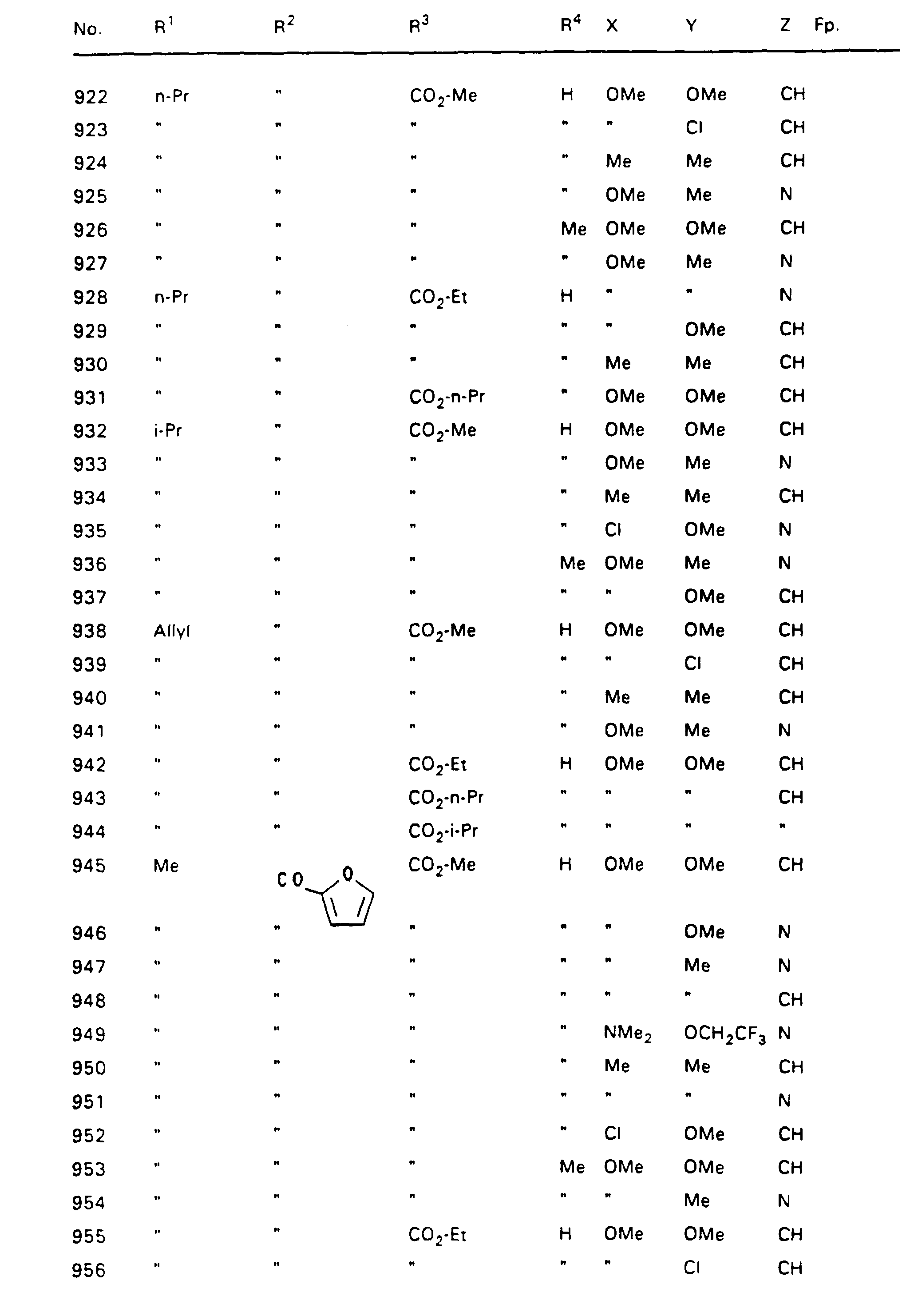

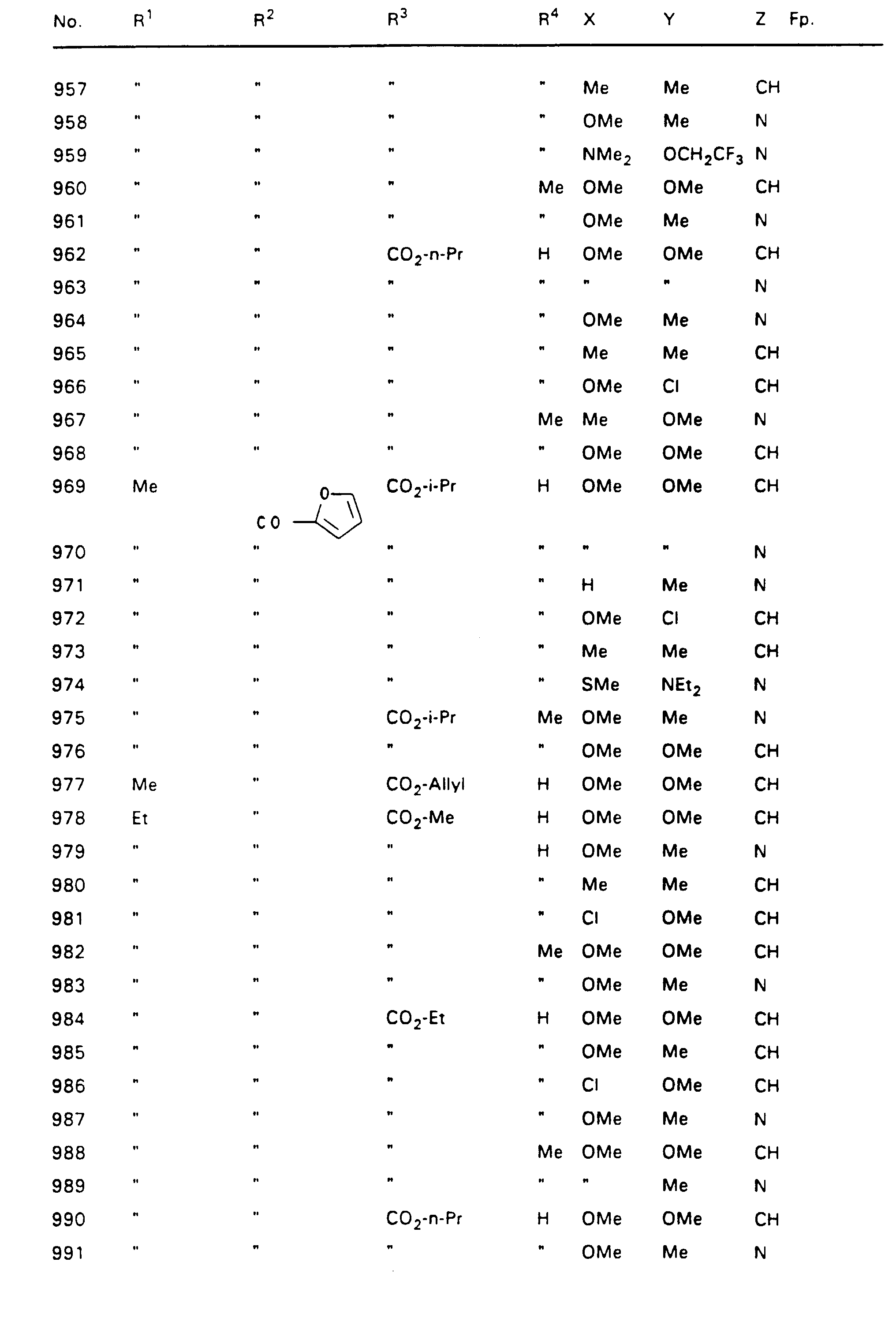

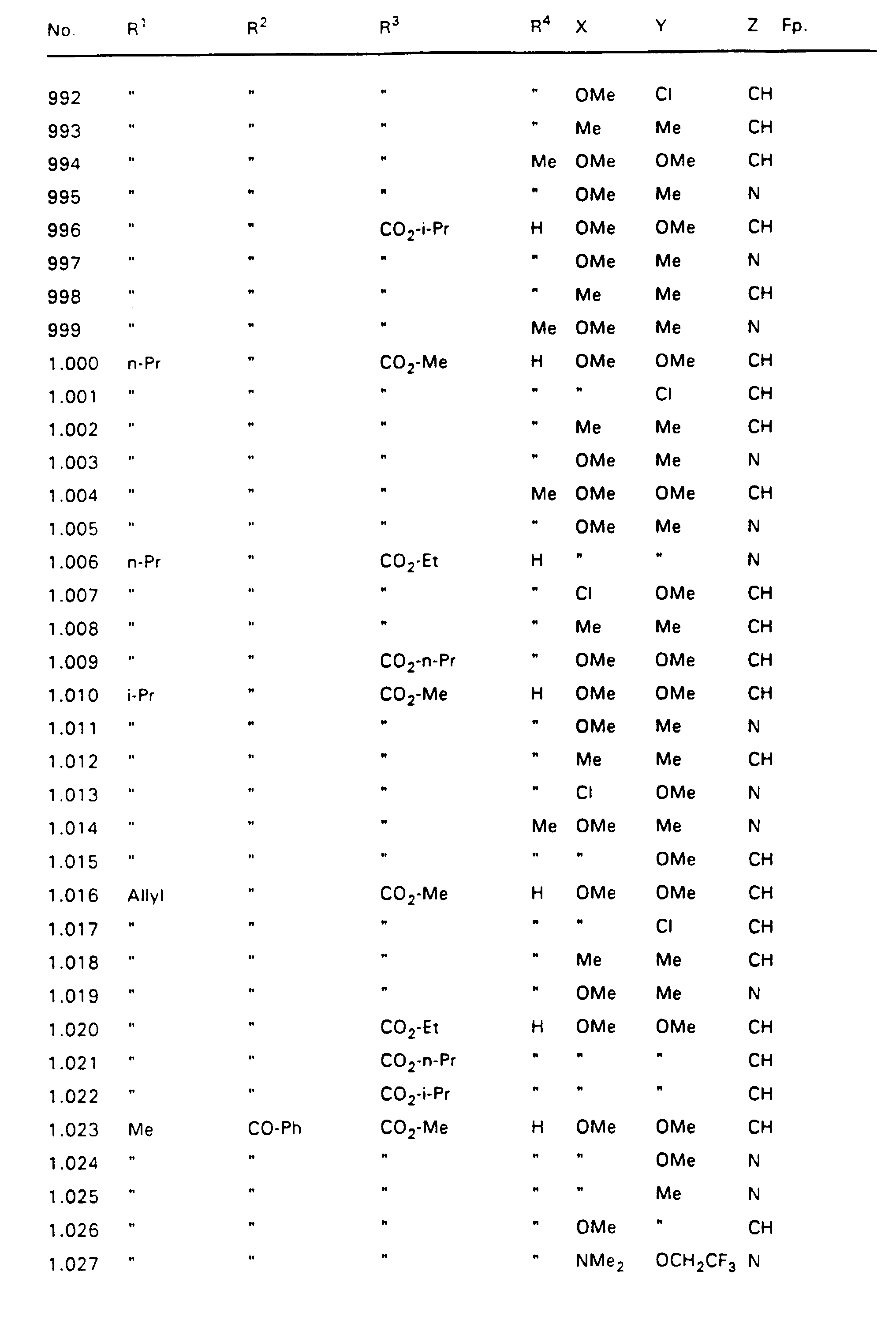

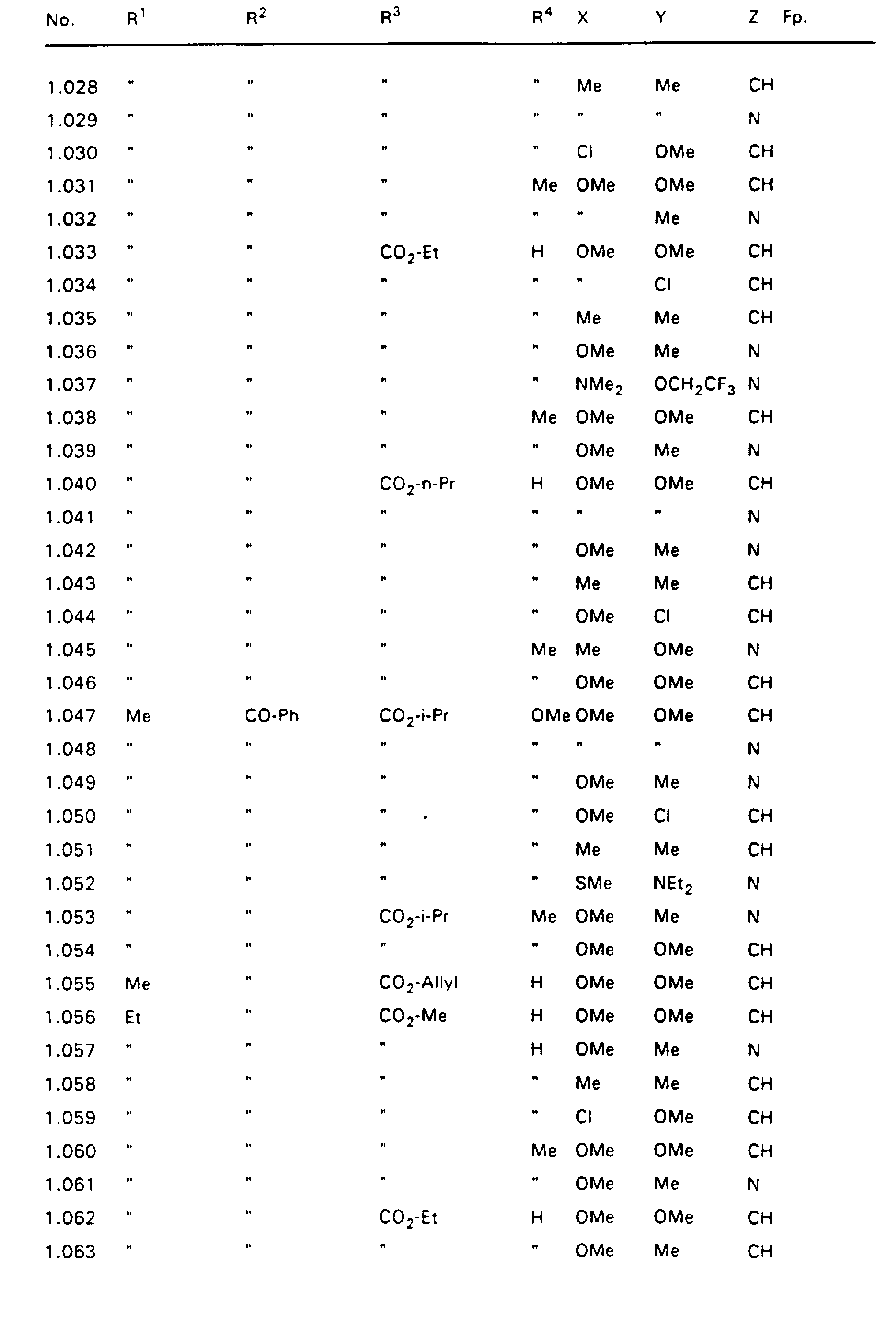

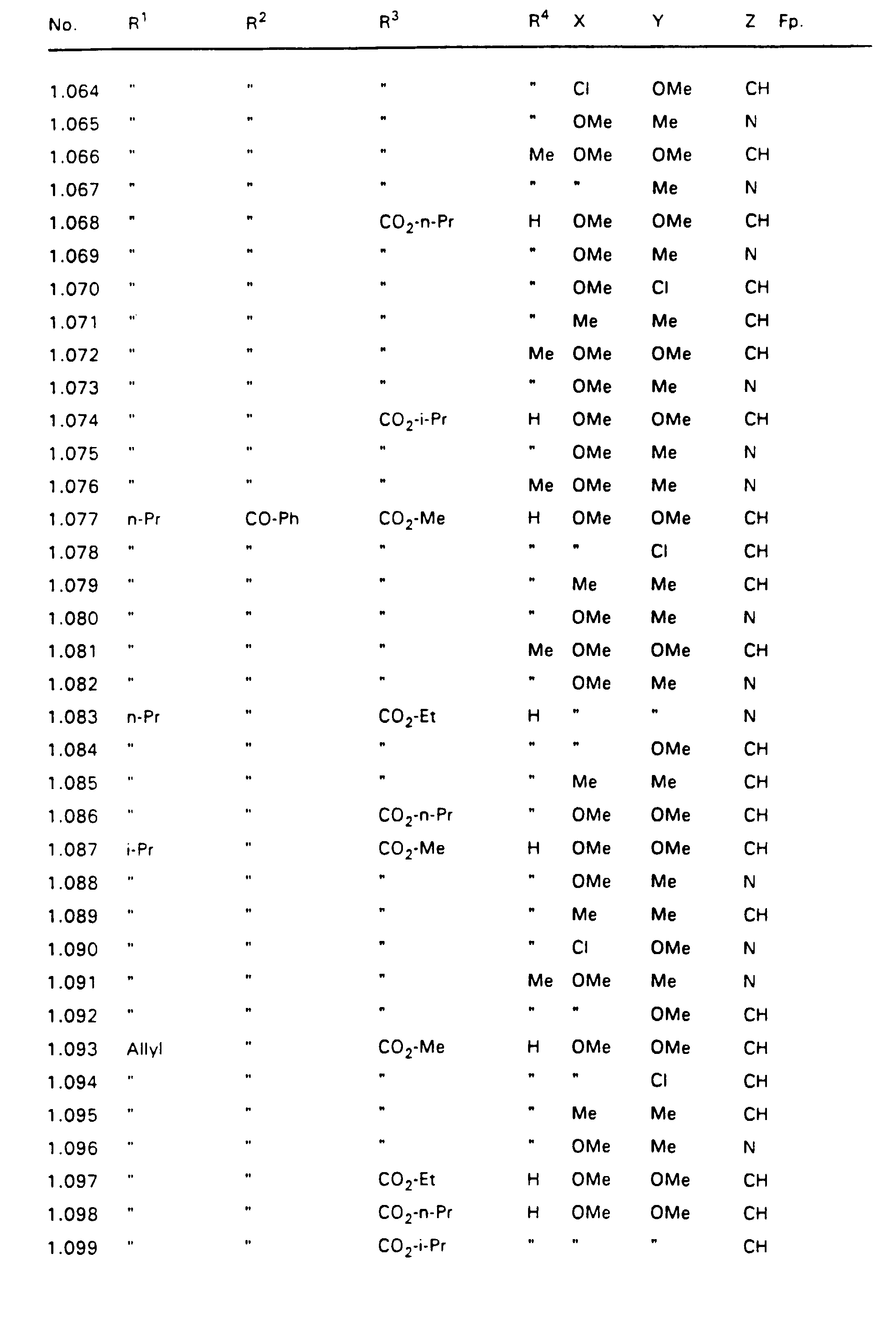

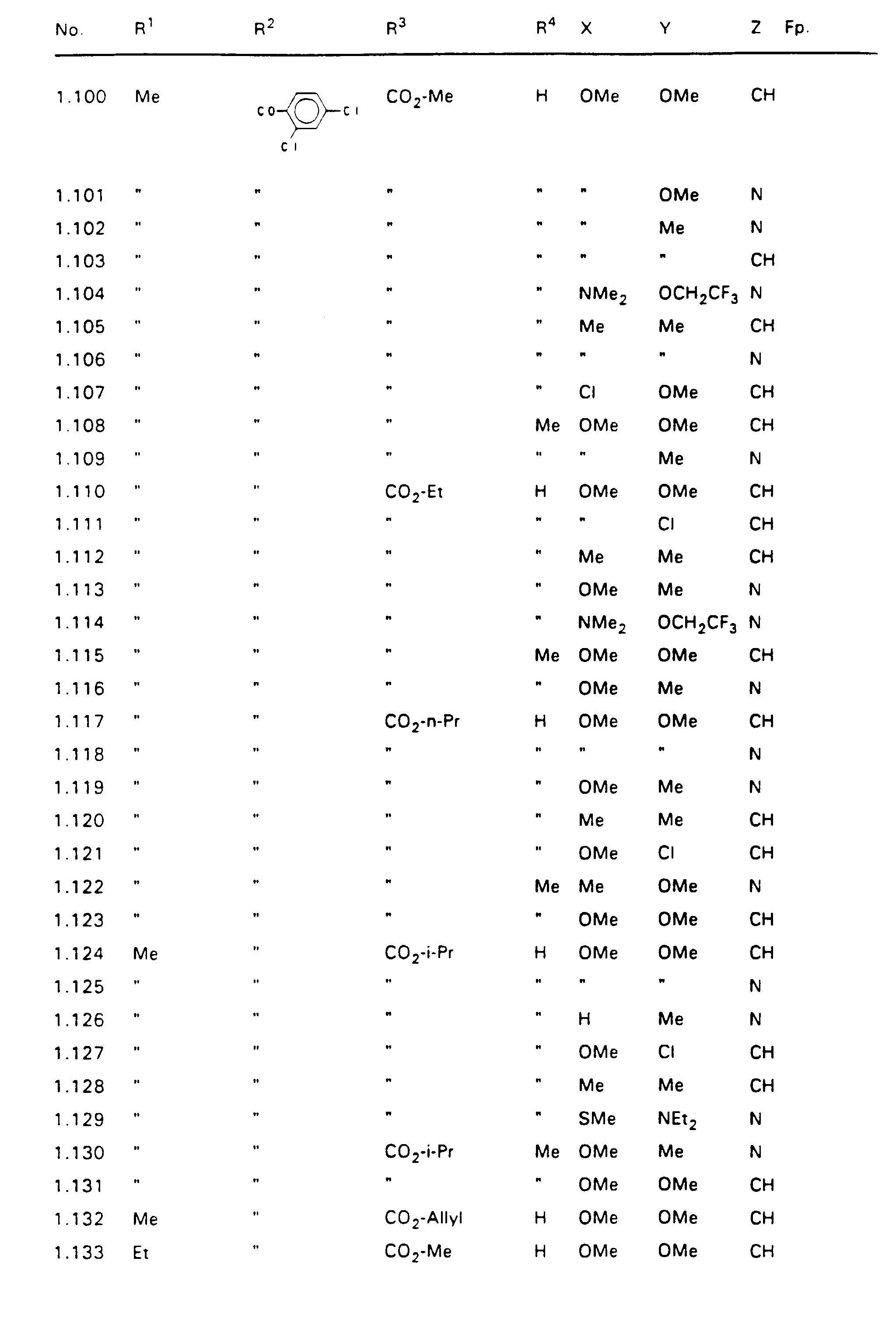

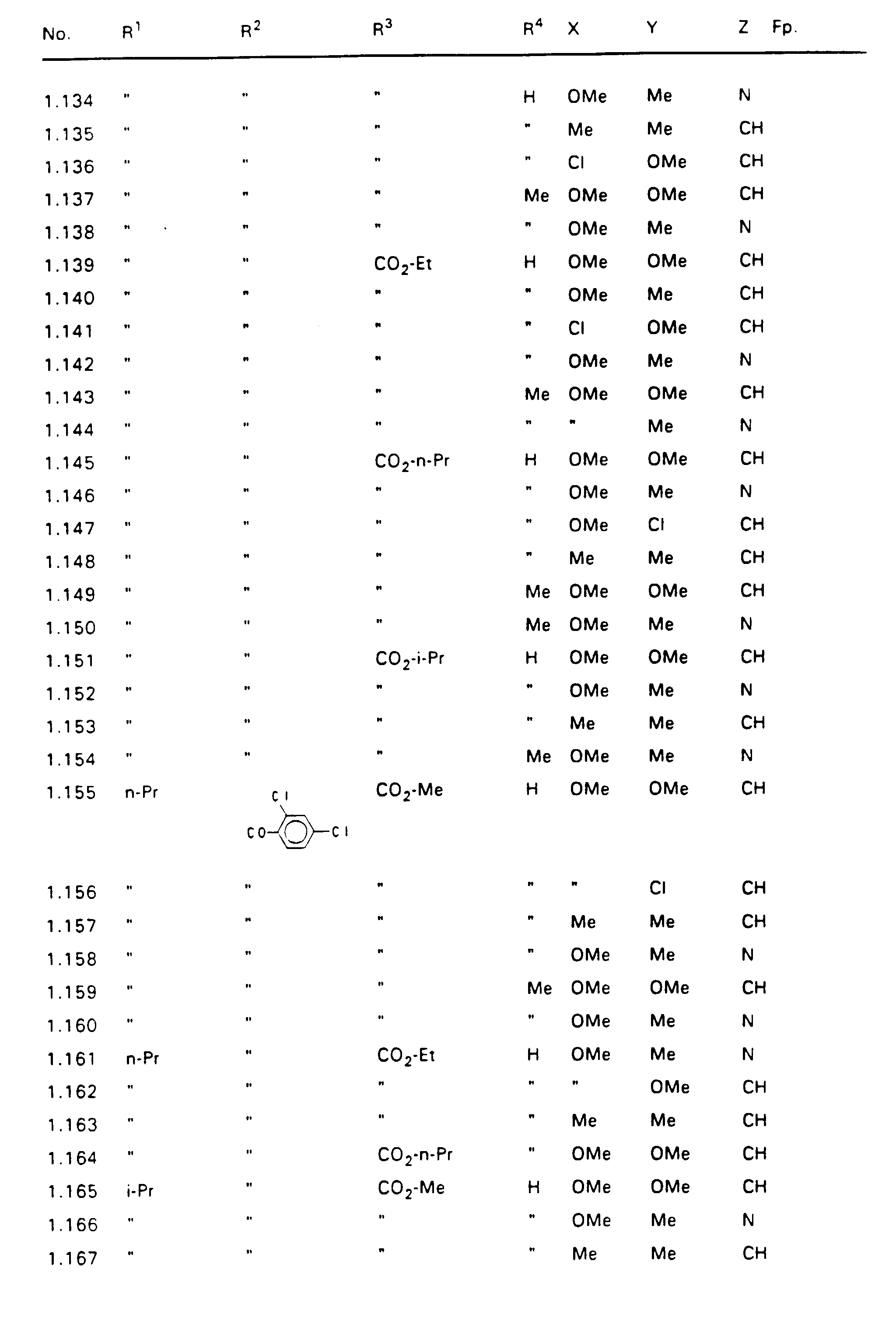

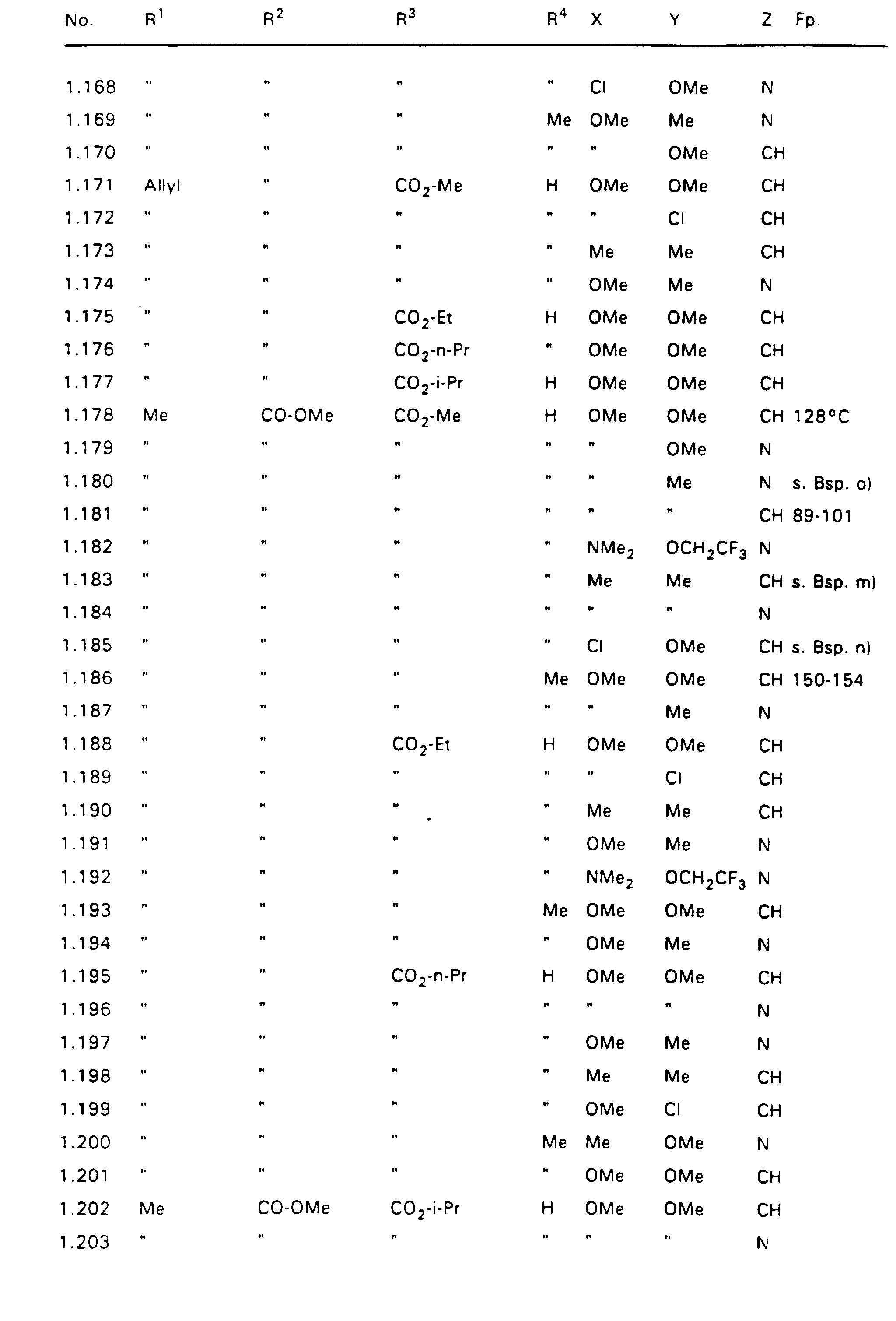

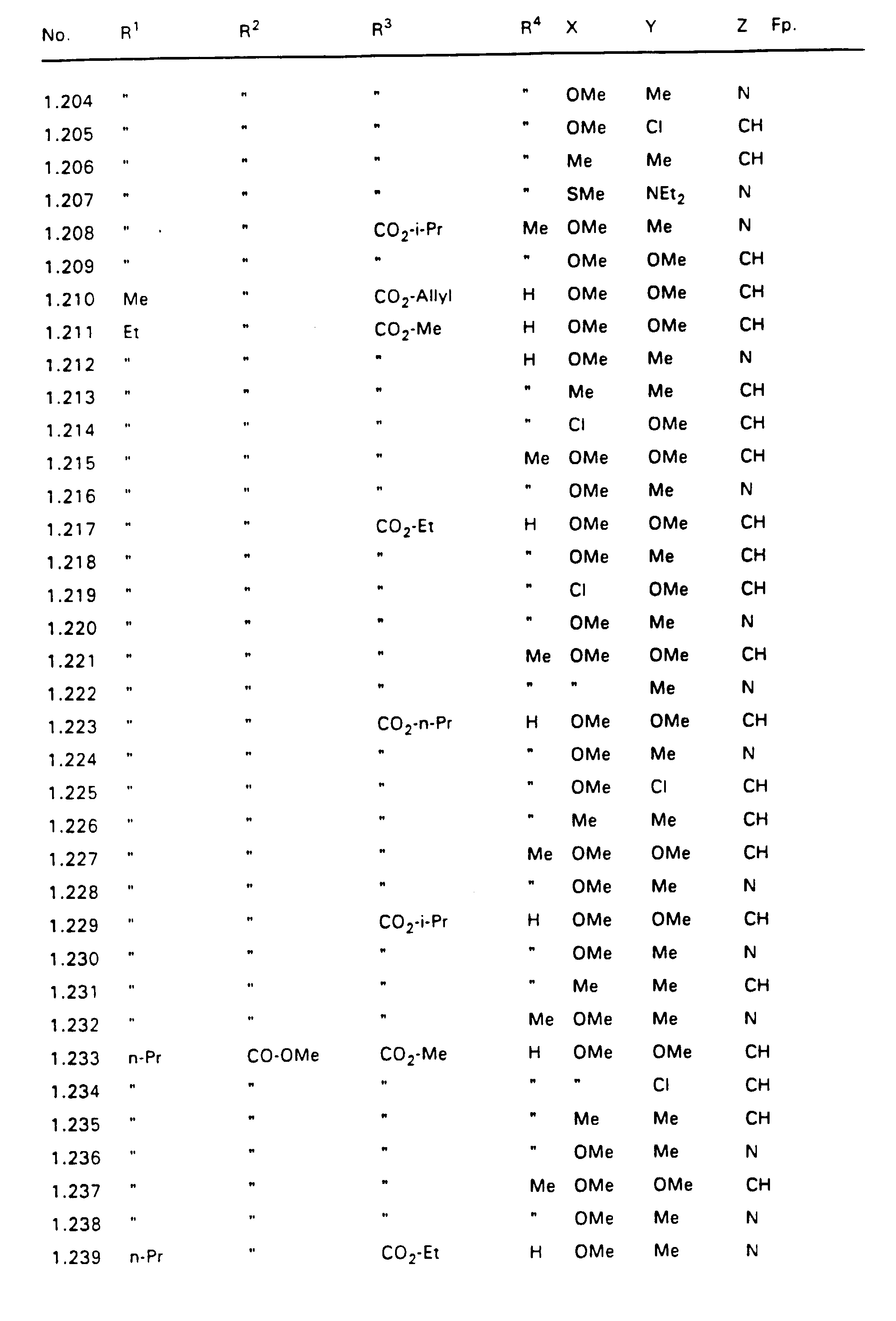

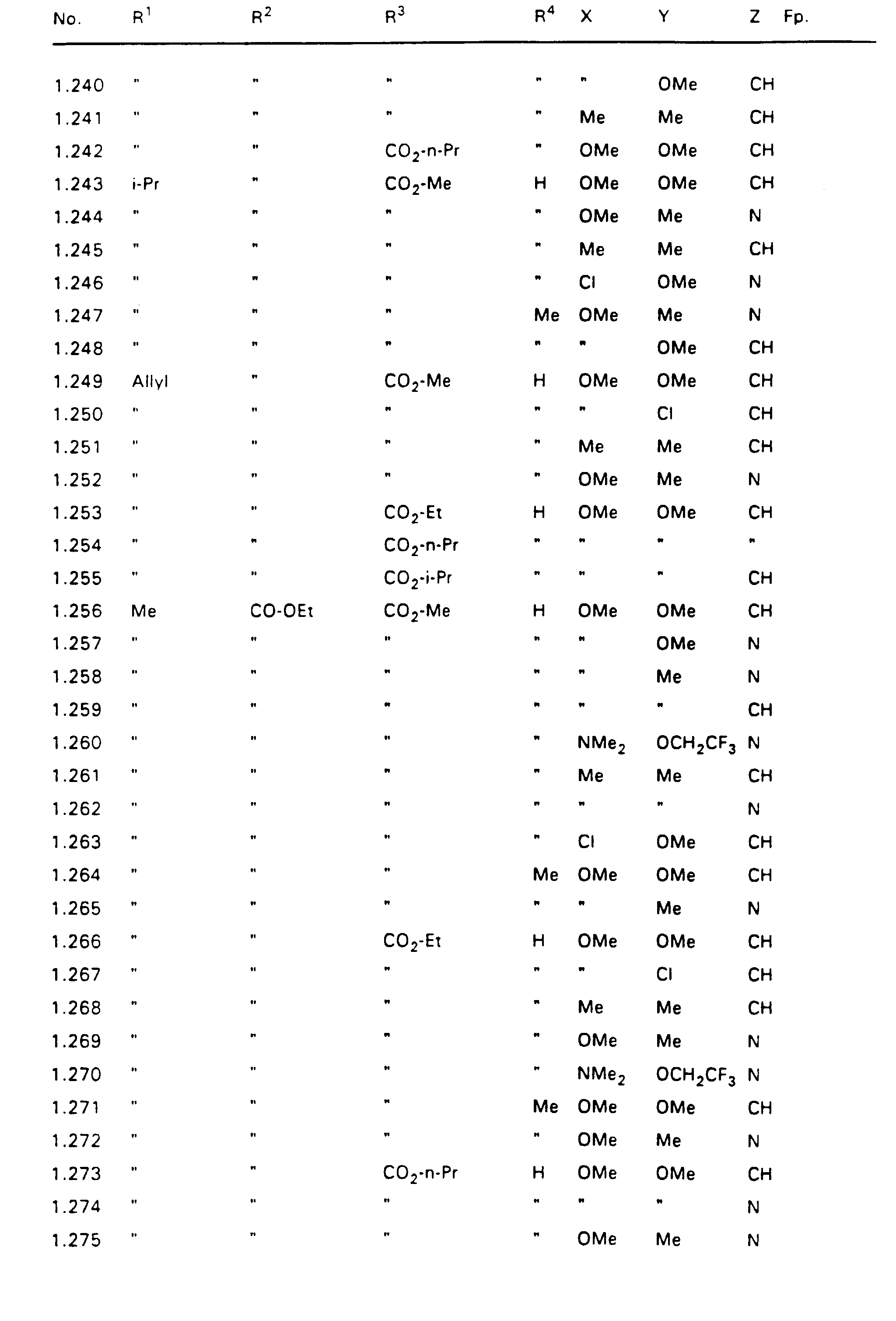

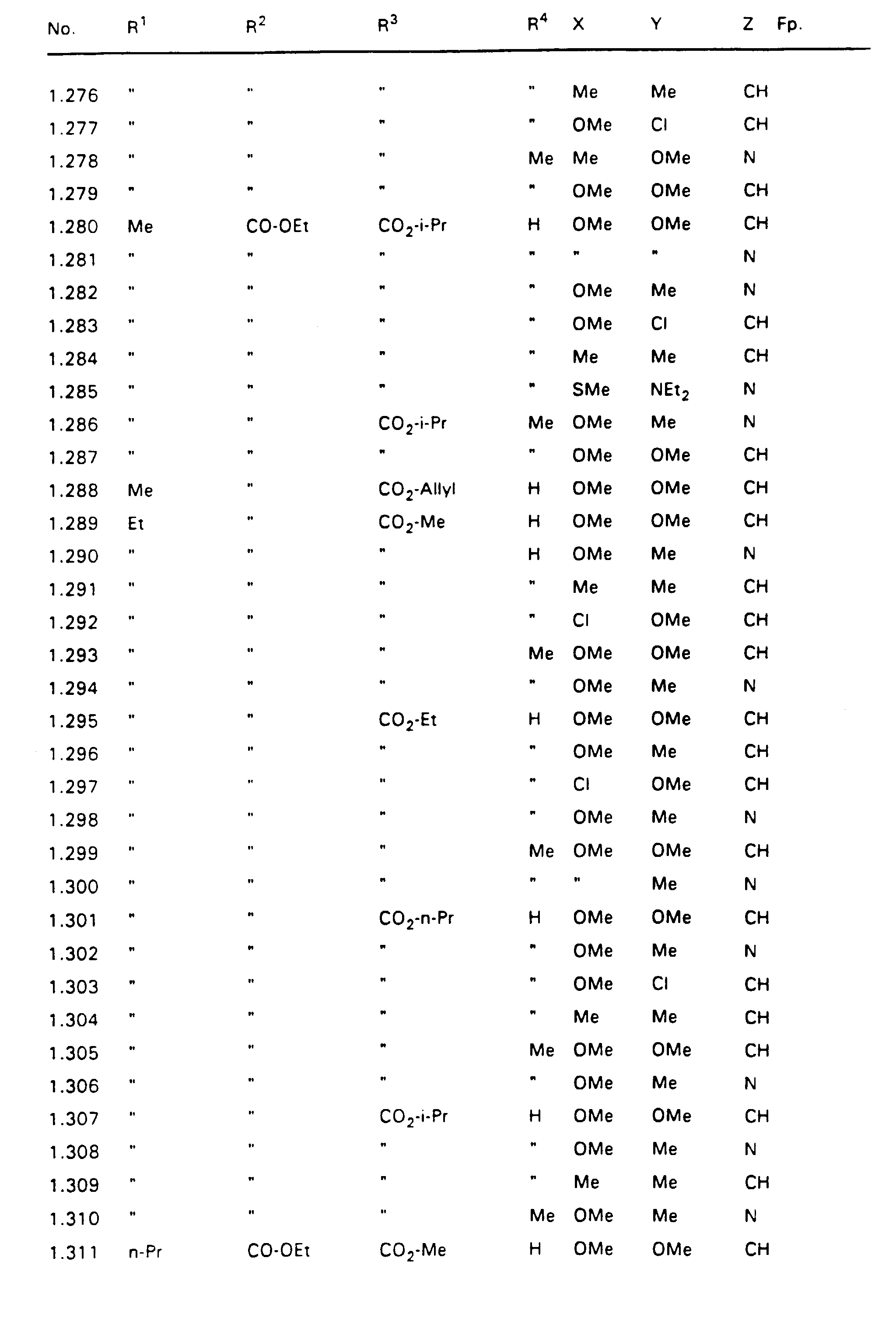

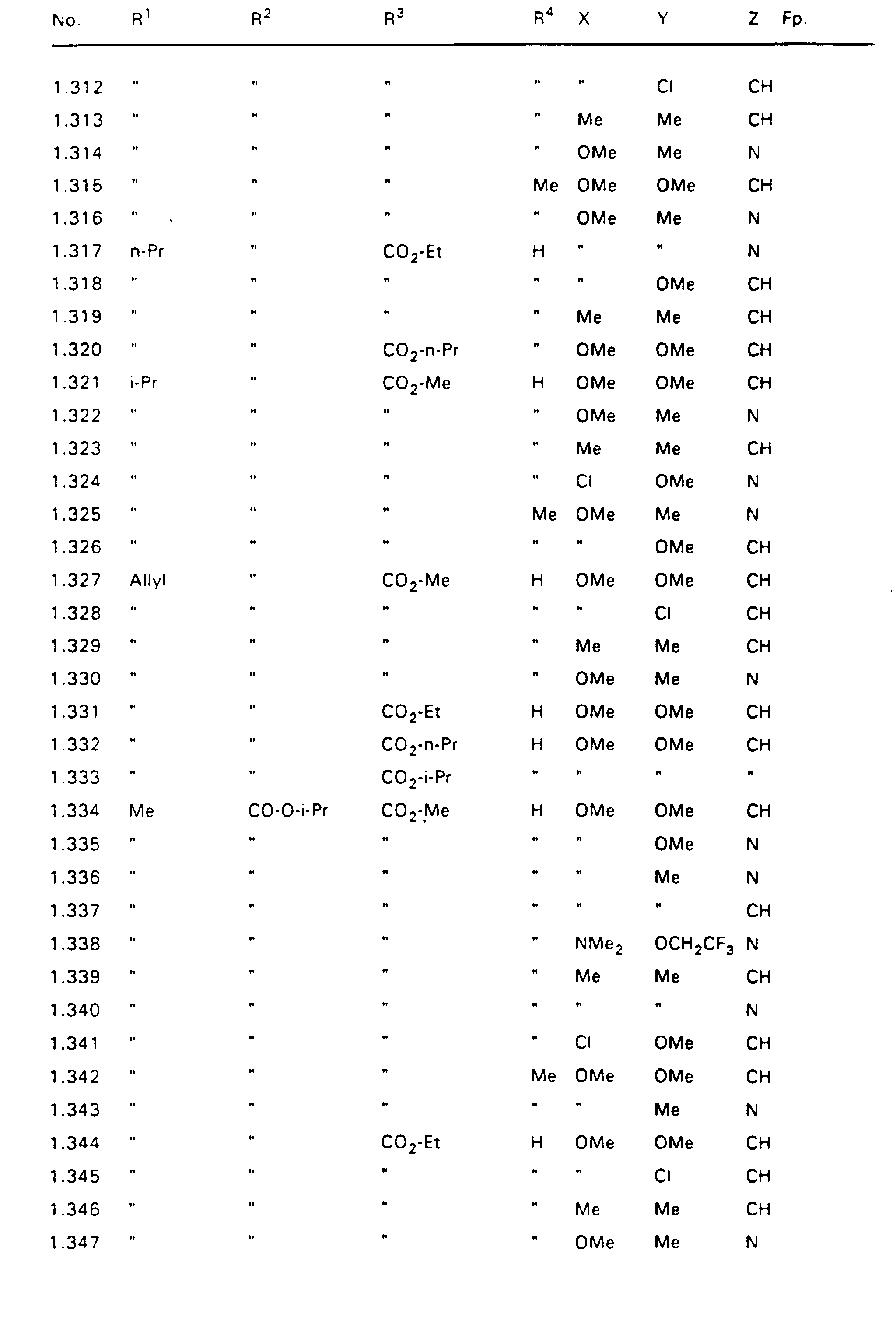

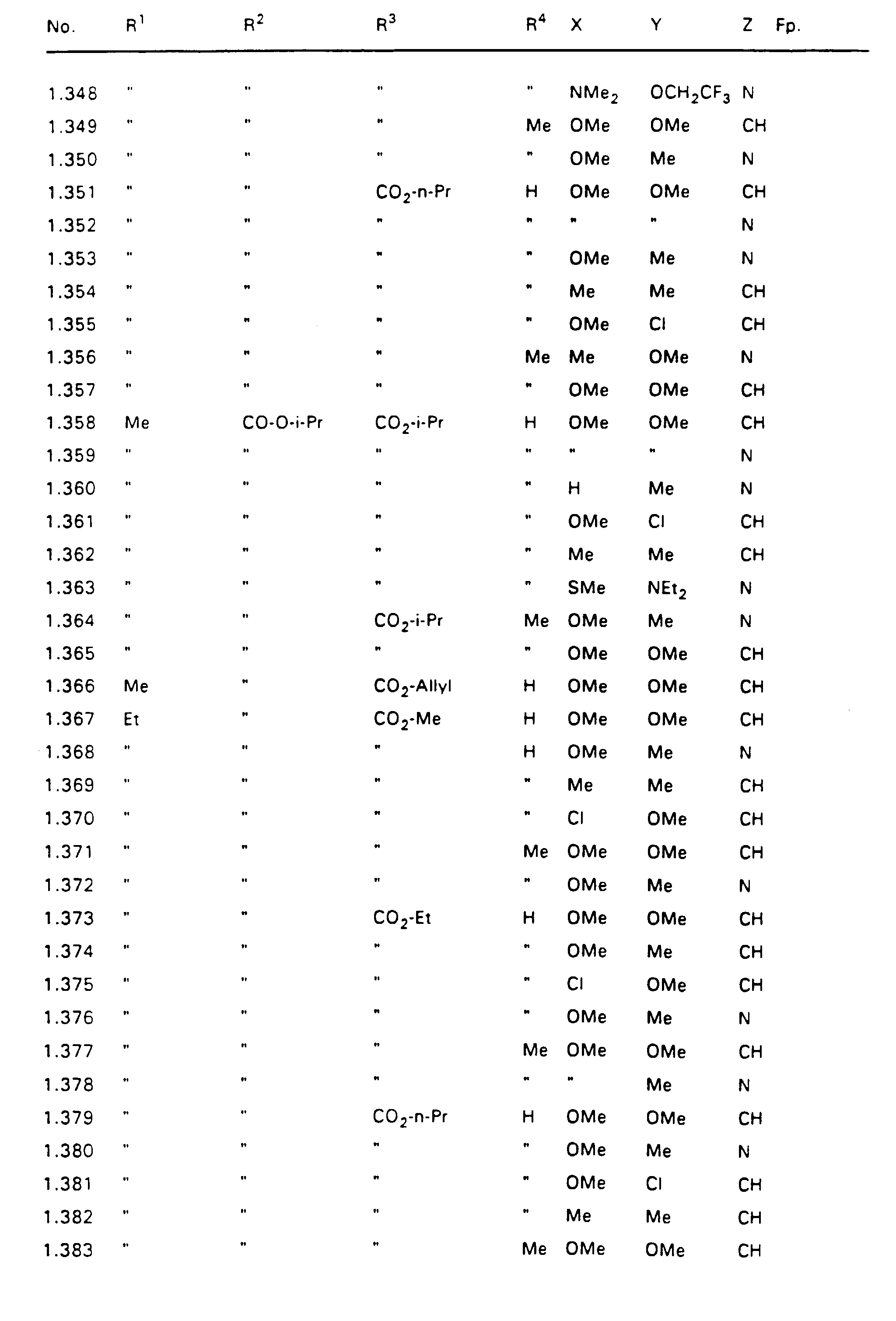

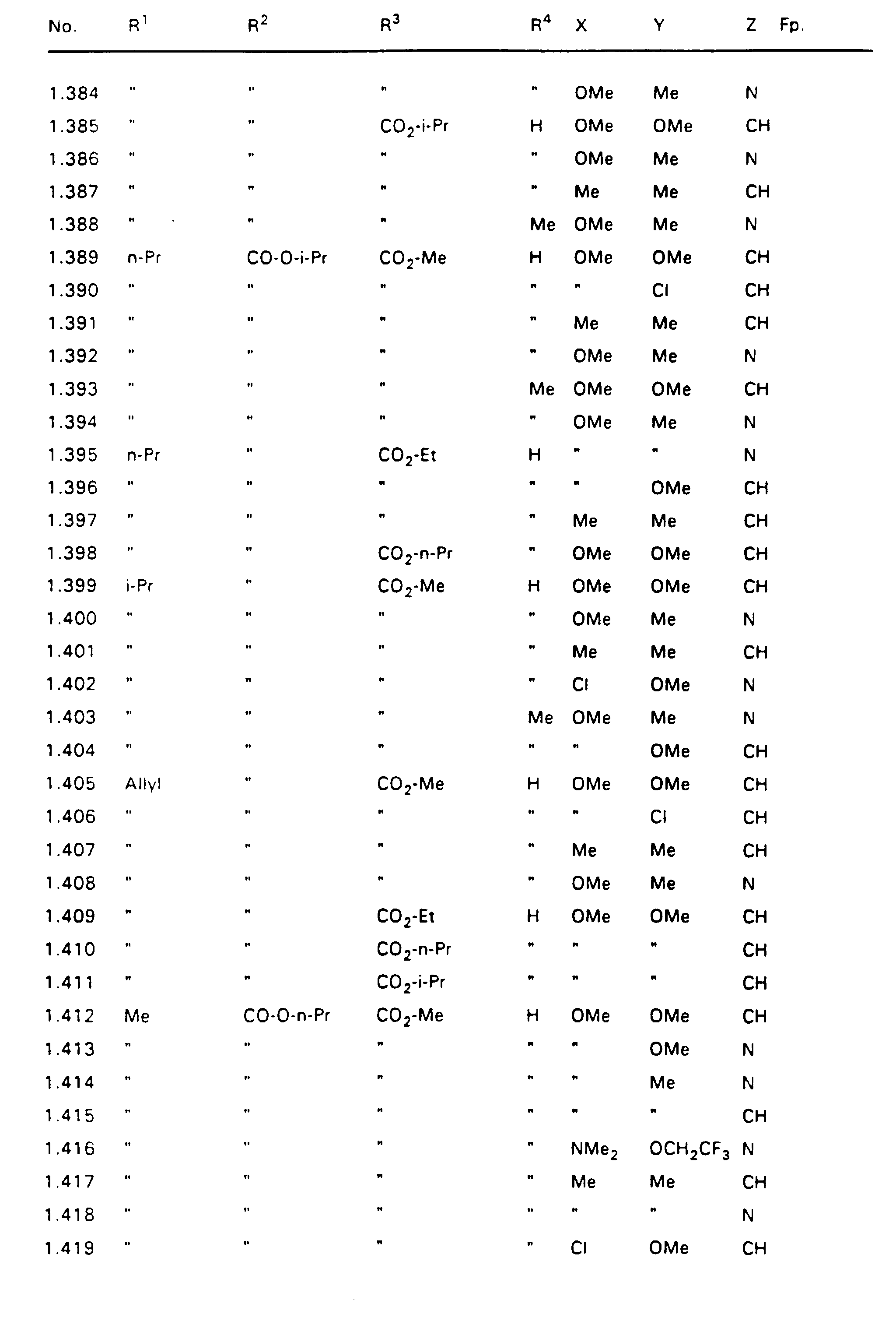

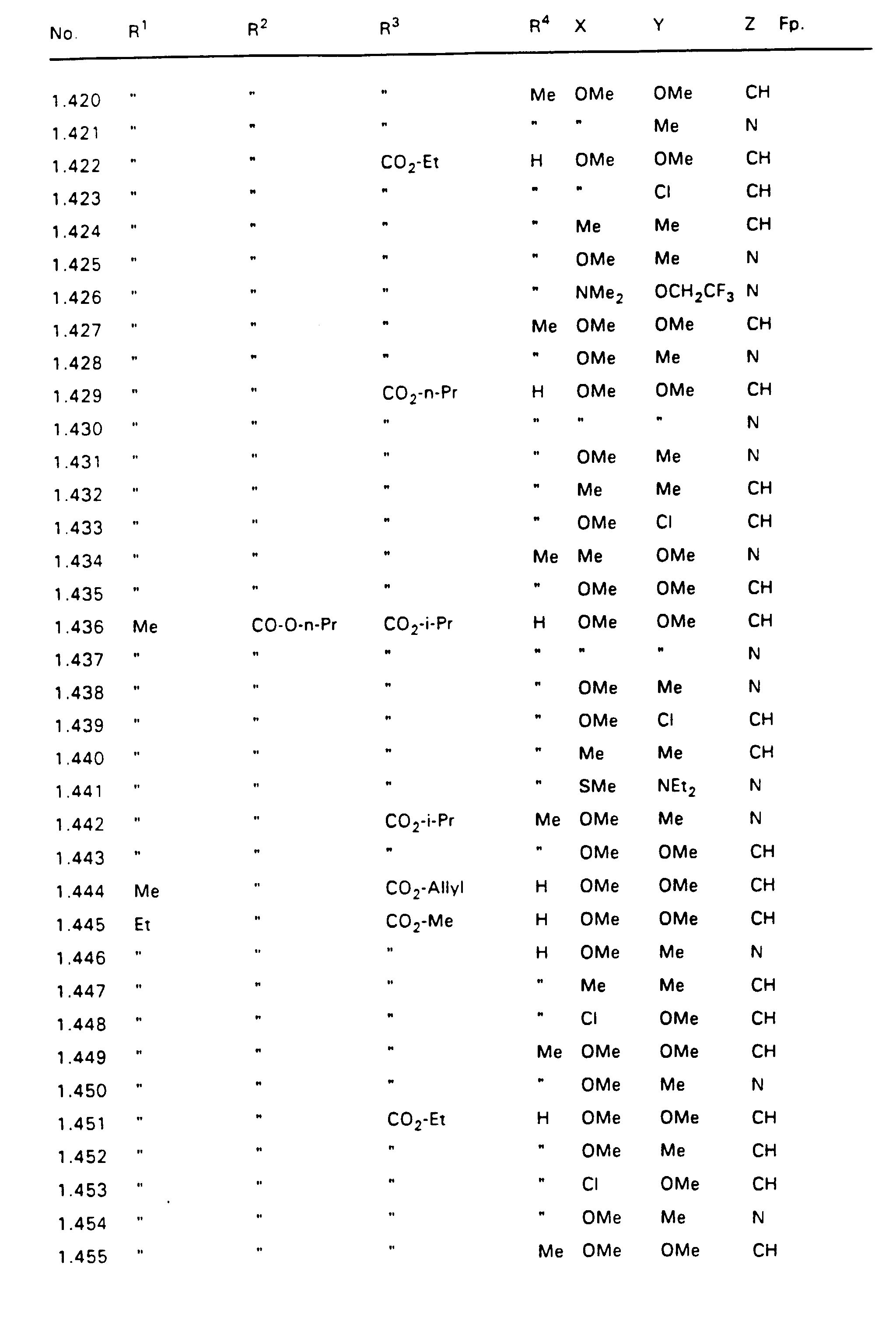

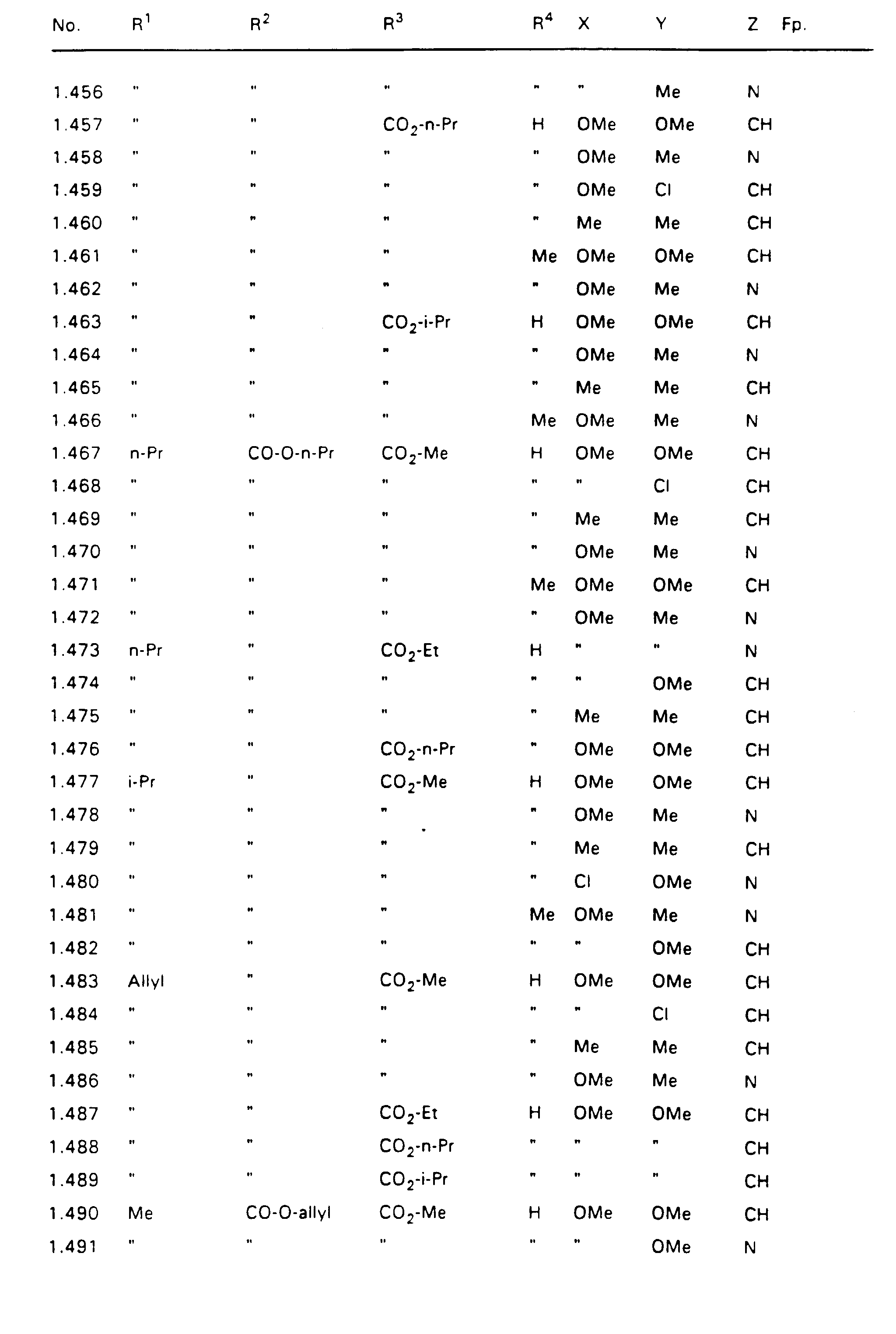

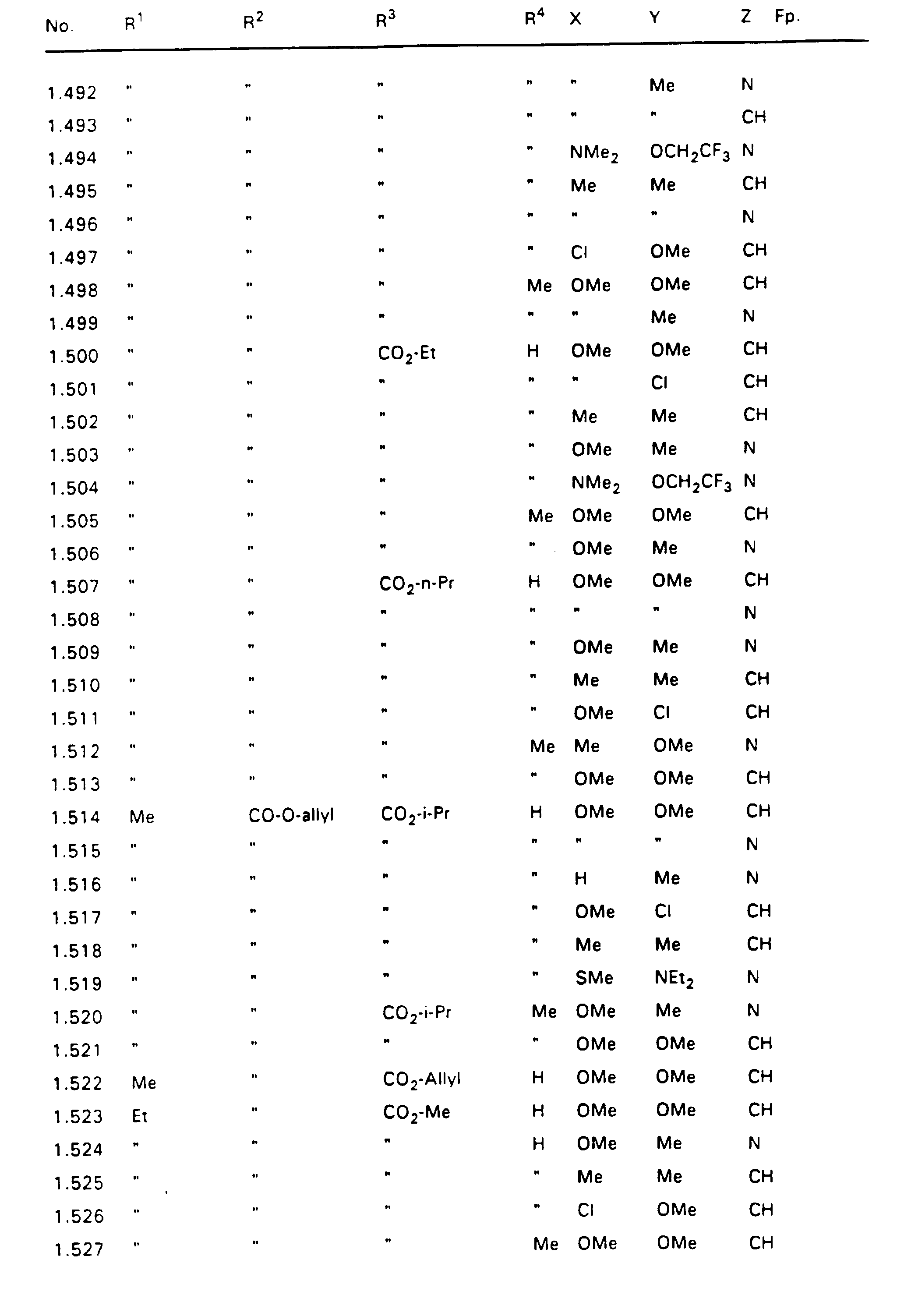

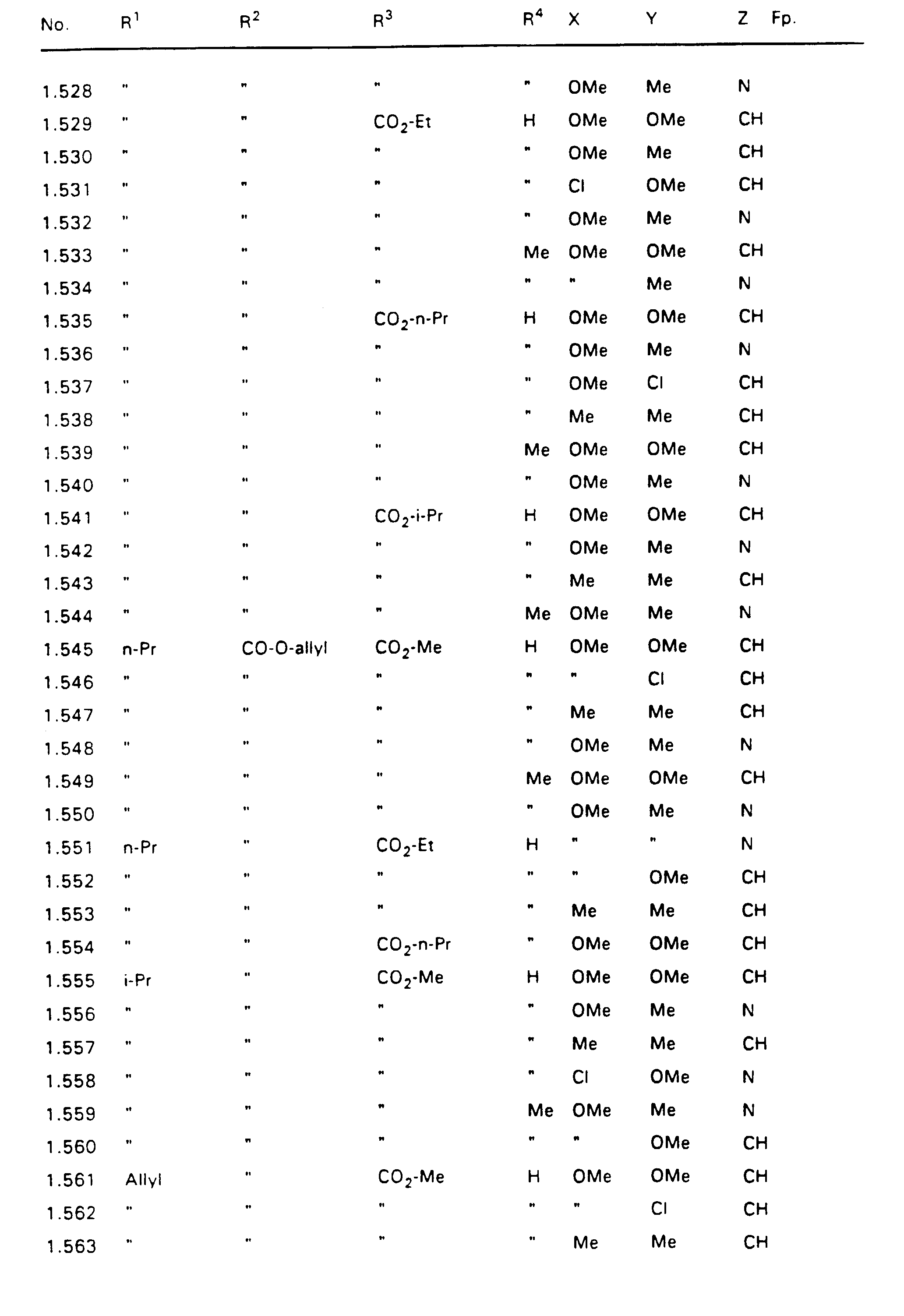

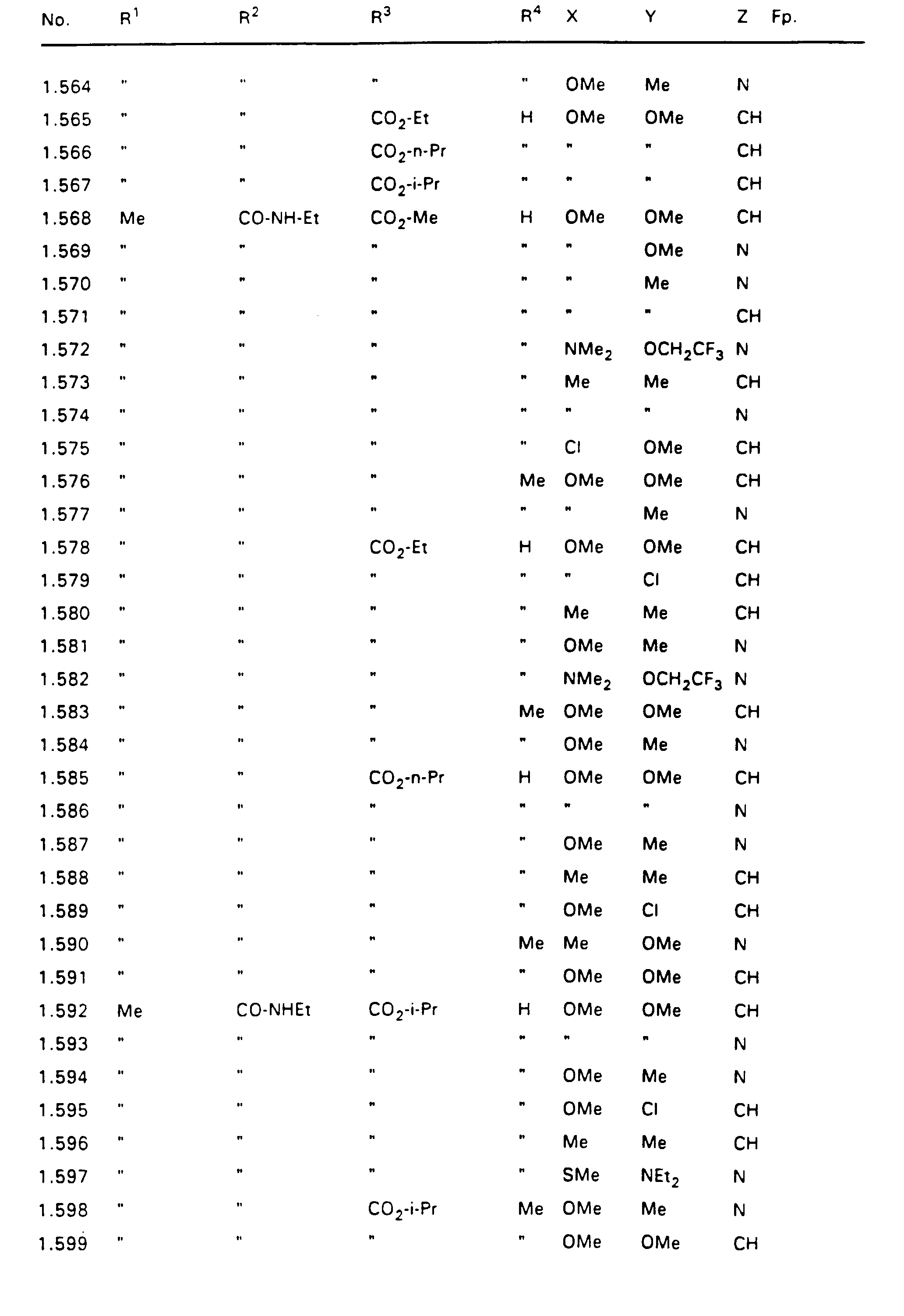

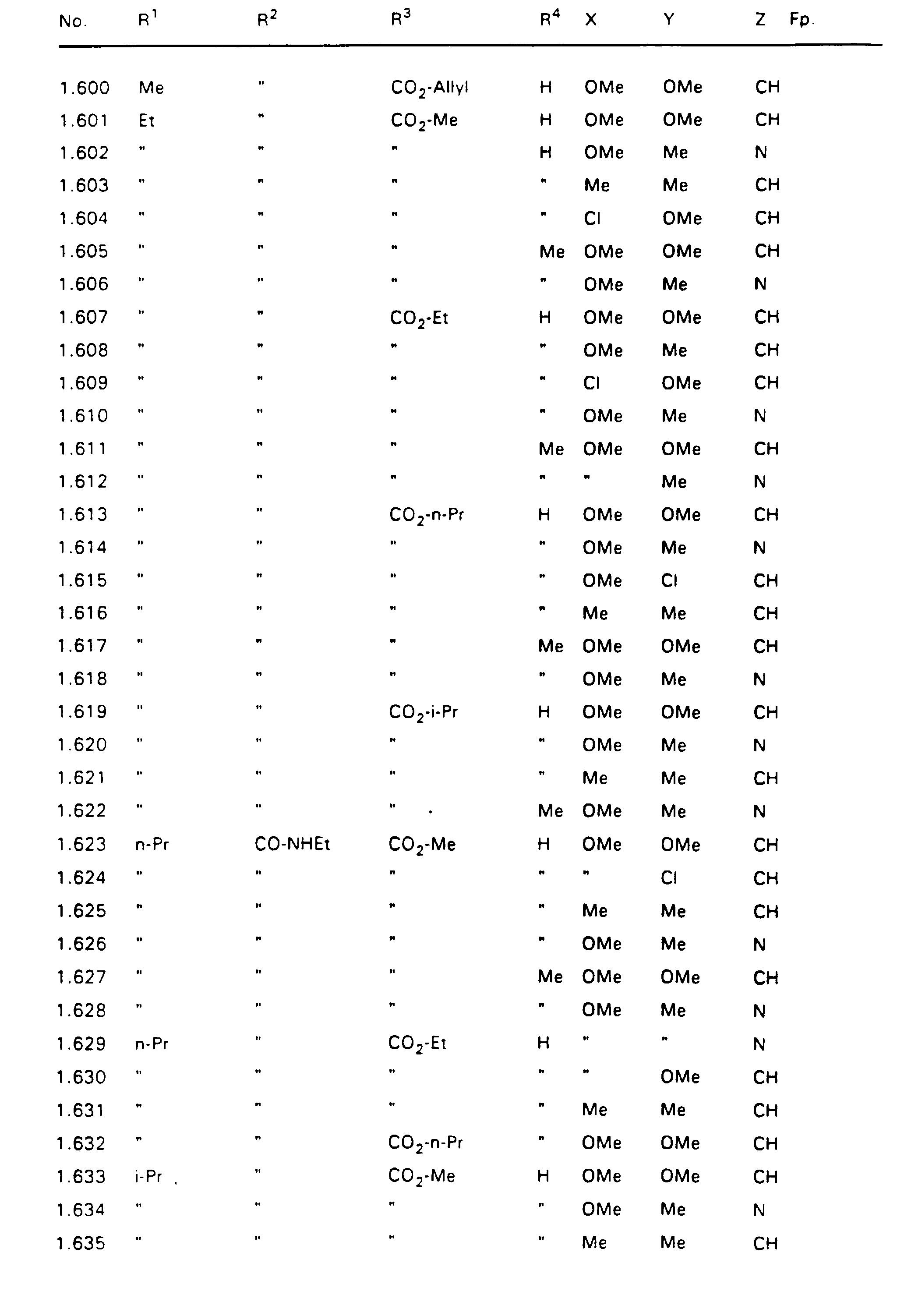

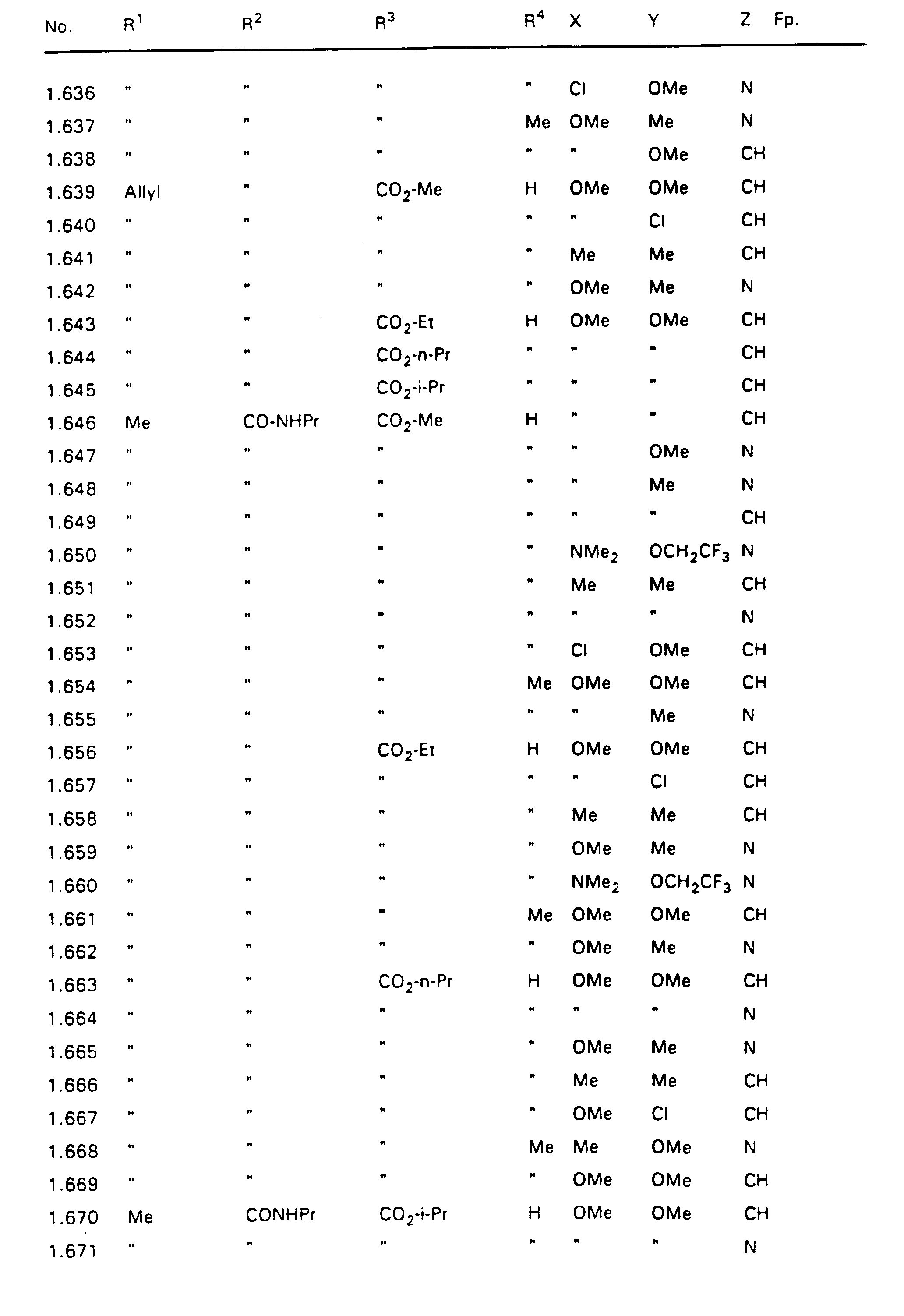

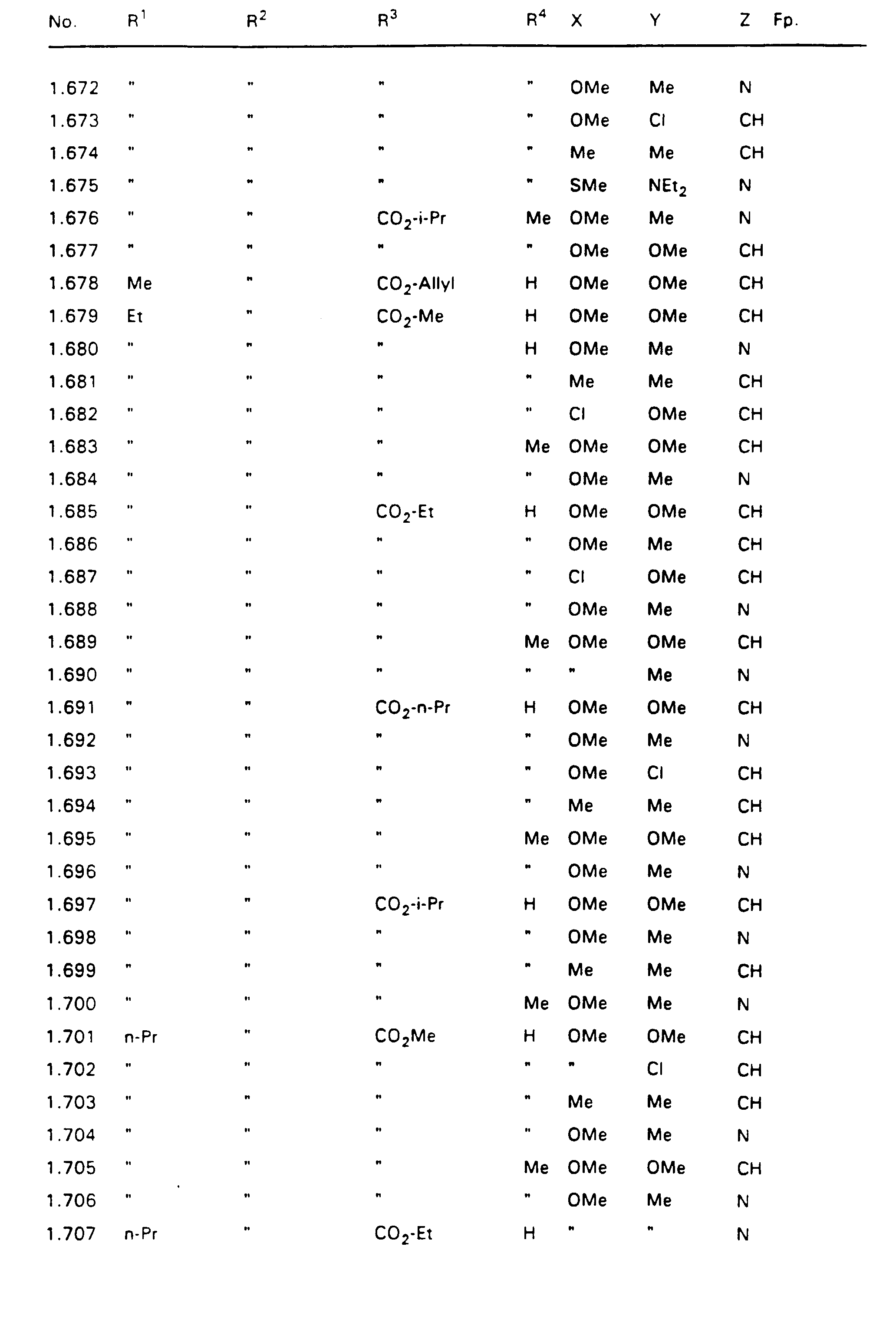

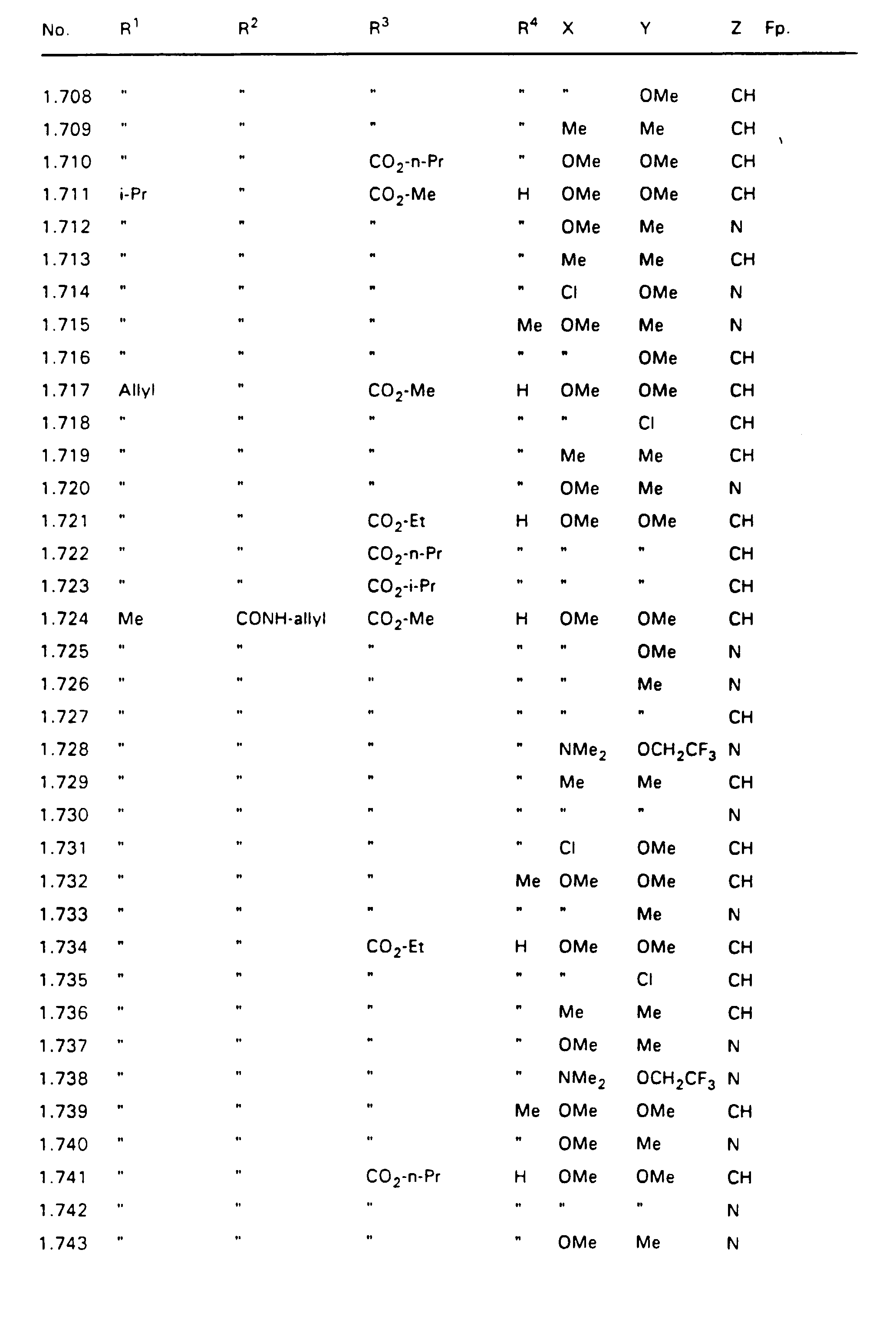

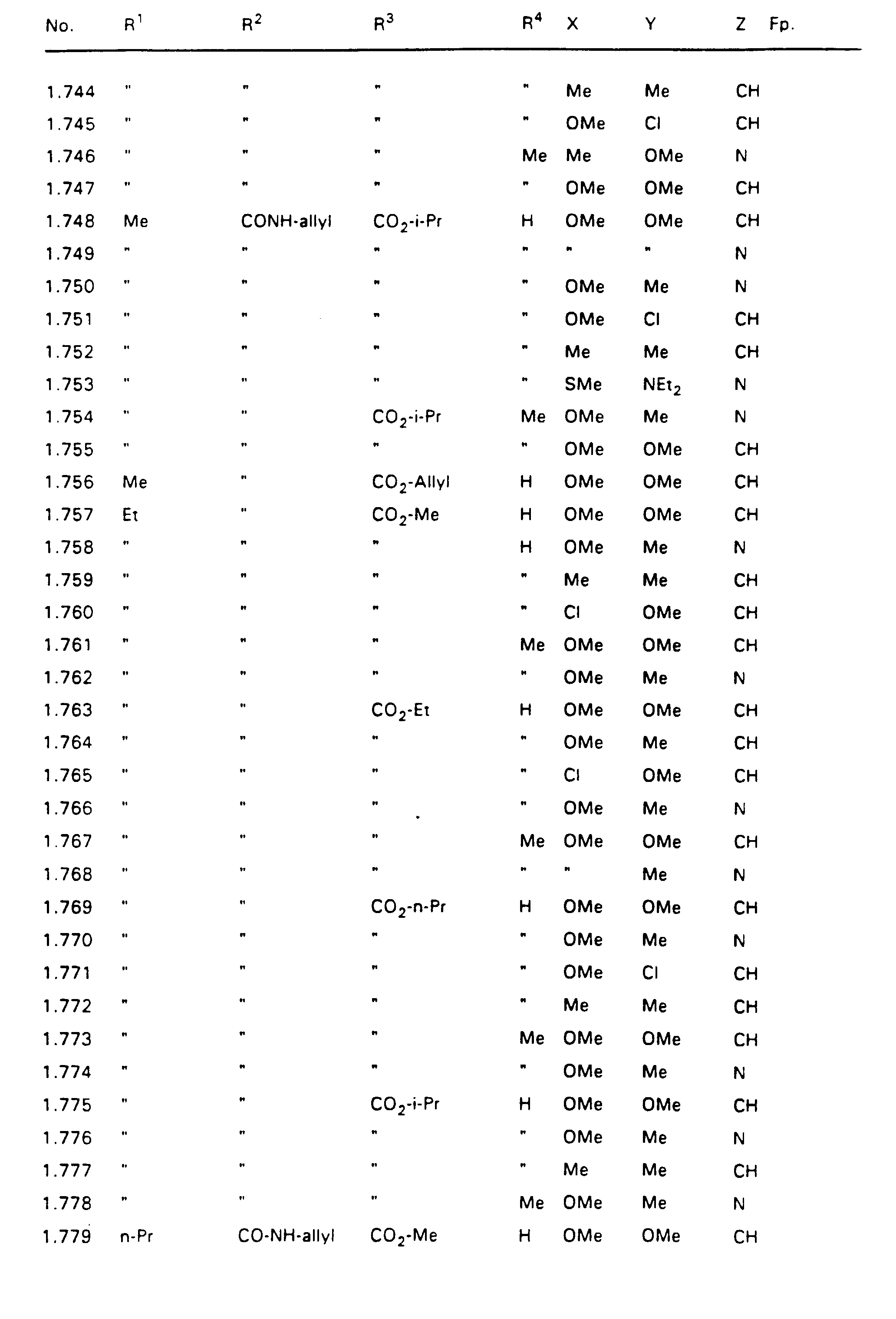

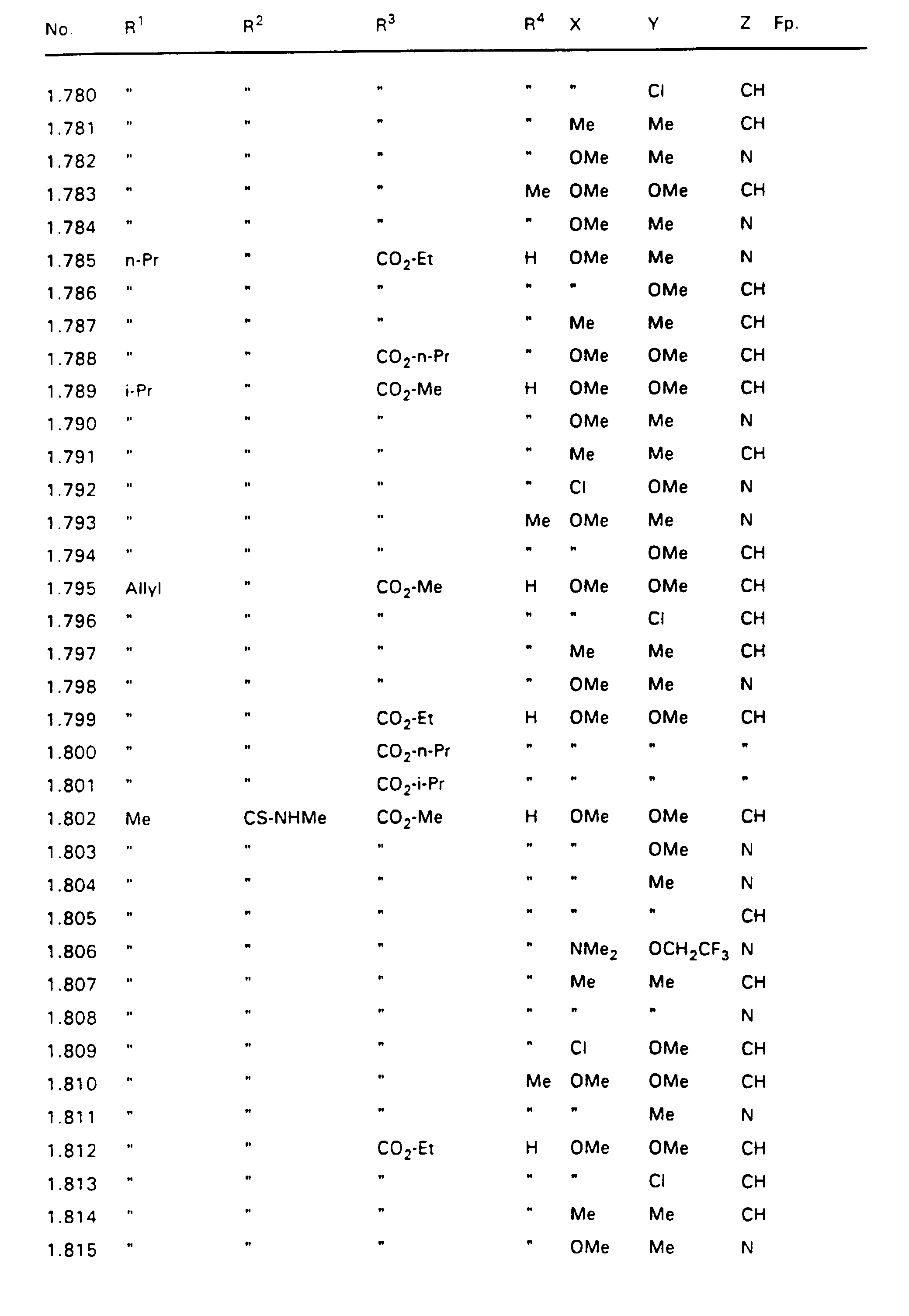

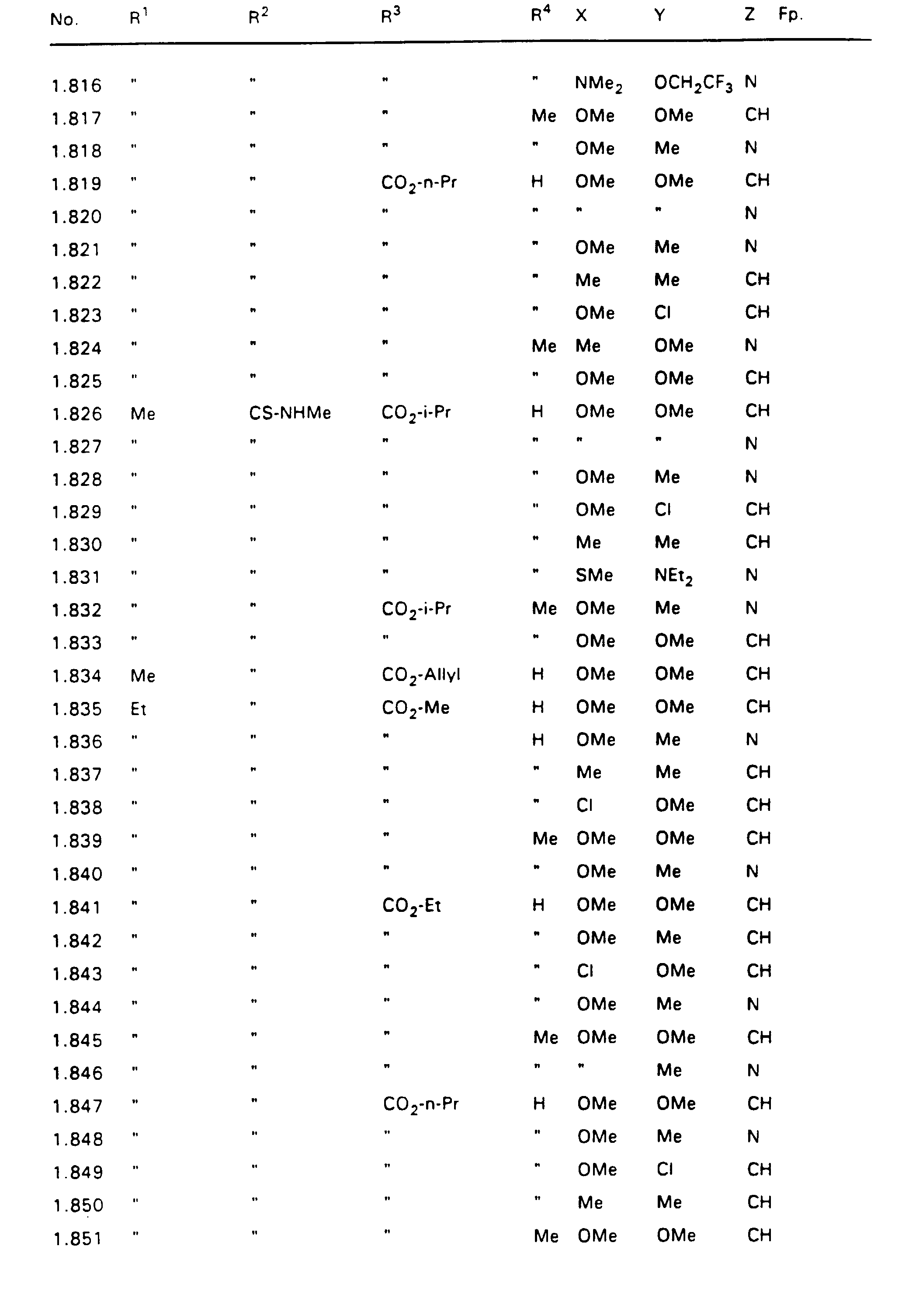

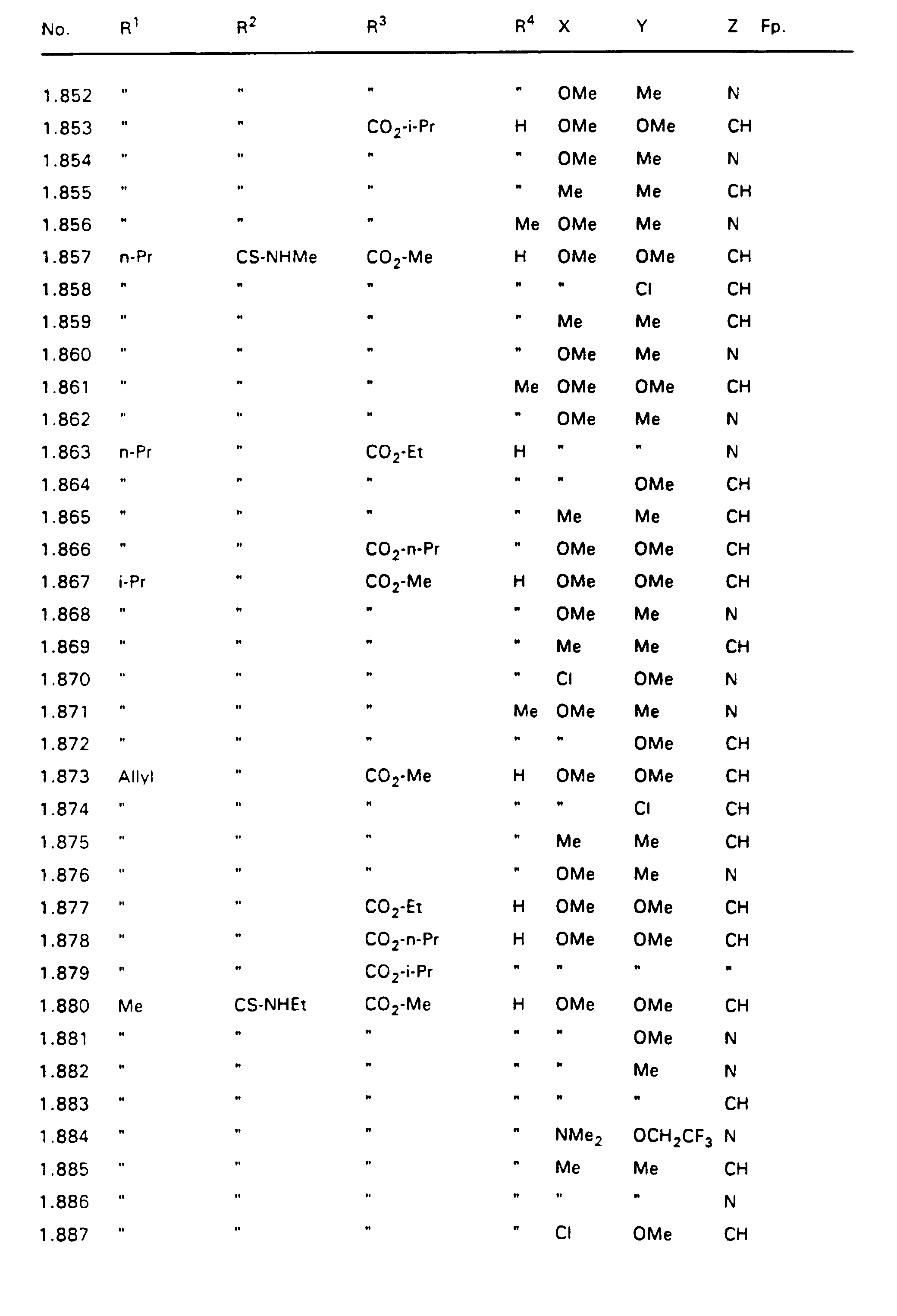

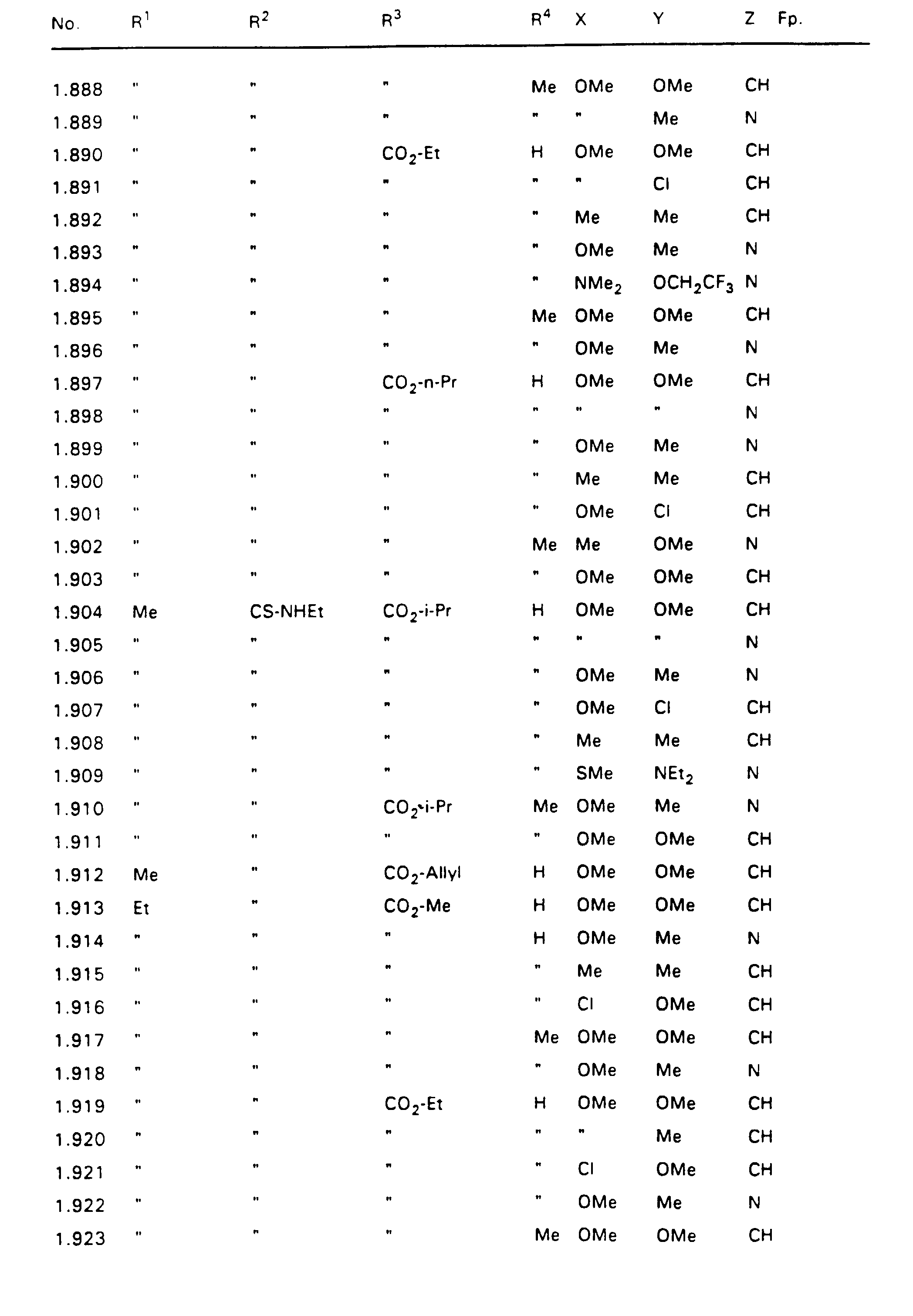

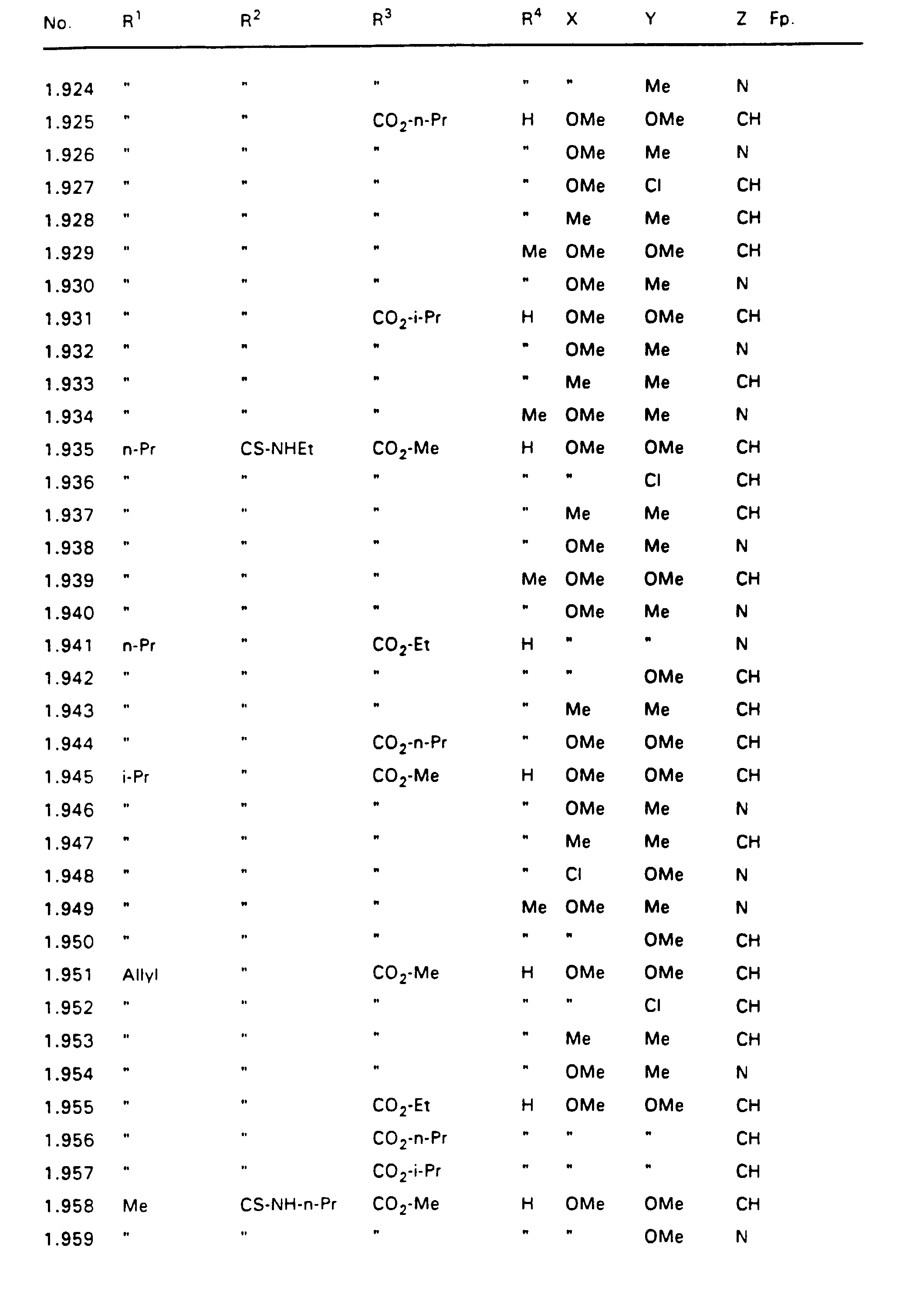

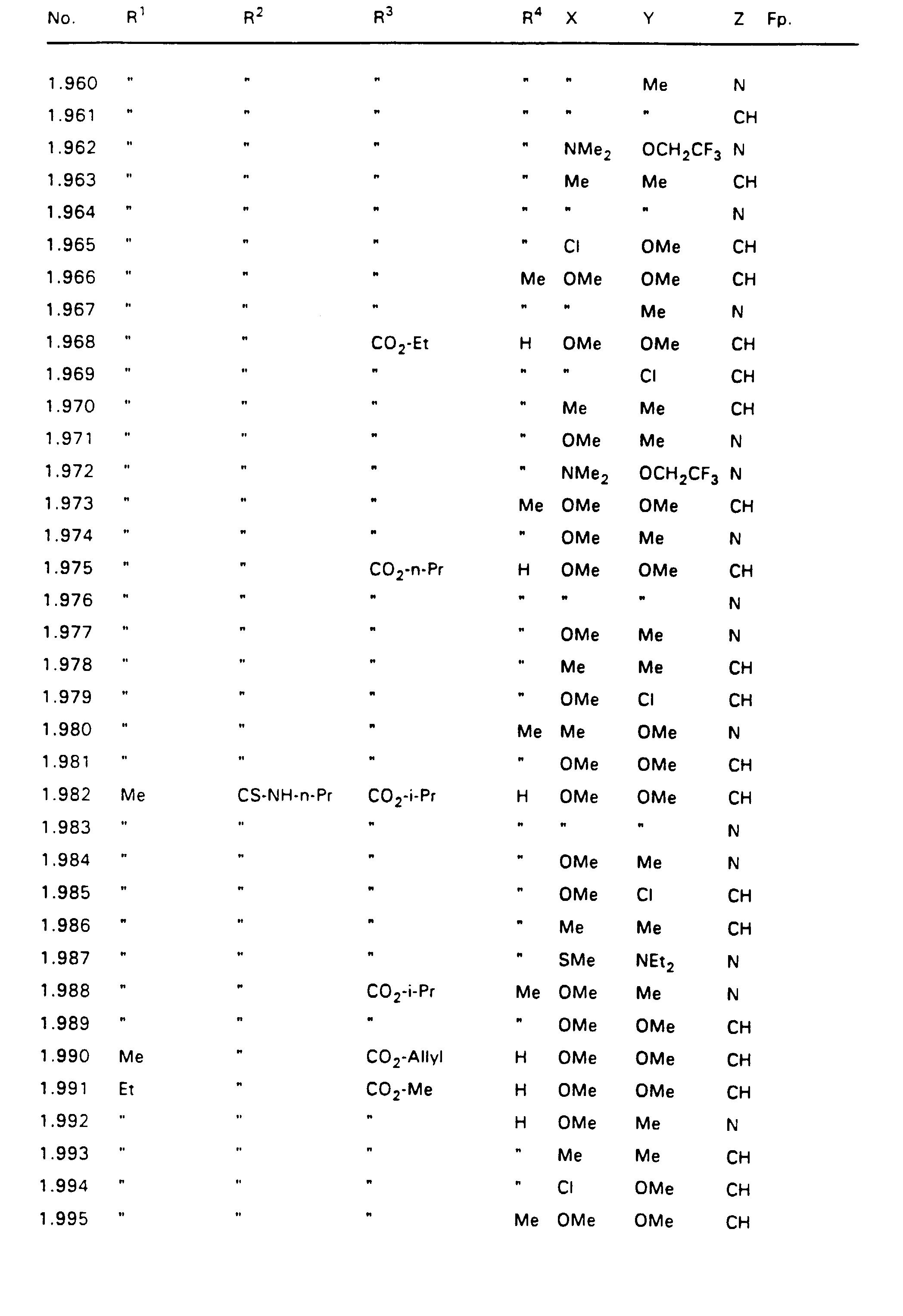

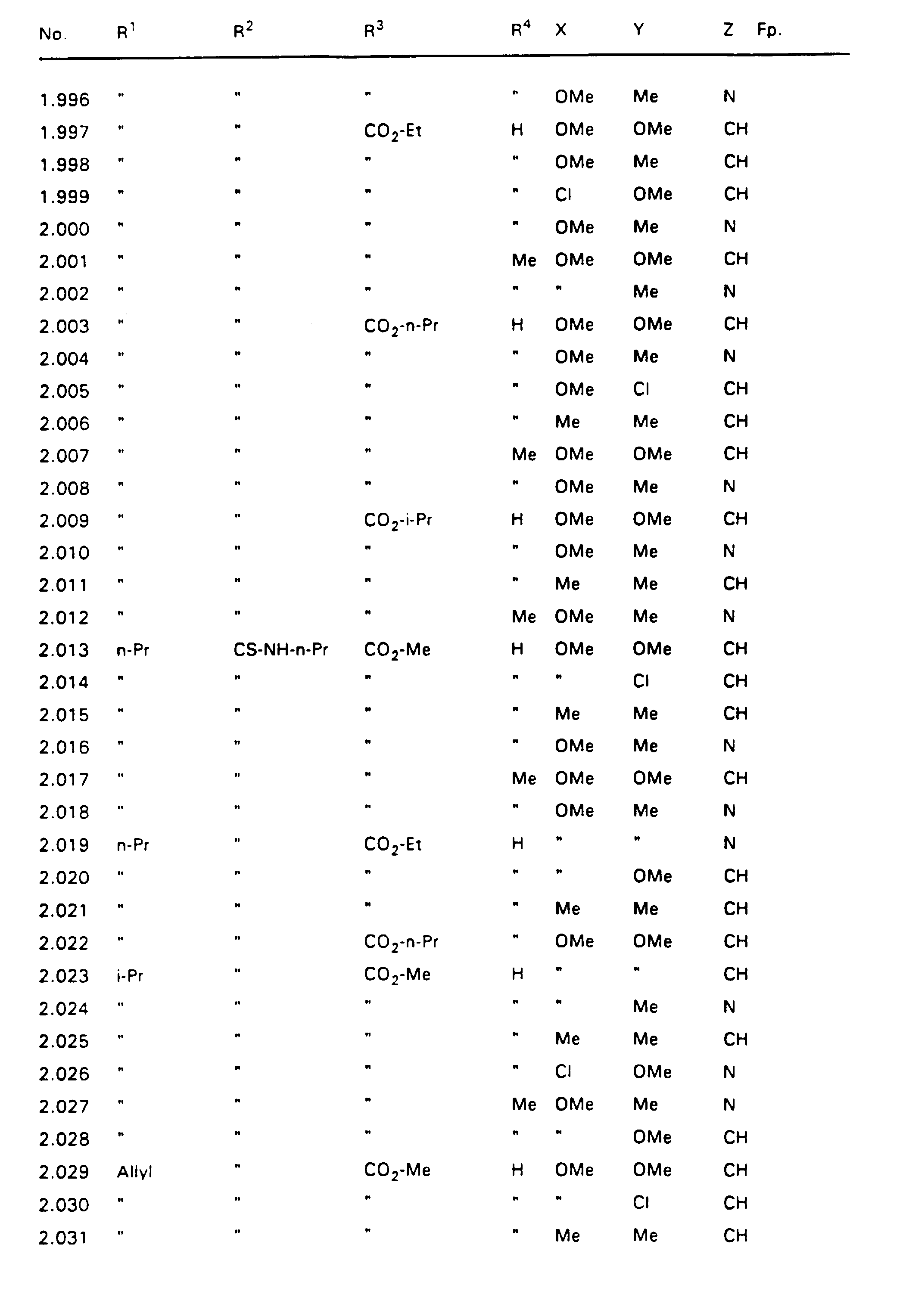

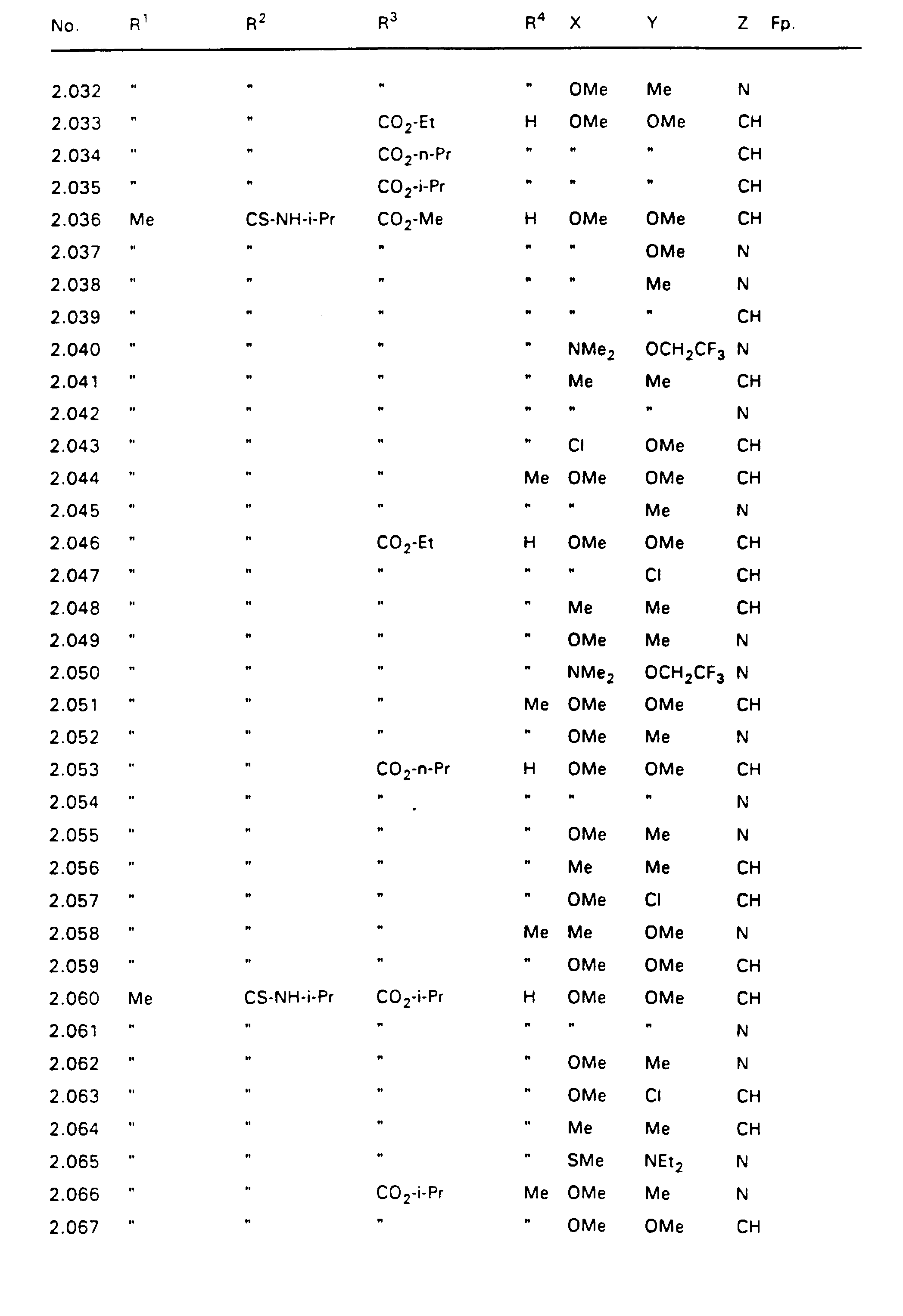

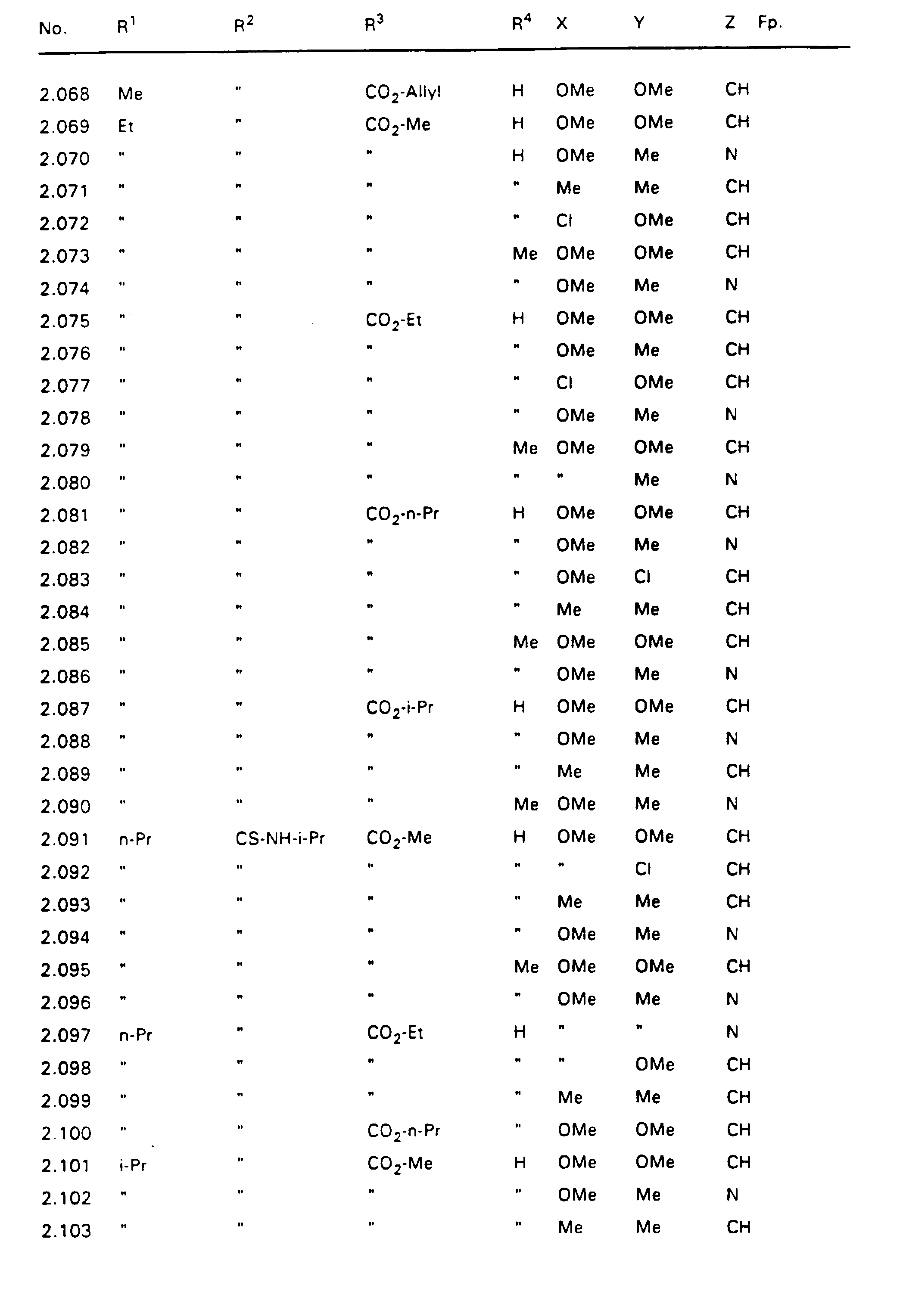

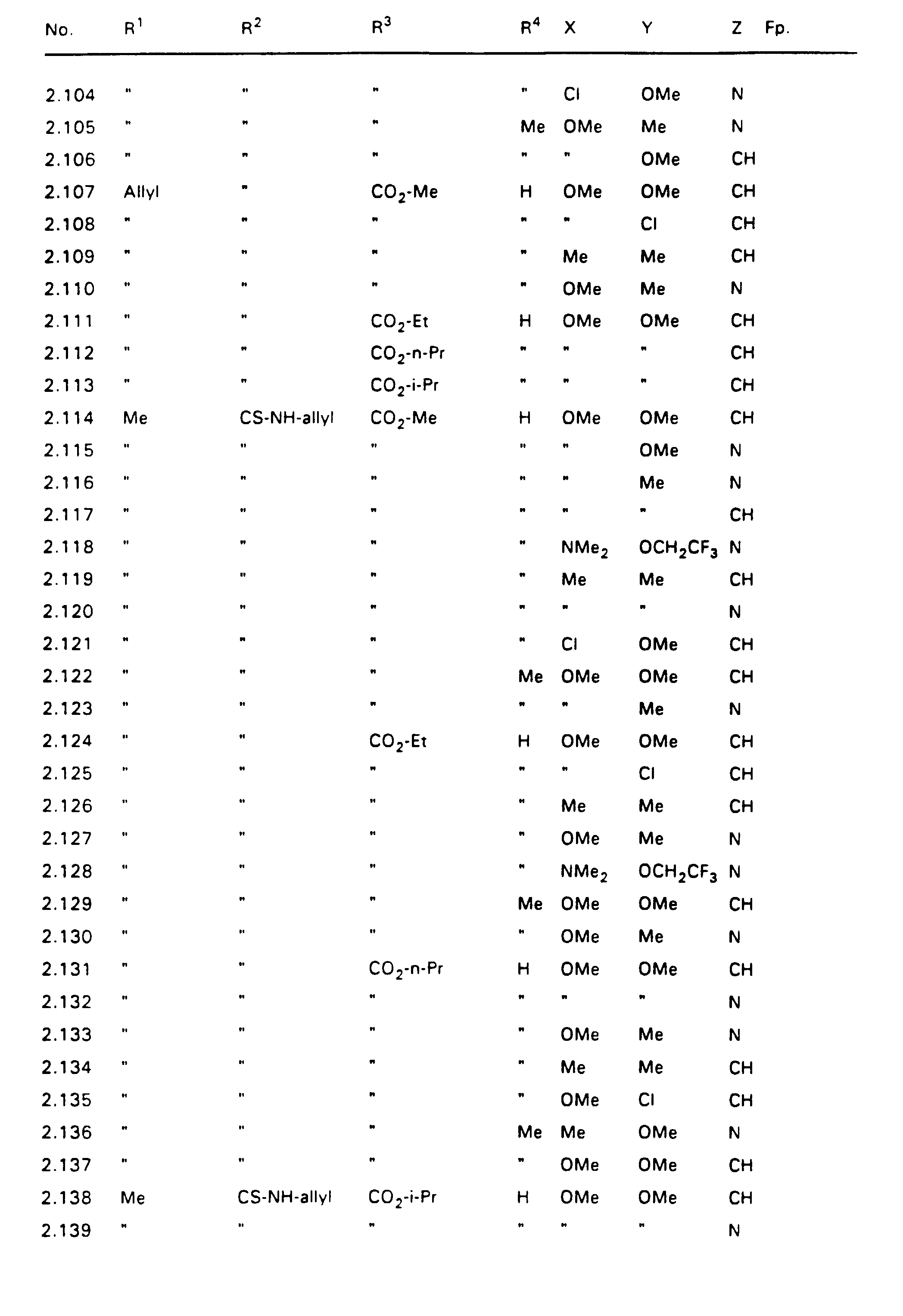

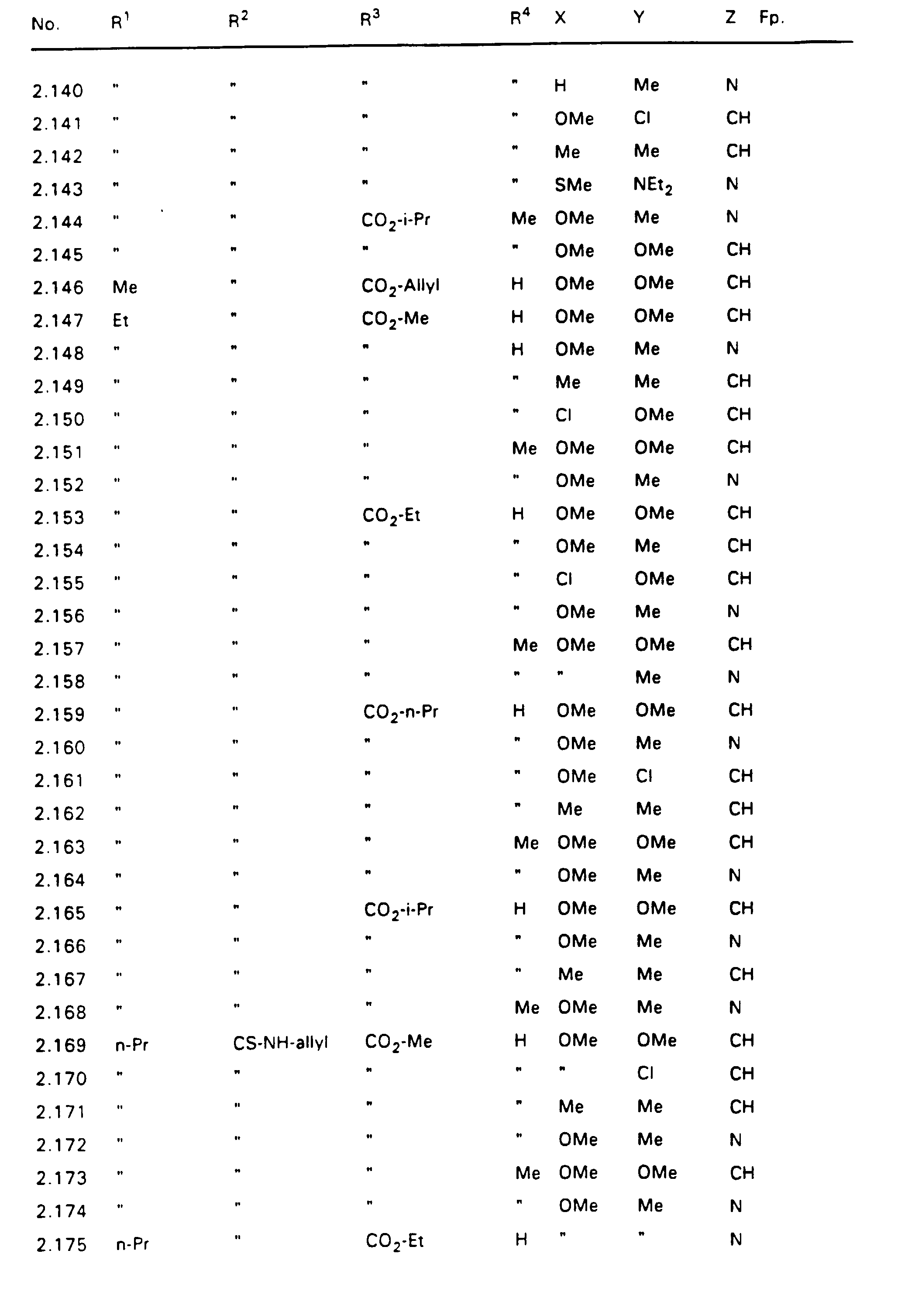

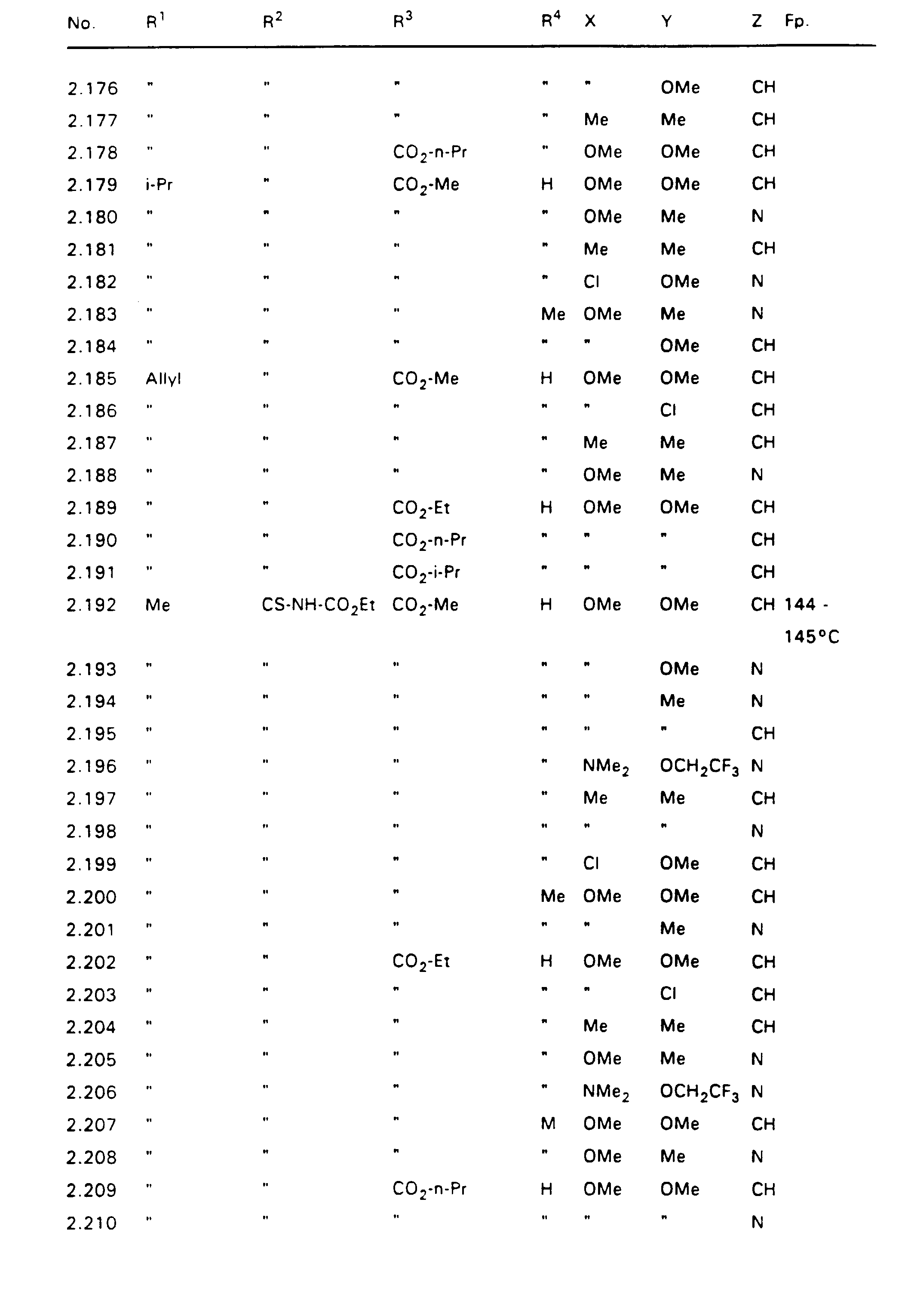

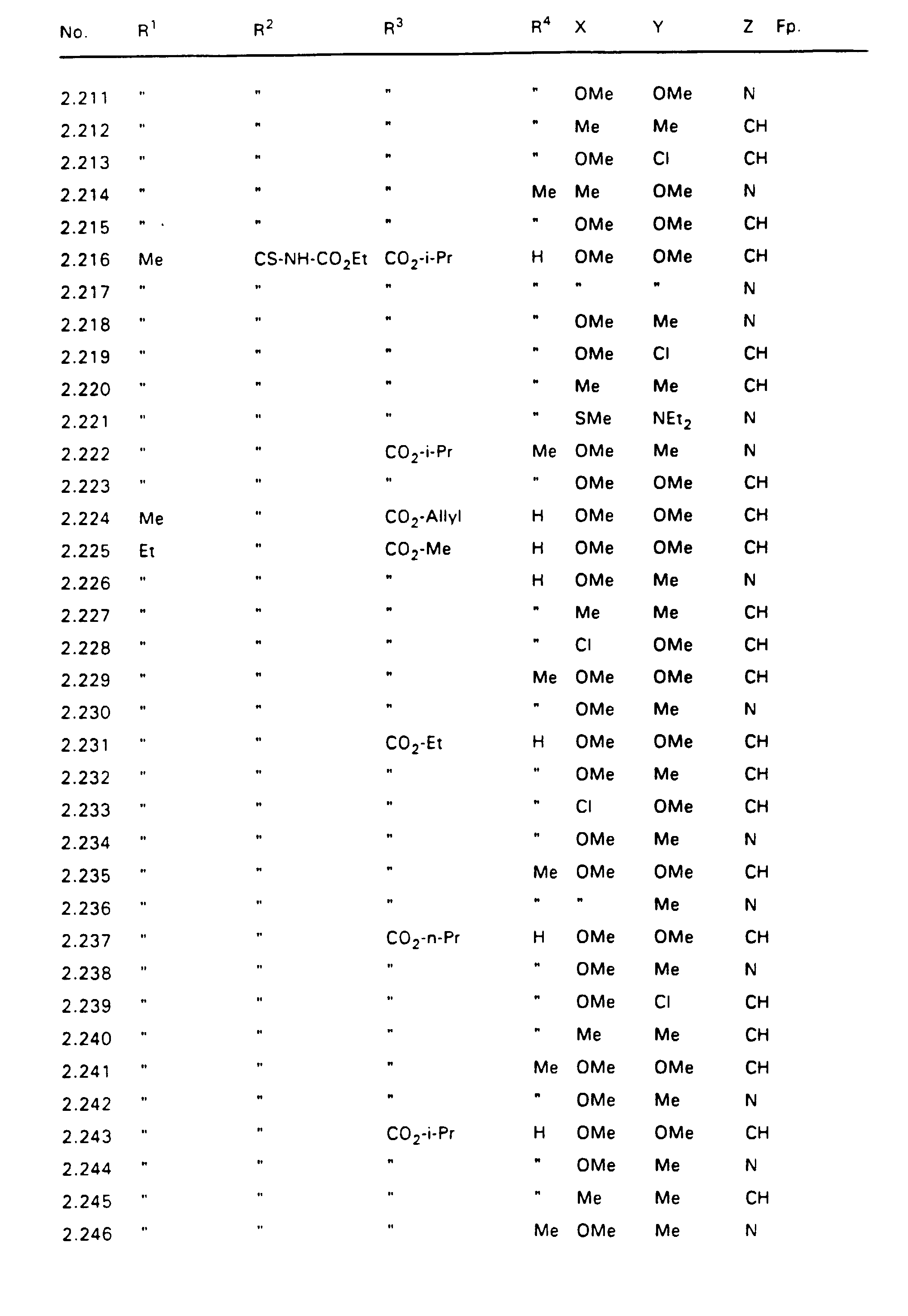

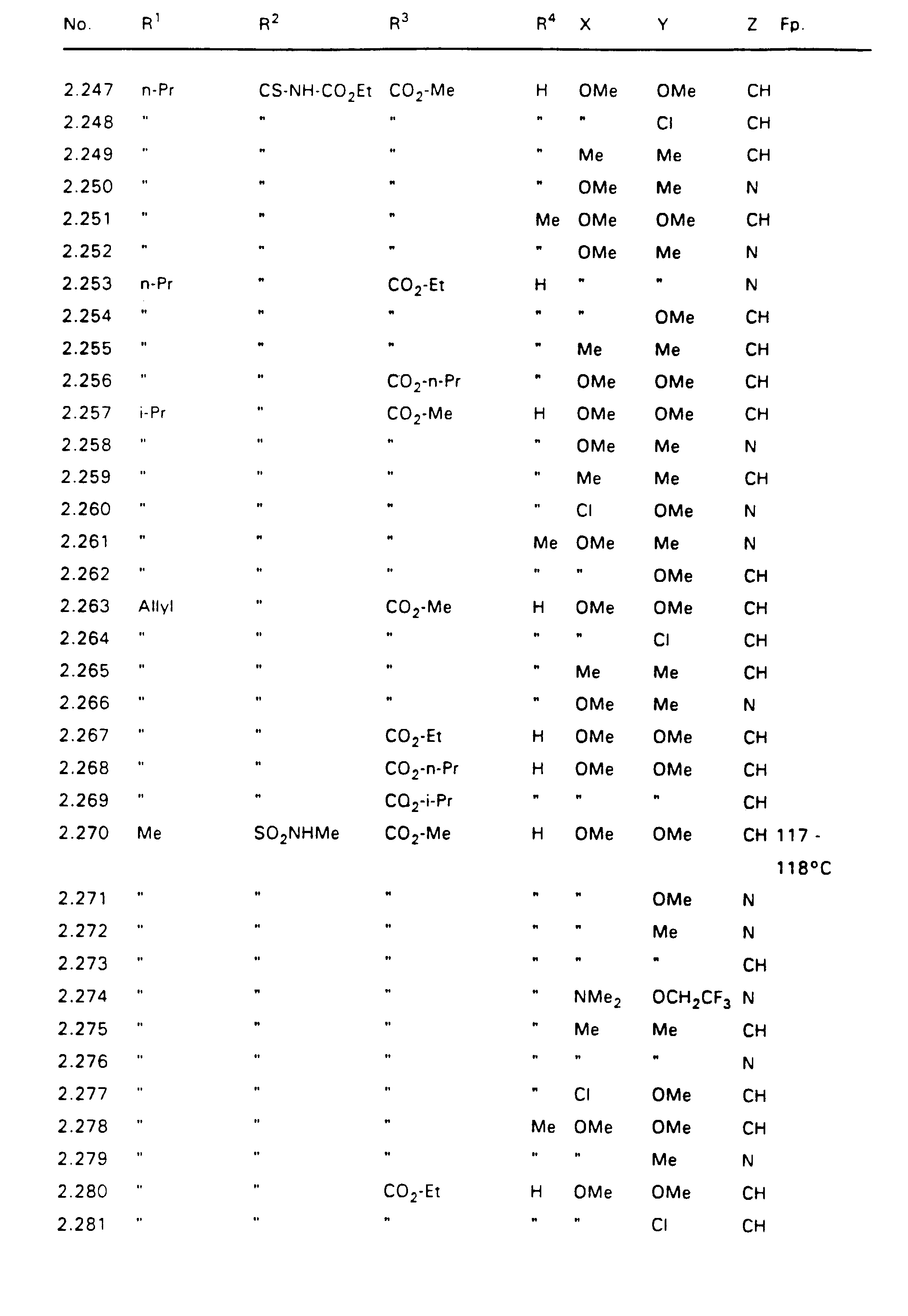

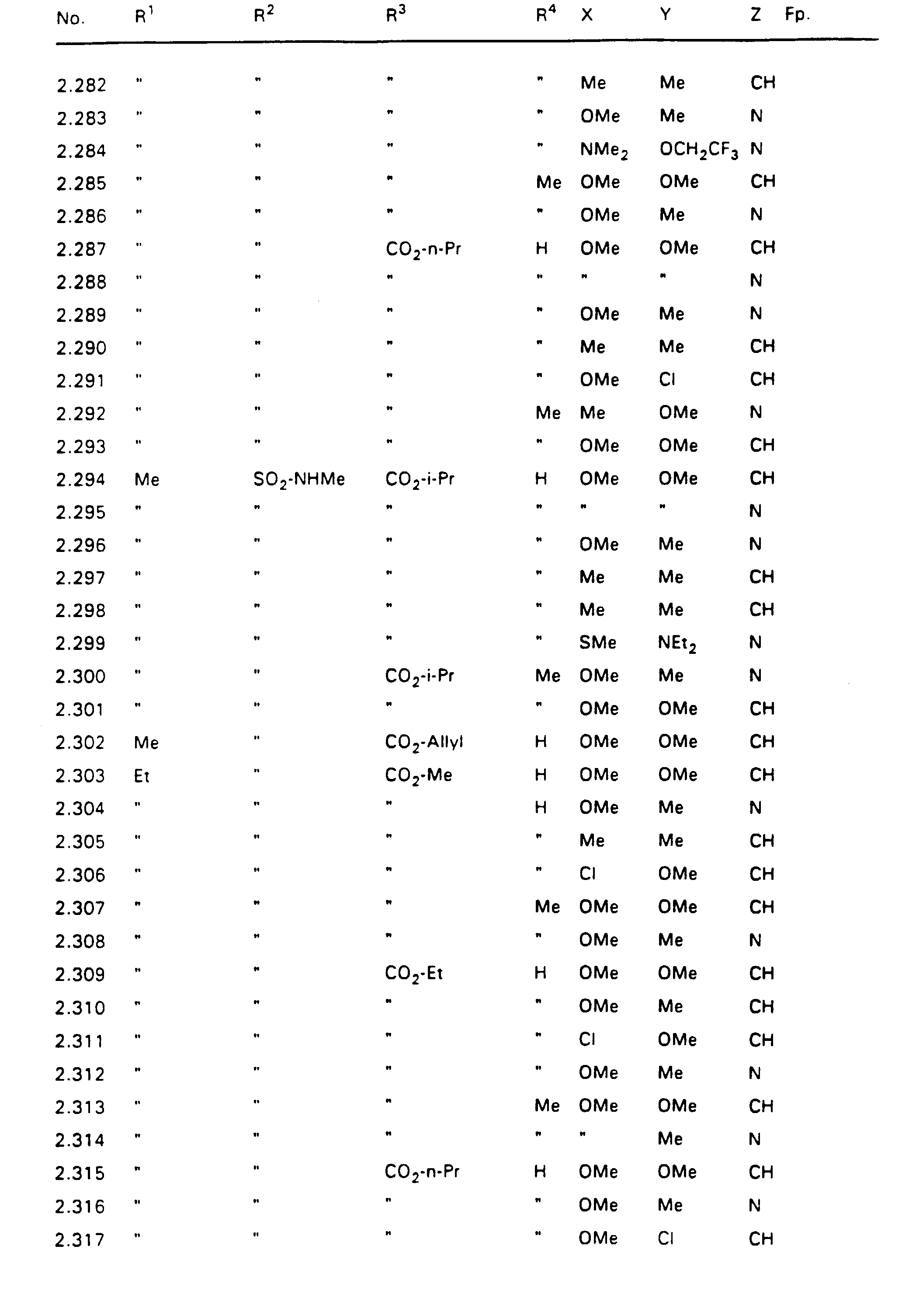

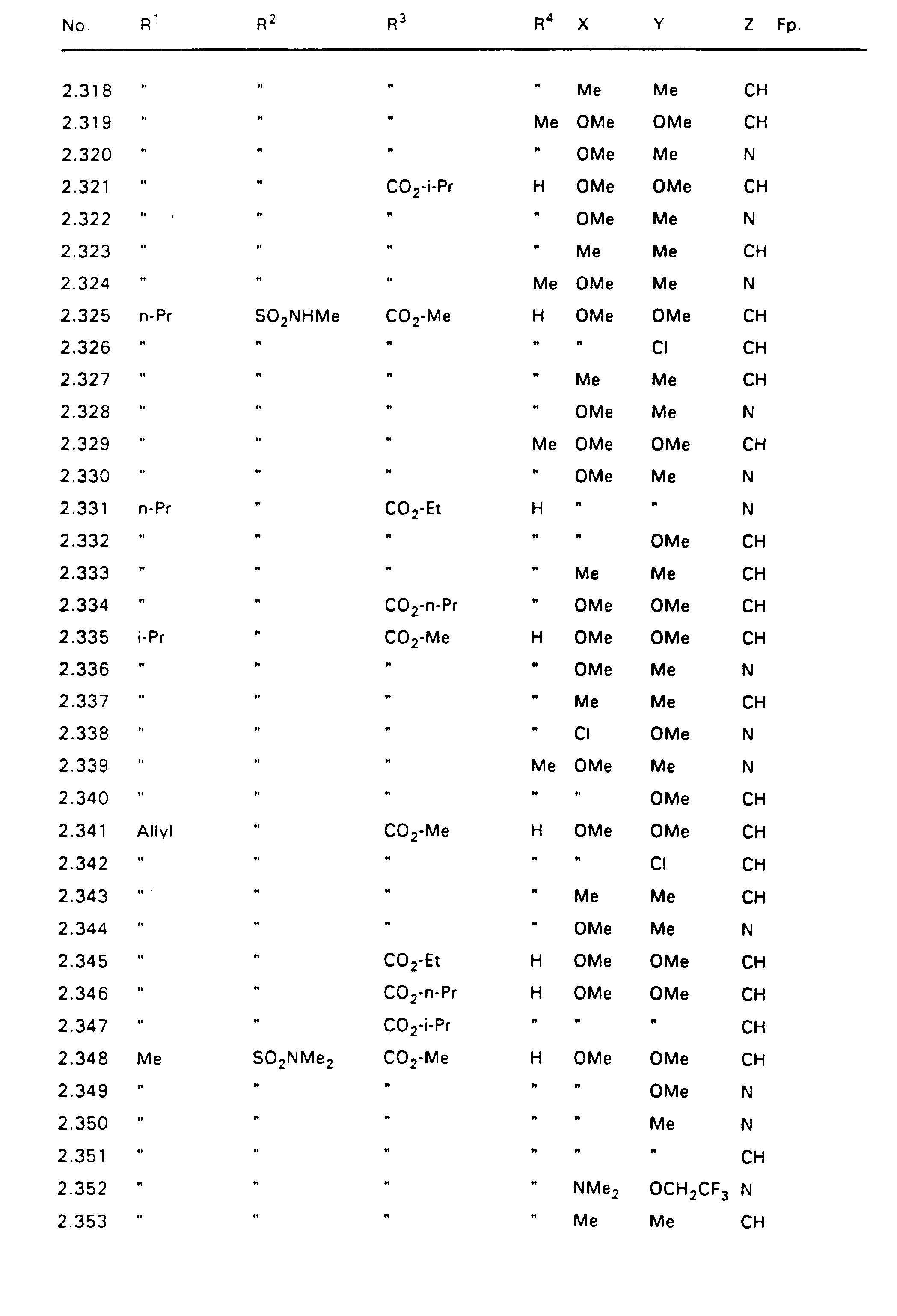

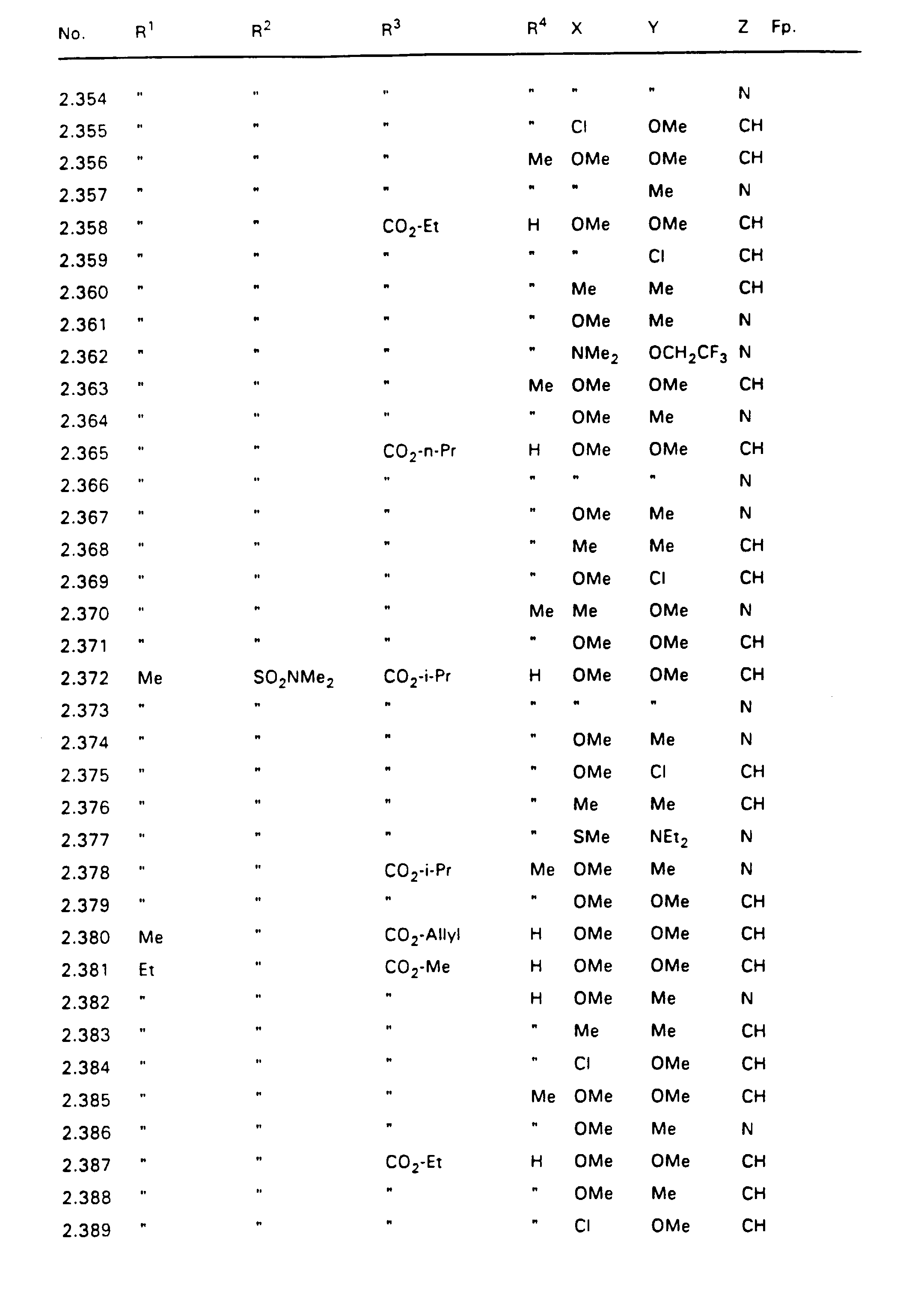

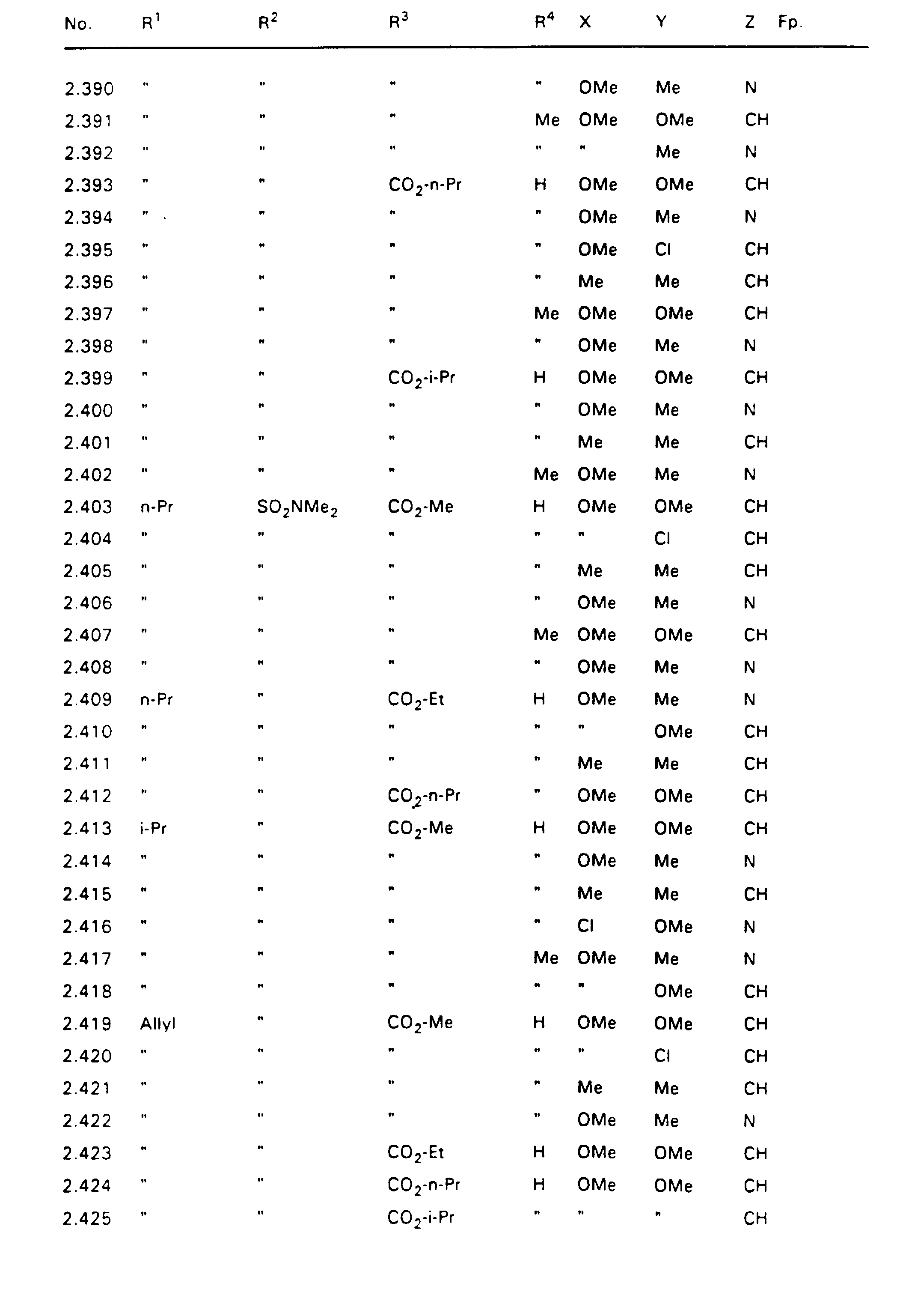

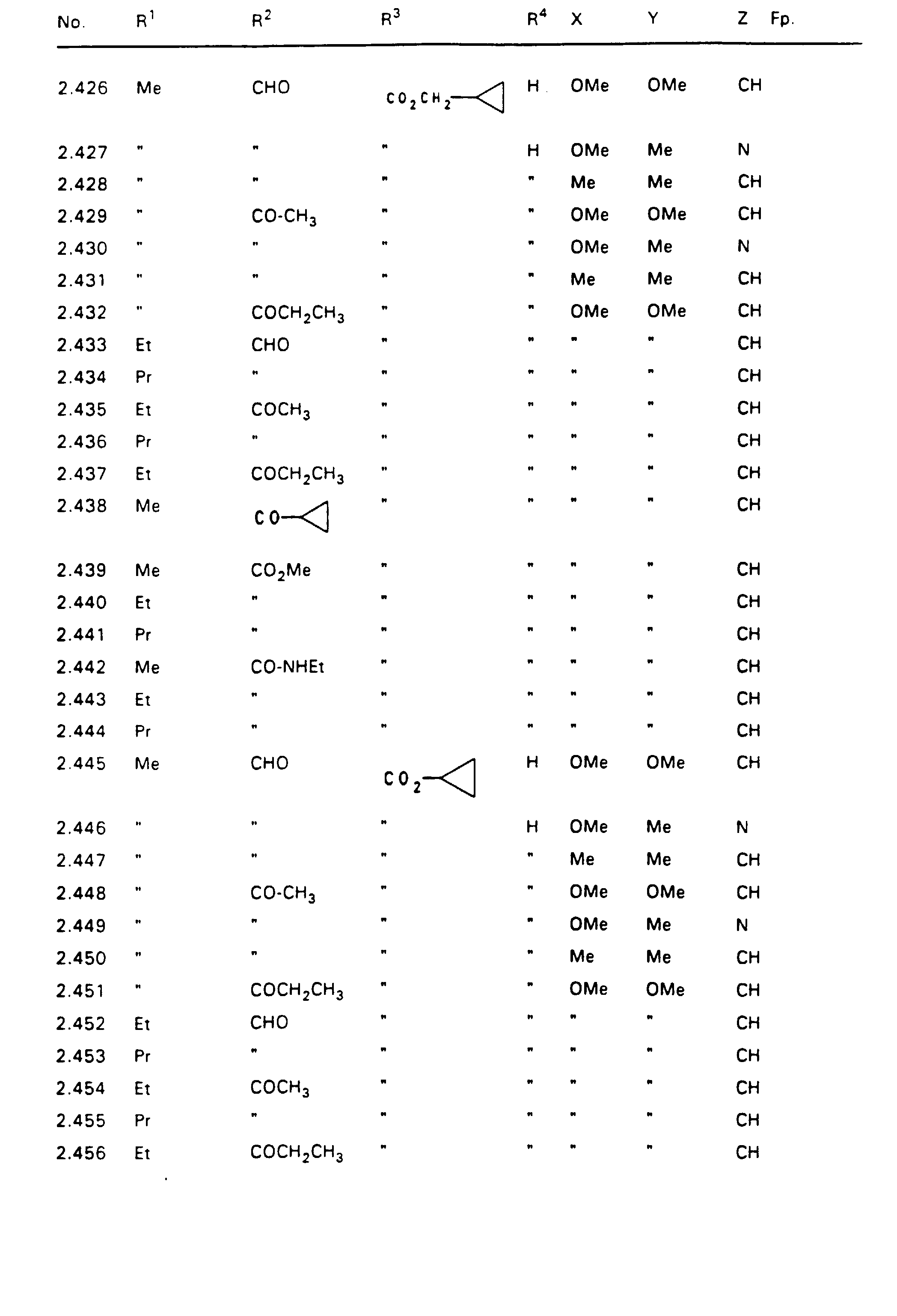

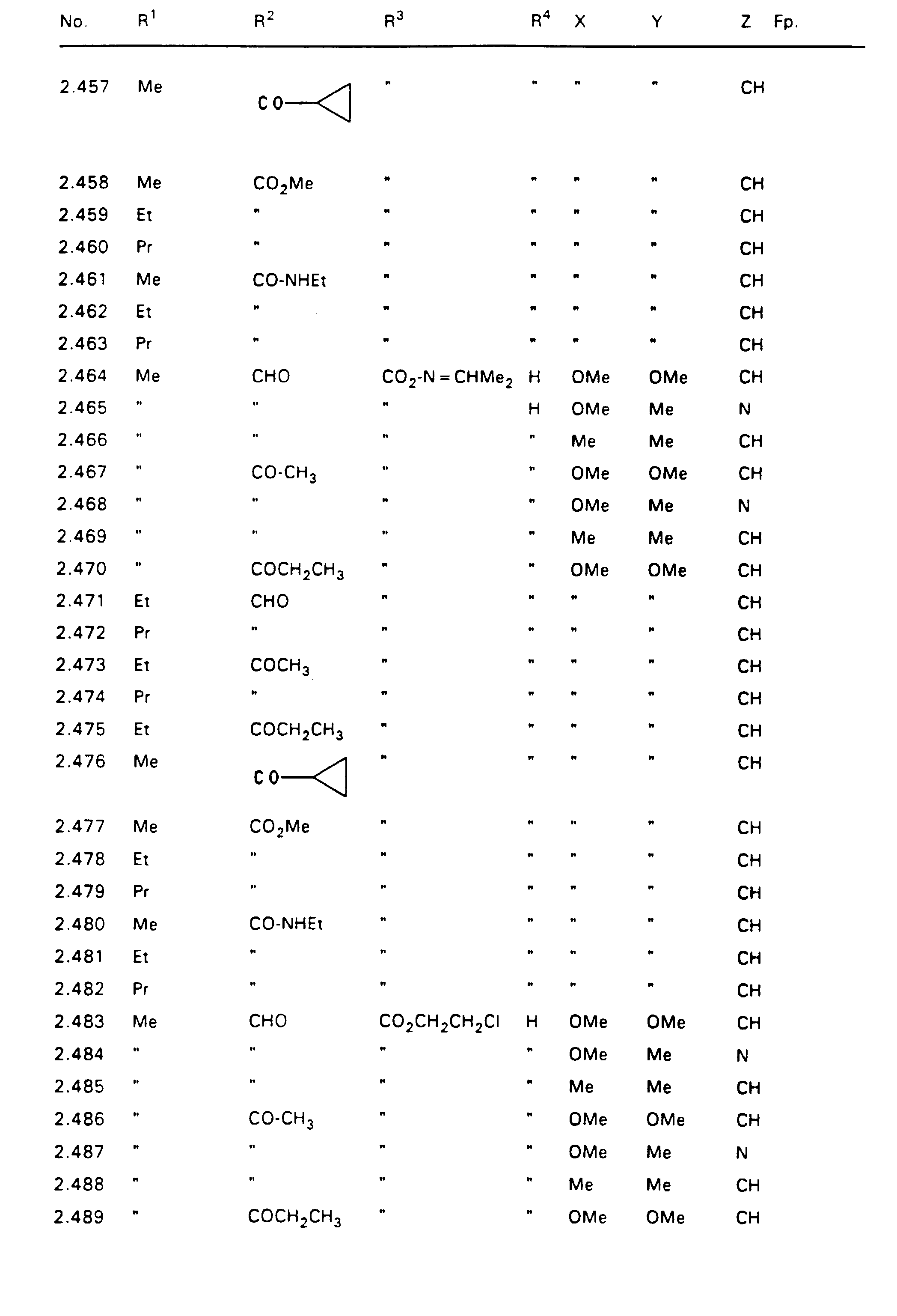

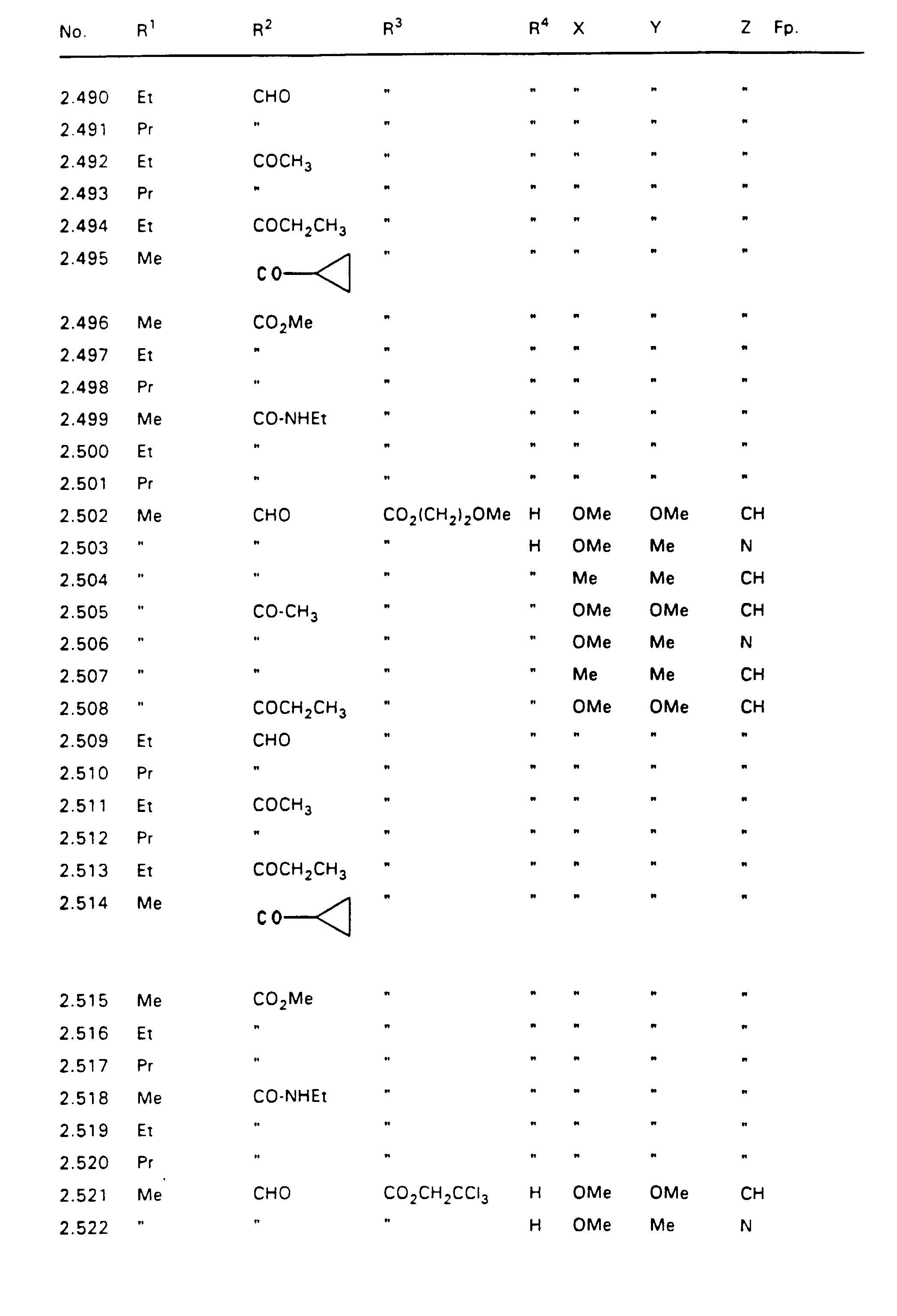

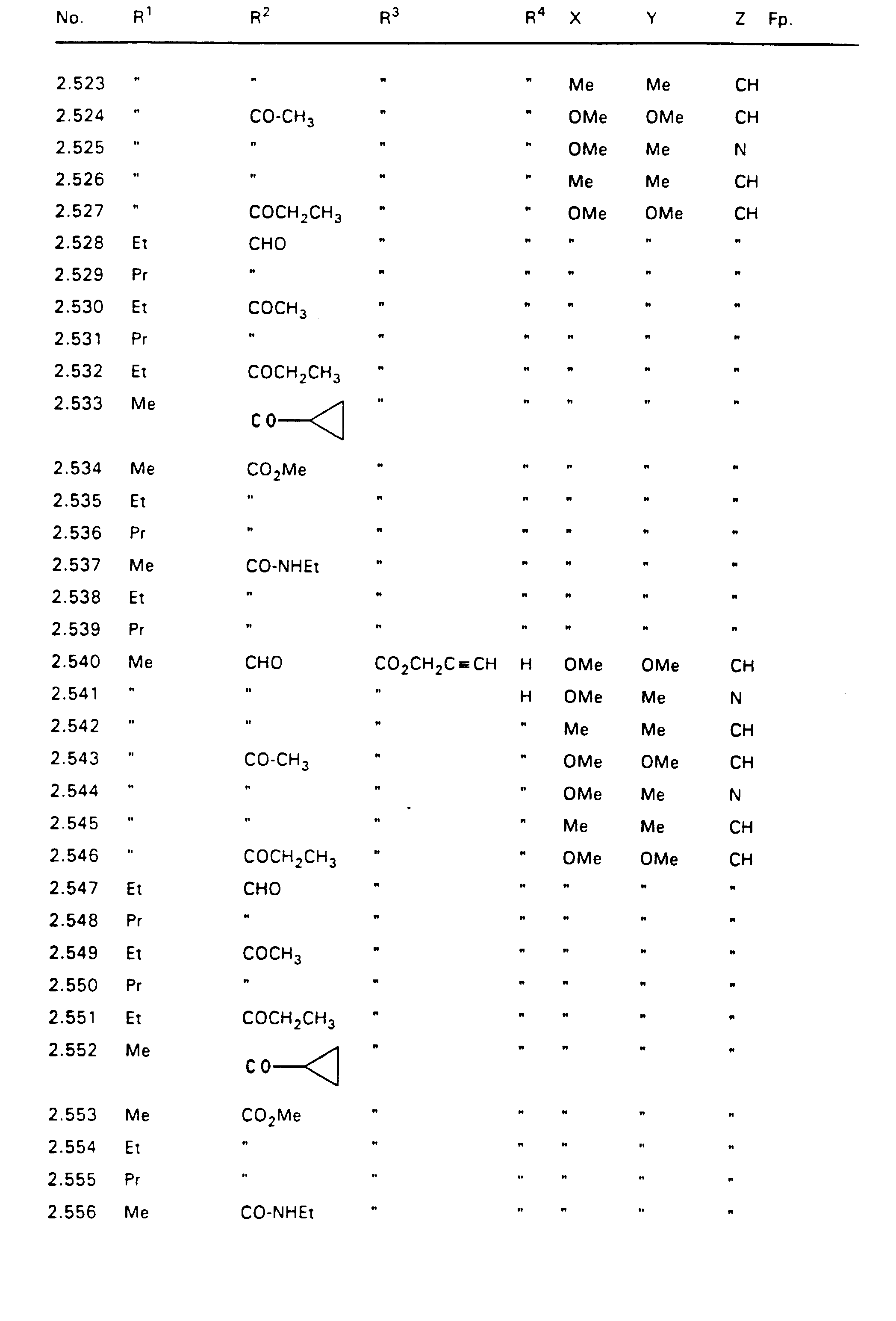

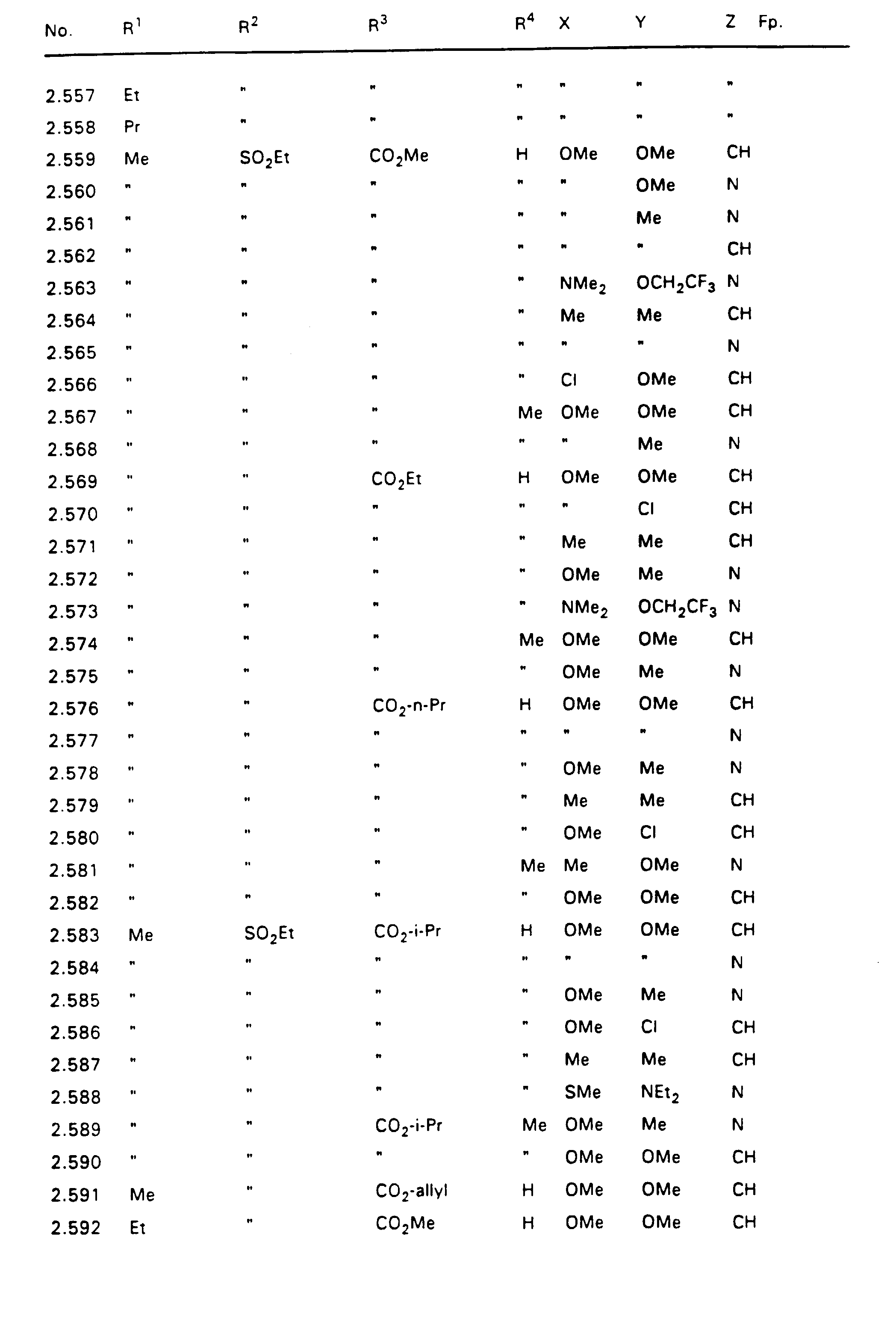

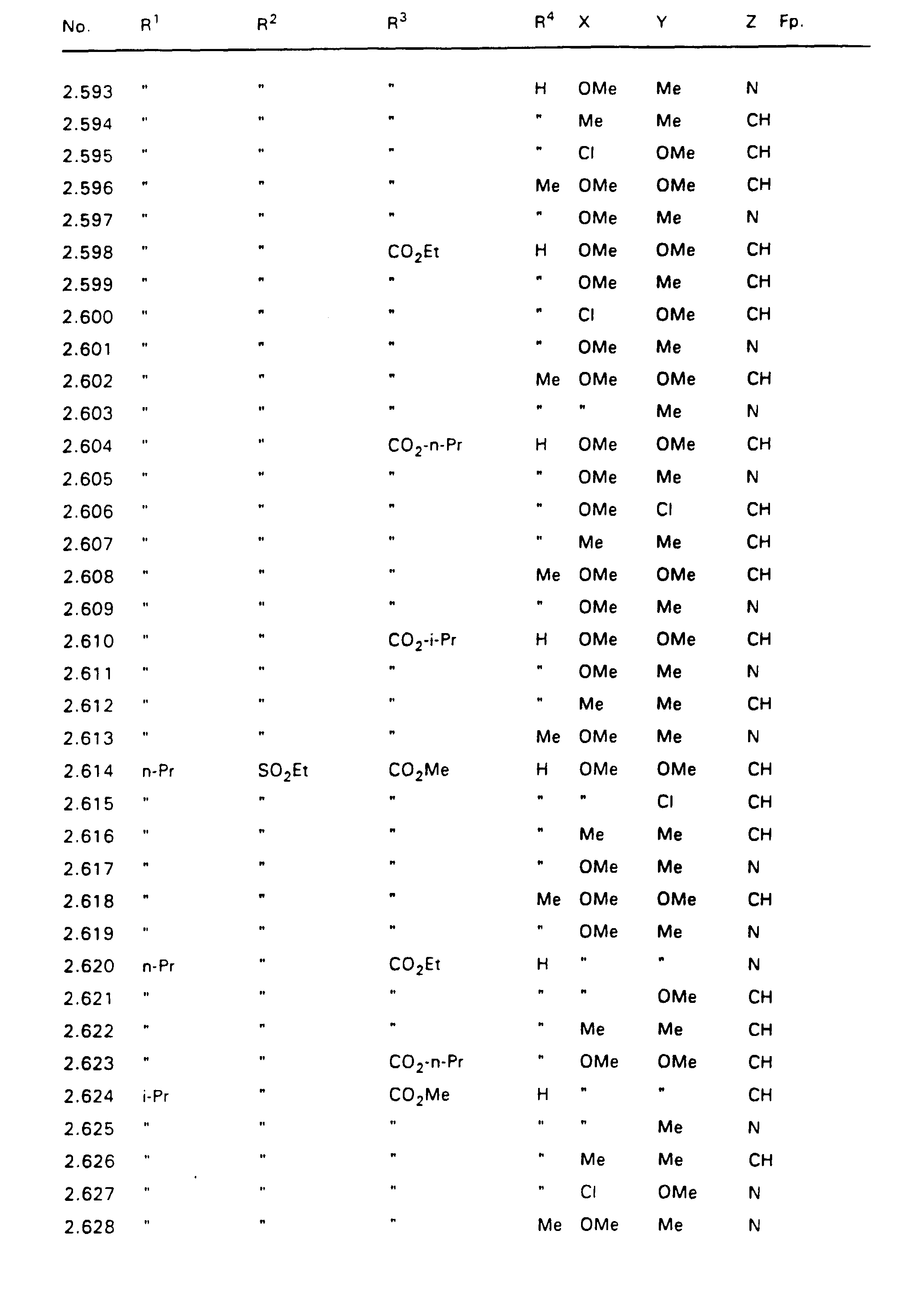

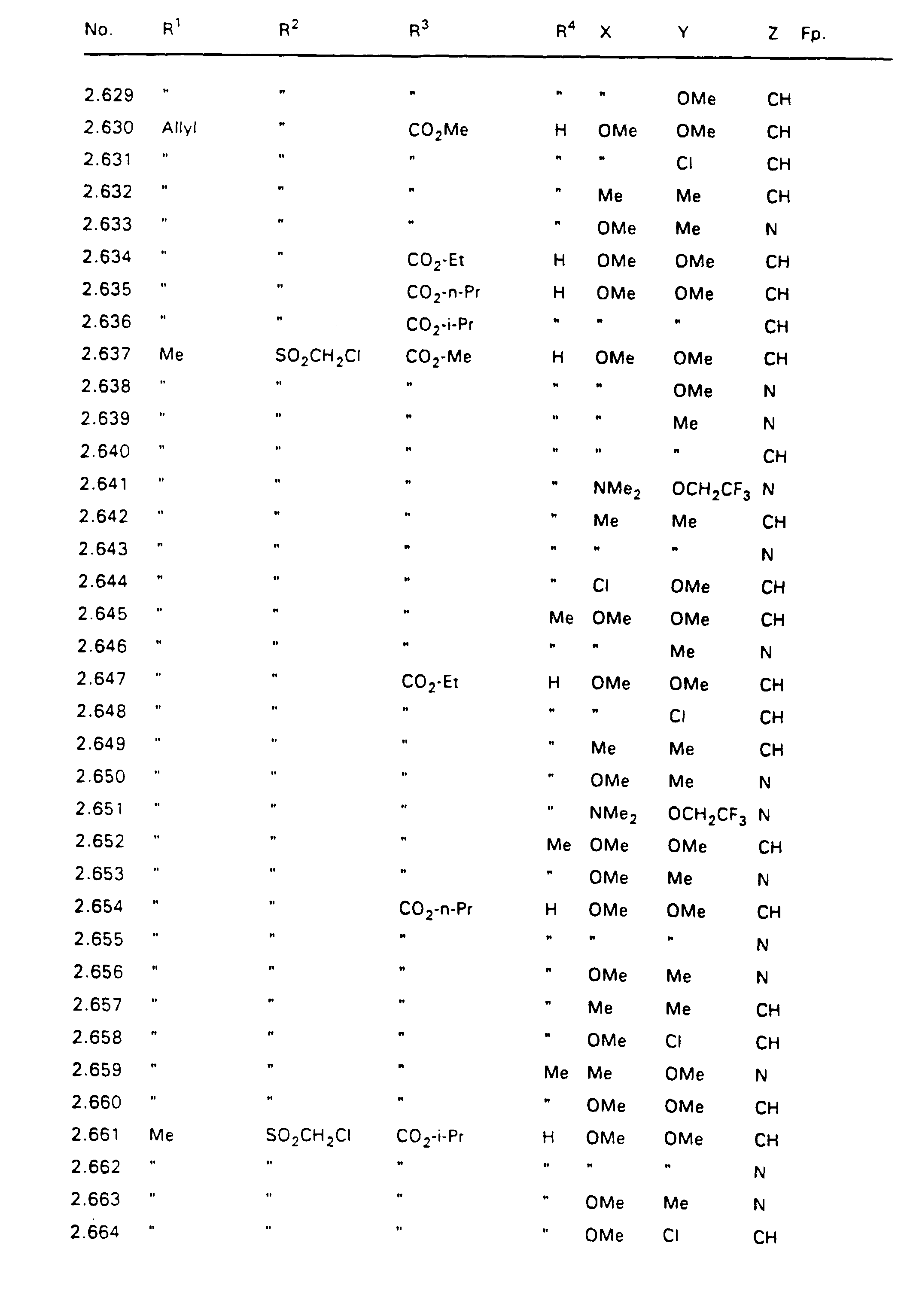

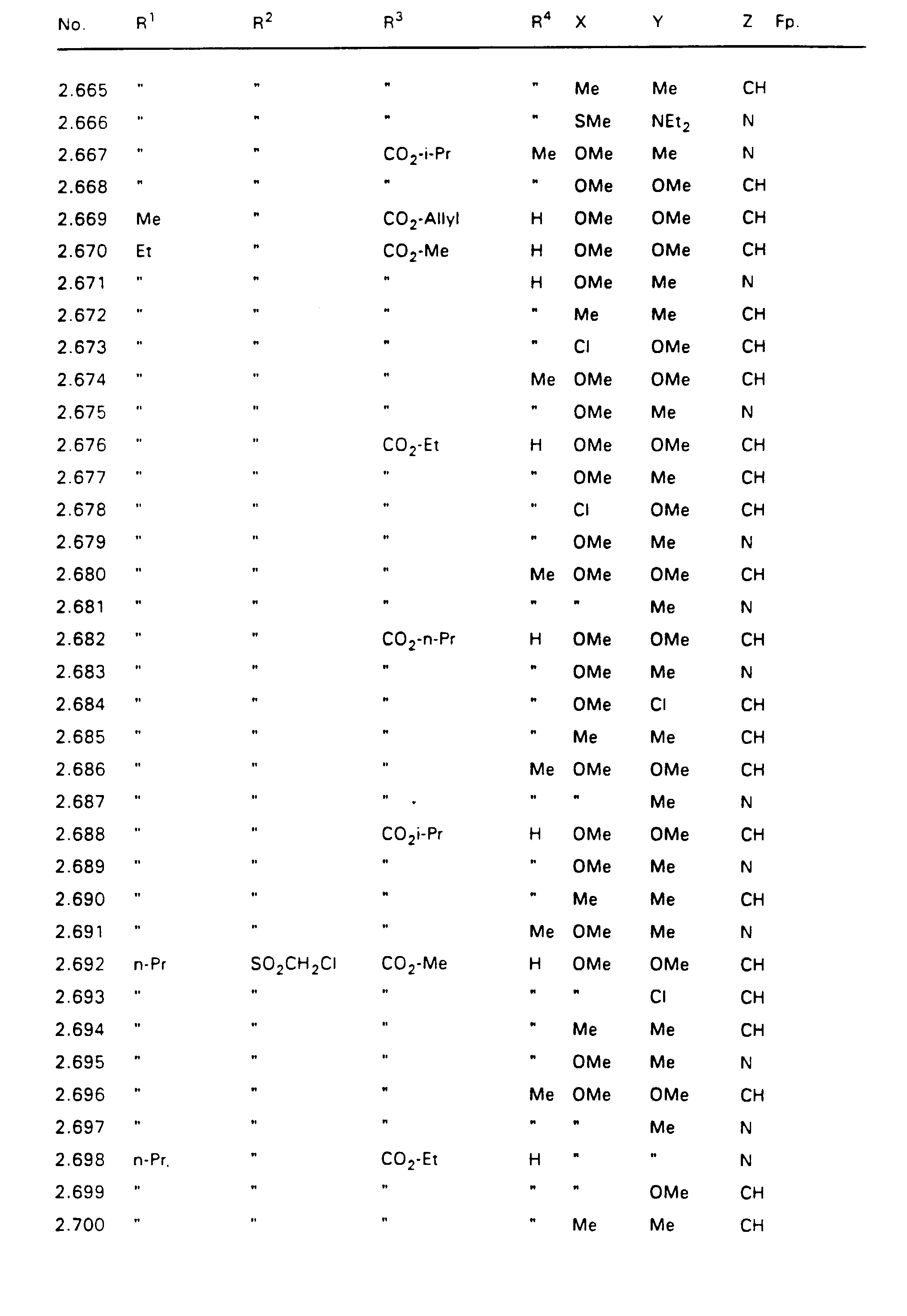

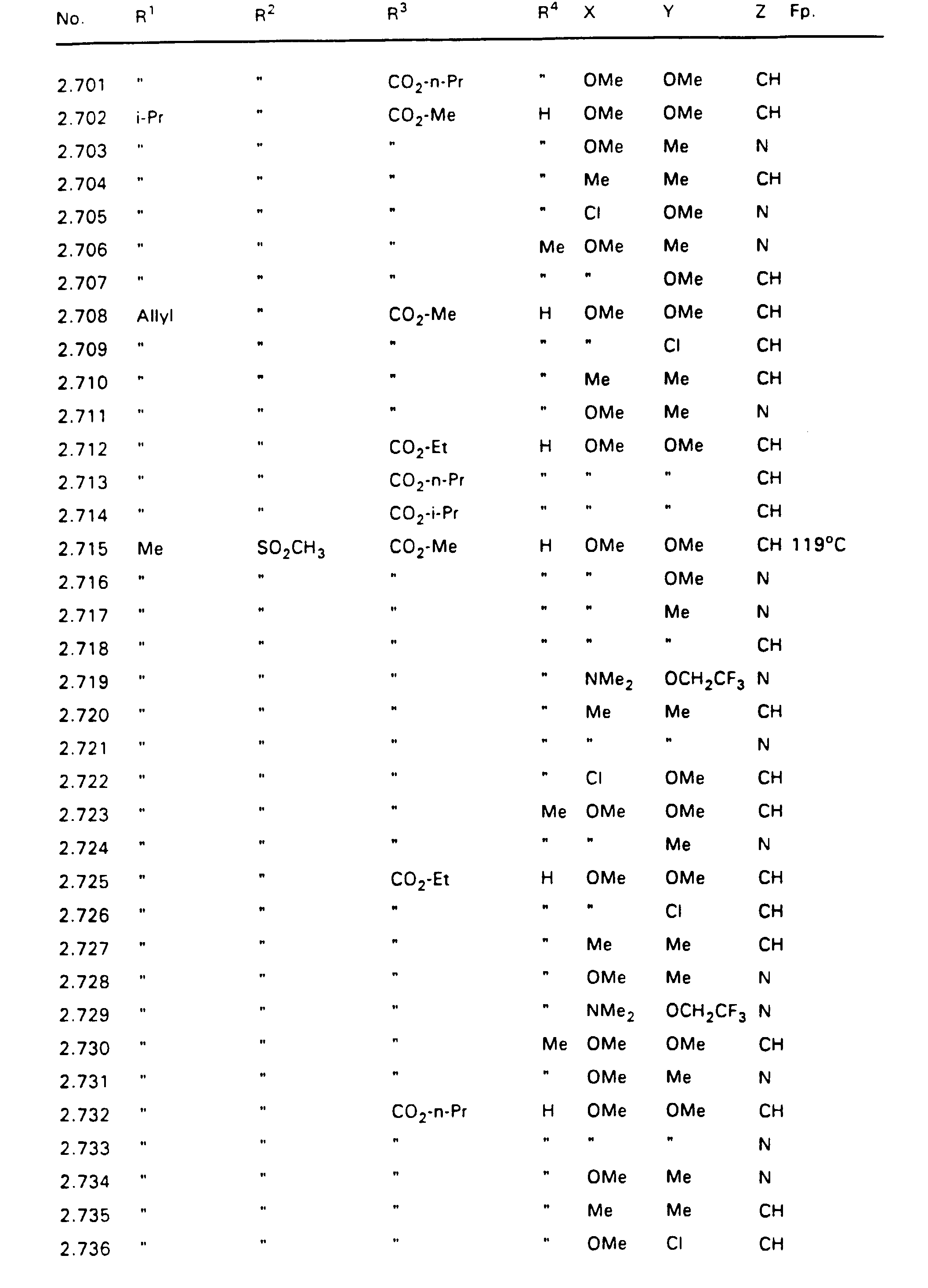

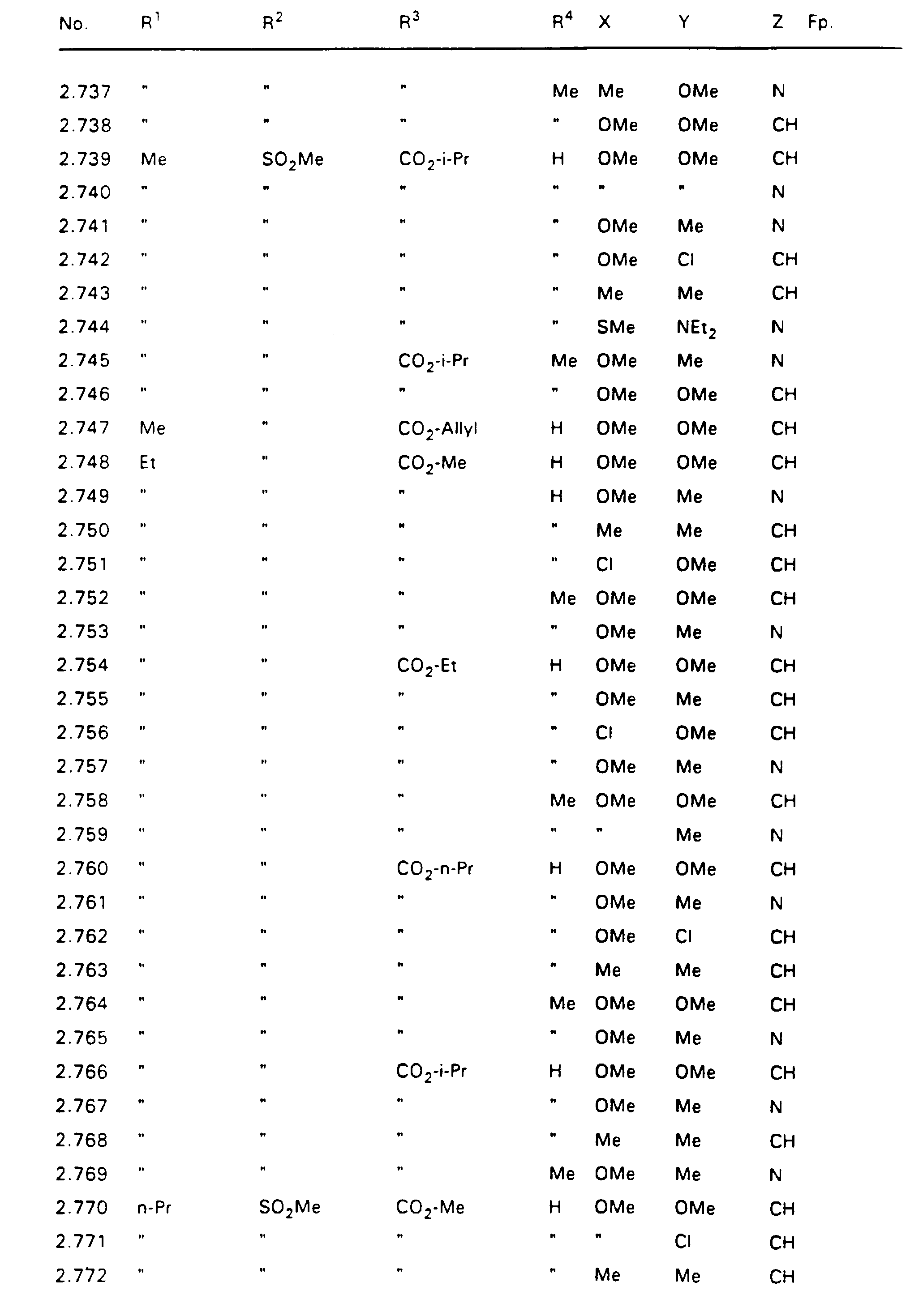

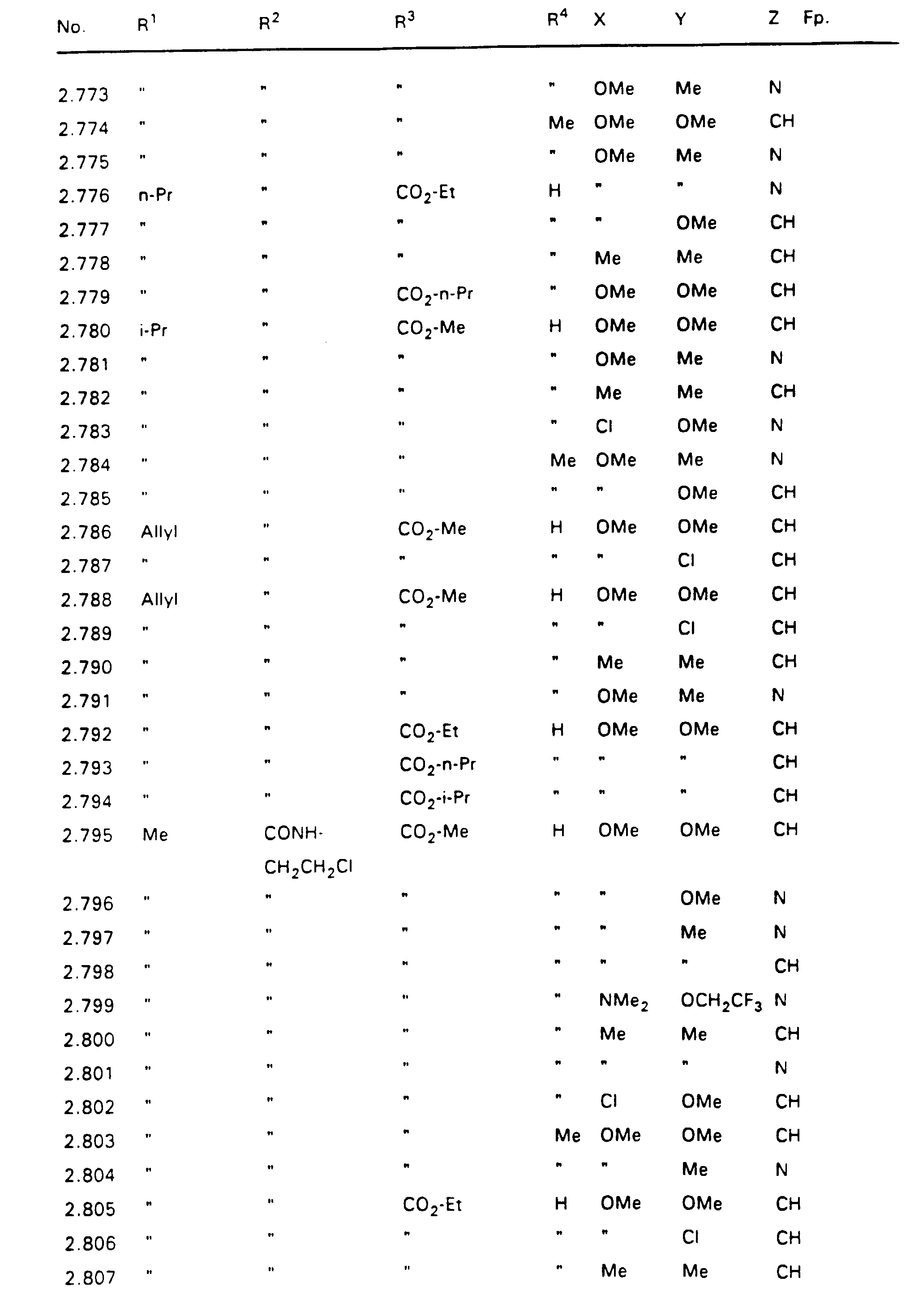

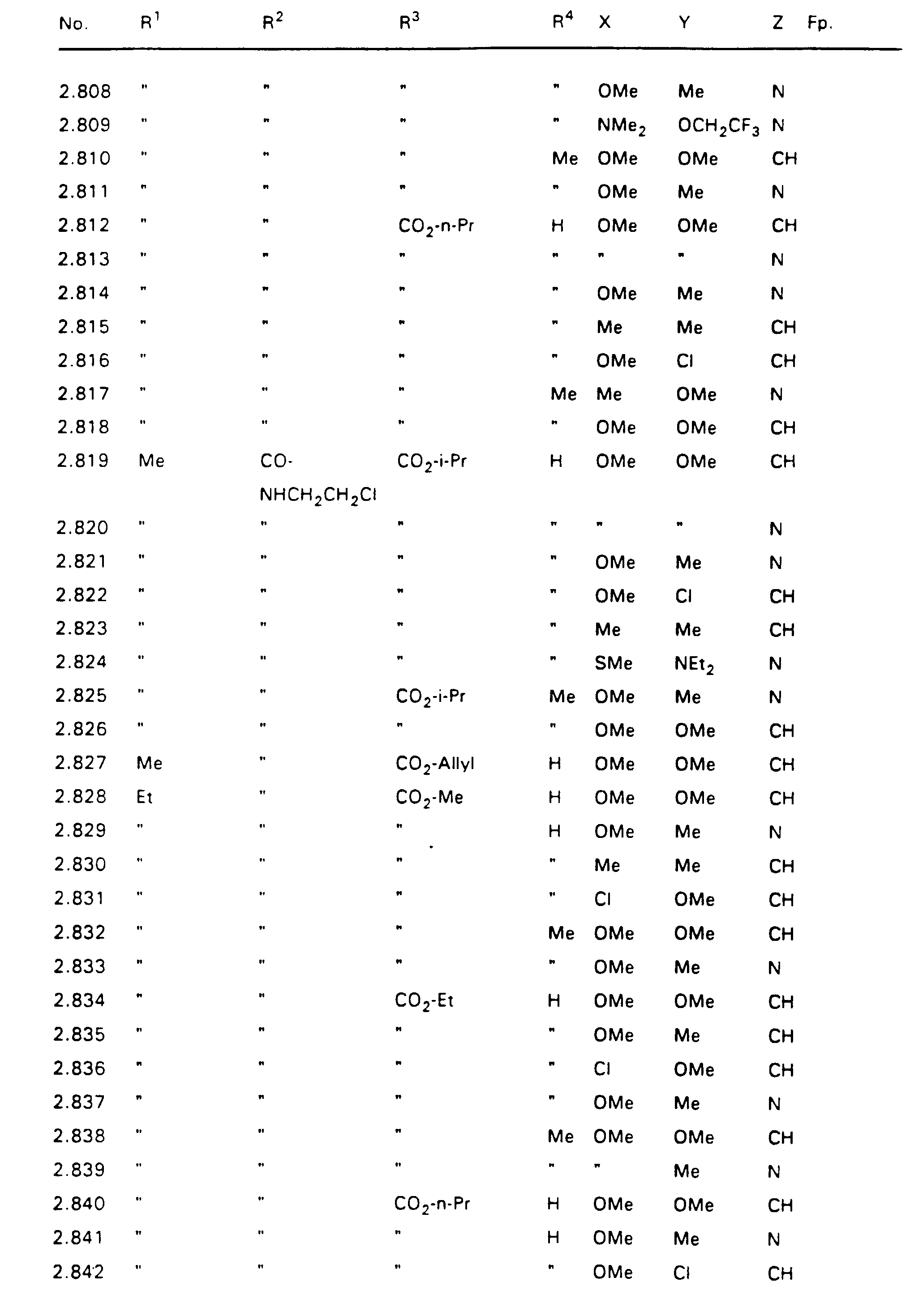

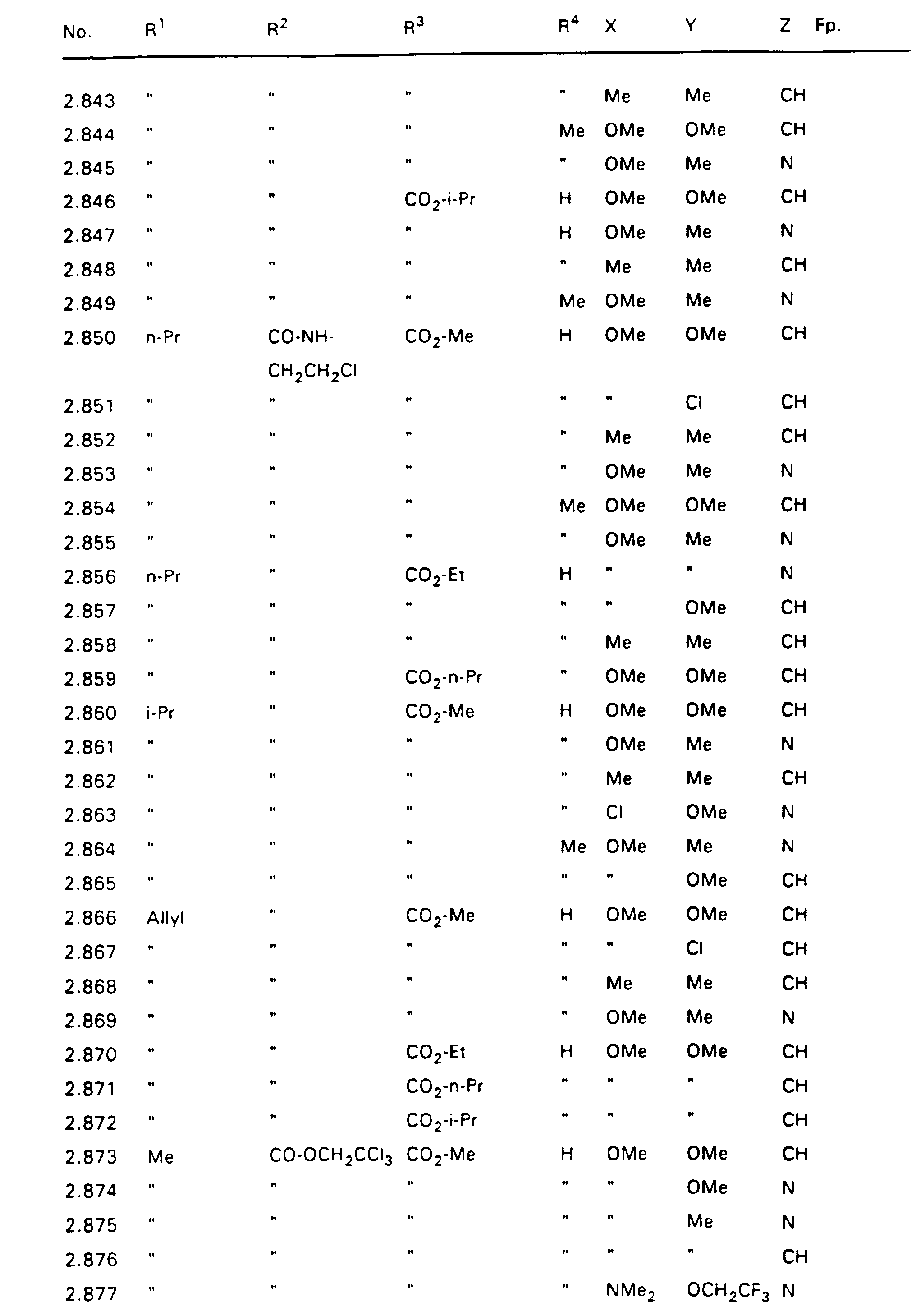

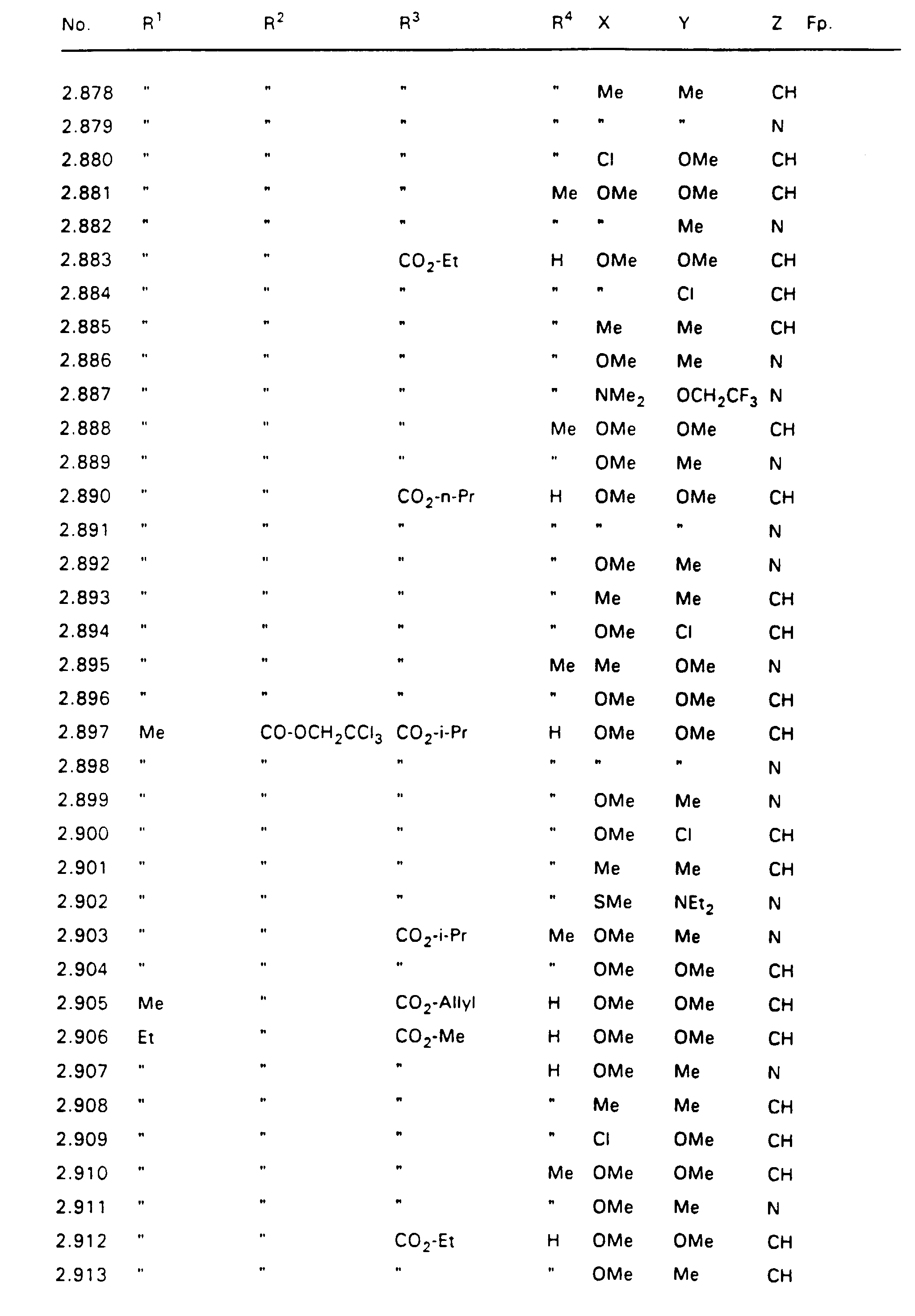

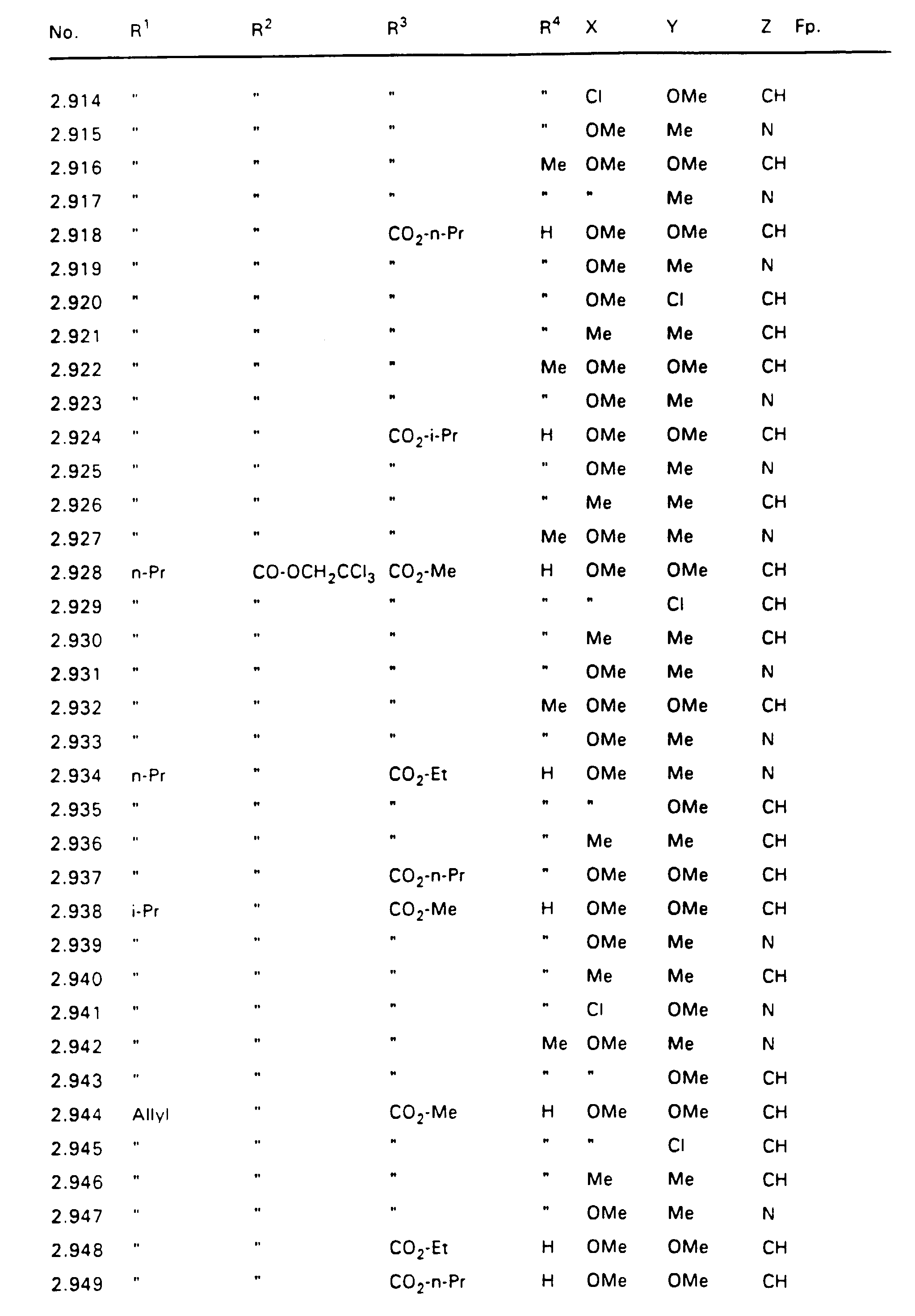

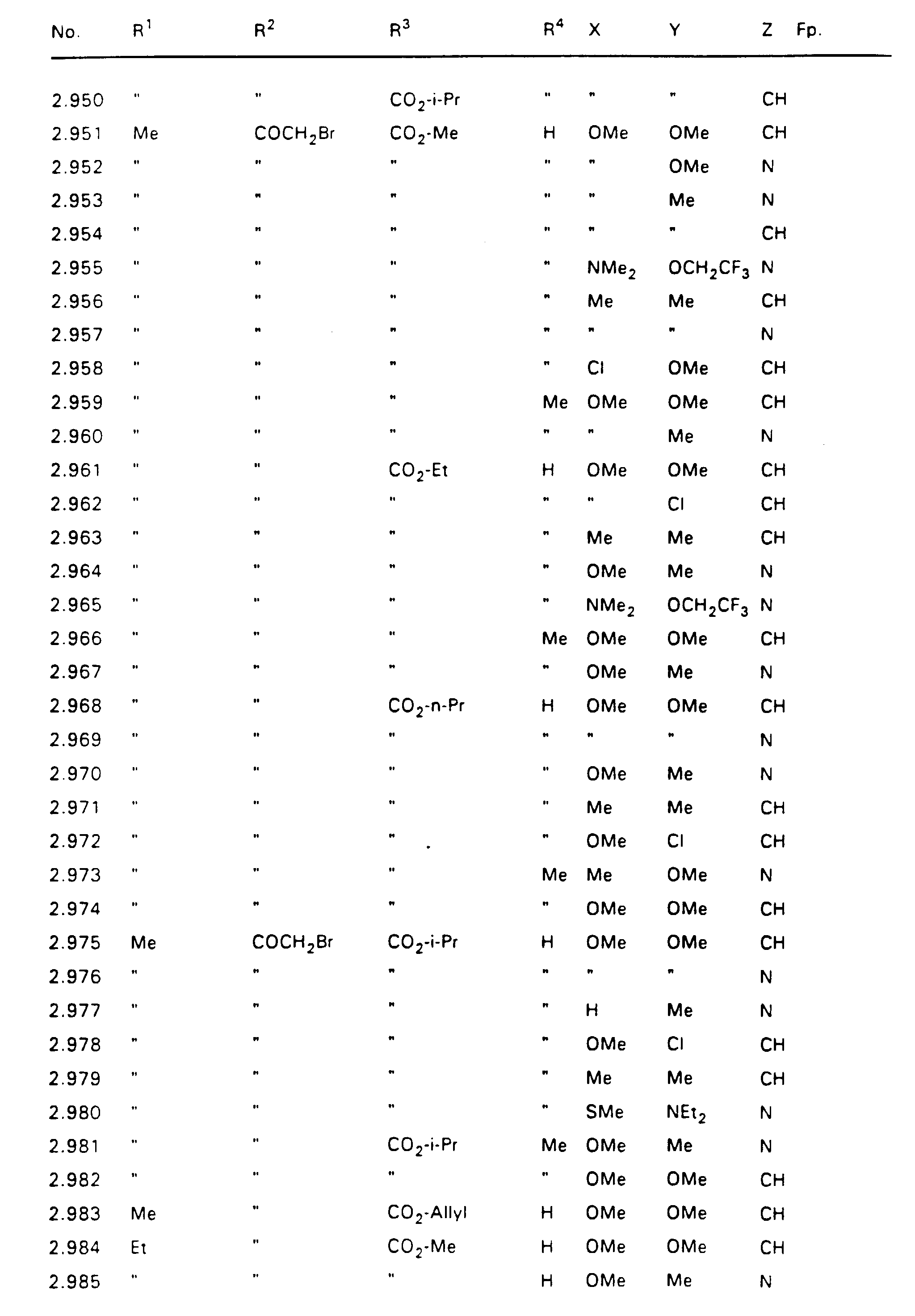

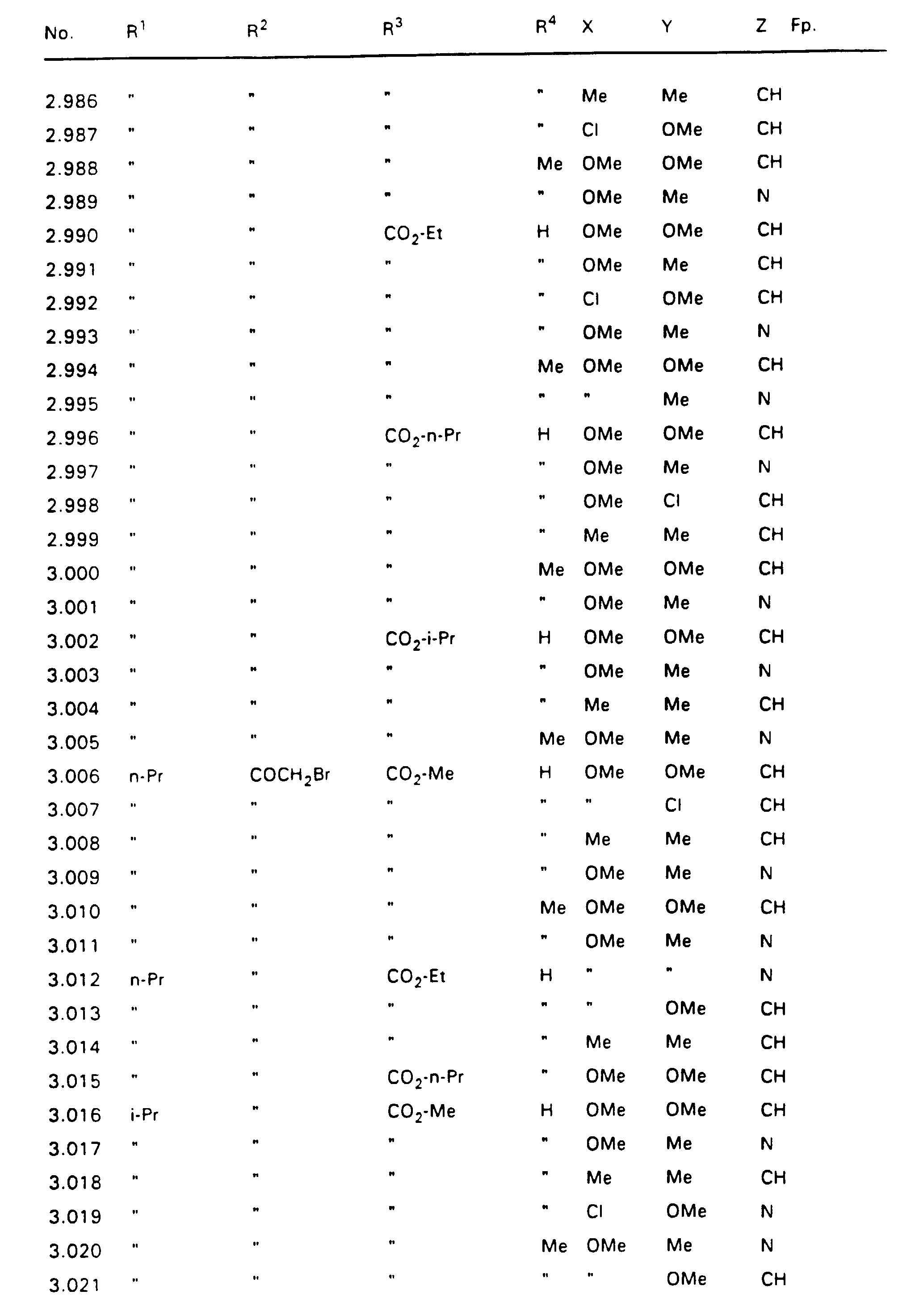

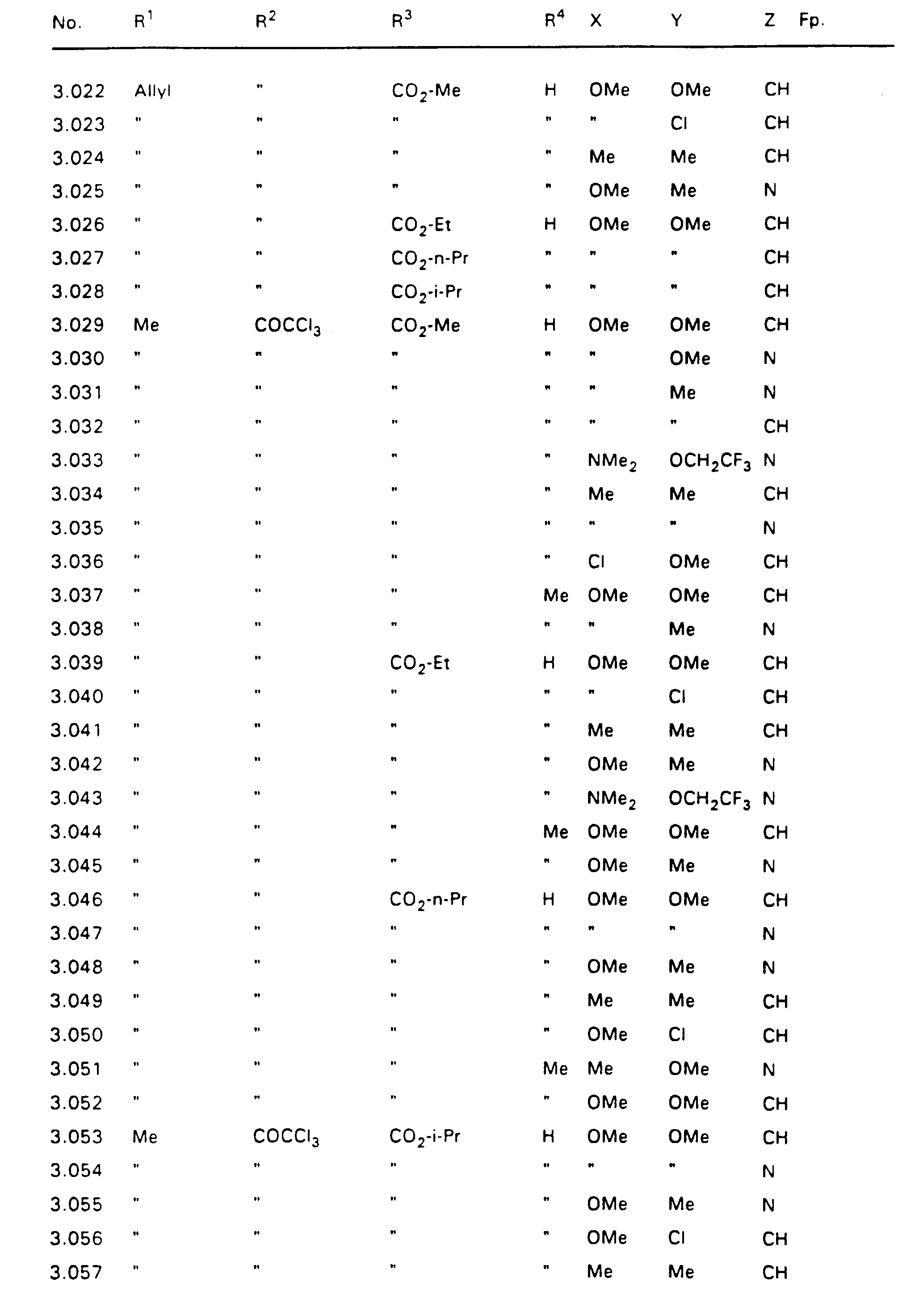

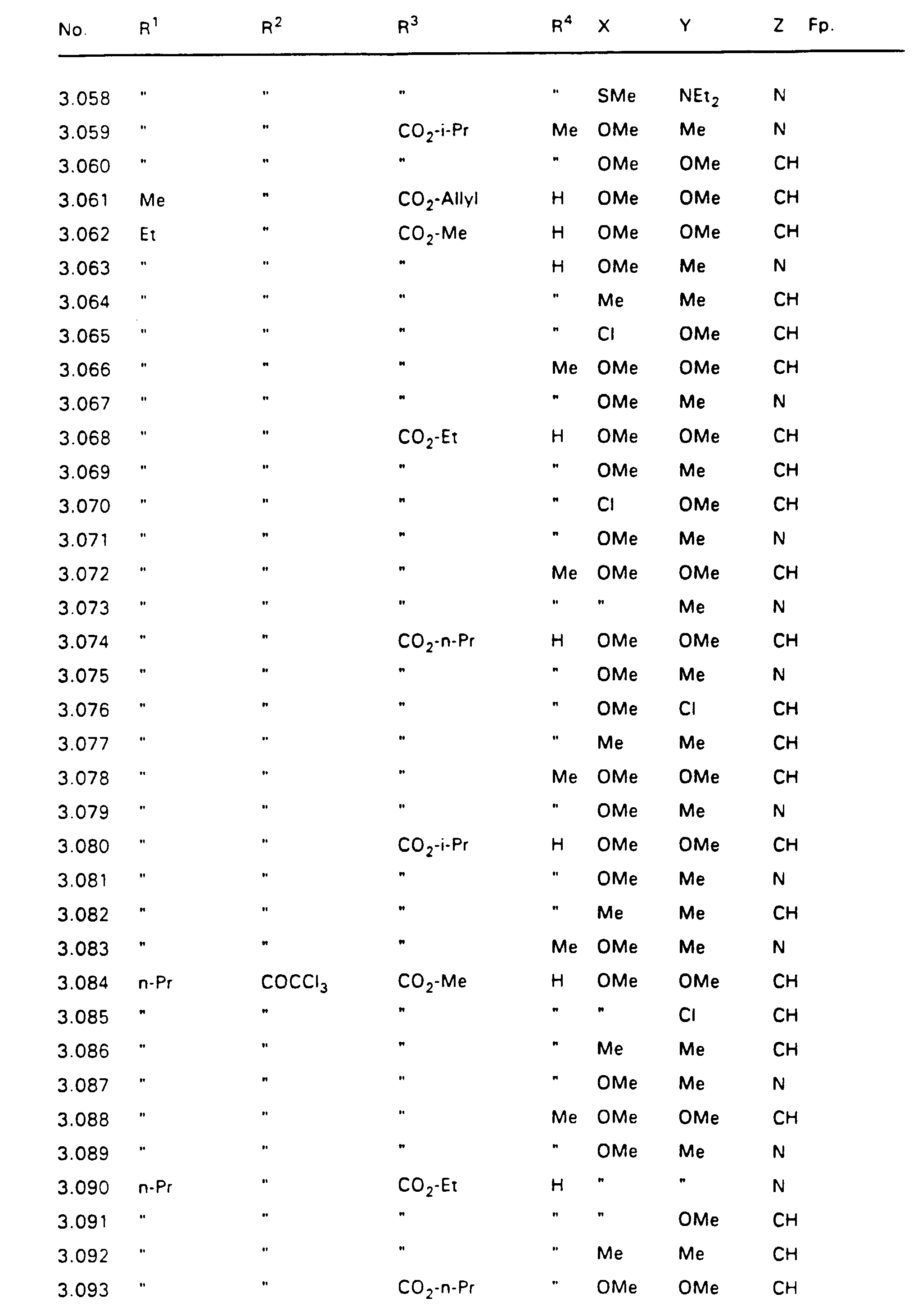

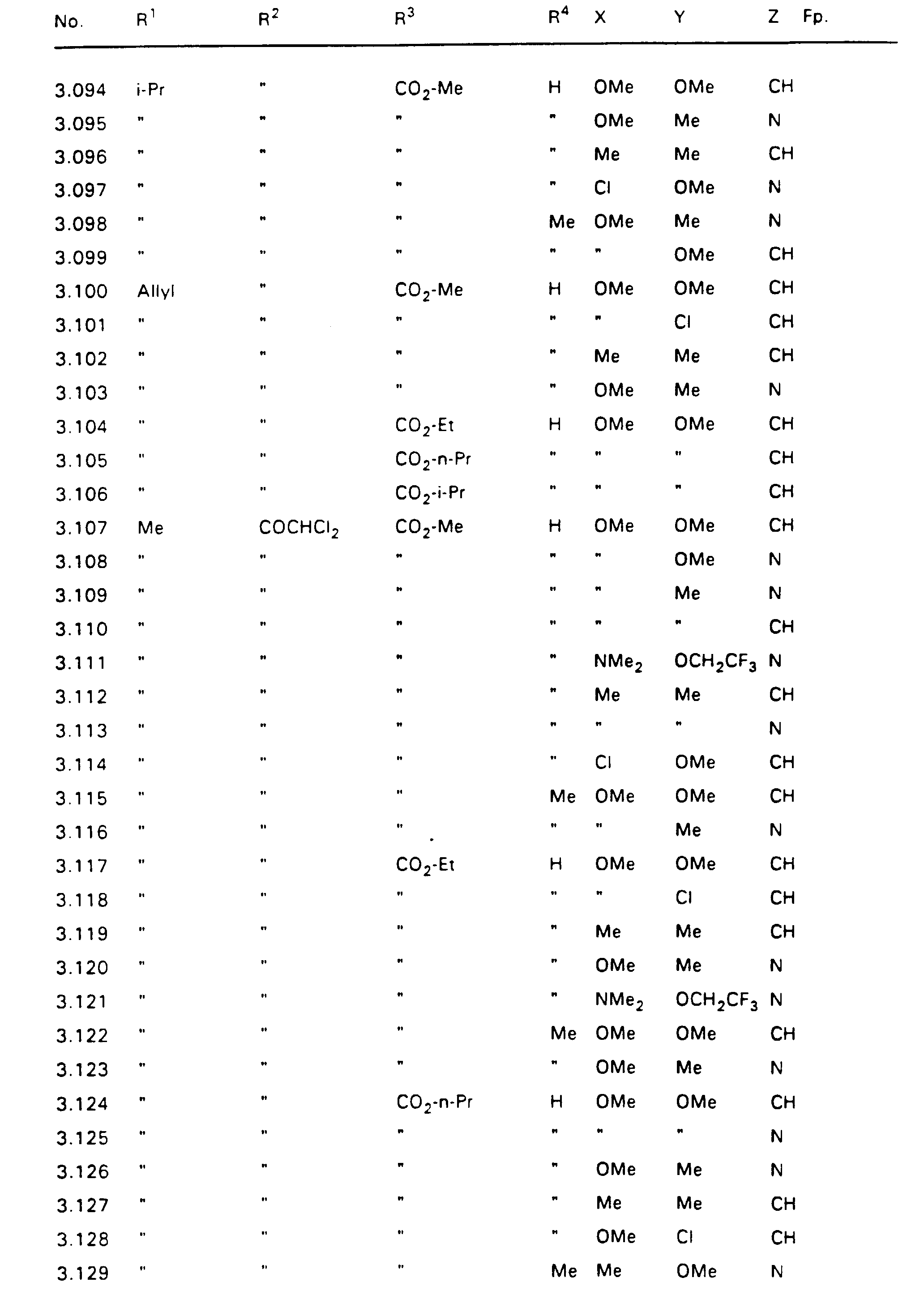

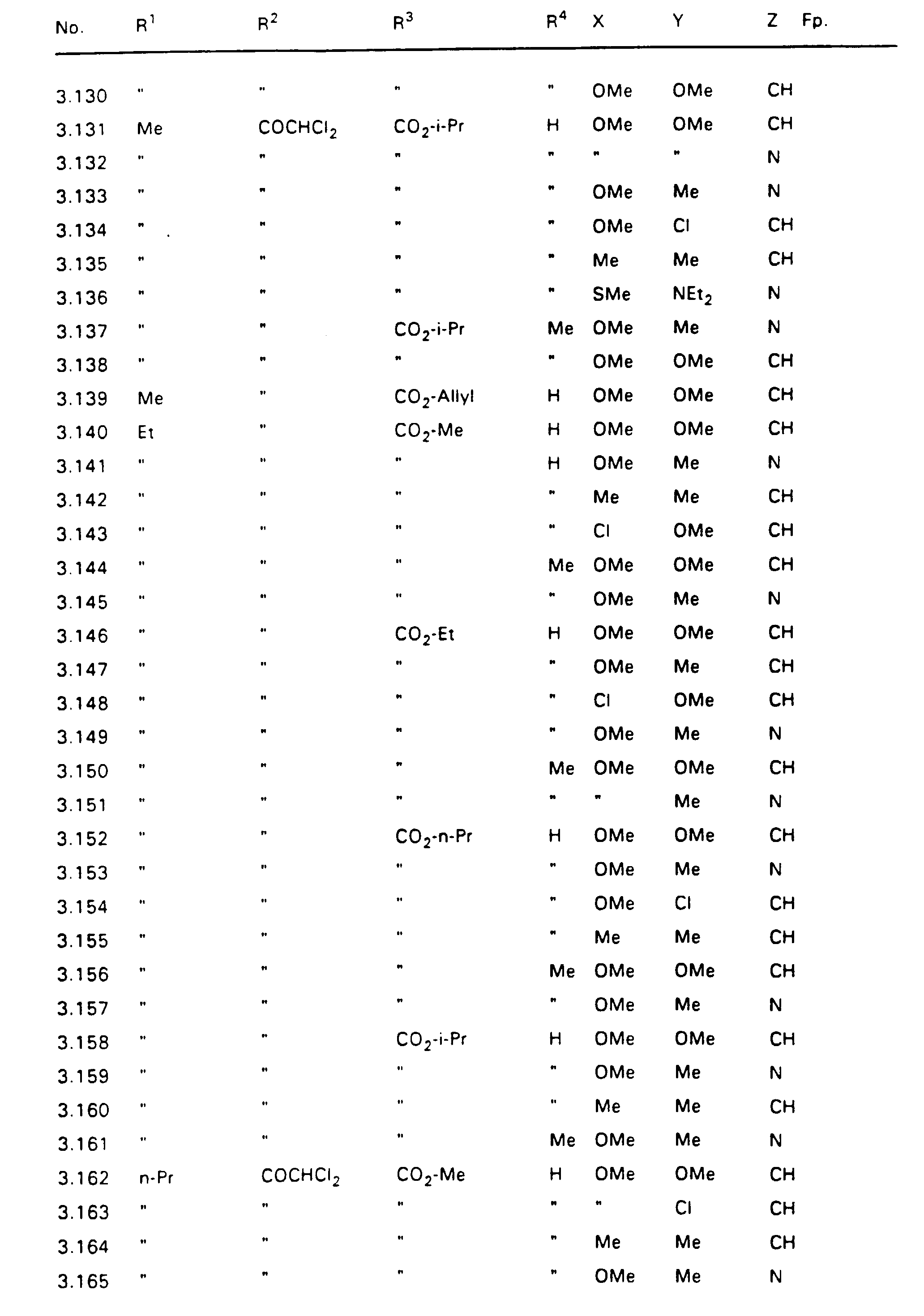

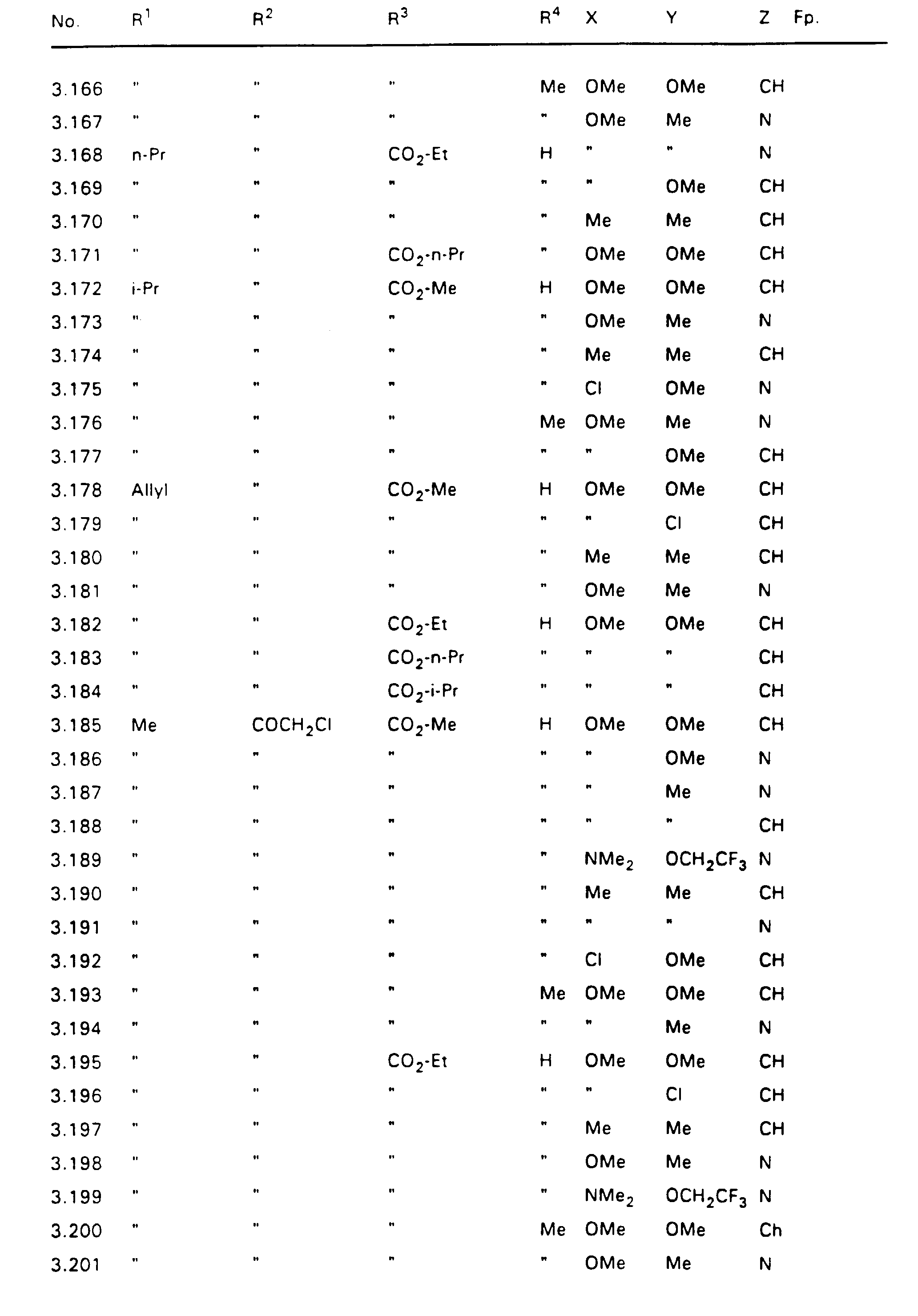

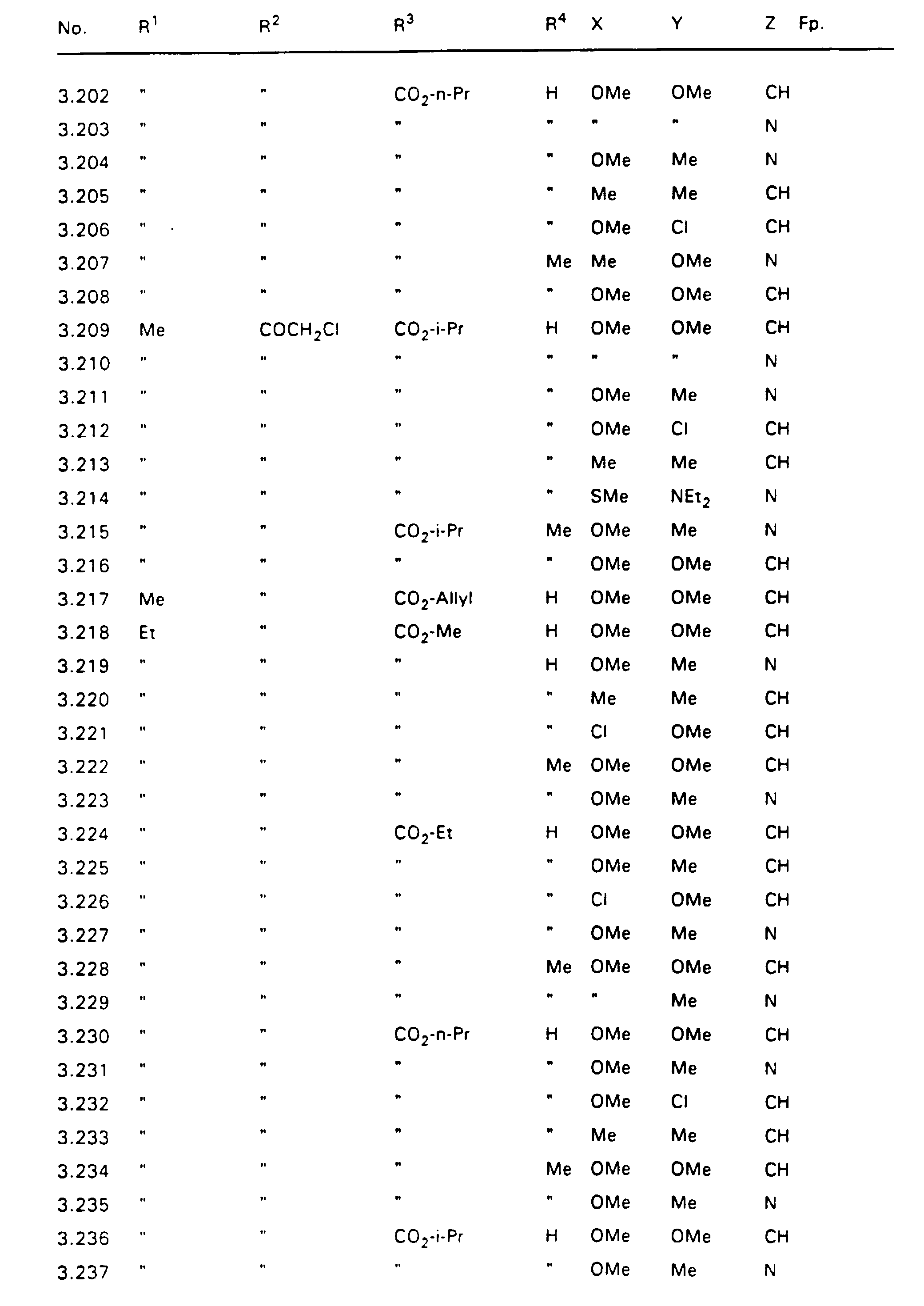

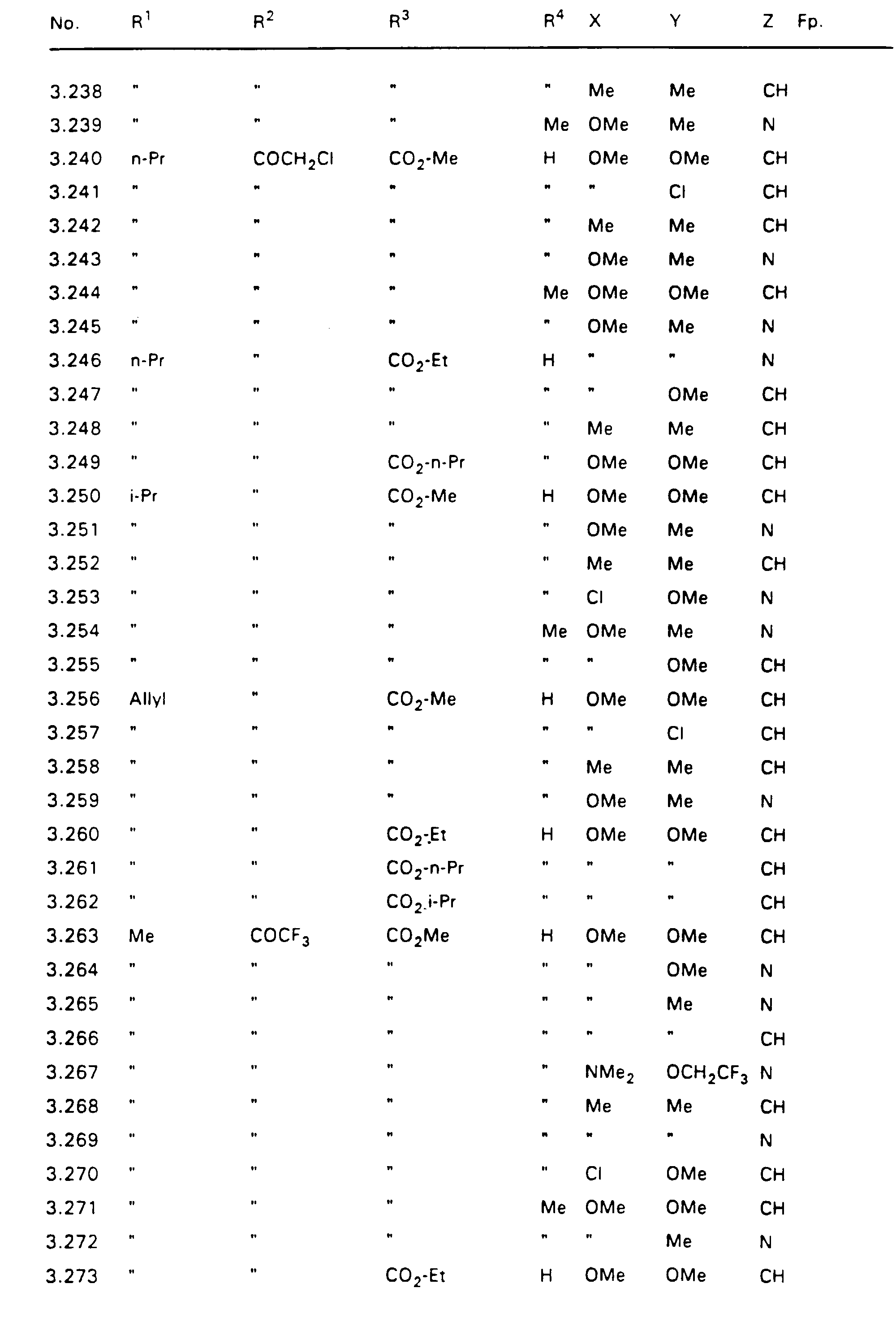

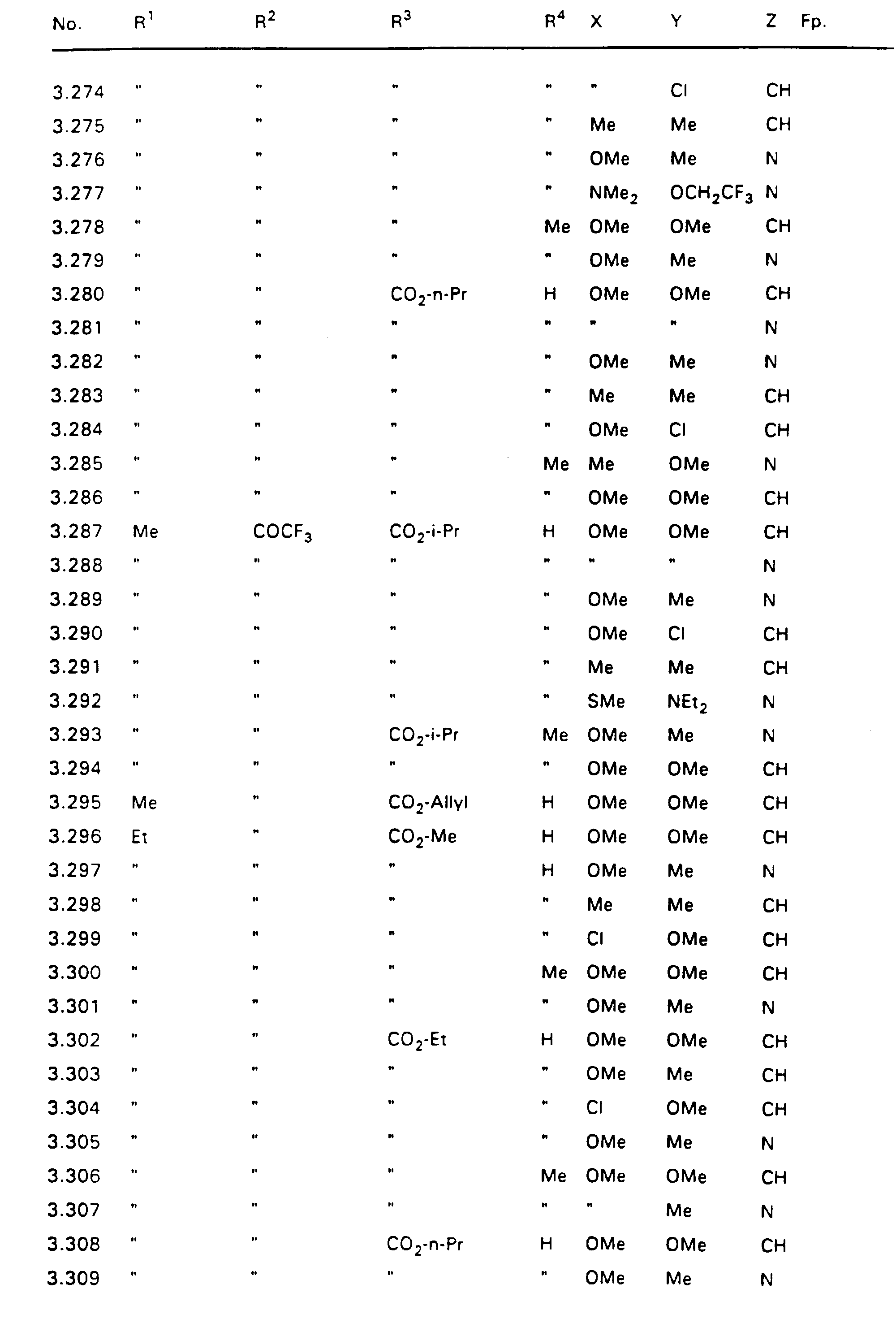

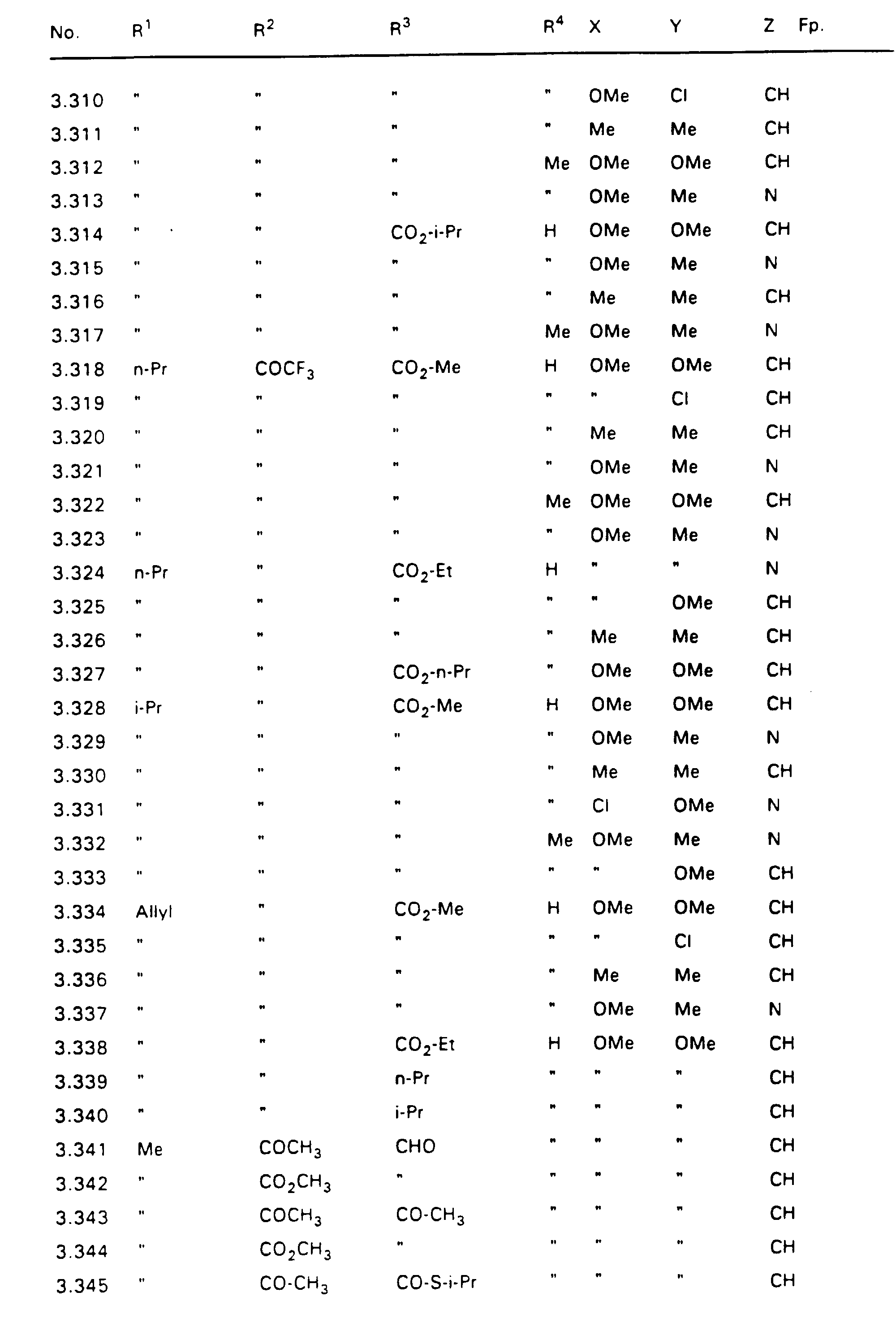

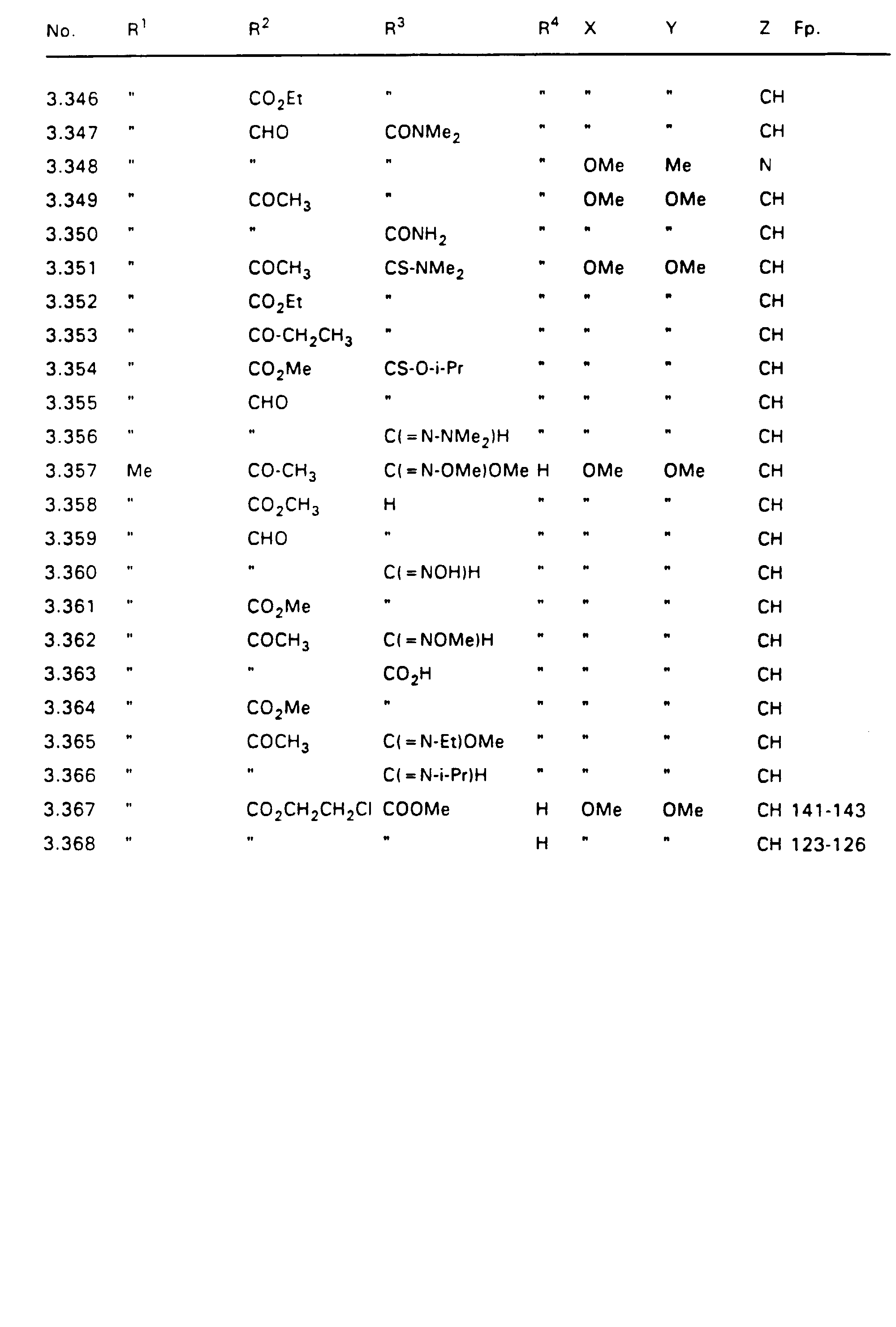

### B) Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gewichtsteilen Alkylphenolpolyglykolether (® Triton X 207), 3 Gewichtsteilen Isotridecanolpolyglykolether (8 EO) und 71 Gewichtsteilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gewichtsteilen einer Verbindung der Formel (1), 75 Gewichtsteilen Cyclohexanon als Lösungsmittel und 10 Gewichtsteilen oxethyliertes Nonylphenol als Emulgator.
e) Ein in Wasser dispergierbares Granulat wird erhalten indem man
   75 Gewichtsteile einer Verbindung der Formel (I),
   10 Gewichtsteile ligninsulfonsaures Calcium,
   5 Gewichtsteile Natriumlaurylsulfat,
   3 Gewichtsteile Polyvinylalkohol und
   7 Gewichtsteile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gewichtsteile einer Verbindung der Formel (I),
   5 Gewichtsteile 2,2'-dinaphthylmethan-6,6'-disulfonsaures Natrium
   2 Gewichtsteile oleoylmethyltaurinsaures Natrium,
   1 Gewichtsteil Polyvinylalkohol,
   17 Gewichtsteile Calciumcarbonat und
   50 Gewichtsteile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C) Biologische Beispiele

### 1. Unkrautwirkung im Vorauflauf

Samen bzw. Rhizomstücke von mono- und dikotylen Unkraufpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspension bzw. Emulsion mit einer Wasseraufwandmenge von umgerechnet 600 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 3 bis 4 Wochen im Vergleich zu unbehandelten Kontrollen. Wie die Testergebnisse zeigen, weisen die erfindungsgemäßen Verbindungen eine gute herbizide Vorauflaufwirksamkeit gegen ein breites Spektrum von Ungräsern und Unkräutern auf. Beispielsweise haben die Verbindungen der Beispiele 89, 477, 1.178, 1.180, 1.182, 1.183, 1.185, 1.186, 2.192, 2.270, 2.715, 3.367 und 3.386 aus Tabelle 1 sehr gute herbizide Wirkung gegen Schadpflanzen wie Sinapis alba, Chrysanthemum segetum, Avena sativa, Stellaria media, Echinochloa crus-galli und Lolium multiflorum im Vorauflaufverfahren bei einer Aufwandmenge von 0,3 kg und weniger Aktivsubstanz pro Hektar.

### 2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise haben die Verbindungen der Beispiele 89, 477, 1.178, 1.180, 1.182, 1.183, 1.185, 1.186, 2.192, 2.270, 2.715, 3.367 und 3.368 aus Tabelle 1 sehr gute herbizide Wirkung gegen Schadpflanzen wie Sinapis alba, Stellaria media, Echinochloa crus-galli, Lolium multiflorum, Chrysanthemum segetum und Avena sativa im Nachauflaufverfahren bei einer Aufwandmenge von 0,3 kg und weniger Aktivsubstanz pro Hektar.

### 3. Kulturptlanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt. Ein Teil der Töpfe wurde sofort wie unter Abschnitt 1 beschrieben behandelt, die übrigen im Gewächshaus aufgestellt, bis die Pflanzen zwei bis drei echte Blätter entwickelt hatten und dann wie unter Abschnitt 2 beschrieben mit den erfindungsgemäßen Substanzen der Formel (1) in unterschiedlichen Dosierungen besprüht. Vier bis fünf Wochen nach der Applikation und Standzeit im Gewächshaus wurde mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten besonders gut Gramineen-Kulturen wie z.B. Gerste, Weizen, Roggen, Sorghum-Hirsen, Mais oder Reis.

Die Verbindungen der Formel (I) weisen somit eine hohe Selektivität bei Anwendung zur Bekämpfung von unerwünschtem Pflanzenwuchs in landwirtschaftlichen Kulturen auf.

## Patentansprüche

1. Verbindungen der Formel (I) oder deren Salze, 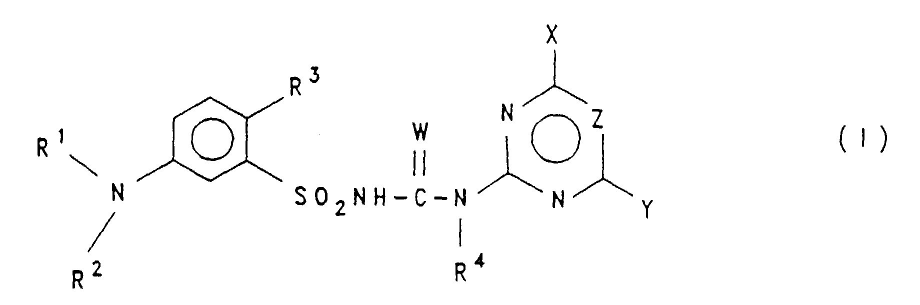 worin
R¹ C₁-C₆-Alkyl, C₂-C₆-Alkenyl, C₂-C₆-Alkinyl, wobei die 3 letztgenannten Reste unabhängig voneinander unsubstituiert oder durch einen oder mehrere Reste aus der Gruppe Halogen, C₁-C₄-Alkoxy, CONH₂, C₁-C₄-Alkylthio, CN, CHO, (C₁-C₆-Alkyl)-carbonyl, (C₃-C₆-Cycloalkyl)-carbonyl, C₁-C₄-Alkylsulfonyl, Carboxy, (C₁-C₄-Alkoxy)-carbonyl, (C₂-C₄-Alkenyloxy)-carbonyl, (C₂-C₄-Alkinyloxy)-carbonyl, NO₂, NH₂, Mono- und Di-(C₁-C₆)-Alkylamino substituiert sind,
R² CO-R⁵, COO-R⁶, CO-SR⁷, CO-NR⁸R⁹, CS-NR¹⁰R¹¹, CS-OR¹², CS-SR¹³, SO₂R¹⁴, SO₂NR¹⁵R¹⁶,
R³ COR¹⁷, CO-OR¹⁸, CO-NR¹⁹R²⁰, CO-SR²¹, COO-N =CR²²R²³, CSR²⁴, CSSR²⁵, CS-OR²⁶, CS-NR²⁷R²⁸, C(=NR²⁹)R³⁰,
R⁴ H, C₁-C₆-Alkyl, C₂-C₆-Alkenyl, C₂-C₆-Alkinyl, wobei die 3 letztgenannten Reste unabhängig voneinander unsubstituiert oder durch einen oder mehrere Reste aus der Gruppe Halogen substituiert sind,
R⁵ H, C₁-C₆-Alkyl, C₂-C₆-Alkenyl, C₂-C₆-Alkinyl, wobei die drei letztgenannten Reste unabhängig voneinander unsubstituiert oder durch einen oder mehrere Reste aus der Gruppe Halogen, C₁-C₄-Alkoxy, C₁-C₄-Alkylthio und NR³¹R³² substituiert sind, oder unsubstituiertes oder substituiertes C₃-C₈-Cycloalkyl, unsubstituiertes oder substituiertes Phenyl, unsubstituiertes oder substituiertes Heteroaryl oder Phenyl-C₁-C₄-alkyl, das am Phenylring unsubstituiert oder substituiert ist,
R⁶ C₁-C₆-Alkyl, C₂-C₅-Alkenyl, C₂-C₆-Alkinyl, wobei die drei letztgenannten Reste unabhängig voneinander unsubstituiert oder durch einen oder mehrere Reste aus der Gruppe Halogen, C₁-C₄-Alkoxy, C₁-C₄-Alkylthio und NR³¹R³² substituiert sind, oder C₃-C₆-Cycloalkyl, das unsubstituiert oder durch einen oder mehrere Reste aus der Gruppe Halogen, C₁-C₄-Alkyl und C₁-C₄-Alkoxy substituiert ist, oder C₃-C₆-Cycloalkyl-C₁-C₃-alkyl,
R⁷ analog R⁶,
R⁸ H, C₁-C₆-Alkyl, C₂-C₆-Alkenyl, C₂-C₆-Alkinyl, wobei die 3 letztgenannten Reste unabhängig voneinander unsubstituiert oder durch einen oder mehrere Reste aus der Gruppe Halogen substituiert sind, oder (C₁-C₆-Alkoxy)-carbonyl oder C₁-C₄-Alkoxy,
R⁹ H, C₁-C₆-Alkyl, C₂-C₆-Alkenyl, C₂-C₆-Alkinyl, wobei die 3 letztgenannten Reste unabhängig voneinander unsubstituiert oder durch ein oder mehrere Reste aus der Gruppe Halogen, C₁-C₄-Alkoxy, C₁-C₄-Alkylthio und NR³¹R³² substituiert sind, oder CO-R³³, CO-OR³⁴, CO-NR³⁵R³⁶, CS-NR³⁵R³⁶, CS-R³³ oder CS-OR³⁴ oder
R⁸, R⁹ zusammengenommen einen bivalenten Rest der Formel -(CH₂)₄-, -(CH₂)₅-, -CH₂CH₂-O-CH₂CH₂-, -CH₂CH₂NHCH₂CH₂-, wobei die letztgenannten 4 Reste unsubstituiert oder durch C₁-C₄-Alkyl substituiert sind,
R¹⁰ analog R⁸,
R¹¹ analog R⁹,
R¹² analog R⁶,
R¹³ analog R⁶,
R¹⁴ C₁-C₆-Alkyl, C₂-C₆-Alkenyl, C₂-C₆-Alkinyl, wobei die drei letztgenannten Reste unabhängig voneinander unsubstituiert oder durch einen oder mehrere Reste aus der Gruppe Halogen, C₁-C₄-Alkoxy, C₁-C₄-Alkylthio und NR³¹R³² substituiert sind,
R¹⁵ analog R⁸,
R¹⁶ H, C₁-C₆-Alkyl, C₂-C₆-Alkenyl, C₂-C₆-Alkinyl, wobei die 3 letztgenannten Reste unabhängig voneinander unsubstituiert oder durch einen oder mehrere Reste aus der Gruppe Halogen substituiert sind,
R¹⁷ analog R⁵,
R¹⁸ C₁-C₆-Alkyl, C₂-C₆-Alkenyl, C₂-C₆-Alkinyl, wobei die drei letztgenannten Reste unabhängig voneinander unsubstituiert oder durch einen oder mehrere Reste aus der Gruppe Halogen, C₁-C₄-Alkoxy, C₁-C₄-Alkylthio und NR³¹R³² substituiert sind, oder C₃-C₆-Cycloalkyl, das unsubstituiert oder durch einen oder mehrere Reste aus der Gruppe Halogen, C₁-C₄-Alkyl und C₁-C₄-Alkoxy substituiert ist, oder C₃-C₆-Cycloalkyl-C₁-C₃-alkyl oder H,
R¹⁹ analog R⁸,
R²⁰ analog R⁹,
R²¹ analog R¹⁸,
R²² H, C₁-C₄-Alkyl, C₃-C₅-Alkenyl oder C₃-C₅-Alkinyl,
R²³ H, C₁-C₄-Alkyl, C₃-C₅-Alkenyl oder C₃-C₅-Alkinyl,
R²⁴ analog R¹⁷,
R²⁵ analog R¹⁸,
R²⁶ analog R¹⁸,
R²⁷ analog R⁸,
R²⁸ analog R⁹,
R²⁹ H, OH, NH₂, NHR³⁷, N(R³⁷)₂, C₁-C₄-Alkyl, C₁-C₄-Alkoxy, C₂-C₄-Alkenyl oder C₂-C₄-Alkinyl, wobei die letztgenannten 4 Reste unabhängig voneinander unsubstituiert oder durch einen oder mehrere Reste aus der Gruppe Halogen, C₁-C₃-Alkoxy und C₁-C₃-Alkylthio substituiert sind,
R³⁰ H, C₁-C₆-Alkyl, C₁-C₆-Alkoxy, C₂-C₆-Alkenyl, C₂-C₆-Alkinyl, wobei die letztgenannten vier Reste unabhängig voneinander unsubstituiert oder durch einen oder mehrere Reste aus der Gruppe Halogen, C₁-C₃-Alkoxy und C₁-C₃-Alkylthio substituiert sind,
die R³¹ unabhängig voneinander H, C₁-C₄-Alkyl, (C₁-C₄-Alkyl)-carbonyl, (C₁-C₄-Alkoxy)-carbonyl oder CHO,
die R³² unabhängig voneinander H oder C₁-C₄-Alkyl,
R³³ analog R⁵,
R³⁴ analog R⁶,
R³⁵, R³⁶ unabhängig voneinander H, C₁-C₆-Alkyl, C₂-C₆-Alkenyl, C₂-C₆-Alkinyl, wobei die 3 letztgenannten Reste unabhängig voneinander unsubstituiert oder durch einen oder mehrere Reste aus der Gruppe Halogen substituiert sind,
R³⁷ C₁-C₄-Alkyl, C₂-C₄-Alkenyl oder C₂-C₄-Alkinyl, wobei die drei genannten Reste unabhängig voneinander unsubstituiert oder durch einen oder mehrere Reste aus der Gruppe Halogen, C₁-C₄-Alkoxy und C₁-C₄-Alkylthio substituiert sind,
W O oder S,
X, Y unabhängig voneinander Wasserstoff, Halogen, C₁-C₆-Alkyl, C₁-C₆-Alkoxy oder C₁-C₆-Alkylthio, wobei die letztgenannten drei Reste unsubstituiert oder durch einen oder mehrere Reste aus der Gruppe Halogen, C₁-C₄-Alkoxy und C₁-C₄-Alkylthio substituiert sind, oder Mono- oder Di-(C₁-C₄-alkyl)amino, C₃-C₆-Cycloalkyl, C₃-C₅-Alkenyl, C₃-C₅-Alkenyloxy oder C₃-C₅-Alkinyloxy und
Z CH oder N
bedeuten.

2. Verbindungen oder deren Salze gemäß Anspruch 1, dadurch gekenzeichnet, daß
R¹ C₁-C₄-Alkyl, C₂-C₄-Alkenyl oder C₂-C₄-Alkinyl, oder
R² CO-R⁵, COOR⁶, CO-NR⁸R⁹, CS-N¹⁰R¹¹, SO₂R¹⁴ oder SO₂NR¹⁵R¹⁶ oder
R³ COR¹⁷, COOR¹⁸, CONR¹⁹R²⁰ oder CO-ON=CR²²R²³, oder
R⁴ H oder C₁-C₄-Alkyl, oder
R⁵ H, C₁-C₆-Alkyl, das unsubstituiert oder durch einen oder mehrere Reste aus der Gruppe Halogen oder durch C₁-C₄-Alkoxy, C₁-C₄-Alkylthio oder NR³¹R³² substituiert ist, oder C₃-C₆-Cycloalkyl, Phenyl, Benzyl oder Heteroaryl, wobei die letztgenannten drei Reste unsubstituiert oder durch einen oder mehrere Reste aus der Gruppe C₁-C₄-Alkyl, C₁-C₄-Alkoxy und Halogen substituiert sind, oder
R⁶ C₁-C₆-Alkyl, C₂-C₆-Alkenyl, C₂-C₆-Alkinyl, C₁-C₆-Haloalkyl oder C₃-C₆-Cycloalkyl, oder
R⁷ C₁-C₄-Alkyl, oder
R⁸ Wasserstoff, C₁-C₆-Alkyl, C₁-C₄-Haloalkyl, C₁-C₄-Alkoxy oder (C₁-C₄-Alkoxy)-carbonyl, oder
R⁹ Wasserstoff, C₁-C₆-Alkyl, das unsubstituiert oder durch einen oder mehrere Reste aus der Gruppe Halogen, C₁-C₄-Alkoxy und NR³¹R³² substituiert ist, oder CO-R³³, CO-OR³⁴ oder CO-NR³⁵R³⁶ oder
R⁸ und R⁹ zusammengenommen einen bivalenten Rest der Formel -(CH₂)₄-, -(CH₂)₅- oder -CH₂CH₂-O-CH₂CH₂- oder
R¹⁴ C₁-C₆-Alkyl, C₁-C₆-Haloalkyl, oder
R¹⁵, R¹⁶ unabhängig voneinander Wasserstoff oder C₁-C₄-Alkyl oder
R¹⁷ Wasserstoff oder C₁-C₄-Alkyl, C₁-C₄-Haloalkyl, C₃-C₆-Cycloalkyl, Phenyl oder Heteroaryl, wobei die letztgenannten zwei Reste unsubstituiert oder substituiert sind, oder
R¹⁸ Wasserstoff, C₁-C₄-Alkyl, C₂-C₆-Alkenyl oder C₂-C₆-Alkinyl, wobei die letztgenannten 3 Reste unsubstituiert oder durch einen oder mehrere Reste aus der Gruppe Halogen, C₁-C₄-Alkoxy, C₁-C₄-Alkylthio und NR³¹R³² substituiert sind, oder C₃-C₆-Cycloalkyl oder C₃-C₆-Cycloalkyl-C₁-C₃-alkyl oder
R²² Wasserstoff oder C₁-C₂-Alkyl oder
R²³ Wasserstoff oder C₁-C₂-Alkyl oder
R²⁹ Wasserstoff, Hydroxy, Amino, NHCH₃, N(CH₃)₂, C₁-C₄-Alkyl oder C₁-C₄-Alkoxy, oder
R³⁰ Wasserstoff oder C₁-C₄-Alkyl oder
die R³¹ unabhängig voneinander H oder C₁-C₄-Alkyl oder
die R³² unabhängig voneinander H oder C₁-C₄-Alkyl oder
R³³ Wasserstoff, C₁-C₄-Alkyl, C₁-C₄-Haloalkyl, C₃-C₆-Cycloalkyl oder Phenyl, das unsubstituiert oder durch ein oder mehrere Reste aus der Gruppe Halogen, C₁-C₄-Alkyl und C₁-C₄-Alkoxy substituiert ist, oder
R³⁴ C₁-C₄-Alkyl, Allyl, Propargyl oder Cycloalkyl oder
R³⁵ Wasserstoff oder C₁-C₄-Alkyl oder
R³⁶ Wasserstoff oder C₁-C₄-Alkyl oder
X C₁-C₄-Alkyl, C₁-C₄-Alkoxy, C₁-C₄-Haloalkyl, C₁-C₄-Alkylthio, Halogen oder Mono- oder Di-(C₁-C₂-alkyl)-amino, oder
Y C₁-C₄-Alkyl, C₁-C₄-Alkoxy, C₁-C₄-Haloalkyl oder C₁-C₄-Alkylthio,
bedeutet, oder solche Verbindungen der Formel (I) oder deren Salze, worin zwei oder mehrere der vorstehend genannten Bedeutungen für die Reste R¹ bis R³⁶ in Formel (I) kombiniert vorhanden sind.

3. Verbindungen oder deren Salze, gemäß Anspruch 1 oder 2, dadurch gekennzeichnet, daß
R¹ Methyl, Ethyl, n-Propyl, i-Propyl oder Allyl,
R² CO-R⁵, COOR⁶, CO-NR⁸R⁹, CS-NR¹⁰R¹¹, SO₂R¹⁴ oder SO₂NR¹⁵R¹⁶,
R⁵ H, C₁-C₄-Alkyl, C₁-C₂-Haloalkyl, Cyclopropyl, Phenyl, Benzyl oder Heteroaryl mit 5 oder 6 Ringatomen, wobei die letztgenannten 3 Reste unsubstituiert oder durch einen oder mehrere Halogenatome substituiert sind,
R⁶ C₁-C₄-Alkyl, Allyl, Propargyl oder Cyclopropyl,
R⁸ H, C₁-C₄-Alkyl, C₁-C₄-Haloalkyl oder (C₁-C₄-Alkoxy)-carbonyl,
R⁹ H oder C₁-C₄-Alkyl,
R¹⁰ H oder C₁-C₄-Alkyl,
R¹¹ H oder C₁-C₄-Alkyl,
R¹⁴ C₁-C₄-Alkyl,
R¹⁵ H oder C₁-C₄-Alkyl und
R¹⁶ H oder C₁-C₄-Alkyl
bedeuten.

4. Verfahren zur Herstellung der Verbindungen der Formel (I) oder deren Salze, wie sie in Anspruch 1 definiert sind, dadurch gekennzeichnet, daß man
a) eine Verbindung der Formel (II) 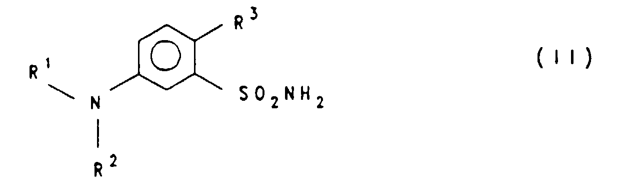 mit einem heterocyclischen Carbamat der Formel (III) 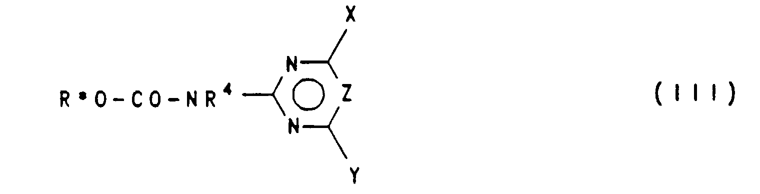 worin R* gegebenenfalls substituiertes Phenyl oder C₁-C₄-Alkyl bedeutet, umsetzt oder
b) ein Sulfonylisocyanat der Formel (IV) 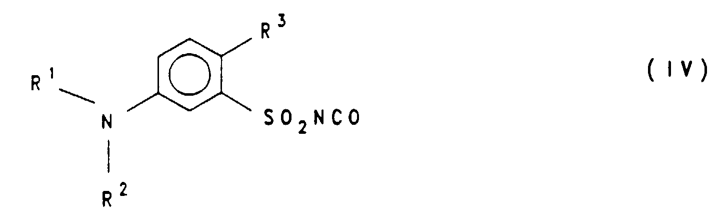 mit einem Aminoheterocyclus der Formel (V) 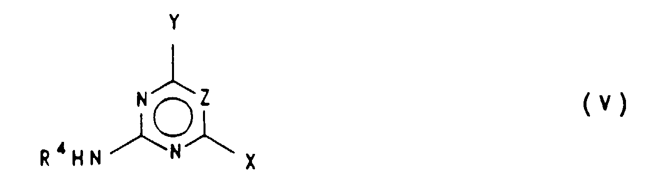 umsetzt oder
c) eine Verbindung der Formel (II) mit einem (Thio)lsocyanat der Formel (X) 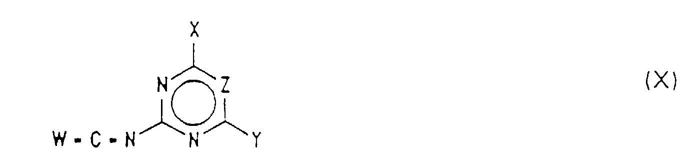
in Gegenwart einer Base umsetzt,
wobei in den obigen Formeln (II) bis (V) und (X) die Reste R¹ bis R⁴, W, X, Y und Z wie in Formel (I) definiert sind und bei den Varianten a) und b) zunächst Verbindungen der Formel (I) erhalten werden, in denen W = 0 ist.

5. Herbizide oder pflanzenwachstumsregulierende Mittel, dadurch gekennzeichnet, daß sie eine Verbindung der Formel (I) nach einem der Ansprüche 1 bis 3 und übliche Formulierungshilfsmittel enthalten.

6. Verwendung der Verbindungen der Formel (I) oder deren Salze nach einem der Ansprüche 1 bis 3 als Herbizide oder Pflanzenwachstumsregulatoren.

7. Verfahren zur Bekämpfung von Schadpfianzen oder zur Wachstumsregulierung von Pflanzen, dadurch gekennzeichnet, daß man eine wirksame Menge einer oder mehrerer Verbindungen der Formel (I) oder deren Salze nach einem der Ansprüche 1 bis 3 auf die Pflanzen, Pflanzenteile oder den landwirtschaftlich oder industriell genutzten Boden appliziert.

8. Verbindungen der Formel (II) 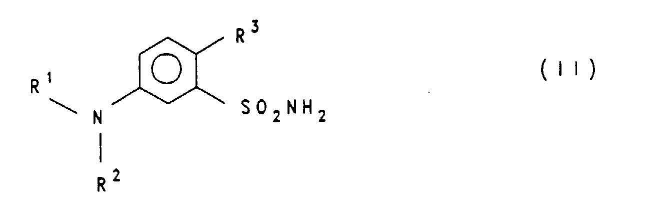 worin
R¹, R² und R³ wie in Formel (I) nach Anspruch 1, 2 oder 3 definiert sind

9. Verbindungen der Formel (IV) 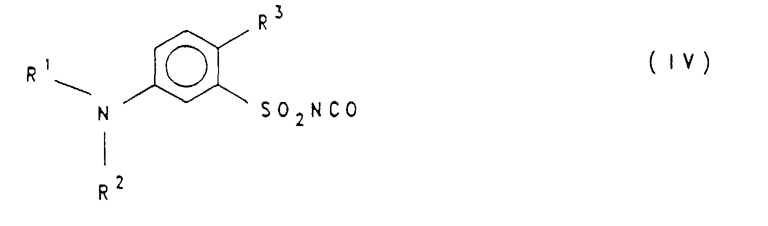 worin
R¹, R² und R³ wie in Formel (I) nach einem der Ansprüche 1, 2 oder 3 definiert sind.

## Claims

1. A compound of the formula (I) or a salt thereof 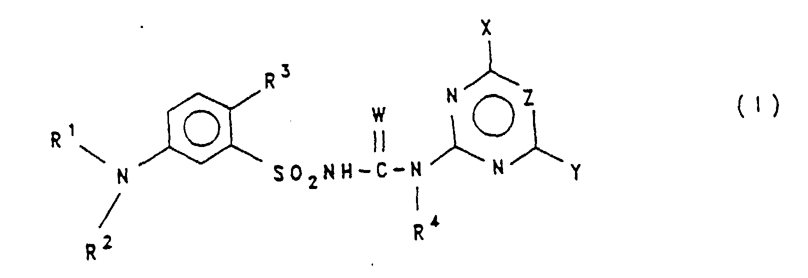 in which
R¹ is C₁-C₆-alkyl, C₂-C₆-alkenyl, C₂-C₆-alkynyl, the 3 latter radicals independently of one another being unsubstituted or substituted by one or more radicals from the group comprising halogen, C₁-C₄-alkoxy, CONH₂, C₁-C₄-alkylthio, CN, CHO, (C₁-C₆-alkyl)car-bonyl, (C₃-C₆-cycloalkyl)carbonyl, C₁-C₄-alkylsulfonyl, carboxyl, (C₁-C₄-alkoxy)-carbonyl, (C₂-C₄-alkenyloxy)carbonyl, (C₂-C₄-alkynyloxy)carbonyl, NO₂, NH₂, mono- and di-(C₁-C₆)-alkylamino,
R² is CO-R⁵, COO-R⁶, CO-SR⁷, CO-NR⁸R⁹, CS-NR¹⁰R¹¹, CS-OR¹², CS-SR¹³, SO₂R¹⁴, SO₂NR¹⁵R¹⁶,
R³ is COR¹⁷, CO-OR¹⁸, CO-NR¹⁹R²⁰, CO-SR²¹, COO-N=CR²²R²³, CSR²⁴, CSSR²⁵, CS-OR²⁶, CS-NR²⁷R²⁸, C(=NR²⁹)R³⁰,
R⁴ is H, C₁-C₆-alkyl, C₂-C₆-alkenyl, C₂-C₆-alkynyl, the 3 latter radicals independently of one another being unsubstituted or substituted by one or more halogen radicals,
R⁵ is H, C₁-C₆-alkyl, C₂-C₆-alkenyl, C₂-C₆-alkynyl, the three latter radicals independently of one another being unsubstituted or substituted by one or more radicals from the group comprising halogen, C₁-C₄-alkoxy, C₁-C₄-alkylthio and NR³¹R³², or unsubstituted or substituted C₃-C₈-cycloalkyl, unsubstituted or substituted phenyl, unsubstituted or substituted heteroaryl or phenyl-C₁-C₄-alkyl which is unsubstituted or substituted on the phenyl ring,
R⁶ is C₁-C₆-alkyl, C₂-C₆-alkenyl, C₂-C₆-alkynyl, the three latter radicals independently of one another being unsubstituted or substituted by one or more radicals from the group comprising halogen, C₁-C₄-alkoxy, C₁-C₄-alkylthio and NR³¹R³², or C₃-C₆-cycloalkyl which is unsubstituted or substituted by one or more radicals from the group comprising halogen, C₁-C₄-alkyl- and C₁-C₄-alkoxy, or C₃-C₆-cycloalkyl-C₁-C₃-alkyl,
R⁷ is analogous to R⁶
R⁸ is H, C₁-C₆-alkyl, C₂-C₆-alkenyl, C₂-C₆-alkynyl, the 3 latter radicals independently of one another being unsubstituted or substituted by one or more halogen radicals, or (C₁-C₆-alkoxy)carbonyl or C₁-C₄-alkoxy,
R⁹ is H, C₁-C₆-alkyl, C₂-C₆-alkenyl, C₂-C₆-alkynyl, the 3 latter radicals independently of one another being unsubstituted or substituted by one or more radicals from the group comprising halogen, C₁-C₄-alkoxy, C₁-C₄-alkylthio and NR³¹R³², or CO-R³³, CO-OR³⁴, CO-NR³⁵R³⁶, CS-NR³⁵R³⁶, CS-R³³ or CS-OR³⁴, or
R⁸, R⁹ taken together are a divalent radical of the formula -(CH₂)₄-, -(CH₂)₅-, -CH₂CH₂-O-CH₂CH₂-, -CH₂CH₂NHCH₂CH₂-, the latter 4 radicals being unsubstituted or substituted by C₁-C₄-alkyl,
R¹⁰ is analogous to R⁸,
R¹¹ is analogous to R⁹,
R¹² is analogous to R⁶,
R¹³ is analogous to R⁶,
R¹⁴ is C₁-C₆-alkyl, C₂-C₆-alkenyl, C₂-C₆-alkynyl, the three latter radicals independently of one another being unsubstituted or substituted by one or more radicals from the group comprising halogen, C₁-C₄-alkoxy, C₁-C₄-alkylthio and NR³¹R³²,
R¹⁵ is analogous to R⁸,
R¹⁶ is H, C₁-C₆-alkyl, C₂-C₆-alkenyl, C₂-C₆-alkynyl, the 3 latter radicals independently of one another being unsubstituted or substituted by one or more halogen radicals,
R¹⁷ is analogous to R⁵,
R¹⁸ is C₁-C₆-alkyl, C₂-C₆-alkenyl, C₂-C₆-alkynyl, the three latter radicals independently of one another being unsubstituted or substituted by one or more radicals from the group comprising halogen, C₁-C₄-alkoxy, C₁-C₄-alkylthio and NR³¹R³², or C₃-C₆-cycloalkyl which is unsubstituted or substituted by one or more radicals from the group comprising halogen, C₁-C₄-alkyl and C₁-C₄-alkoxy, or C₃-C₆-cycloalkyl-C₁-C₃-alkyl or H,
R¹⁹ is analogous to R⁸,
R²⁰ is analogous to R⁹,
R²¹ is analogous to R¹⁸,
R²² is H, C₁-C₄-alkyl, C₃-C₅-alkenyl or C₃-C₅-alkynyl,
R²³ is H, C₁-C₄-alkyl, C₃-C₅-alkenyl or C₃-C₅-alkynyl,
R²⁴ is analogous to R¹⁷,
R²⁵ is analogous to R¹⁸,
R²⁶ is analogous to R¹⁸,
R²⁷ is analogous to R⁸,
R²⁸ is analogous to R⁹,
R²⁹ is H, OH, NH₂, NHR³⁷, N(R³⁷)₂, C₁-C₄-alkyl, C₁-C₄-alkoxy, C₂-C₄-alkenyl or C₂-C₄-alkynyl, the latter 4 radicals independently of one another being unsubstituted or substituted by one or more radicals from the group comprising halogen, C₁-C₃-alkoxy and C₁-C₃-alkylthio,
R³⁰ is H, C₁-C₆-alkyl, C₁-C₆-alkoxy, C₂-C₆-alkenyl, C₂-C₆-alkynyl, the latter four radicals independently of one another being unsubstituted or substituted by one or more radicals from the group comprising halogen, C₁-C₃-alkoxy and C₁-C₃-alkylthio,
each R³¹ independently of the others is H, C₁-C₄-alkyl, (C₁-C₄-alkyl)carbonyl, (C₁-C₄-alkoxy)carbonyl or CHO,
each R³² independently of the others is H or C₁-C₄-alkyl,
R³³ is analogous to R⁵,
R³⁴ is analogous to R⁶,
R³⁵, R³⁶ independently of one another are H, C₁-C₆-alkyl, C₂-C₆-alkenyl, C₂-C₆-alkynyl, the 3 latter radicals independently of one another being unsubstituted or substituted by one or more halogen radicals,
R³⁷ is C₁-C₄-alkyl, C₂-C₄-alkenyl or C₂-C₄-alkynyl, the three radicals mentioned, independently of one another, being unsubstituted or substituted by one or more radicals from the group comprising halogen, C₁-C₄-alkoxy and C₁-C₄-alkylthio,
W is O or S,
X and Y independently of one another are hydrogen, halogen, C₁-C₆-alkyl, C₁-C₆-alkoxy or C₁-C₆-alkylthio, the latter three radicals being unsubstituted or substituted by one or more radicals from the group comprising halogen, C₁-C₄-alkoxy and C₁-C₄-alkylthio, or mono- or di-(C₁-C₄-alkyl)amino, C₃-C₆-cycloalkyl, C₃-C₅-alkenyl, C₃-C₅-alkenyloxy or C₃-C₅-alkynyloxy, and
Z is CH or N.

2. A compound or a salt thereof as claimed in claim 1, wherein
R¹ is C₁-C₄-alkyl, C₂-C₄-alkenyl or C₂-C₄-alkynyl, or
R² is CO-R⁵, COOR⁶, CO-NR⁸R⁹, CS-NR¹⁰R¹¹, SO₂R¹⁴ or SO₂NR¹⁵R¹⁶, or
R³ is COR¹⁷, COOR¹⁸, CONR¹⁹R²⁰ or CO-ON=CR²²R²³, or
R⁴ is H or C₁-C₄-alkyl, or
R⁵ is H, C₁-C₆-alkyl which is unsubstituted or substituted by one or more halogen radicals or by C₁-C₄-alkoxy, C₁-C₄-alkylthio or NR³¹R³², or C₃-C₆-cycloalkyl, phenyl, benzyl or heteroaryl, the latter three radicals being unsubstituted or substituted by one or more radicals from the group comprising C₁-C₄-alkyl, C₁-C₄-alkoxy and halogen, or
R⁶ is C₁-C₆-alkyl, C₂-C₆-alkenyl, C₂-C₆-alkynyl, C₁-C₆-haloalkyl or C₃-C₆-cycloalkyl, or
R⁷ is C₁-C₄-alkyl, or
R⁸ is hydrogen, C₁-C₆-alkyl, C₁-C₄-haloalkyl, C₁-C₄-alkoxy or (C₁-C₄-alkoxy)carbonyl, or
R⁹ is hydrogen, C₁-C₆-alkyl which is unsubstituted or substituted by one or more radicals from the group comprising halogen, C₁-C₄-alkoxy and NR³¹R³², or CO-R³³, CO-OR³⁴ or CO-NR³⁵R³⁶, or
R⁸ and R⁹ taken together are a divalent radical of the formula -(CH₂)₄-, -(CH₂)₅- or -CH₂CH₂-O-CH₂CH₂-, or
R¹⁴ is C₁-C₆-alkyl or C₁-C₆-haloalkyl, or
R¹⁵, R¹⁶ independently of one another are hydrogen or C₁-C₄-alkyl, or
R¹⁷ is hydrogen or C₁-C₄-alkyl, C₁-C₄-haloalkyl, C₃-C₆-cycloalkyl, phenyl or heteroaryl, the latter two radicals being unsubstituted or substituted, or
R¹⁸ is hydrogen, C₁-C₄-alkyl, C₂-C₆-alkenyl or C₂-C₆-alkynyl, the latter 3 radicals being unsubstituted or substituted by one or more radicals from the group comprising halogen, C₁-C₄-alkoxy, C₁-C₄-alkylthio and NR³¹R³², or C₃-C₆-cycloalkyl or C₃-C₆-cycloalkyl-C₁-C₃-alkyl, or
R²² is hydrogen or C₁-C₂-alkyl, or
R²³ is hydrogen or C₁-C₂-alkyl, or
R²⁹ is hydrogen, hydroxyl, amino, NHCH₃, N(CH₃)₂, C₁-C₄-alkyl or C₁-C₄-alkoxy, or
R³⁰ is hydrogen or C₁-C₄-alkyl, or
each R³¹ independently of the others is H or C₁-C₄-alkyl, or
each R³² independently of the others is H or C₁-C₄-alkyl, or
R³³ is hydrogen, C₁-C₄-alkyl, C₁-C₄-haloalkyl, C₃-C₆-cycloalkyl or phenyl which is unsubstituted or substituted by one or more radicals from the group comprising halogen, C₁-C₄-alkyl and C₁-C₄-alkoxy, or
R³⁴ is C₁-C₄-alkyl, allyl, propargyl or cycloalkyl, or
R³⁵ is hydrogen or C₁-C₄-alkyl, or
R³⁶ is hydrogen or C₁-C₄-alkyl, or
X is C₁-C₄-alkyl, C₁-C₄-alkoxy, C₁-C₄-haloalkyl, C₁-C₄-alkylthio, halogen or mono- or di-(C₁-C₂-alkyl)amino, or
Y is C₁-C₄-alkyl, C₁-C₄-alkoxy, C₁-C₄-haloalkyl or C₁-C₄-alkylthio,
or a compound of the formula (I) or a salt thereof in which two or more of the definitions mentioned above for the radicals R¹ to R³⁶ in formula (I) are present in combination.

3. A compound or a salt thereof as claimed in claim 1 or 2, wherein
R¹ is methyl, ethyl, n-propyl, i-propyl or allyl,
R² is CO-R⁵, COOR⁶, CO-NR⁸R⁹, CS-NR¹⁰R¹¹, SO₂R¹⁴ or SO₂NR¹⁵R¹⁶,
R⁵ is H, C₁-C₄-alkyl, C₁-C₂-haloalkyl, cyclopropyl, phenyl, benzyl or heteroaryl having 5 or 6 ring atoms, the latter 3 radicals being unsubstituted or substituted by one or more halogen atoms,
R⁶ is C₁-C₄-alkyl, allyl, propargyl or cyclopropyl,
R⁸ is H, C₁-C₄-alkyl, C₁-C₄-haloalkyl or (C₁-C₄-alkoxy)carbonyl,
R⁹ is H or C₁-C₄-alkyl,
R¹⁰ is H or C₁-C₄-alkyl,
R¹¹ is H or C₁-C₄-alkyl,
R¹⁴ is C₁-C₄-alkyl,
R¹⁵ is H or C₁-C₄-alkyl and
R¹⁶ is H or C₁-C₄-alkyl.

4. A process for the preparation of a compound of the formula (I) or of a salt thereof as defined in claim 1, which comprises
a) reacting a compound of the formula (II) 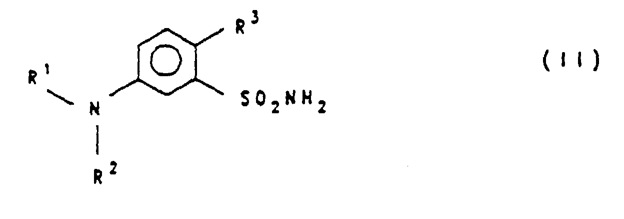 with a heterocyclic carbamate of the formula (III) 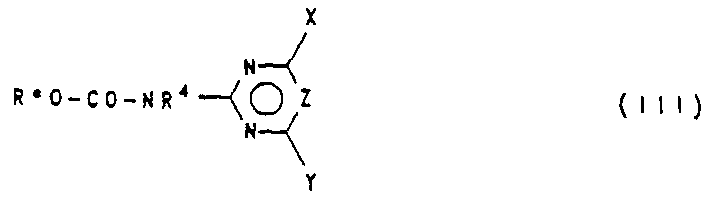 in which R* is unsubstituted or substituted phenyl or C₁-C₄-alkyl, or
b) reacting a sulfonyl isocyanate of the formula (IV) 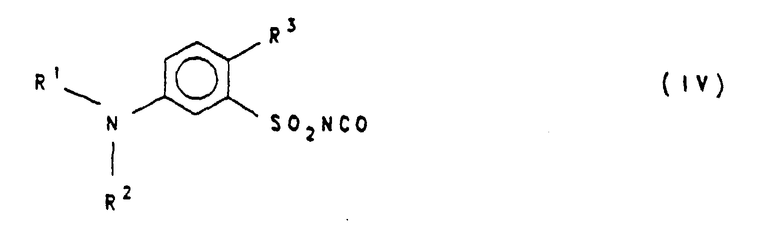 with an amino heterocycle of the formula (V) 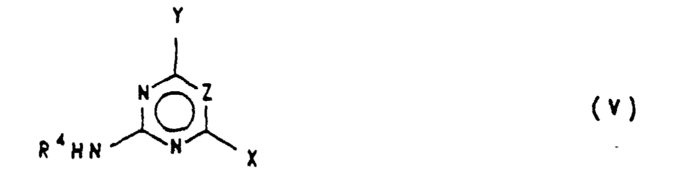 or
c) reacting a compound of the formula (II) with a (thio)isocyanate of the formula (X) 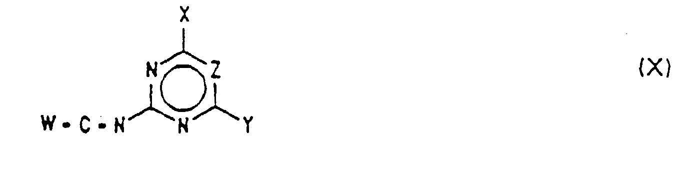
in the presence of a base,
the radicals R¹ to R⁴, W, X, Y and Z in the above formulae (II) to (V) and (X) being as defined in formula (I) and, in the case of variants a) and b), the compounds initially obtained being those of the formula (I) in which W = O.

5. A herbicidal or plant growth-regulating agent, which comprises a compound of the formula (I) as claimed in any of claims 1 to 3 and conventional formulation auxiliaries.

6. The use of a compound of the formula (I) or a salt thereof as claimed in any of claims 1 to 3 as a herbicide or plant growth regulator.

7. A method of controlling harmful plants or of regulating the growth of plants, which comprises applying an effective amount of one or more compounds of the formula (I) or of salts thereof as claimed in any of claims 1 to 3 to the plants, parts of plants or the agricultural or industrial land.

8. A compound of the formula (II) 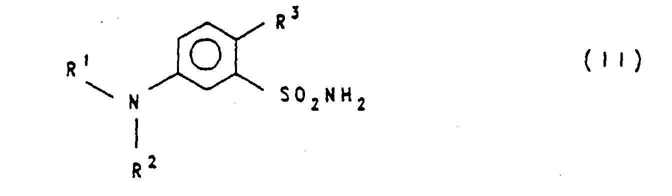 in which
R¹, R² and R³ are as defined in formula (I) as claimed in claim 1, 2 or 3.

9. A compound of the formula (IV) 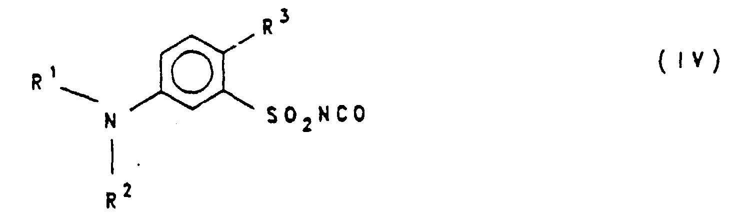 in which
R¹, R² and R³ are as defined in formula (I) as claimed in any of claims 1, 2 or 3.

## Revendications

1. Composés de £ormule I, ou leurs sels 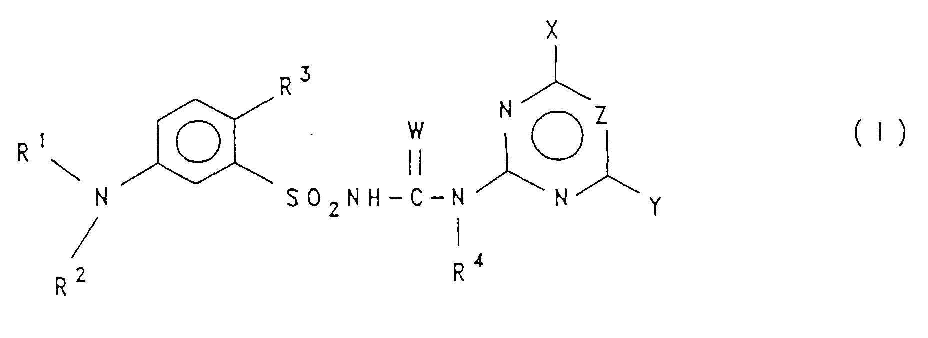 dans lesquels :
R¹ est un groupe alkyle en C₁-C₆, alcényle en C₂-C₆, alcynyle en C₂-C₆, ces trois derniers résidus étant, indépendamment les uns des autres, non substitués, ou substitués par un ou plusieurs substituants choisis parmi l'ensemble comprenant les groupes halogéno, alcoxy en C₁-C₄, CONH₂, alkylthio en C₁-C₄, CN, CHO, (alkyle en C₁-C₆)-carbonyle, (cycloalkyle en C₃-C₆)-carbonyle, alkylsulfonyle en C₁-C₄, carboxy, (alcoxy en C₁-C₄)-carbonyle, (alcényloxy en C₂-C₄)-carbonyle, (alcynyloxy en C₂-C₄)-carbonyle, NO₂, NH₂, mono- et di-(alkyle en C₁-C₆)amino,
R² est CO-R⁵, COO-R⁶, CO-SR⁷, CO-NR⁸R⁹, CS-NR¹⁰R¹¹, CS-OR¹², CS-SR¹³, SO₂R¹⁴, SO₂NR¹⁵R¹⁶,
R³ est COR¹⁷, CO-OR¹⁸, CO-NR¹⁹R²⁰, CO-SR²¹, CO-O-N=CR²²R²³, CSR²⁴, CSSR²⁵, CS-OR²⁶, CS-NR²⁷R²⁸, C(=NR²⁹)R³⁰,
R⁴ est H ou un groupe alkyle en C₁-C₆, alcényle en C₂-C₆, alcynyle en C₂-C₆, ces trois derniers radicaux étant, indépendamment l'un de l'autre, non substitués ou substitués par un ou plusieurs substituants choisis parmi les halogènes,
R⁵ est H ou un groupe alkyle en C₁-C₆, alcényle en C₂-C₆, alcynyle en C₂-C₆, ces trois derniers radicaux, indépendamment l'un de l'autre, étant non substitués ou substitués par un ou plusieurs substituants choisis parmi l'ensemble constitué des groupes halogéno, alcoxy en C₁-C₄, alkylthio en C₁-C₄ et NR³¹R³², ou encore cycloalkyle en C₃-C₈ non substitué ou substitué, phényle non substitué ou substitué, hétéroaryle non substitué ou substitué, ou encore phényl-(alkyle en C₁-C₄) qui est non substitué ou substitué sur le noyau phényle,
R⁶ est un groupe alkyle en C₁-C₆, alcényle en C₂-C₆, alcynyle en C₂-C₆, ces trois derniers radicaux étant, indépendamment les uns des autres, non substitués ou substitués par un ou plusieurs substituants choisis parmi l'ensemble comprenant les groupes halogéno, alcoxy en C₁-C₄, alkylthio en C₁-C₄ et NR³¹R³², ou encore cycloalkyle en C₃-C₆, qui est non substitué ou est substitué par un ou plusieurs substituants choisis parmi l'ensemble comprenant les groupes halogéno, alkyle en C₁-C₄ et alcoxy en C₁-C₄, ou encore (cycloalkyle en C₃-C₆)-(alkyle en C₁-C₃),
R⁷ est analogue à R⁶,
R⁸ est H ou un groupe alkyle en C₁-C₆, alcényle en C₂-C₆, alcynyle en C₂-C₆, ces trois derniers radicaux étant, indépendamment les uns des autres, non substitués ou substitués par un ou plusieurs substituants choisis parmi les halogènes, ou encore (alcoxy en C₁-C₆)-carbonyle ou alcoxy en C₁-C₄,
R⁹ est H ou un groupe alkyle en C₁-C₆, alcényle en C₂-C₆, alcynyle en C₂-C₆, ces trois derniers radicaux étant, indépendamment les uns des autres, non substitués ou substitués par un ou plusieurs substituants choisis parmi l'ensemble constitué des groupes halogéno, alcoxy en C₁-C₄, alkylthio en C₁-C₄ et NR³¹R³², ou encore CO-R³³, CO-OR³⁴, CO-NR³⁵R³⁶, CS-NR³⁵R³⁶, CS-R³³ ou CS-OR³⁴, ou bien
R⁸ et R⁹ forment ensemble un radical bivalent de formule -(CH₂)₄-, -(CH₂)₅-, -CH₂CH₂-O-CH₂CH₂-, -CH₂CH₂NH-CH₂CH₂-, ces quatre derniers radicaux étant non substitués ou substitués par des groupes alkyle en C₁-C₄,
R¹⁰ est analogue à R⁸,
R¹¹ est analogue à R⁹,
R¹² est analogue à R⁶,
R¹³ est analogue à R⁶,
R¹⁴ est un groupe alkyle en C₁-C₆, alcényle en C₂-C₆, alcynyle en C₂-C₆, ces trois derniers radicaux étant, indépendamment les uns des autres, non substitués ou substitués par un ou plusieurs substituants choisis parmi l'ensemble constitué des groupes halogéno, alcoxy en C₁-C₄, alkylthio en C₁-C₄ et NR³¹R³²,
R¹⁵ est analogue à R⁸,
R¹⁶ est H ou un radical alkyle en C₁-C₆, alcényle en C₂-C₆, alcynyle en C₂-C₆, ces trois derniers radicaux étant, indépendamment les uns des autres, non substitués ou substitués par un ou plusieurs substituants choisis parmi les halogènes,
R¹⁷ est analogue à R⁵,
R¹⁸ est un groupe alkyle en C₁-C₆, alcényle en C₂-C₆, alcynyle en C₂-C₆, ces trois derniers radicaux étant, indépendamment les uns des autres, non substitués ou substitués par un ou plusieurs substituants choisis parmi l'ensemble constitué des groupes halogéno, alcoxy en C₁-C₄, alkylthio en C₁-C₄ et NR³¹R³², ou encore cycloalkyle en C₃-C₆ qui est non substitué ou est substitué par un ou plusieurs substituants choisis parmi l'ensemble constitué des groupes halogéno, alkyle en C₁-C₄ et alcoxy en C₁-C₄, ou encore (cycloalkyle en C₃-C₆)-(alkyle en C₁-C₃) ou H,
R¹⁹ est analogue à R⁸,
R²⁰ est analogue à R⁹,
R²¹ est analogue à R¹⁸,
R²² est H , un groupe alkyle en C₁-C₄, alcényle en C₃-C₅ ou alcynyle en C₃-C₅,
R²³ est H , un groupe alkyle en C₁-C₄, alcényle en C₃-C₅ ou alcynyle en C₃-C₅,
R²⁴ est analogue à R¹⁷,
R²⁵ est analogue à R¹⁸,
R²⁶ est analogue à R¹⁸,
R²⁷ est analogue à R⁸,
R²⁸ est analogue à R⁹,
R²⁹ est H, OH, NH₂, NHR³⁷, N(R³⁷)₂, ou un groupe alkyle en C₁-C₄, alcoxy en C₁-C₄, alcényle en C₂-C₄ ou alcynyle en C₂-C₄, ces quatre derniers radicaux étant, indépendamment les uns des autres, non substitués ou substitués par un ou plusieurs substituants choisis parmi l'ensemble constitué des groupes halogéno, alcoxy en C₁-C₃ et alkylthio en C₁-C₃,
R³⁰ est H ou un groupe alkyle en C₁-C₆, alcoxy en C₁-C₆, alcényle en C₂-C₆, alcynyle en C₂-C₆, ces quatre derniers radicaux étant, indépendamment les uns des autres, non substitués ou substitués par un ou plusieurs substituants choisis parmi l'ensemble constitué des groupes halogéno, alcoxy en C₁-C₃ et alkylthio en C₁-C₃,
les radicaux R³¹, indépendamment les uns des autres, sont H ou des groupes alkyle en C₁-C₄, (alkyle en C₁-C₄)-carbonyle, (alcoxy en C₁-C₄)-carbonyle ou CHO,
les radicaux R³², indépendamment les uns des autres, sont H ou des radicaux alkyle en C₁-C₄,
R³³ est analogue à R⁵,
R³⁴ est analogue à R⁶,
R³⁵ et R³⁶, indépendamment l'un de l'autre, sont chacun H , un groupe alkyle en C₁-C₆, alcényle en C₂-C₆, alcynyle en C₂-C₆, ces trois derniers radicaux étant, indépendamment les uns des autres, non substitués
ou substitués par un ou plusieurs substituants choisis parmi les halogènes,
R³⁷ est un groupe alkyle en C₁-C₄, alcényle en C₂-C₄ ou alcynyle en C₂-C₄, ces trois derniers radicaux étant, indépendamment les uns des autres, non substitués ou substitués par un ou plusieurs substituants choisis parmi l'ensemble constitué des groupes halogéno, alcoxy en C₁-C₄ et alkylthio en C₁-C₄,
W est O ou S,
X et Y, indépendamment l'un de l'autre, sont chacun un hydrogène ou un groupe halogéno, alkyle en C₁-C₆ alcoxy en C₁-C₆ ou alkylthio en C₁-C₆, ces trois derniers radicaux étant non substitués ou substitués par un ou plusieurs substituants choisis parmi l'ensemble constitué des groupes halogéno, alcoxy en C₁-C₄ et alkylthio en C₁-C₄, ou encore mono- ou di-(alkyle en C₁-C₄)amino, cycloalkyle en C₃-C₆, alcényle en C₃-C₅, alcényloxy en C₃-C₅ ou alcynyloxy en C₃-C₅, et
Z est CH ou N.

2. Composés, ou leurs sels, selon la revendication 1, caractérisés en ce que :
R¹ est un groupe alkyle en C₁-C₄, alcényle en C₂-C₄ ou alcynyle en C₂-C₄, ou bien
R² est CO-R⁵, COOR⁶, CO-NR⁸R⁹, CS-NR¹⁰R¹¹, SO₂R¹⁴ ou SO₂NR¹⁵R¹⁶, ou bien
R³ est COR¹⁷, COOR¹⁸, CONR¹⁹R²⁰ ou CO-ON=CR²²R²³, ou bien
R⁴ est H ou un groupe alkyle en C₁-C₄, ou bien
R⁵ est H ou un groupe alkyle en C₁-C₆, qui est non substitué ou est substitué par un ou plusieurs substituants choisis parmi les halogènes, ou par des groupes alcoxy en C₁-C₄, alkylthio en C₁-C₄ ou NR³¹R³², ou encore cycloalkyle en C₃-C₆, phényle, benzyle, ou hétéroaryle, auquel cas ces trois derniers radicaux sont non substitués ou sont substitués par un ou plusieurs substituants choisis parmi l'ensemble constitué des groupes alkyle en C₁-C₄, alcoxy en C₁-C₄, et halogéno, ou bien
R⁶ est un groupe alkyle en C₁-C₆ alcényle en C₂-C₆, alcynyle en C₂-C₆, halogénalkyle en C₁-C₆ ou cycloalkyle en C₃-C₆, ou bien
R⁷ est un groupe alkyle en C₁-C₄, ou bien
R⁸ est un hydrogène ou un groupe alkyle en C₁-C₆, halogénalkyle en C₁-C₄, alcoxy en C₁-C₄ ou encore (alcoxy en C₁-C₄)-carbonyle, ou bien
R⁹ est un hydrogène ou un groupe alkyle en C₁-C₆, qui est non substitué ou est substitué par un ou plusieurs substituants choisis parmi l'ensemble constitué des groupes halogéno, alcoxy en C₁-C₄ et NR³¹R³², ou encore CO-R³³, CO-OR³⁴ ou CO-NR³⁵R³⁶, ou bien
R⁸ et R⁹ forment ensemble un radical bivalent de formule -(CH₂)₄-, -(CH₂)₅- ou -CH₂CH₂-O-CH₂CH₂-, ou bien
R¹⁴ est un groupe alkyle en C₁-C₆, halogénalkyle en C₁-C₆, ou bien
R¹⁵ et R¹⁶, indépendamment l'un de l'autre, sont chacun un hydrogène ou un groupe alkyle en C₁-C₄, ou bien
R¹⁷ est un hydrogène ou un groupe alkyle en C₁-C₄, halogénalkyle en C₁-C₄, cycloalkyle en C₃-C₆, phényle ou hétéroaryle, ces deux derniers radicaux étant non substitués ou substitués, ou bien
R¹⁸ est un hydrogène ou un groupe alkyle en C₁-C₄, alcényle en C₂-C₆ ou alcynyle en C₂-C₆, ces trois derniers radicaux étant non substitués, ou étant substitués par un ou plusieurs substituants choisis parmi l'ensemble constitué des groupes halogéno, alcoxy en C₁-C₄, alkylthio en C₁-C₄ et NR³¹R³², ou cycloalkyle en C₃-C₆ ou (cycloalkyle en C₃-C₆)-(alkyle en C₁-C₃), ou bien
R²² est un hydrogène ou un groupe alkyle en C₁-C₂, ou bien
R²³ est un hydrogène ou un groupe alkyle en C₁-C₂, ou bien
R²⁹ est un hydrogène , un groupe hydroxy, amino, NHCH₃, N(CH₃)₂, alkyle en C₁-C₄ ou alcoxy en C₁-C₄, ou bien
R³⁰ est un hydrogène ou un groupe alkyle en C₁-C₄, ou bien
les radicaux R₃₁, indépendamment les uns des autres, sont chacun H ou un groupe alkyle en C₁-C₄, ou bien
les radicaux R₃₂, indépendamment l'un de l'autre, sont chacun H ou un groupe alkyle en C₁-C₄, ou bien
R³³ est un hydrogène, un groupe alkyle en C₁-C₄, halogénalkyle en C₁-C₄, cycloalkyle en C₃-C₆ ou phényle, qui est non substitué ou est substitué par un ou plusieurs substituants choisis parmi l'ensemble constitué des groupes halogéno, alkyle en C₁-C₄ et alcoxy en C₁-C₄, ou bien
R³⁴ est un groupe alkyle en C₁-C₄, allyle, propargyle ou cycloalkyle, ou bien
R³⁵ est un hydrogène ou un groupe alkyle en C₁-C₄, ou bien
R³⁶ est un hydrogène ou un groupe alkyle en C₁-C₄, ou bien
X est un groupe alkyle en C₁-C₄, alcoxy en C₁-C₄, halogénalkyle en C₁-C₄, alkylthio en C₁-C₄, halogéno ou mono- ou di-(alkyle en C₁-C₂)-amino, ou bien
Y est un groupe alkyle en C₁-C₄, alcoxy en C₁-C₄, halogénoalkyle en C₁-C₄ ou alkylthio en C₁-C₄, ou encore les composés de formule I ou leurs sels dans lesquels au moins deux des significations ci-dessus des radicaux R¹ à R³⁶ de la formule I sont présentes en combinaison.

3. Composés ou leurs sels selon la revendication 1 ou 2, caractérisés en ce que :
R¹ est le radical méthyle, éthyle, n-propyle, isopropyle ou allyle,
R² est CO-R⁵, COOR⁶, CO-NR⁸R⁹, CS-NR¹⁰R¹¹, SO₂R¹⁴ ou SONR¹⁵R¹⁶,
R⁵ est H, ou un groupe alkyle en C₁-C₄, halogénalkyle en C₁-C₂, cyclopropyle, phényle, benzyle ou hétéroaryle ayant 5 ou 6 atomes dans le noyau, ces trois derniers radicaux étant non substitués ou étant substitués par un ou plusieurs atomes d'halogène,
R⁶ est un groupe alkyle en C₁-C₄, allyle, propargyle ou cyclopropyle,
R⁸ est H, un groupe alkyle en C₁-C₄, halogénalkyle en C₁-C₄, ou (alcoxy en C₁-C₄)-carbonyle,
R⁹ est H ou un groupe alkyle en C₁-C₄,
R¹⁰ est H ou un groupe alkyle en C₁-C₄,
R¹¹ est H ou un groupe alkyle en C₁-C₄,
R¹⁴ est un groupe alkyle en C₁-C₄,
R¹⁵ est H ou un groupe alkyle en C₁-C₄, et
R¹⁶ est H ou un groupe alkyle en C₁-C₄.

4. Procédés pour préparer les composés de formule I ou leurs sels tels que définis dans la revendication 1, caractérisés en ce que :
a) on fait réagir un composé de formule II : 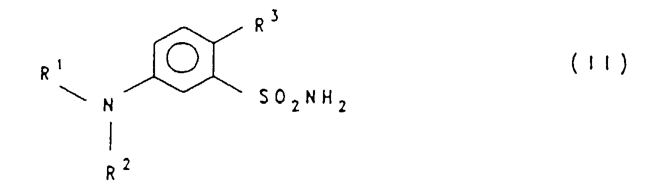 avec un carbamate hétérocyclique de formule III : 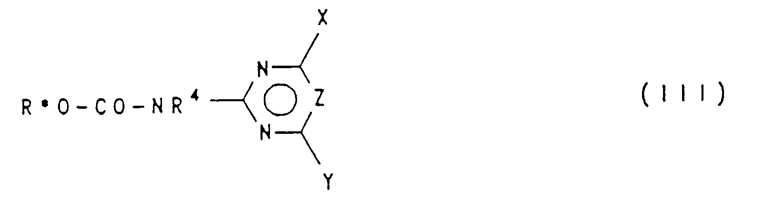 dans laquelle R* est un groupe phényle éventuellement substitué ou alkyle en C₁-C₄, ou bien
b) on fait réagir un sulfonylisocyanate de formule IV : 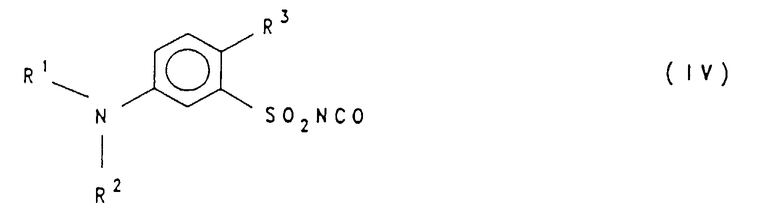 avec un composé amino-hétérocyclique de formule V : 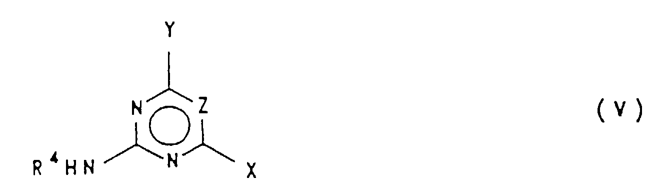 ou bien
c) on fait réagir en présence d'une base un composé de formule II avec un (thio)isocyanate de formule X : 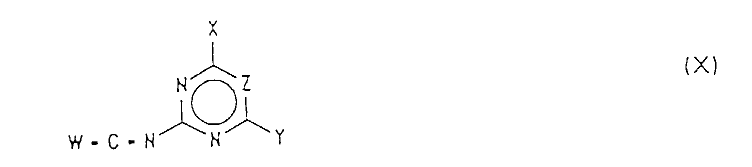
où, dans les formules II à V et X ci-dessus, les radicaux R¹ à R⁴, W, X, Y et Z sont tels que définis dans la formule I, et, dans les variantes a) et b), on obtient d'abord des composés de formule I dans lesquels W est O.

5. Herbicides ou produits régulateurs de croissance des végétaux, caractérisés en ce qu'ils contiennent un composé de formule I selon l'une des revendications 1 à 3 et les adjuvants de formulation usuels.

6. Utilisation des composés de formule I ou de leurs sels selon l'une des revendications 1 à 3, en tant qu'herbicides ou régulateurs de croissance des végétaux.

7. Procédés pour maîtriser les plantes nuisibles ou pour réguler la croissance de plantes, caractérisés en ce qu'on applique une quantité efficace d'un ou plusieurs composés de formule I ou de leurs sels selon l'une des revendications 1 à 3 sur les végétaux, les parties de végétaux ou sur le sol à usage agricole ou industriel.

8. Composés de formule II : 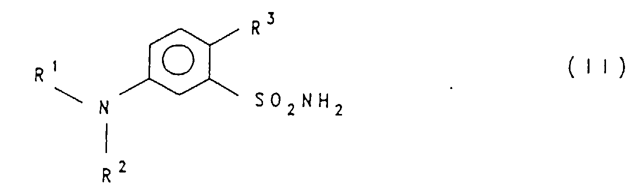 dans laquelle R¹, R² et R³ sont tels définis dans la formule I selon la revendication 1, 2 ou 3.

9. Composés de formule IV : 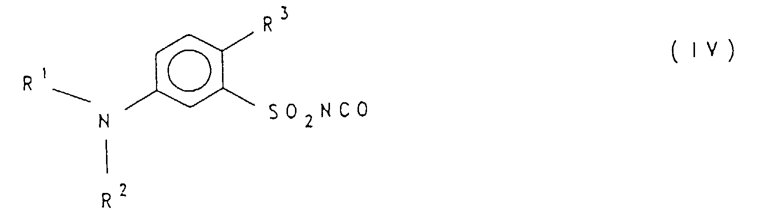 dans laquelle R¹, R² et R³ sont tels que définis dans la formule I selon l'une des revendications 1, 2 ou 3.
